# EUROPEAN PATENT APPLICATION

(11) **EP 1 223 176 A2**
(43) Date of publication of application: **17.07.2002**
(21) Application number: 01307075.0
(22) Date of filing: 20.08.2001
(51) Int. Cl.: C07K 14/435, C07K 16/40, C12N 9/04, G01N 33/68

(54) **Peptide mutant of human ERAB/HADH2, its X-ray crystal structure, and materials and method for identification of inhibitors thereof**

(30) Priority: 18.08.2000 US 226123 P
(71) Applicant: Agouron Pharmaceuticals, Inc., La Jolla, CA 92037 (US)
(72) Inventor: Abreo, Melwyn A., Jamul, California 91935 (US); Agree, Charles S., San Diego, California 92121 (US); Pelletier, Laura A., Escondido, California 92029 (US); Aust, Robert M., Alpine, California 91901 (US); Rejto, Paul Abraham, Carlsbad, California 92009 (US); Thomson, James Arthur, San Diego, California 92123 (US); Meng, Jerry J., San Diego, California 92129 (US); Vanderpool, Darin Louis, San Diego, California 92117 (US); Kissinger, Charles R., San Diego, California 92121 (US); Margosiak, Stephen, Escondido, California 92025 (US); Showalter, Richard Edward, Santee, California 92071 (US); Villafranca, Jesus Ernesto, San Diego, California 92103 (US); Tempczyk-Russel, Anna, San Diego, California 92130 (US)
(74) Representative: Jones, Alan John

(57) **Abstract**

The identification, isolation and modification of human ERAB or HADH2 is described. A crystal structure of ERAB or HADH2 is described which may be used in the discovery, identification and characterization of inhibitors or modulators of ERAB or HADH2. This structure provides a three-dimensional description of binding sites of ERAB or HADH2 for structure-based design of inhibitors or modulators thereof as therapeutic agents, for example in the treatment of Alzheimer's disease).

## Description

### CROSS REFERENCES OF RELATED APPLICATIONS

This application claims the benefit of U.S. Provisional Application, Serial No. 60/226123, filed August 18, 2000.

### TECHNICAL FIELD OF THE INVENTION

The present invention relates to the three-dimensional crystal structure of human ERAB or HADH2 with NAD⁺ cofactor and its use in the design and development of inhibitors thereof.

### BACKGROUND OF THE INVENTION

Endoplasmic reticulum-associated amyloid β-peptide-binding protein (ERAB), a member of the short-chain dehydrogenase/reductase (SDR) family of enzymes, has been implicated in the development of Alzheimer's disease (AD). As the name suggests, ERAB, also known as L-3-hydroxyacyl-CoA dehydrogenase Type II (HADH2), is a dehydrogenase enzyme capable of binding amyloid-β (Aβ) peptide. Inhibition of ERAB or HADH2 finds therapeutic utility in the treatment of AD and research and diagnostic utility in the delineation of the role of the enzyme in both normal cellular function and in AD pathogenesis.

AD is a progressive neurodegencrative disease of the brain resulting in diminished cognitive abilities, dementia, and ultimately death. A strong link has been established between the development of AD and the accumulation of Aβ in the brain [Storey et al., *Neuropathology And Applied Neurobiology*, Vol. 25, pp. 81-97 (1999); Selkoe, *Annual Review of Neuroscience*, Vol. 17, pp. 489-517 (1994), Small et al., *J. Neurochemistry,* Vol. 73, pp. 443-9 (1999)]. Aβ is a proteolytic fragment of the integral membrane glycoprotein, amyloid-β precursor protein (APP) [Kang et al., *Nature*, Vol. 325, pp. 733-736 (1987)]. Aβ is also the principal component of the extracellular plaques that are diagnostic of AD and species of the peptide have been shown to be engaged by intracellular targets [Yan et al., *Nature*, Vol. 389, pp. 689-95 (1997)]. Although aggregated Aβ appears to be toxic to neuronal cells in culture, the mechanisms of Aβ neurotoxicity *in vivo* are not completely understood.

Aβ accumulates both inside and outside nerve cells [Wilson et al., *Journal of Neuropathology And Experimental Neurology*, Vol. 58, pp. 787-94 (1999)]. There is evidence that the interaction of Aβ with intracellular proteins could lead to cytotoxic events prior to the formation of extracellular plaques. ERAB or HADH2, an intracellular protein, has been found to bind Aβ in the yeast two-hybrid system [Yan et al., *Nature*, Vol. 389, pp. 689-95 (1997)]. HADH2, a protein identical to ERAB, has been independently identified as an L-3-hydroxyacyl-CoA dehydrogenase with an apparent role in the mitochondrial fatty acid β-oxidation pathway [He et al., *Journal of Biochemistry*, Vol. 273. No. 17, pp. 10741-10746 (1998)].

ERAB or HADH2 is reported to be an NAD⁺ dependent dehydrogenase which catalyzes the reversible oxidation of L-3-hydroxyacyl-CoA. The human short chain L-3-hydroxyacyl-CoA dehydrogenase gene is organized into six exoris and five introns and maps to chromosone Xp 11.2 [He et al. (1998), *supra].* Sequence comparisons show that ERAB or HADH2 belongs to the SDR family of enzymes.

ERAB or HADH2 has been cloned, expressed, purified, and characterized from human brain [He et al. (1998), *supra*]. ERAB OR HADH2 messenger RNA (mRNA) is expressed ubiquitously in normal human tissues. Its expression is highest in liver and heart but ERAB or HADH2 mRNA is also expressed in normal brain. In normal brain, ERAB or HADH2 antigen is present at low levels, being predominantly localized in neurons. However, in neurons affected in AD, ERAB or HADH2 is reported to be overexpressed relative to non-AD age-matched controls, especially near deposits of Aβ [Yan et al., *Nature*, Vol. 389, pp. 689-95 (1997); USP 6,268,479].

A variety of experimental evidence suggests that ERAB or HADH2 interacts with the Aβ peptide and can mediate its cytotoxic effects [Yan et al. (1997), *supra*]. For example, ERAB or HADH2, normally found in the endoplasmic reticulum and mitochondria, has been shown to become redistributed to the plasma membrane fraction of cells in the presence of Aβ peptide [Yan et al.(1997), *supra*]. Likewise, it has been shown that the cytotoxic effects of Aβ on neuroblastoma cells in cultures, can be blocked by anti-ERAB or HADH2 antibodies. Cells which overexpress ERAB or HADH2 and Aβ show elevated markers of cytotoxiciry and cell stress compared to mock transfected controls; conversely, cells overexpressing catalytically inactive mutants of ERAB or HADH2 were no more sensitive than controls which overexpressed Aβ alone [Yan et al., *Journal of Biochemistry*, Vol. 274, pp- 2145-56 (1999)].

These observations support the theory that ERAB or HADH2 mediates the intraneuronal toxicity of Aβ by acting on inappropriate substrates, possibly generating toxic aldehydes [Yan et al., *Journal of Biochemistry*, Vol. 274, pp. 2145-56 (1999)]. It has also been suggested that ERAB or HADH2 could contribute to Aβ-associated pathogenesis of AD by reducing neuroprotective estrogen levels in the brain, based on the finding that the enzyme can also utilize estrogen as a substrate [Yan et al., *Journal of Biochemistry*, Vol. 274, pp. 2145-56 (1999); He et al., *Journal of Biochemistry*, Vol. 274, pp. 15014-9 (1999)]. Although the two proposed roles for ERAB or HADH2 in AD are quite distinct, enzymatic activity of the protein is a prerequisite in either case.

Several attempts have been made to elucidate the three-dimensional structure of proteins to design candidate drugs. For example, Davis *et al*., Prevention of Chemotherapy-Induced Alopecia in Rats by CDK Inhibitors (2001) *Science* 291:134-137; Zhu et al., Structural Analysis of the Lymphocyte-Specific Kinase Lck in Complex with Non-selective and Src Family selective Kinase Inhibitors (1999) *Structure* 7: 651-661; and Ymaguchi et al., Structural Basis for Activation of Human Lymphocyte Kinase Lck Upon Tyrosine Phosphorylation (1996) *Nature* 384: 484-489. U.S. Patent Nos. 5,322,933, 5,556,778, 5,616,555, 5,872,011 and 6,020,162 disclose the three-dimensional structure of proteins, protein-ligand complex, or enzyme-cofactor complex.

### SUMMARY OF THE INVENTION

The present invention relates to an isolated, purified polynucleotide that encodes a mutant ERAB or HADH2 peptide, wherein the mutant peptide is engineered to avoid cysteine oxidation. It further relates to an isolated, purified polypeptide comprising a mutant ERAB or HADH2 peptide, which is engineered to avoid cysteine oxidation. According to one embodiment, the polypeptide has an amino acid other than cysteine at one or all of positions 5, 58, and/or 214 of SEQ ID NO: 2, and particularly the polypeptide has an amino acid sequence selected from the group consisting of SEQ ID NO: 2, SEQ ID NO: 4, SEQ ID NO: 6, SEQ ID NO: 20, and SEQ ID NO: 23 and functional variants thereof.

The present invention further relates to a crystalline mutant ERAB or HADH2 peptide. For one embodiment of the invention, the crystalline peptide diffracts x-rays at a reolution value of ≤ 3 Å, and preferably ≤ 2 Å.

The present invention further relates to a crystal of a mutant ERAB or HADH2 peptide:ligand:NAD⁺ complex comprising a mutant ERAB or HADH2 peptide:ligand:NAD⁺.

The present invention still further relates to a mutant ERAB or HADH2 peptide comprising a fold, wherein the fold comprises: a core β-sheet of seven strands sandwiched between two sets of three α-helices; and first and second insert domains, relative to other members of the short-chain dehydragenase/reductase (SDR) family, the first insert domain forming a β hairpin that extends the surface of the ERAB or HADH2 peptide on one side of a substrate-binding cleft, and the second insert domain extending a loop between an α-helix comprising residues 123-136 and a β-sheet comprising residues 148-153 of SEQ ID NO: 2.

The present invention relates to an expression vector comprising a ploynucleotide that encodes a mutant ERAB or HADH2 peptide; transcriptional regulatory sequences functional in the host cell operably linked to the polynucleotide; and a selectable marker, as well as to a host cell stably transfected and transformed with such polynucleotide.

The present invention further relates to a method for identifying a candidate compound for its ability to interact with the human ERAB or HADH2 peptide comprising:
(a) expressing an isolated polynucleotide sequence which encodes a mutant ERAB or HADH2 peptide in a host cell capable of producing the peptide;
(b) exposing the mutant ERAB or HADH2 peptide to the candidate compound; and
(c) evaluating the interaction of the mutant ERAB or HADH2 peptide with the candidate compound.

The present invention further relates to a process of drug design for compounds which interact with ERAB or HADH2 comprising:
(a) crystallizing a mutant ERAB or HADH2 peptide;
(b) resolving the x-ray crystallography of the peptide;
(c) applying the data generated from resolving the x-ray crystallography of the peptide to a computer algorithm which generates a model of the peptide suitable for use in designing molecules that will act as agonists or antagonists to the peptide; and
(d) applying an interative process whereby various molecular structures are applied to the computer-generated model to identify potential agonists or antagonists of the peptide.

The present invention also relates to a method of assessing compounds which are agonists or antagonists of the activity of the human ERAB or HADH2 enzyme comprising:
(a) crystallizing a mutant ERAB or HADH2 peptide; obtaining crystallography coordinates for the crystallized peptide;
(b) applying the crystallography coordinates for the peptide to a computer algorithm such that the algorithm generates a model of the peptide, the model suitable for use in designing molecules that will act as agonists or antagonists to the peptide; and
(c) applying an iterative process whereby various molecular structures are applied to the computer-generated model to identify potential agonists or antagonists to the peptide.

The invention further relates to a method of identifying a compound that associates with ERAB or HADH2 comprising the step of using the structure coordinates of Table II to computationally analyze a molecular structure to evaluate a chemical compound for associating with the substrate-binding site of ERAB or HADH2 and identifying those chemical entities that associate with the substrate-binding site, wherein the step of computationally analyzing the molecular sturucture comprises:
(a) using a machine readable data storage medium comprising a data storage material encoded with machine readable data wherein the data comprises crystal coordinates of mutant ERAB or HADH2 peptide molecule or complex of the peptide;
(b) employing a working memory for string instructions for processing the machine readable data,
(c) using a central processor unit (CPU) coupled to the working memory and the machine readable data for performing a Fourier transformation of the machine readable data and for processing such data into crystal coordinates of three-dimensional molecule or complex of the peptide, and
(d) displaying a display coupled to the CPU for displaying the crystal corrinates of the three-dimensional moleculer or complex.

Other aspects, features, and advantages of the invention will be become apparent upon consideration of the detailed description below in conjuction with the appended figures.

### BRIEF DESCRIPTION OF THE DRAWINGS

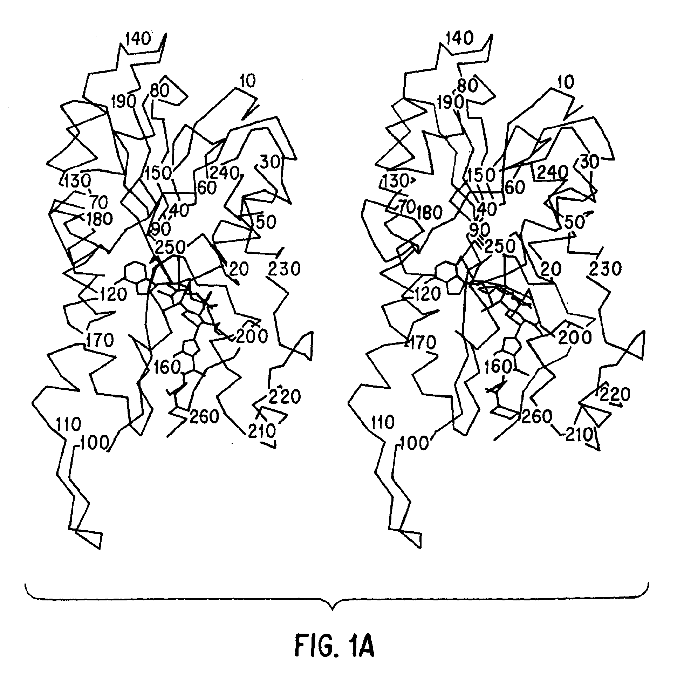

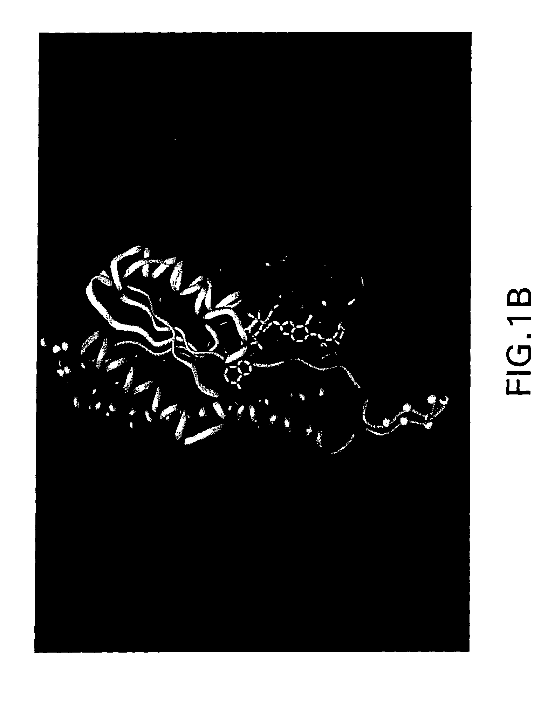

Figures 1A and 1B depict the three-dimensional structure of the ERAB or HADH2 monomer. Figure 1A shows the stereo Cα trace of the ERAB or HADH2 monomer. Every tenth residue is numbered. The bound NAD-inhibitor adduct is also shown. Figure 1B is a ribbon diagram of the ERAB or HADH2 monomer, with the NAD-inhibitor adduct shown in ball-and-stick representation. The ribbon is white at the amino terminus and becomes darker blue moving toward the carboxy terminus. Alpha-carbon positions of residues in the insertion regions of ERAB or HADH2 are shown as yellow spheres.

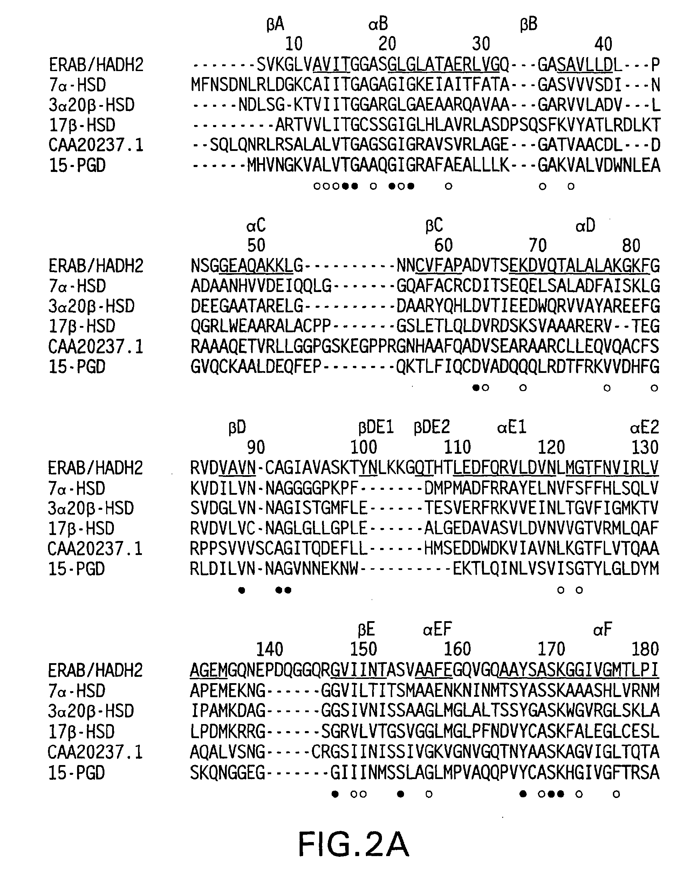

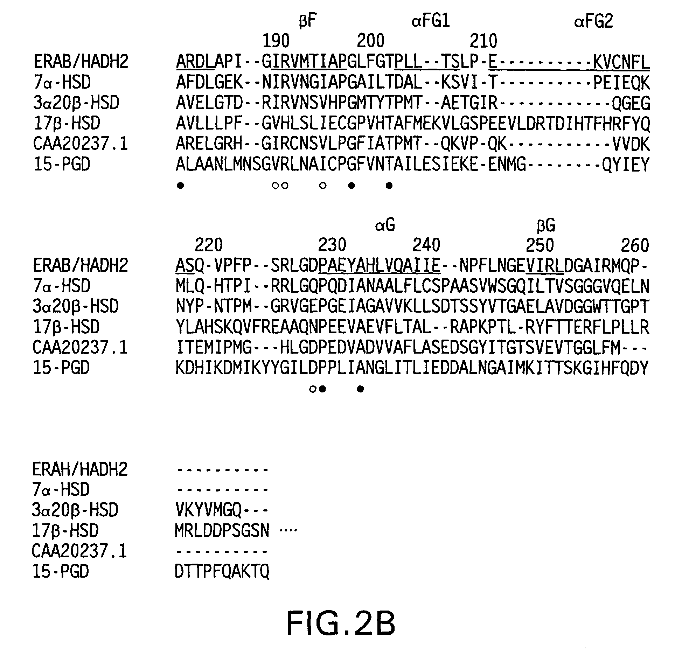

Figure 2 shows the sequence alignment of wild-type ERAB or HADH2 and representative members of the SDR family. The wild-type ERAB or HADH2 sequence is aligned with five related members of the SDR family: *E. coli* 7α-hydroxysteroid dehydrogenase (7α-HSD), *Streptomyces hydrogenans* 3α-20β-hydroxysteroid dehydrogenase (3α-20β-HSD), human 17β-hydrodroxysteroid dehydrogenase (17β-HSD), human 15-hydroxyprostaglandin dehydrogenase (15-PGD; P15428) (Ensor et al., *Journal of Biochemistry*, Vol. 265, pp.14888-91 (1990)) and a human protein of unknown function, GenBank accession number CAA20237.1. 7α-HSD (1fmc) and 3α-20β-HSD (1hdc) are two proteins with known structures that are closely related to ERAB or HADH2; 17β-HSD (1fdt) is a similar human protein of known structure. A gapped sequence alignment for comparison is derived using Gapped BLAST [Altschul et al., *Nucleic Acids Research*, Vol. 25, pp. 3389-3402 (1997)] to search NCBI's GenBank database. Results indicate that 15-PGD is a human protein sequence similar to ERAB or HADH2; CAA20237.1 is a human nucleotide sequence that is similar when translated to its corresponding protein sequence. The alignment of ERAB or HADH2 to 17β-HSD, 7α-HSD, and 3α-20β-HSD was derived from a structural superposition using the tools in Insight II [available through Molecular Simulations Inc. (1999)] coupled with visual inspection. The sequences of 15-PGD and translated CAA20237.1 were aligned to the resulting profile using CLUSTALW [Thompson et al., *Nucleic Acids Research*, Vol. 22, pp. 4673-4680 (1994)]. Every tenth residue in ERAB or HADH2 is labeled. The secondary structure elements of ERAB or HADH2, as identified by PROCHECK (Laskowski et al., *Journal of Applied Crystallography*, Vol. 26, pp. 283-291 (1993)), are underlined and labeled above the sequences. Residues that are identical (•) or similar (o) in all six proteins are marked beneath the sequences. The first six residues of ERAB or HADH2, which are disordered in the crystal structure, are not shown. The final 53 residues of 17β-HSD are also not shown.

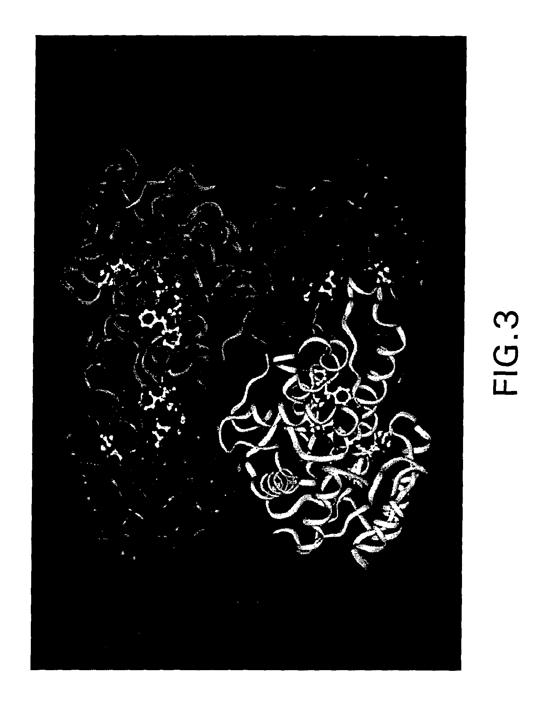

Figure 3 is a ribbon representation of the ERAB or HADH2 tetramer. The tetramer is viewed down one of three mutually perpendicular two-fold axes. Individual monomers are shown in red, grcen, blue and yellow. The bound NAD⁺ and NAD-inhibitor adduct molecules are shown in ball-and-stick representation.

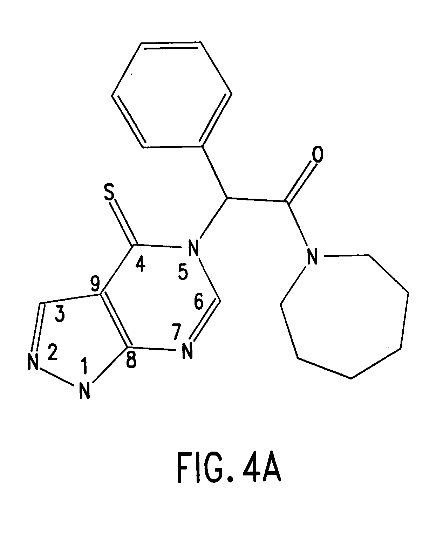

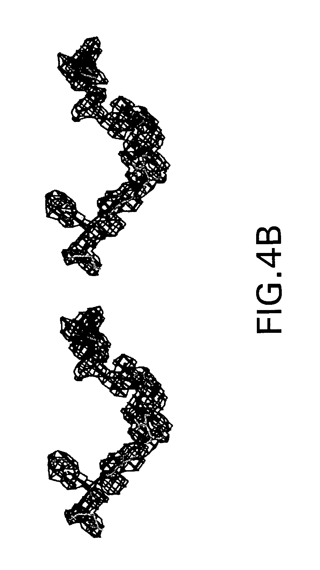

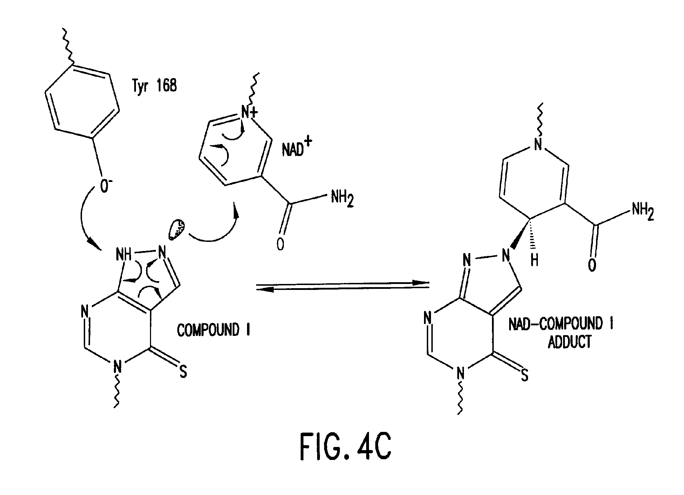

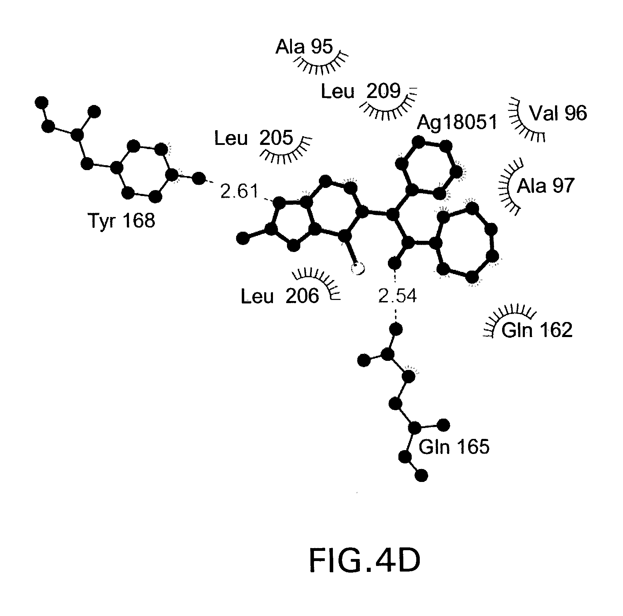

Figure 4A shows the structural formula and binding enantiomer of Compound I. Figure 4B is a stereoview of the electron density for the NAD-Compound I adduct. Figure 4C depicts the proposed reaction scheme occurring between the NAD⁺ and Compound I. Figure 4D is a schematic LIGPLOT (Wallace et al., *Protein England* Vol. 8, pp. 127-134 (1995)) view of ERAB or HADH2-Compound I interactions. Compound I, the C4N atom (see Table III) of NAD⁺, and the enzyme side-chains that form hydrogen bonds to the ligand are shown in ball-and-stick representation.

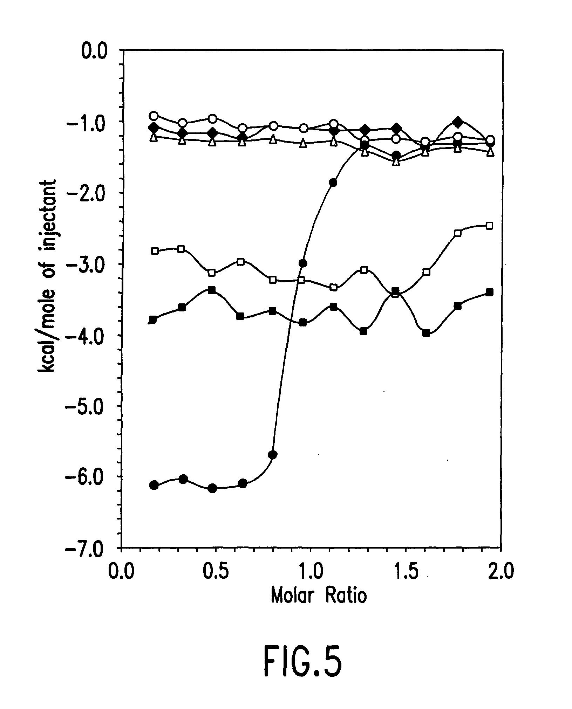

Figure 5 is a plot of integrated-binding data obtained for a calorimetric titration of wild-type ERAB or HADH2 into Compound I in the presence and absence of cofactor. Wild-type ERAB or HADH2 (415 µM) was titrated into 20 µM inhibitor in the presence and absence of 20 µM cofactor at 15°C. The closed symbols represent the integrated-data points for 10 µL injections of ERAB or HADH2 into inhibitor in the absence of cofactor (◆); in the presence of NAD⁺ (●); and in the presence of NADH (■). The open symbols represent the integrated data points for the corresponding control titrations of ERAB or HADH2 into buffer (Δ); in the presence of NAD⁺ (o); and in the presence of NADH (□). Lines connecting the data points have been added for visual clarity.

### SEQUENCE LISTINGS

- SEQ ID NO.1-: Full-length mutant ERAB or HADH2 C214R (nucleotide sequence - 786 base pairs)
- SEQ ID NO. 2-: Full-length mutant ERAB or HADH2 C214R (peptide sequence- 261 AA)
- SEQ ID NO. 3-: Full-length mutant ERAB or HADH2 C5V (nucleotide sequence - 786 base pairs)
- SEQ ID NO. 4 -: Full-length mutant ERAB or HADH2 C5V (peptide sequence - 261 AA)
- SEQ ID NO. 5 -: Full-length mutant ERAB or HADH2 C58V (nucleotide sequence- 786 base pairs)
- SEQ ID NO. 6 -: Full-length mutant ERAB or HADH2 C58V (peptide sequence - 261 AA)
- SEQ ID NO. 7 -: DNA sequence of wild-type ERAB or HADH2 (nucleotide sequence - 786 base pairs)
- SEQ ID NO. 8 -: Peptide sequence of wild-type ERAB or HADH2 (peptide sequence - 261 AA)
- SEQ ID NO. 9 -: PCR primer
- SEQ ID NO. 10 -: PCR primer
- SEQ ID NO. 11 -: Oligonucleotide for C5V
- SEQ ID NO. 12 -: Oligonucleotide for C58V
- SEQ ID NO. 13 -: Oligonucleotide for C214R
- SEQ ID NO. 14 -: Peptide sequence of 7α-HDS
- SEQ ID NO. 15 -: Peptide sequence of 3α20β -HDS
- SEQ ID NO. 16 -: Peptide sequence of 17β-HDS
- SEQ ID NO. 17 -: Peptide sequence of CAA20237.1
- SEQ ID NO. 18 -: Peptide sequence of 15- PGD
- SEQ ID NO. 19 -: Full-length mutant ERAB or HADH2 C214A (nucleotide sequence - 786 base pairs)
- SEQ ID NO. 20 -: Full-length mutant ERAB or HADH2 C214A (peptide sequence- 261 AA)
- SEQ ID NO. 21 -: Oligonucleotide for ERAB or HADH2 C214A
- SEQ ID NO. 22-: Full-length mutant ERAB or HADH2 C214S (nuclcotide sequence - 786 base pairs)
- SEQ ID NO. 23 -: Full-length mutant ERAB or HADH2 C214S (peptide sequence - 261 AA)
- SEQ ID NO. 24 -: Oligonucleotide for ERAB or HADH2 C214S

### DETAILED DESCRIPTION OF THE INVENTION

ERAB or HADH2 is a member of the short-chain dehydrogenase/reductase (SDR) family of enzymes. We have facilitated overexpression of several mutants of the enzyme in E. *coli.* The mutants having advantageous physical characteristics as compared to the wild-type ERAB or HADH2. The mutant ERAB or HADH2 peptides of the present invention have sufficient activity, stability and solubility so as to allow for peptide crystallization and subsequent x-ray crystallography characterization. Such properties facilitate the resolution of the three-dimensional crystal structure of human ERAB or HADH2 as well as the characterization of several structural features of ERAB or HADH2 responsible for interactions associated with high-affinity binding of substrates to ERAB or HADH2. Such interactions include the formation of a covalent linkage between the bound substrate and the NAD⁺ cofactor.

We have determined the crystal structure of human ERAB or HADH2 with NAD and an inhibitory small molecule bound thereto. The inhibitor occupies the substrate-binding site and forms a covalent adduct with the NAD⁺ cofactor. The crystal structure provides a basis for the design of ERAB or HADH2 inhibitors with potential application in the treatment of Alzheimer's disease. Crystals of ERAB or HADH2 with bound NAD⁺ and Compound I were obtained using the C214R mutant of the enzyme. This mutant was produced to avoid potential problems with cysteine oxidation in the protein, with arginine selected as the replacement amino acid because of its occurrence at this position in other mammalian ERAB or HADH2 sequences [He et al. (1998), *supra*]. Enzymatic activity of the C214R mutant protein is comparable to that of the wild-type protein. The crystal structure of the ERAB or HADH2:NAD⁺:Compound 1 complex was determined at 2.0 Å using molecular replacement techniques. The search model for molecular replacement was our 1.9 Å resolution structure of the mutant ERAB or HADH2:NAD⁺ complex. The structure was refined to an R factor of 0.215 for all data to 2.0 Å resolution. Data collection, structure determination, and refinement statistics are summarized in Table I. The resolution of the ERAB or HADH2 crystal structure allows for efficient design and development of inhibitors or modulators of human ERAB or HADH2. Inhibition of ERAB or HADH2 finds therapeutic utility in the treatment of AD. In addition to any therapeutic application, ERAB or HADH2 inhibitors can facilitate the delineation of the role of the enzyme in both normal cellular function and in Aβ pathogenesis.

The invention encompasses both the mutant peptides *per se* and salts thereof. The peptide may be synthetically or naturally produced. The invention further relates to an isolated, purified polynucleotide which encodes an enzymatically active peptide mutant of ERAB or HADH2 in the active conformation. The polynucleotide may be natural or recombinant.

The invention provides an expression vector for producing an enzymatically active mutant of human ERAB or HADH2 peptide in a host cell. The invention further relates to a host cell stably transformed and transfected with a polynucleotide encoding an enzymatically active peptide mutant of ERAB or HADH2, or fragment thereof, or an active fimctional analog thereof, in a manner allowing the expression of the enzymatically active peptide mutant of ERAB or HADH2 in the active conformation.

According to the present invention, an inhibitor (Compound I) of ERAB or HADH2 and the crystal structure of the ERAB or HADH2:NAD⁺:inhibitor complex are provided. The availability of a potent specific inhibitor of ERAB or HADH2 can facilitate the detailed analysis of the role of this enzyme in AD pathogenesis and may ultimately provide a new therapeutic approach for treatment of the disease. The crystal structure of the enzyme:inhibitor:cofactor complex provides a basis for the design of additional inhibitors and for elucidating the nature of the interactions between ERAB or HADH2 and the amyloid-β peptide.

The molecular structure of the x-ray crystallography data may be used to model the binding of candidate compounds in order to screen candidate compounds. In accordance with the invention, there is also provided several structural features unique to ERAB or HADH2 as compared to other members of the short-chain dehydrogenase/reductase (SDR) family. Some of these features are responsible for the high-affinity binding of an inhibitor compound to the ERAB or HADH2 substrate-binding site, including the formation of a covalent linkage between inhibitor and the NAD⁺ cofactor.

Potential inhibitors or modulators of the human ERAB or HADH2 may be identified according to the method of the present invention. Potential therapeutic compounds for the treatment of various disease conditions associated with the ERAB or HADH2, for example neurodegenerative diseases and cancers may be designed and screened according to the present invention. In the designing and screening processes, the DNA sequence or variants thereof encoding the mutant ERAB or HADH2 peptide is expressed, and may be assayed for interaction of the enzyme with the candidate compound by exposing the enzyme to the candidate compound and evaluating the interaction of the enzyme with the candidate compound. The designing and synthesis as well as screening may be automated, for example, utilizing robotic techniques.

Three-dimensional molecular structure of ERAB or HADH2 of the present invention may be used to model the binding of candiatate compounds to the ERAB or HADH2 binding site, facilitating screening candidate compounds. The x-ray crystallographic coordinates disclosed herein, allow for the generation of three-dimentsional models of the catalytic region and binding site of the human ERAB or HADH2. Design of inhibitors generally involves the generation of molecules via the use of computer programs, which build and link fragments or atoms into a site based upon steric and electrostatic complementary, without reference to substrate analog structures. The drug design process begins after the structure of the target (human ERAB or HADH2) is solved to at least a resolution of 3 Å, more preferably 2.5 Å, even more preferably 2 Å. Refinement ofthe structure to a resolution of 2 Å or better with fixed water molecules in place provides more optimal conditions to undertake drug design.

In one preferred embodiment, the method of screening utilized the steps of (a) computergenerating a virtual-binding cavity, the binding cavity defined by the binding sites; (b) computer-generating a compound structure that spatially conforms to the binding cavity; and (c) testing to see whether said compound binds to at least one of the stated-binding sites.

The invention also has utility in the iterative drug design process. The process screens potential inhibitors or modulators of the ERAB or HADH2 enzyme by determining their ability to bind to and inhibit or modulate the ERAB or HADH2. The x-ray crystallographic coordinates disclosed herein allow advantageously generation of three-dimensional models of the enzymatically active site and the drug-binding site of the ERAB or HADH2 protein. *De novo* design comprises of the generation of molecules via the use of computer programs which build and link fragments or atoms into a site based upon steric and electrostatic complementarily, without reference to substrate analog structures. The determination of the structure of the target (human ERAB or HADH2) is solved to at least a resolution of 3 Å, more preferably 2.5 Å, even more preferably 2 Å. Refinement of the structure to a resolution of 2 Å or better with fixed water molecules in place provides more optimal conditions to undertake drug design.

In one preferred embodiment, the method for identifying potential inhibitors utilizes the steps of (a) designing a poteintial inhibitor or modulator for ERAB or HADH2 that will interact with amino acids in the ERAB or HADH2 substrate-binding site based on the crystal coordinates of the mutant ERAB or HADH2 peptide:ligand:NAD⁺ complex set forth in Table II; and (b) synthesizing the potential inhibitor or modulator and assaying it to determine whether it inhibits or modulates the activity of ERAB or HADH2.

Rapidly screening methods to assay those compounds that inhibit or modulate the ERAB or HADH2 enzyme or core structure for further ERAB or HADH2 inhibitor design may be used. The high throughput-screening assay is capable of being fully automated on robotic workstations. The assay may employ radioactivity or other materials useful for detection.

A computer processor may be used for analyzing a molecular structure comprising by using a machine-readable data storage medium comprising a data storage material encoded with machine-readable data. The data comprises crystal coordinates of mutant ERAB or HADH2 peptide molecule or its molecular complex. In this method, a CPU coupled to a working memory and the machine readable data may be used to perform a Fourier transformation of the machine readable data and to process such data into crystal coordinates of three-dimensional molecule or complex of said peptide. The x-ray coordinate data may be processed into three-dimensional graphical display using a computer-based method. The machine-readable data storage medium may include CD-ROM, magneto-optic disk, or other storage media.

The terms as used herein are explained below. Also, as used herein, the terms "comprising" and "including" are used in the conventional, non-limiting sense.

### A. Peptides, Proteins and Antibodies

The present invention provides isolated peptide and protein molecules that consist of, consist essentially of or are comprised of the amino acid sequences of the mutant peptides encoded by the nucleic acid sequences disclosed in the SEQ ID NO: 1, SEQ ID NO: 3, SEQ ID NO: 5, SEQ ID NO: 19 and SEQ ID NO: 22 as well as all obvious variants of these peptides that the within the art to make and use. Some of these variants are described in detail below.

The invention also includes a modified, enzymatically active ERAB or HADH2 peptide comprising a fold, wherein said fold comprises: a core β-sheet of seven strands sandwiched between two sets of three α-helices; and first and second insert domains, relative to other members of the SDR family, the first insert domain forming a β hairpin that extends the surface of said ERAB or HADH2 peptide on one side of a substrate-binding cleft, and the second insert domain extending a loop between an α-helix comprising residues 123-136 and a β-sheet comprising residues 148-153 of SEQ ID NO: 2. The invention provides a peptide having an amino acid sequence comprising amino acids 90 to 261 of SEQ ID NO. 2 or an active or functional variant thereof.

As used herein, a peptide is said to be "isolated" or "purified" when it is substantially free of homologous cellular material or free of chemical precursors or other chemicals. The peptides of the present invention can be purified to homogeneity or other degrees of purity. The level of purification will be based on the intended use. The critical feature is that the preparation allows for the desired function of the peptide, even if in the presence of considerable amounts of other components.

In some uses, "substantially free of cellular material" includes preparations of the peptide having less than 30% (by dry weight) other proteins (i.e., contaminating protein), preferably less than 20% other proteins, more preferably less than 10% other proteins, or most preferably less than 5% other proteins. When the peptide is recombinantly produced, it can also be substantially free of culture medium, i.e., culture medium represents less than 20% of the volume of the protein preparation.

The language "substantially free of chemical precursors or other chemicals" includes preparations of the peptide in which it is separated from chemical precursors or other chemicals that are involved in its synthesis. In one embodiment, the language "substantially free of chemical precursors or other chemicals" includes preparations of the mutant peptide having less than 30% (by dry weight) chemical precursors or other chemicals, preferably less than 20% chemical precursors or other chemicals, more preferably less than 10% chemical precursors or other chemicals, or most preferably less than 5% chemical precursors or other chemicals.

The isolated mutant peptide described herein can be purified from cells that naturally express it, purified from cells that have been altered to express it (recombination), or synthesized using known protein synthesis methods. For example, a nucleic acid molecule encoding the mutant peptide is cloned into an expression vector, the expression vector introduced into a host cell and the peptide expressed in the host cell. The peptide can then be isolated from the cells by an appropriate purification scheme using standard peptide/protein purification techniques. Many of these techniques are described in detail below.

As mentioned above, the present invention also provides and enables obvious variants of the amino acid sequence of the peptides of the present invention, such as naturally occurring mature forms of the peptides, allelic/sequence variants of the peptides, non-naturally occurring recombinantly derived variants of the peptides, and orthologs and paralogs of the peptides. Such variants can be generated using techniques that are known by those skilled in the fields of recombinant nucleic acid technology and protein biochemistry.

Such variants can readily be identified/made using molecular techniques and the sequence information disclosed herein. Further, such variants can readily be distinguished from other peptides based on sequence and/or structural homology to the peptides of the present invention. The degree of homology/identity present will be based primarily on whether the peptide is a functional variant or non-functional variant, the amount of divergence present in the paralog family and the evolutionary distance between the orthologs.

To determine the percent identity of two amino acid sequences or two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes is at least 30%, preferably 40%, more preferably 50%, even more preferably 60% or more of the length of the reference sequence. In a preferred embodiement, the length of a reference sequence aligned for comparison purposes is at least 70%, preferably 80%, more preferably 90% or more of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid 'identity' is equivalent to amino acid or nucleic acid 'homology'). The percent identity between the two sequences is a function of the number of identical positions shared by the sequences, taking into account the number of gaps, and the length of each gap, which need to be introduced for optimal alignment of the two sequences.

The comparison of sequences and determination of percent identity and similarity between two sequences can be accomplished using a mathematical algorithm. (See, e.g., *Computational Molecular Biology*, Lesk, A.M., ed., Oxford University Press, New York, 1988; *Biocomputing: Informatics and Genome Projects*, Smith, D.W., ed., Academic Press, New York, 1993; *Computer Analysis of Sequence Data, Part 1*, Griffin, A.M., and Griffin, H.G., eds., Humana Press, New Jersey, 1994; *Sequence Analysis in Molecular Biology*, von Heinje, G., Academic Press, 1987; and *Sequence Analysis Primer*, Gribskov, M. and Devereux, J., eds., M Stockton Press, New York, 1991). In a preferred embodiment, the percent identity between two amino acid sequences is determined using the Needleman and Wunsch (*J. Mol. Biol.* (48):444-453 (1970)) algorithm which has been incorporated into commercially available computer programs, such as GAP in the GCG software package, using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences can be determined using the commercially available computer programs including the GAP program in the GCG software package (Devereux, J., et al., *Nucleic Acids Res*. *12(1)*:387 (1984)), the NWS gap DNA CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4:11-17 (1989)) which has been incorporated into commercially available computer programs, such as ALIGN (version 2.0), using a PAM120 weight residue table, a gap length penalty of 12 and a gap penalty of 4.

The nucleic acid and protein sequences of the present invention can further be used as a "query sequence" to perform a search against sequence databases to, for example, identify other family members or related sequences. Such searches can be performed using commercially available search engines, such as the NBLAST and XBLAST programs (version 2.0) of Altschul, et al. (*J. Mol. Biol*. 215:403-10 (1990)). BLAST nucleotide searches can be performed with the NBLAST program, score = 100, wordlength = 12 to obtain nucleotide sequences homologous to the nucleic acid molecules of the invention. BLAST protein searches can be performed with the XBLAST program, score = 50, wordlength = 3 to obtain amino acid sequences homologous to the proteins of the invention. To obtain gapped alignments for comparison purposes, Gapped BLAST can be utilized as described in Altschul et al. (*Nucleic Acids Res*. 25(17):3389-3402 (1997)). When utilizing BLAST programs, the default parameters of the respective programs (e.g., XBLAST and NBLAST) can be used.

Full-length clones comprising one of the peptides of the present invention can readily be identified as having complete sequence identity to one of the mutant peptides of human ERAB or HADH2 of the present invention as well as being encoded by the same genetic locus as the mutant peptide provided herein.

Allelic variants of a peptide can readily be identified as having a high degree (significant) of sequence homology/identity to at least a portion of the peptide as well as being encoded by the same genetic locus as the mutant peptide provided herein. As used herein, two proteins (or a region of the proteins) have significant homology when the amino acid sequences are typically at least 70%, preferably 75%, more preferably 80% or 85%, and typically at least 90%, more preferably 95% or more homologous. A significanlly homologous amino acid sequence, according to the present invention, will be encoded by a nucleic acid sequence that will hybridize to a peptide encoding nucleic acid molecule under stringent conditions as described below.

Paralogs of a peptide can readily be identified as having some degree of significant sequence homology/identity to at least a portion of the mutant peptide as well as being encoded by a gene from human, and as having similar activity or function. Two proteins will typically be considered paralogs when the amino acid sequences are typically at least 70%, preferably 80%, more preferably at least 90%, most preferably 95% or more homologous through a given region or domain. Such paralogs will be encoded by a nucleic acid sequence that will hybridize to a mutant peptide encoding nucleic acid molecule under stringent conditions as described in the section "B. Nucleic Acids and Polynucleotides" below.

Orthologs of a mutant peptide can readily be identified as having some degree of significant sequence homology/identity to at least a portion of the mutant peptide as well as being encoded by a gene from another organism. Preferred orthologs will be isolated from mammals other than human, for the development of human therapeutic targets and agents, or other invertebrates, particularly insects of economical/agriculture importance, e.g. members of the Lepidopteran and Colcopteran orders, for the development of insecticides and insecticidal targets. Such orthologs will be encoded by a nucleic acid sequence that will hybridize to a mutant peptide encoding nucleic acid molecule under moderate to highly stringent conditions, as more fully described below, depending on the degree of relatedness of the two organisms yielding the proteins.

Non-naturally occurring variants of the mutant peptides of the present invention can readily be generated using recombinant techniques. Such variants include, but are not limited to deletions, additions and substitutions in the amino acid sequence of the mutant peptide. For example, one class of substitutions involves conserved amino acid changes. Such substitutions are those that substitute a given amino acid in a peptide by another amino acid of like characteristics. Typically seen as conservative substitutions are the replacements, one for another, among the aliphatic amino acids Ala, Val, Leu, and Ile, interchange of the hydroxyl residues Ser and Thr; exchange of the acidic residues Asp and Glu; substitution between the amide residues Asn and Gln; exchange of the basic residues Lys and Arg; and replacements among the aromatic residues Phe, Tyr. Guidance concerning which amino acid changes are likely to be phenotypically silent are found in Bowie et al., *Science 247*:1306-1310 (1990).

Variants of the mutant peptide can be fully functional or can lack function in one or more activities. Fully functional variants typically contain only conservative variation or variation in non-critical residues or in non-critical regions Functional variants can also contain substitution of similar amino acids, which result in no change or an insignificant change in function. Alternatively, such substitutions may positively or negatively affect function to some degree.

Non-functional variants typically contain one or more non-conservative amino acid substitutions, deletions, insertions, inversions, or truncation or a substitution, insertion, inversion, or deletion in a critical residue or critical region.

Amino acids that are essential for function can be identified by methods known in the art, such as site-directed mutagenesis or alanine-scanning mutagenesis (Cunningham et al., *Science 244*:1081-1085 (1989)). The latter procedure introduces single alanine mutations at every residue in the molecule. The resulting mutant molecules are then tested for biological activity such as receptor binding or *in vitro* proliferative activity. Sites that are critical for binding can also be determined by structural analysis such as x-ray crystallography, nuclear magnetic resonance or photoaffinity labeling (Smith et al., *J. Mol. Biol. 224*:899-904 (1992); de Vos et al. *Science 255*:306-312 (1992)). Accordingly, the polypeptides also encompass derivatives or analogs in which a substituted amino acid residue is not one encoded by the genetic code; in which a substituent group is included, in which the mature polypeptide is fused with another compound, such as a compound to increase the half-life of the polypeptide (for example, polyethylene glycol), or in which the additional amino acids are fused to the mature polypeptide, such as a leader or secretory sequence or a sequence for purification of the mature polypeptide or a pro-protein sequence.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. The terms "peptide", "polypeptide" and "protein" are used interchangeably herein. Polypeptides may contain amino acids other than the 20 naturally occurring amino acids. Further, many amino acids, including the terminal amino acids, may be modified by natural processes, such as processing and other post-translational modifications, or by chemical modification techniques well known in the art. Common modifications that occur naturally in polypeptides are described in basic texts, detailed monographs, and the research literature, and they are well known to those of skill in the art. Generally known modifications include, but are not limited to, acetylation, acylation. ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, phenylation, racemization, selenoylation, sulfation, transfer-RNA mediated addition of amino acids to proteins such as arginylation, and ubiquitination. Several particularly common modifications, glycosylation, lipid attachment, sulfation, gamma-carboxylation of glutamic acid residues, hydroxylation and ADP-ribosylation, for instance, are described in most basic texts, such as Creighton, *Proteins - Structure and Molecular Properties*, 2nd Ed., W. H. Freeman and Company, New York (1993). Many reviews are available on this subject, such as Wold, *Posttranslational Covalent Modification of Proteins*, B.C. Johnson, Ed., Academic Press, New York 1-12 (1983); Seifter et al. (*Meth. Enzymol. 182*: 626-646 (1990)) and Rattan *et al*. (*Ann. N.Y. Acad. Sci. 663*:48-62 (1992)).

The present invention further provides for fragments of the mutant pcptides, in addition to proteins and peptides that comprises and consist of such fragments.

As used herein, a fragment comprises at least 8 or more contiguous amino acid residues from the mutant peptide of ERAB or HADH2. Such fragments can be chosen based on the ability to retain one or more of the biological activities of the ERAB or HADH2 or could be chosen for the ability to perform a function, e.g. act as an immunogen. Particularly important fragments are biologically acitive fragments, peptides which are, for example about 8 or more amino acids in length. Such fragments will typically comprise a domain or motif of the mutant peptide, e.g., active site. Further, possible fragments include, but are not limited to, domain or motif containing fragments, soluble peptide fragments, and fragments containing immunogenic structures. Predicted domains and functional sites are readily identifiable by computer programs well-known and readily available to those of skill in the art (e.g., by PROSITE analysis).

The peptides of the present invention can be attached to heterologous sequences to form chimeric or fusion proteins. Such chimeric and fusion proteins comprise a peptide operatively linked to a heterologous protein having an amino acid sequence not substantially homologous to the mutant peptide of ERAB or HADH2. "Operatively linked" indicates that the peptide and the heterologous protein are fused in-frame. The heterologous protein can be fused to the N-terminus or C-terminus of the mutant peptide of ERAB or HADH2. The two peptides linked in a fusion peptide are typically derived from two independent sources, and therefore a fusion peptide comprises two linked peptides not normally found linked in nature. The two peptides may be from the same or different genome.

In some uses, the fusion protein does not affect the activity of the peptide *per se*. For example, the fusion protein can include, but is not limited to, enzymatic fusion proteins, for example beta-galactosidase fusions, yeast two-hybrid GAL fusions, poly-His fusions, MYC-tagged, HI-tagged and Ig fusions. Such fusion proteins, particularly poly-His fusions, can facilitate the purification of recombinant peptide. In certain host cells (e.g., mammalian host cells), expression and/or secretion of a protein can be increased by using a heterologous signal sequence.

A chimeric or fusion protein can be produced by standard recombinant DNA techniques. For example, DNA fragments coding for the different protein sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence [see Ausubel et al., *Current Protocols in Molecular Biology* (1992)]. Moreover, many expression vectors are commercially available that already encode a fusion moiety (e.g., a GST protein). A mutant ERAB or HADH2 peptide-encoding nucleic acid can be cloned into such an expression vector such that the fusion moiety is linked in-frame to the mutant peptide.

### B. Nucleic Acids and Polynucleotides

The present invention provides isolated nucleic acid molecules that encode the functional, active mutant peptides of ERAB or HADH2 of the present invention. Such nucleic acid molecules will consist of, consist essentially of, or comprise a nucleotide sequence that encodes one of the peptides of the present invention, an allelic variant thereof, or an ortholog or paralog thereof.

As used herein, an "isolated" nucleic acid molecule is one that is separated from other nucleic acid present in the natural source of the nucleic acid. Preferably, an "isolated" nucleic acid is free of sequences which naturally flank the nucleic acid (i.e., sequences located at the 5' and 3' ends of the nucleic acid) in the genomic DNA or cDNA of the organism from which the nucleic acid is derived. However, there can be some flanking nucleotide sequences, for example up to about 5KB, particularly contiguous peptide encoding sequences and peptide encoding sequences within the same gene but separated by introns in the genomic sequence. The important point is that the nucleic acid is isolated from remote and unimportant flanking sequences such that it can be subjected to the specific manipulations described herein such as recombinant expression, preparation of probes and primers, and other uses specific to the nucleic acid sequences.

Moreover, an "isolated" nucleic acid molecule, such as a cDNA molecule, can be substantially free of other cellular material, or culture medium when produced by recombinant techniques, or chemical precursors or other chemicals when chemically synthesized. However, the nucleic acid molecule can be fused to other coding or regulatory sequences and still be considered isolated.

For example, recombinant DNA molecules contained in a vector are considered isolated. Further examples of isolated DNA molecules include recombinant DNA molecules maintained in heterologous host cells or purified (partially or substantially) DNA molecules in solution, Isolated RNA molecules include *in vivo* or *in vitro* RNA transcripts of the isolated DNA molecules of the present invention. Isolated nucleic acid molecules according to the present invention further include such molecules produced synthetically.

Full-length genes may be cloned from known sequence using any one of a number of methods known in the art. For example, a method which employs XL-PCR (Perkin-Elmer, Foster City, Calif.) to amplify long pieces of DNA may be used. Other methods for obtaining full-length sequences are known in the art

The isolated nucleic acid molecules can encode the active protein plus additional amino or carboxyl-terminal amino acids, or amino acids interior to the mature peptide (when the mature form has more than one peptide chain, for instance). Such sequences may play a role in processing of a protein from precursor to an active form, facilitate protein trafficking, prolong or shorten protein half-life or facilitate manipulation of a protein for assay or production, among other things. As generally is the case *in situ*, the additional amino acids may be processed away from the mature protein by cellular enzymes. As mentioned above, the isolated nucleic acid molecules include, but are not limited to, the sequence encoding the active mutant peptide of ERAB or HADH2 alone or in combination with coding sequences, such as a leader or secretory sequence (e.g., a pre-pro or pro-protein sequence), the sequence encoding the active mutant peptide, with or without the additional coding sequences, plus additional non-coding sequences, for example introns and non-coding 5' and 3' sequences such as transcribed but non-translated sequences that play a role in transcription, including mRNA processing (including splicing and polyadenylation signals), ribosome binding sites and sequences important for stability of mRNA. In addition, the nucleic acid molecule may be fused to a marker sequence encoding, for example, a peptide that facilitates purification.

Isolated nucleic acid molecules can be in the form of RNA, such as mRNA, or in the form of DNA, including cDNA and genomic DNA, obtained by cloning or produced by chemical synthetic techniques or by a combination thereof. The nucleic acid, especially DNA, can be double-stranded or single-stranded. Single-stranded nucleic acid can be the coding strand (sense strand) or the non-coding strand (antisense strand).

The invention further provides nucleic acid molecules that encode fragments of the peptides of the present invention and that encode obvious variants of the peptides of the present invention that are described herein. Such nucleic acid molecules may be naturally occurring, such as allelic variants (same locus), paralogs (different locus), and orthologs (different organism), or may be constructed by recombinant DNA methods or by chemical synthesis. Such non-naturally occurring variants may be made by mutagenesis techniques, including those applied to nucleic acid molecules, cells, or organisms. Accordingly, as discussed above, the variants can contain nucleotide substitutions, deletions, inversions and insertions. Variation can occur in either or both the coding and non-coding regions. The variations can produce both conservative and non-conservative amino acid substitutions.

A fragment comprises a contiguous nucleotide sequence greater than 12 or more nucleotides. Further, a fragment could be at least 30, preferably 40, more preferably 50, even more preferably 100, most preferably 250 or 500 nucleotides in length. The length of the fragment will be based on its intended use. For example, the fragment can encode epitope bearing regions of the peptide, or can be useful as DNA probes and primers. Such fragments can be isolated using the known nucleotide sequence to synthesize an oligonucleotide probe. A labeled probe can then be used to screen a cDNA library, genomic DNA library, or mRNA to isolate nucleic acid corresponding to the coding region. Further, primers can be used in PCR reactions to clone specific regions of gene.

A probe/primer typically comprises a substantially purified oligonucleotide or oligonucleotide pair. The oligonucleotide typically comprises a region of nucleotide sequence that hybridizes under stringent conditions to at least 12, preferably 20, more preferably 25, even more preferably 40, most preferably 50 or more consecutive nucleotides.

Orthologs, homologs, and allelic variants can be identified using methods known in the art. As described above, these variants comprise a nucleotide sequence encoding a peptide that is typically 60%, preferably 70%, more preferably 80%, even more preferably 85%, and typically at least 90%, preferably 95% or more homologous to the nucleotide sequence provided in SEQ ID NO: 1, SEQ ID NO: 3, SEQ ID NO: 5, SEQ ID NO: 19 or SEQ ID NO: 22, or a fragment of these sequences, Such nucleic acid molecules can readily be identified as being able to hybridize under moderate to highly stringent conditions, to the nucleotide sequences shown in SEQ ID NO: 1, SEQ ID NO: 3, SEQ ID NO: 5, SEQ ID NO: 19 or SEQ ID NO: 22, or a fragment thereof.

As used herein, the term "hybridizes under stringent conditions" is intended to describe conditions for hybridization and washing under which nucleotide sequences encoding a peptide at least 50%, preferably 55% or more homologous to each other typically remain hybridized to each other. The conditions can be such that sequences at least 65%, preferably 70%, more preferably 75% or more homologous to each other typically remain hybridized to each other. Standard hybridization conditions from moderate to highly stringent conditions are known to those skilled in the art (See e.g., *Current Protocols in Molecular Biology*, John Wiley & Sons, N.Y. (1989), 6.3.1-6.3.6). Moderate hybridization conditions are defined as equivalent to hybridization in 2X sodium chloride/sodium citrate (SSC) at 30 °C, followed by one or more washes in 1X SSC, 0.1% SDS at 50-60 °C. Highly stringent conditions are defined as equivalent to hybridization in 6X sodium chloride/sodium citrate (SSC) at 45 °C, followed by one or more washes in 0.2X SSC, 0.1% SDS at 50-65 °C.

The nucleic acid molecules of the present invention are useful for probes, primers, chemical intermediates, and in biological assays. The nucleic acid molecules are useful as a hybridization probe for cDNA and genomic DNA to isolate full-length cDNA and genomic clones encoding the peptide described herein and to isolate cDNA and genomic clones that correspond to variants (alleles, orthologs, etc.) producing the same or related peptides described herein.

The probe can correspond to any sequence along the entire length of the nucleic acid molecules provided in the SEQ ID NO: 1, SEQ ID NO: 3. SEQ ID NO: 5, SEQ ID NO: 19 or SEQ ID NO: 22. Accordingly, it could be derived from 5' noncoding regions, the coding region, and 3' noncoding regions.

The nucleic acid molecules are also useful as primers for PCR to amplify any given region of a nucleic acid molecule and are useful to synthesize antisense molecules of desired length and sequence.

The nucleic acid molecules are also useful for constructing recombinant vectors. Such vectors include expression vectors that express a portion of, or all of, the peptide sequences. Vectors also include insertion vectors, used to integrate into another nucleic acid molecule sequence, such as into the cellular genome, to alter *in situ* expression of a gene and/or gene product. For example, an endogenous coding sequence can be replaced via homologous recombination with all or part of the coding region containing one or more specifically introduced mutations.

The nucleic acid molecules are also useful for expressing antigenic portions of the proteins; for determining the chromosomal positions of the nucleic acid molecules by means of *in situ* hybridization methods; for designing ribozymes corresponding to all, or a part, of the mRNA produced from the nucleic acid molecules described herein; for constructing host cells expressing a part, or all, of the nucleic acid molecules and peptides; for constructing transgenic animals expressing all, or a part, of the nucleic acid molecules and peptides; and for making vectors that express part, or all, of the peptides.

The nucleic acid molecules are also useful as hybridization probes for determining the presence, level, form and distribution of nucleic acid expression. Accordingly, the probes can be used to detect the presence of, or to determine levels of, a specific nucleic acid molecule in cells, tissues, and in organisms. The nucleic acid whose level is determined can be DNA or RNA. Accordingly, probes corresponding to the peptides described herein can be used to assess expression and/or gene copy number in a given cell, tissue, or organism. These uses are relevant for diagnosis of disorders involving an increase or decrease in protein expression relative to normal results.

*In vitro* techniques for detection of mRNA include Northern hybridizations and *in situ* hybridizations. *In vitro* techniques for detecting DNA include Southern hybridizations and *in situ* hybridization.

Probes can be used as a part of a diagnostic test kit for identifying cells or tissues that express a protein.

### C. Vectors and Host Cells

The invention also provides vectors containing the nucleic acid molecules described herein. The term "vector" refers to a vehicle, preferably a nucleic acid molecule, that can transport the nucleic acid molecules. When the vector is a nucleic acid molecule, the nucleic acid molecules are covalently linked to the vector nucleic acid. With this aspect of the invention, the vector includes a plasmid, single or double stranded phage, a single or double stranded RNA or DNA viral vector, or artificial chromosome, such as a BAC, PAC, YAC, OR MAC. Various expression vectors can be used to express polynucleotide encoding the mutant peptide of human ERAB OR HADH2.

A vector can be maintained in the host cell as an extrachromosomal element where it replicates and produces additional copies of the nucleic acid molecules. Alternatively, the vector may integrate into the host cell genome and produce additional copies of the nucleic acid molecules when the host cell replicates.

The invention provides vectors for the maintenance (cloning vectors) or vectors for expression (expression vectors) of the nucleic acid molecules. The vectors can function in prokaryotic or eukaryotic cells or in both (shuttle vectors).

Expression vectors contain cis-acting regulatory regions that are operably linked in the vector to the nucleic acid molecules such that transcription of the nucleic acid molecules is allowed in a host cell. The nucleic acid molecules can be introduced into the host cell with a separate nucleic acid molecule capable of affecting transcription. Thus, the second nucleic acid molecule may provide a trans-acting factor interacting with the cis-regulatory control region to allow transcription of the nucleic acid molecules from the vector. Alternatively, a trans-acting factor may be supplied by the host cell. Finally, a trans-acting factor can be produced from the vector itself. It is understood, however, that in some embodiments, transcription and/or translation of the nucleic acid molecules can occur in a cell-free system.

The regulatory sequences to which the nucleic acid molecules described herein can be operably linked include promoters for directing mRNA transcription. These include, but are not limited to, the left promoter from bacteriophage λ, the lac, TRP, and TAC promoters from *E. coli,* the early and late promoters from SV40, the CMV immediate early promoter, the adenovirus early and late promoters, and retrovirus long-terminal repeats (LTR).

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination and, in the transcribed region a ribosome-binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook et al., (*Molecular Cloning: A Laboratory Manual, 2nd. ed*., Cold Spring Harbor Laboratory Press, Cold Spring Harbor, NY, (1989)).

A variety of expression vectors can be used to express a nucleic acid molecule. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses, adenoviruses, poxviruses, pseudorabies viruses, and retroviruses. Vectors may also be derived from combinations of these sources such as those derived from plasmid and bacteriophage genetic elements, e.g., cosmids and phagemids. Appropriate cloning and expression vectors for prokaryotic and eukaryotic hosts are described in Sambrook et al., *Molecular Cloning: A Laboratory Manual. 2nd. ed*., Cold Spring Harbor Laboratory Press, Cold Spring Harbor, NY, (1989).

The regulatory sequence may provide constitutive expression in one or more host cells (i.e. tissue specific) or may provide for inducible expression in one or more cell types such as by temperature, nutrient additive, or exogenous factor such as a hormone or other ligand. A variety of vectors providing for constitutive and inducible expression in prokaryotic and eukaryotic hosts are known to those of ordinary skill in the art.

The nucleic acid molecules can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are known to those of ordinary skill in the art.

The vector containing the appropriate nucleic acid molecule can be introduced into an appropriate host cell for propagation or expression using well-known techniques. Bacterial cells include, but are not limited to, *E. coli, Streptomyces*, and *Salmonella typhimurium.* Eukaryotic cells include, but are not limited to, yeast, insect cells such as *Drosophila*, animal cells such as COS and CHO cells, and plant cells.

It may be desirable to express a peptide of the present invention as a fusion protein. Accordingly, the invention provides fusion vectors that allow for the production of such peptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired peptide can ultimately be separated from the fusion moiety. Proteolytic enzymes include, but are not limited to, factor Xa, thrombin, and enterokinase. Typical fusion expression vectors include pGEX (Smith et al., (1988) *Gene 67*:31-40), pMAL (New England Biolabs, Beverly, MA) and pRfT5 (Pharmacia, Piscataway, NJ) which fuse glutathione S-transferase (GST), maltose E binding protein, or protein A, respectively, to the target recombinant protein. Examples of suitable inducible non-fusion *E. coli* expression vectors include pTrc (Amann et al., (1988) *Gene 69*:301-315) and pET 11d (Studier et al., (1990) *Gene Expression Technology: Methods in Enzymology* 185:60-89).

Recombinant protein expression can be maximized in a host bacteria by providing a genetic background wherein the host cell has an impaired capacity to proteolytically cleave the recombinant protein. (Gottesman, S., *Gene Expression Technology: Methods in Enzymology 185,* Academic Press, San Diego, California (1990) 119-128). Alternatively, the sequence of the nucleic acid molecule of interest can be altered to provide preferential codon usage for a specific host cell, for example *E. coli*. (Wada et al., (1992) *Nucleic Acids Res. 20*:2111-2118).

The nucleic acid molecules can also be expressed by expression vectors that are operative in yeast. Examples of vectors for expression in yeast e.g., *S. cerevisiae* include pYepScc1 (Baldari, et al., (1987) *EMBO J. 6*:229-234), pMFa (Kurjan et al., (1982) *Cell 30*:933-943), pJRY88 (Schultz et al., (1987) *Gene 54*:113-123), and pYES2 (Invitrogen Corporation, San Diego, CA).

The nucleic acid molecules can also be expressed in insect cells using, for example, baculovirus expression vectors. Baculovirus vectors available for expression of proteins in cultured insect cells (e.g., Sf9 cells) include the pAc series (Smith et al., (1983) *Mol. Cell Biol. 3*:2156-2165) and the pVL series (Lucklow et al, (1989) *Virology 170*:31-39).

In certain embodiments of the invention, the nucleic acid molecules described herein are expressed in mammalian cells using mammalian expression vectors. Examples of mammalian expression vectors include pCDM8 (Seed, B, (1987) *Nature 329*:840) and pMT2PC (Kaufman et al., (1987) *EMBO J. 6*:187-195).

The expression vectors listed herein are provided by way of example only of the well-known vectors available to those of ordinary skill in the art that would be useful to express the nucleic acid molecules. Preferred vectors include the pET28a (Novagen, Madison, WI), pAcSG2 (Pharmingen, San Diego, CA), and pFastBac (Life Technologics, Gaithersburg, MD). The person of ordinary skill in the art would be aware of other vectors suitable for maintenance propagation or expression of the nucleic acid molecules described herein. These are found for example in Sambrook, J., Fritsh, E. F., and Maniatis, T. *Molecular Cloning: A Laboratory Manual. 2nd, ed, Cold Spring Harbor Laboratory*, Cold Spring Harbor Laboratory Press, Cold Spring Harbor, NY, 1989.

The invention also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the nucleic acid molecule sequences described herein, including both coding and non-coding regions. Expression of this antisense RNA is subject to each of the parameters described above in relation to expression of the sense RNA (regulatory sequences, constitutive or inducible expression, tissue-specific expression).

The invention also relates to recombinant host cells containing the vectors described herein. Host cells therefore include prokaryotic cells, lower eukaryotic cells such as yeast, other eukaryotic cells such as insect cells, and higher eukaryotic cells such as mammalian cells. Preferred host cells of the instant invention include *E. coli* and Sf9.

The recombinant host cells are prepared by introducing the vector constructs described herein into the cells by techniques readily available to the person of ordinary skill in the art These include, but arc not limited to, calcium phosphate transfection, DEAE-dextran-mediated transfection, cationic lipid-mediated transfection, electroporation, transduction, infection, lipofection, and other techniques, such as those found in Sambrook et al. (*Molecular Cloning: A Laboratory Manual. 2nd, ed., Cold Spring Harbor Laboratory*, Cold Spring Harbor Laboratory Press, Cold Spring Harbor, NY, 1989).

Host cells can contain more than one vector. Thus, different nucleotide sequences can be introduced on different vectors of the same cell. Similarly, the nucleic acid molecules can be introduced either alone or with other nucleic acid molecules that are not related to the nucleic acid molecules such as those providing trans-acting factors for expression vectors. When more than one vector is introduced into a cell, the vectors can be introduced independently, co-introduced or joined to the nucleic acid molecule vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the nucleic acid molecules described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the active proteins can be produced in bacteria, yeast, mammalian cells, and other cells under the control of the appropriate regulatory sequences, cell- free transcription and translation systems can also be used to produce these proteins using RNA derived from the DNA constructs described herein.

Where secretion of the peptide is desired, appropriate secretion signals are incorporated into the vector. The signal sequence can be endogenous to the peptides or heterologous to these peptides.

It is also understood that depending upon the host cell in recombinant production of the peptides described herein, the peptides can have various glycosylation patterns, depending upon the cell, or maybe non-glycosylated as when produced in bacteria. In addition, the peptides may include an initial modified methionine in some cases as a result of a host-mediated process.

The recombinant host cells expressing the peptides described herein have a variety of uses. First, the cells are useful for producing a peptide or protein that can be further purified to produce desired amounts of protein or fragments. Thus, host cells containing expression vectors are useful for peptide production.

Host cells are also useful for conducting cell-based assays involving the ERAB or HADH2 protein/peptjde, or its fragments. Thus, a recombinant host cell expressing a native protein is useful for assaying compounds that stimulate or inhibit protein function.

Host cells are also useful for identifying ERAB or HAHD2 protein mutants in which these functions are affected. If the mutants naturally occur and give rise to a pathology, host cells containing the mutations are useful to assay compounds that have a desired effect on the mutant protein (for example, stimulating or inhibiting function) which may not be indicated by their effect on the native protein.

### EXAMPLES

### A. Synthesis of an ERAB OR HADH2 Inhibitor (Compound I)

The structure of Compound I as a single enantiomer is shown in Figure 4A. Compound I was synthesized using two reaction schemes. The abbreviations employed in the Schemes have the following meaning unless otherwise indicated: Me: methyl; Et: ethyl; Ac: acetyl; Boc: butyloxycarbonyl; EtoAc: ethyl acetate; TFA: trifluoroacetic acid; DCC: dicyclohexylcarbodimide; rt: room temperature; HATU: [O-(7-azabcnzotriazol-1-yl)-1,1,3,3-tetra-triethyl-uronium hexafluorophsphate; DMAP: N,N-dimethyl-4-aminopyridine; and DMF: dimethylformamide.

### 1. Preparation of 1-azepan-1-yl-2-phenyl-2-(4-thioxo-1,4-dihydro-pyrazolo[3,4-d]pyrimidin-5-yl)-ethanone (Compound I) according to Scheme 1.

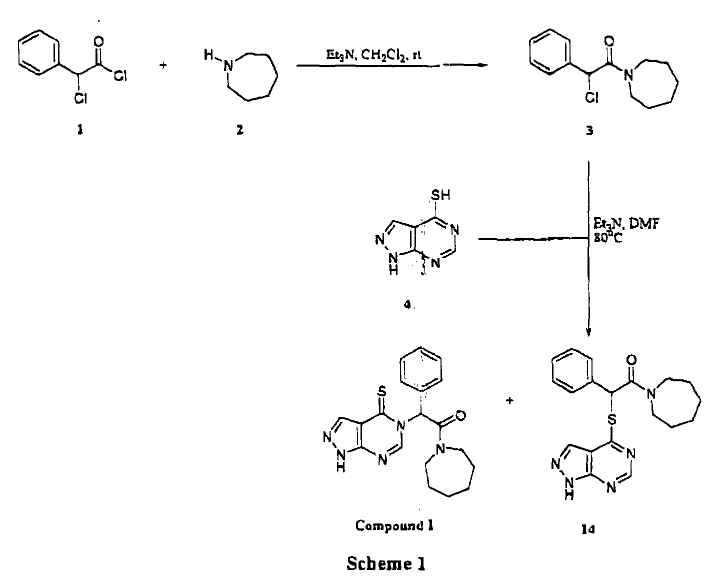

### 1-a. General description of Scheme 1.

2-Chloro-2-phenyl-acetyl chloride (1) and hexamethyleneimine (2) are reacted in dichloromethane in the presence of triethylamine to afford the intermediate (3). This intermediate (3) is then carried forward and reacted with commercially available 4-mercapto-1*H*-pyrazolo[3,4-*d*] pyrimidine (4) to afford a mixture of products Compound I and 14. The two products are separated via column chromatography to yield 1-azepan-1-yl-2-phenyl-2-(4-thioxo-1,4-dihydro-pyrazolo[3,4-d]pyrimidin-5-yl)-ethanone (Compound I) as the minor and 14 major products respectively. The desired Compound I is further purified via preparatory HPLC to yield the product in >95% purity. Compound I, made according to Scheme 1, when tested against ERAB as described below, exhibited an IC₅₀ = 88 nM.

### 1-b. Experimental Section to Scheme 1.

2-Chloro-2-phenyl-acetyl chloride (1) (2.50 ml, 15.75 mmol) was dissolved in CH₂Cl₂ (50 ml), the mixture was cooled to 0°C, hexamethyleneimine (2) (1.76 ml, 15.75 mmol) was added dropwise, followed by triethylamine (2.19 ml, 15.75 mmol). The resulting mixture containing intermediate (3) was stirred at 0°C for 15 min followed by warming to room temperature and stirring an additional 60 min. The mixture (3) was then concentrated on a rotary evaporator, the residual oil was taken up in dimethylformamide (DMF) (50 ml), triethylamine (2.19 ml, 15.75 mmol) was added, followed by addition of 4-mercapto-1*H*-pyrazolo[3,4-*d*] pyrimidine (4) (2.33g, 15.44 mmol) in one portion as a solid. The reaction product was warmed to 80°C and stirred for approximately 17 h. The reaction product was cooled to room temperature, diluted with water, and extracted with EtOAc (3 x 30 ml), and the combined organics were washed with brine, dried over anhyd. Na₂SO₄, filtered and concentrated to obtain crude products (Compound I and 14). The crude products were purified on silica gel eluting with 30-40-80 % ethyl acetate/hexane to yield two products. 3.55 g of the product (14) with the lower R_{f} was isolated as a white solid. The desired compound (0.266g) with higher R_{f} was obtained as a tan solid. This product was further purified by preparatory HPLC (5-95% CH₃CN/H₂O, 10 min, followed by 95% CH₃CN/H₂O, 0.1% trifluoroacetic acid (TFA), 10 min)). The desired HPLC fractions were combined, diluted with brine (30 ml), 50% aqueous NaHCO₃ solution (30 ml) was added, and the aqueous layer extracted with EtOAc (3 x 30 ml), combined organics washed with brine, dried over anhyd. Na₂SO₄, filtered and concentrated to yield (0.178 g, 0.484 mmol) >95% purity of Compound I. mp: 201-202 °C; R_{f} = 0.60 (EtOAc:Hexanes = 6:4). ¹H-NMR; (DMSO-d6) δ 14,18 (s, 1H), 8.27 (s, 1H), 8.17 (s, 1H), 7.95 (s, 1H), 7.52-7.54 (m, 3H), 7.39-7.41 (m, 2H), 3.19-3.74 (m, 4H), 1.34-1.90 (m, 8H). Anal. Calcd for C₁₉H₂₁N₅OS • 0.2 TFA • 0.4 H₂O: C, 62.10; H, 5.76; N, 19.06. Found: C, 58.59; H, 5.53; N, 17.84; MALDI HRMS Calcd for C₁₉H₂₁N₅OS (M+H) = 368.1545, observed (M+H) = 368.1535; HPLC: 10% CH₃CN/H₂O (0.1 %TFA)-90% CH₃CN/H₂O (0.1 %TFA): Retention time = 13.80 min.

### 2. Preparation of 1-azepan-1-yl-2-phenyl-2-(4-thioxo-1,4-dihydro-pyrazolo[3,4-d]pyrimidin-5-yl)-ethanone (Compound I) according to Scheme 2.

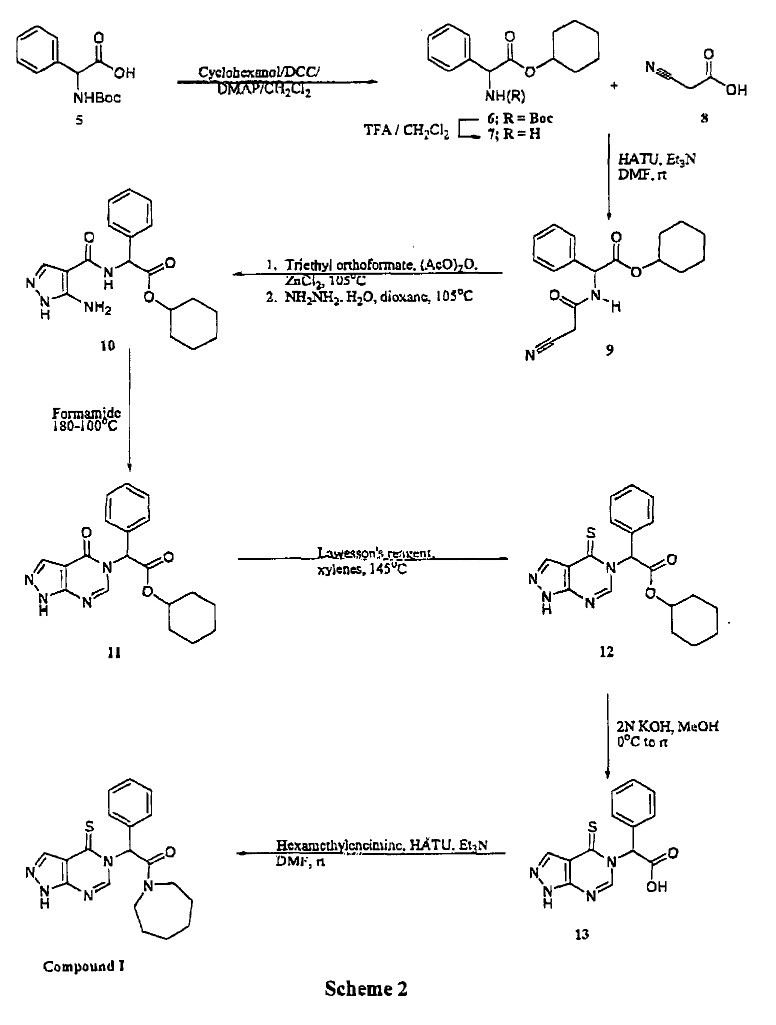

### 2-a. General description of Scheme 2.

Boc-L-α-phenylglycine (5) and cyclohexanol are reacted in dichloromethane in the presence of dicyclohexylcarbodiimide (DCC) and catalytic amount of N,N-dimethyl-4-aminopyridinc (DMAP) to give 6 (R = Boc) in almost quantitative yield. Treatment of 6 with trifluoroacetic acid (TFA) yields 7 (R = H) in 98% yield. 7 is then coupled to alpha-cyanoacetic acid (8) using O-(7-azabenzotriazol-1-yl)-1,1,3,3-tetra-triethyl-uronium hexafluorophosphate (HATU) as the coupling agent to afford (2-cyano-acetylamino)-phenyl-acetic acid cyclohexyl ester (9). The reaction of 9 with triethyl orthoformate in acetic anhydride and catalytic anhydrous ZnCl₂ gives an intermediate which upon reaction with hydrazine in 1,4-dioxane at 105°C affords 10 in 51 % yield. The [(5-amino-1H-pyrazol-4-carbonyl)-amino]-phenyl-acetic acid cyclohexyl ester (10) is then treated with formamide at 180-100°C to give the desired cyclized product (11). Compound 11 is treated with Lawesson's reagent under inert gas atmosphere to afford compound 12. Hydrolysis of compound 12 affords the carboxylic acid 13, which is then coupled to hexamethyeneimine using the coupling reagent HATU to afford Compound 1.

### 2-b. Experimental Section to Scheme 2.

### Step 1: tert- Butoxycarbonylamino-phenyl-acetic acid cyclohexyl ester (6).

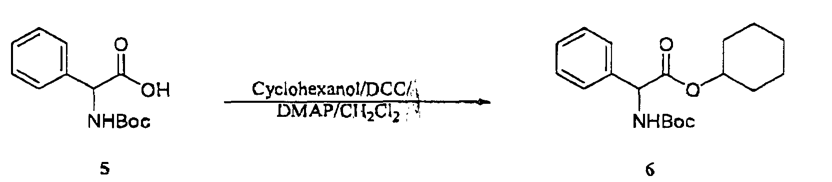

To an ice-cold suspension of Boc-L-α-phenylglycine (5) (14.14g, 40.35 mmol) and DMAP (0.5g, 4.04 mmol) and cyclohexanol (5.12 ml, 48.42 mmol) in 60 ml of CH₂Cl₂ was slowly added DCC (9.16g, 44.4 mmol). The resulting mixture was stirred at 0°C to room temperature and monitored by TLC. Upon completion the reaction was filtered and the precipitate was washed with CH₂Cl₂ to remove most of the N,N'-dicyclohexylurca. The filtrate was then partitioned between CH₂Cl₂ and said. NaHCO₃ and the layers separated. The aqueous phase was extracted with CH₂Cl₂ (2 x 200 ml), and the combined organics were washed with H₂O (150 ml), brine (150 ml) and dried over anhyd. Na₂SO₄, filtered, and concentrated under reduced pressure. The resulting yellowish oil was chromatographed on silica gel using Hexane:EtOAc = 2:1 as the elutant to yield compound 6 (13.37 g 39.95 mmol, 99% yield) as a viscous yellow oil. TLC; R_{f} = 0.7 (EtOAc:Hexanes = 6:4). ¹H-NMR; (CDCl₃) δ 1.27-1.83 (m, 10H), 1.47 (s, 9H) 4.78-4.83 (m, 1H), 5.31-5.33 (d, 1H, J = 8 Hz), 5.61-5.63 (br, s, 1H, NH), 7.32-7.41 (m, 5H). MS Calcd for C₁₉H₂₇NO₄ (M+H) = 334, observed (M+H) = 334; HPLC: 30% CH₃CN/H₂O (0.1%TFA) to 90% CH₃CN/H₂O (0.1%TFA)/20 min: Retention time = 14.61 min.

### Step 2: Amino-phenyl- acetic acid cyclohexyl ester (7).

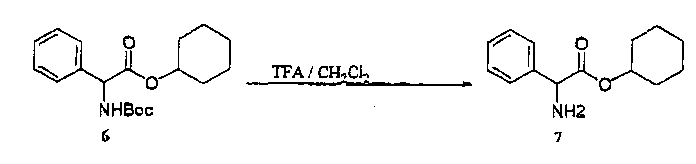

To a solution of 6 (13.27 g, 39.79 mmol) in 50 ml of CH₂Cl₂ at 0°C was added TFA (30 ml, 385.1 mmol) dropwise. The resulting yellowish mixture was stirred at 0°C to room temperature overnight. The reaction was then concentrated under reduced pressure and the resulting yellow oil was partitioned between CH₂Cl₂ (300 ml) and satd. NaHCO₃ (150 ml) and the layers separated. The aqueous phase was extracted with CH₂Cl₂ (2 x 150 ml) and the combined organics were washed with H₂O (150 ml), brine (150 ml) and dried over anhyd. Na₂SO₄, filtered and concentrated under reduced pressure to yield compound 7 (9.05 g, 38.99 mmol, 98% yield) as a yellow oil. TLC; R_{f}=0.7 (CH₂Cl₂:MeOH = 9:1). ¹H-NMR (CDCl₃) δ 1.25-2.03 (m, 10H), 4.62 (s, 1H), 4.81-4.85 (m, 1H), 7.30-7.43 (m, 5H). MS Calcd for C₁₄R₁₉NO₂ (M+H) = 334, observed (M+H) = 334; HPLC: 5% CH₃CN/H₂O (0.1 %TFA) to 90% CH₃CN/H₂O (0.1%TFA)/20min. Retention time = 7.52 min.

### Step 3: (2-Cyano-acctylamino)-phenyl-acetic acid cyclohexyl ester (9).

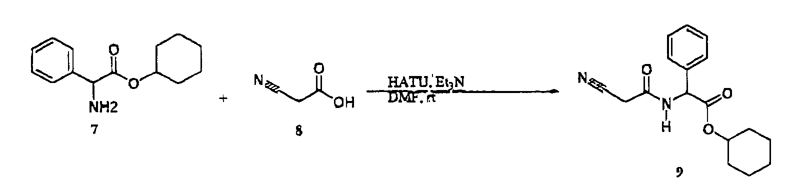

To a solution of 7 (9.01 g, 38.63 mmol), α-cyanoacetic acid (8) (3.58 g, 42.05 mmol) and triethylamine (12 ml, 84.1 mmol) in 60 ml of DMF at 0°C was added HATU (24 g, 63.1 mmol). The yellow solution was stirred at 0°C to room temperature overnight. The reaction was then partitioned between EtOAc (300 ml) and H₂O (150 ml) and the layers separated. The organic phase was washed with said. NaHCO₃ (150 ml), H₂O (150 ml), 0.5 N HCl (150 ml), brine (150 ml), and dried over anhyd. Na₂SO₄, filtered and concentrated under reduced pressure. The resulting yellow oil was chromatographed on silica gel using hexane:EtOAc = 1:1 as the elutant to yield compound 9 (6.57 g, 21.88 mmol, 57% yield) as a white solid. mp: 114-116 ° C; TLC; R_{f}= 0.8 (EtOAc:Hexanes = 1:1). ¹H-NMR; (CDCl₃) δ 1.28-1.85 (m, 10H), 3.45 (s, 2H), 4.87-5.00 (m, 1H), 5.54-5.56 (d, 1H, J = 7 Hz), 7.10-7.29 (br, s, 1H, NH), 7.29-729 (s, 5H). MS Calcd for C₁₇H₂₀N₂O₃ (M+Na) = 323, observed (M+Na) = 323; HPLC: 30% CH₃CN/H₂O (0.1%TFA) to 90% CH₃CN/H₂O (0.1 %TFA)/20min. Retention time = 9.67 min.

### Step 4: (5-Amino-1H-pyrazol-4-carbonyl)amino]-phenyl-acetic acid cyclohexyl ester (10)

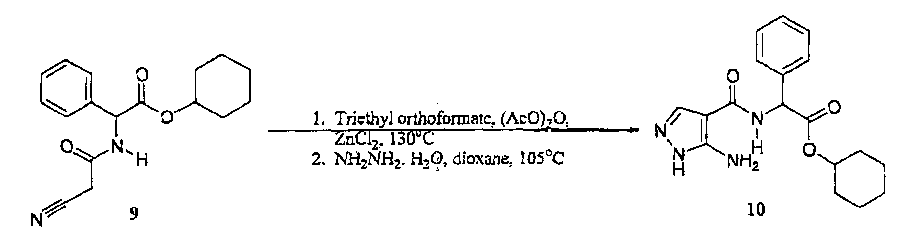

A round-bottom flask was charged with compound 9 (2.3 g, 7.67 mmol), triethyl orthoformate (9.0 ml, 53.7 mmol), acetic anhydride (4.5 ml, 46.02 mmol), and anhyd. ZnCl₂ (1.05 g, 7.67 mmol), and then heated to reflux at 130 °C for 4 h, most preferably 105 °C for 4 h. The yellow reaction mixture was concentrated under vacuum and azeotroped with toluene (3 x 15 ml). The resulting was titrated with CH₂Cl₂ (100 ml) filtered and washed with CH₂Cl₂ (150 ml). The filtrate was then concentrated under vacuum to yield crude residue as yellow grease. The crude residue was treated with hydrazine hydrate (0.56 ml, 11.51 mmol) in 10 ml of 1,4-dioxane and then refluxed at 105°C overnight. The reaction was cooled to room temperature and concentrated under vacuum. The residue was partitioned between EtOAc (250 ml) and satd. NaHCO₃, brine (2 x 100 ml), dried over anhyd. Na₂SO₄, filtered and concentrated under vacuum to afford the crude product as a yellow grease. The crude product was chromatographed using silica gel and eluted with CH₂Cl₂:MeOH = 9:1 to afford compound 10 (1.343 g, 3.92 mmol, 51 % yield) as a white solid. mp: 92-94 °C; TLC; R_{f}= 0.4 (CH₂Cl₂:MeOH = 9:1). ¹H-NMR; (CDCl₃/CD₃OD) δ 1.14-1.37 (m, 4H), 1.38-1.41 (m, 2H), 1.52-1.54 (m, 2H); 1.67-1.71 (m, 2H), 4.68-4.72 (m, 1H), 5.53 (s, 1H), 7.29-7.30 (m, 5H), 7.79 (s, 1H). MS Calcd for C₁₈H₂₂N₄O₃ (M+H) = 343, observed (M+H) = 343; HPLC: 5% CH₃CN/H₂O (0.1%TFA) to 90% CH₃CN/H₂O (0.1%TFA)/20min. Retention time = 9.24 min.

### Step 5: (4-Oxo-1,4-dihydro-pyrazolo[3,4-d] pyrimidin-5-yl)-phenyl-acetic acid cyclohexyl ester (11).

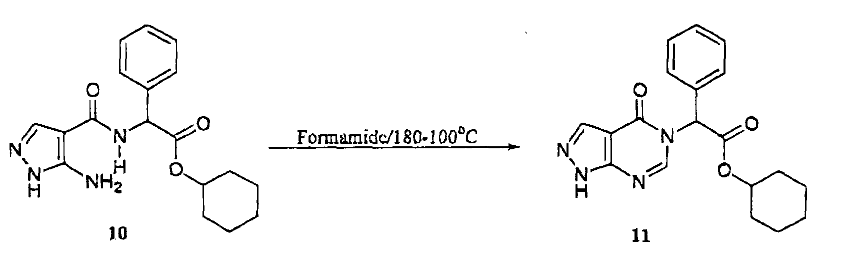

Compound 10 (1.244 g, 3.63 mmol) in 8 ml of formamide was heated to 180°C for 4 h and then at 100°C overnight, most preferably 145° overnight The cooled reaction was filtered and the white precipitate product (11) was washed with and dried in a vacuum oven over P₂O₅. Additionally, the filtrate was concentrated under reduced pressure and the residue was partitioned between EtOAc (250 ml) and satd. NaHCO₃ (2 x 100 ml), wash with brine (100 ml), dried over anhyd. Na₂SO₄, filtered and concentrated under vacuum to afford the crude product as a yellow grease, which was then chromatographed using silica gel and eluted with CH₂Cl₂:EtOAc (1:1) to afford an additional 0.341 g, 0.97 mmol, and 27 % of compound 11 as a white solid. mp: 176-180 °C; TLC; R_{f}=0.5 (CH₂Cl₂: EtOAc = 1:1). ¹H-NMR; (CDCl₃) δ 1.24-1.91 (m, 10H), 4.98-5.03 (m, 1H), 6.84 (s, 1H), 7.29-7.30 (m, 2H), 7.36-7.49 (m, 3H), 7.91 (s, 1H), 8.23 (s, 1H). MS Calcd for C₁₉H₂₀N₄O₃ (M+H) = 353, observed (M+H) = 353; HPLC: 5% CH₃CN/H₂O (0.1%TFA) to 90% CH₃CN/H₂O (0.1%TFA)/20min. Retention time = 12.9 min.

### Step 6: 2-Phenyl-(4-thioxo-1,4-dihydro-pyrazolo[3,4-d]pyrimidin-5-yl)-acetic acid cyclohexyl ester (12).

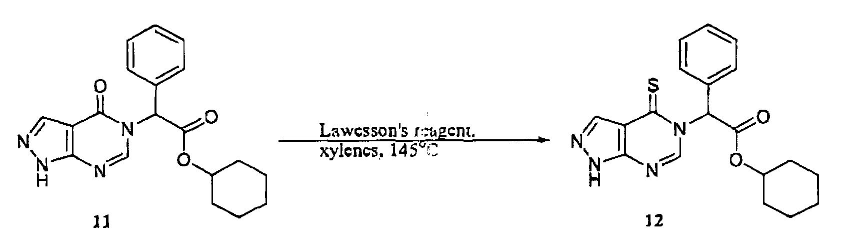

A solution of compound 11 (0.326 g, 0.93 mmol) in 5 ml of xylene was purged with argon for 20 minutes, to which was then added Lawesson's reagent (0.375 g, 0.93 mmol) and the resulting mixture heated at 145°C for 2 h under argon. The yellow reaction mixture was concentrated under vacuum and partitioned between EtOAc (100 ml) and satd. NaHCO₃, washed with brine (2 x 50 ml), dried over anhyd. Na₂SO₄, filtered and concentrated under vacuum. The crude product was chromatographed using silica gel and eluted with CH₂Cl₂: EtOAc (1:1) to afford 0.316 g, 0.86 mmol, 92 % yield of product 12 as a off white solid. mp: 170-173 °C; TLC: R_{f}= 0.8 (CH₂Cl₂:EtOAc= 1:1). ¹H-NMR (CDCl₃) δ 1.30-1.93 (m, 10H), 5.03 (m, 1H), 7.41-7.49 (m, 5H), 7.98 (s, 1H), 8.04 (s, 1H), 8.38 (s, 1H), 10.55 (br, s, 1H, NH). MS Calcd for C₁₉H₂₀N₄O₂S (M+H) = 368, observed (M+H) = 369; HPLC: 5% CH₃CN/H₂O (0.1%TFA) to 90% CH₃CN/H₂O (0.1%TFA)/20min. Retention time = 14.6 min.

### Step 7: 2-Phenyl-(4-thioxo-1,4-dihydro-pyrazolo[3,4-d]pyrimidin-5-yl)acetic acid (13).

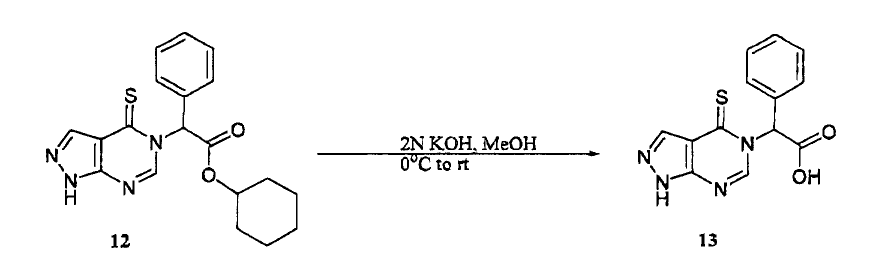

To a solution of 2-phenyl-(4-thioxo-1,4-dihydro-pyrazolo[3,4-d]pyrimidin-5-yl)acetic acid cyclohexyl ester (12), 0.467g, 1.27 mmol, in 10 ml of methanol was added to 2N KOH aqueous solution (1.3ml, 2.53 mmol) at 0°C. The resulting mixture of yellow solution was stirred at 0°C to room temperature overnight. The reaction mixture was concentrated by vacuum to remove most of methanol and water was added. The pH was adjusted to 4.0 with aqueous 10% citric acid solution. The product was extracted with EtOAc (2 x 75ml). The combined organic layer was washed with water, brine and dried over anhyd. Na₂SO₄, then concentrated by vacuum to afford compound 13 (0.354 g, 1.19 mmol, 97 % yield) as an off-white solid. mp: 95-98 °C; TLC; R_{f} = 0.1 (CH₂Cl₂:McOH = 9:1). ¹H-NMR; (CD₃OD) δ 3.23 (s, 1H), 7.50 (s, 5H), 8.08 (s, 1H), 8.09 (s, 1H), 8.34 (s, 1H, NH). MS Calcd for C₁₃H₁₀N₄O₂S (M+H) = 287, observed (M+H) = 287; HPLC: 5% CH₃CN/H₂O (0.1%TFA) to 90% CH₃CN/H₂O (0.1%TFA)/20min. Retention time = 3.9 min.

### Step 8: 1-Azepan-1-yl-2-phenyl-2-(4-thioxo-1,4-dihydro-1,4-dihrdropyrazolo[3,4-d]pyrimidin-5-yl)-ethanone (Compound I).

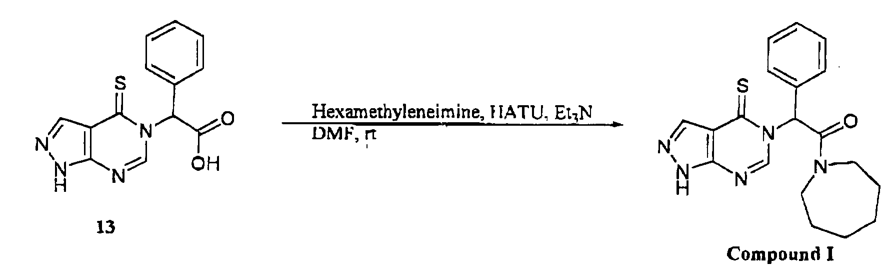

To a solution of 2-phenyl-(4-thioxo-1,4-dihydro-pyrazolo[3,4-d]pyrimidin-5-yl) acetic acid (13), 0.064 g, 0.22 mmol, and hexamethyleneimine (0.023 g, 0.23 mmol) with 4-methylmorpholine (50 µl, 0.44mol) in 3 ml of DMF at 0°C was added HATU [O-(7-azabenzotriazol-1-yl)-1,1,3,3-tetra-triethyl-uronium hexafluorophosphate] (0.125g, 0.33mol). The resulting mixture of yellow solution was stirred at 0°C to room temperature for overnight. The yellow reaction mixture was partitioned between EtOAc (100 ml) and brine, satd. NaHCO₃, brine (3 x 50 ml). The organic layer dried over anhyd. Na₂SO₄, filtered and concentrated under vacuum. The crude product was chomatographed using silica gel and eluted with CH₂Cl₂: EtOAc (1:1) to afford Compound I (0.043 g, 0.12 mmol, 53 % yield) as a yellowish solid. The Compound I obtained via the above procedure appears to be a different crystalline form from that obtained via Scheme 1. This may account for the different melting points. Co-injection of Compound I obtained via step 8, Scheme 2 and that from Scheme 1 gave a single peak via HPLC. mp: >230°C. TLC; R_{f}=0.7 (CH₂Cl₂:EtOAc = 1 :1). ¹H-NMR; (CDCl₃) δ 1.60-2.08 (m, 8H), 3.37. 3.84 (m, 4H), 7.44-7.49 (m, 5H), 8.09 (s, 1H), 8.36 (s, 1H), 8.41 (s, 1H), 10.65 (s, br, 1H, NH). MS Calcd for C₁₉H₂₁N₅OS (M+H) = 368, observed (M+H) = 368; HPLC: 5% CH₃CN/H₂O (0.1%TFA) to 90% CH₃CN/H₂O (0.1%TFA)/20min. Retention time = 11.66 min.

### B. Cloning. Overexpression, and Purificaton of wild type and mutant peptides of ERAB or HADH2.

The coding sequence for full length wild-type ERAB or HADH2 [SEQ ID NO: 7, Yan et al., (1997), *supra*; He et al., (1998), *supra*] was amplified by PCR [Mullis et al., *Cold Spring Harbor Symposium Quantum Biology*, Vol. 51 pp. 263-273 (1986); Saiki et al., *Science,* Vol. 239, pp. 487-491 (1988)] from a Marathon-Ready human lung cDNA library and the Advantage™ cDNA PCR kit, both from Clontech (Palo Alto, CA), using the manufacturer's instructions. The forward primer was
5'-GGGCACACCATGGCAGCAGCGTGTCGGAGCGTGAAGG-3' (SEQ ID NO: 9) and the reverse primer was 5'-AGCTTTCGGCCGTTAAGGCTGCATACGAATAGCCCCATCC-3' (SEQ ID NO: 10).

The amplified DNA was digested with the restriction enzymes *Nco*I and *Eag*I, ligated into the *E. coli* plasmid pMGH4 [Kan et al., *Journal of Protein Chemistry,* Vol. 11, pp. 467-473 (1992); Schoner et al., *Proceedings of National Academy of Science, USA*, Vol. 83, pp. 8506-8510 (1986)], sequence verified, and compared to the sequences in GENBANK Accession numbers AF035555 and U96132.

Mutations were introduced by oligonucleotide site-directed mutagenesis [Kunkel, *Proceedings of National Academy of Science*, *USA*, Vol. 82, pp. 488-492 (1985)] using the Muta-Gene *in vitro* Mutagenesis Kit (obtained from Bio-rad, Hercules, CA). The ssDNA uracil template (minus strand) was created in *E. coli* strain CJ236 supplied in the kit using the pMGH4-ERAB or HADH2 construct. All DNA modification and restriction enzymes were purchased from New England Biolabs and oligonucleotides were purchased from Genosys Biotechnology.

The following oligonucleotides were used for mutagenesis:

C5V 5'-CCATGGCAGCAGCTGTTCGGAGCGTGAAGG-3' (SEQ ID NO: 11)

C58V 5'-GAAGTTAGGAAACAACGTTGTTTCGCCCCAGCC-3' (SEQ ID NO: 12)

C214R 5'-CCAGAGAAAGTGCGTAACTTCTTAGCCAGCCAAG-3' (SEQ ID NO: 13)

C124A 5'-CCAGAGAAAGTAGCAAACTTCCTCGCCAGCCAAG-3' (SEQ ID NO: 21)

C214S 5'-CCAGAGAAAGTGTCTAACTTCTTAGCCAGCCAAG-3' (SEQ ID NO: 24)

All mutants were sequence-verified through the region of the mutations and tested for expression in a spontaneous mutant of *E. coli* strain Bl21(DE3) (obtained from Novagen, Madison, WI) [See Miroux et al., *Journal of Molecular Biology*, Vol. 260, pp. 289-298 (1996)].

Wild-type and mutant proteins were purified in the same manner. The final purification protocol was modified from published reports [Furuta et al., *Biochemistry Biophysics Acta*, Vol. 1350, pp. 317-324 (1997)]. All procedures were carried out at 4 °C. Cell paste was resuspended in half volume 50 mM Tris-HCl pH 7.5, 1 mM EDTA, 10 mM β-mercaptoethanol (BME). The cells were disrupted by micro-fluidization. Cleared supernatant was passed through a Q-Sepharose Fast Flow column. Unbound enzyme was collected in the flow through. This was loaded directly onto a Blue Sepharose Fast Flow column equilibrated in 20 mM Tris - HCl pH 7.5, 10 mM BME, 0.01% NaN₃. ERAB or HADH2 was eluted with a salt gradient increasing to 1 M NaCI over 10 column volumes. Peak fractions were concentrated for size exclusion. The pool was fractionated on a 500 ml Superdex 200 column equilibrated in 20 mM Tris - HCI pH 7.5, 200 mM NaCl, 5 - 10 mM BME, 0.01 % NaN₃. The peak eluted as a tetramer. The peak fractions were concentrated to 20 - 25 mg/ml and stored in aliquots at - 20 °C. All chromatography media were from Pharmacia Biotech.

### C. Isothermal Titration Calorimetry

Titrations described herein were performed with an MCS microcalorimeter (obtained from MicroCal, Inc., Northampton, MS).

For the inhibitor binding experiments, the titrations were performed in 25 mM MOPS (pH 7.5), 125 mM NaCI, 10% glycerol, 2.0% DMSO, 0.5 mM TCEP (Tri(2-carboxyethyl)phosphine), 0.1 mM EDTA at 15°C. The syringe contained 415 µM wild-type ERAB OR HADH2 and the cell contained either 20 µM inhibitor, 20 µM cofactor, or 20 µM each of inhibitor and cofactor. After a preliminary 20 µL injection, 12 injections of 10.0 µL each were made at 4 minute intervals. Dilution control titrations of buffer into buffer, protein into buffer, and buffer into inhibitor, cofactor and inhibitor/cofactor mixture were also performed. For the cofactor binding experiments, the titrations were performed in 50 mM MOPS (3-(N-morpholino) propancsulfonic acid, pH 7.5), 250 mM NaCl, 10% glycerol, 2.0% DMSO, 10 mM TCEP at 15°C.

For the NADH titration, the syringe contained 700 µM cofactor and the cell contained 35 µM wild type ERAB or HADH2. For the NAD⁺ titration, the syringe contained 2.68 mM cofactor and the cell contained 127 µM wild-type ERAB or HADH2.

The IC₅₀ value for Compound I was determined spectrophotometrically by monitoring the reduction of NADH to NAD⁴ with acetoacetyl-CoA as substrate. Enzyme (25 nM) was preincubated with inhibitor for 400 seconds at 30°C in the presence of 36 uM NADH, 25 mM MOPS, pH 7.5, 250 mM NaCl, 2%(v/v) DMSO, and 2.5 mM TCEP. The reaction was initiated by addition of substrate (38 µM). Reduction of NADH was monitored at 340 λ. The IC₅₀ value was determined by non-linear regression analysis using KaleidaGraph (obtained from Synergy Software, Reading. PA).

Compound I made according to Scheme 2 was found to have an average IC₅₀ of 92 ± 5 nM against ERAB or HADH2 (for a racemic mixture of the compound). The experimental data and co-crystal structue of the compound indicates that Compound I inhibits the ERAB or HADH2 via an interaction with NAD⁺, which is the natural co-factor for ERAB or HADH2. This interaction with NAD⁺ enables Compound I to bind ERAB or HADH2, approximately 1000-fold more potently than in the absence of NAD⁺. Further, the tertiary structure analysis of the crystallized ERAB or HADH2:inhibitor complex shows that the inhibitor binds in the active site cavity of the enzyme and reacts with the NAD⁺ cofactor to form a covalent adduct (Figure 4C). The conformation and binding interactions of the inhibitor appear identical in the three ERAB or HADH2 monomers to which it binds in the crystal.

Referring to Figures 4A and 4C, the N2 nitrogen of the inhibitor is covalently linked to the reactive C4 carbon on the nicotinamide ring of the NAD⁺. Adduct formation does not alter the conformation of the bound NAD⁺ cofactor beyond movement of the C4 atom out of the plane of the nicotinamide ring. Consistent with the observed covalent binding, experiments using isothermal titration calorimetry demonstrate that the inhibitor binds weakly to ERAB or HADH2 in the absence of cofactor or in the presence of NADH (Fig. 5). No binding was detected when ERAB or HADH2 was titrated into either inhibitor or NAD⁺ alone. A reaction was detected, however, when ERAB or HADH2 was titrated into an equimolar mixture of inhibitor and NAD⁺. The data display the characteristics of a tight-binding curve involving one mole of ERAB or HADH2 per mole of inhibitor/NAD⁺ mixture. The results obtained when ERAB or HADH2 was titrated into an equimolar mixture of inhibitor and NADH, on the other hand, are comparable to the results obtained when the protein was titratod into NADH alone. Similar results were observed using the C214R mutant of ERAB or HADH2. This observed reaction cannot simply be attributed to NAD⁺ binding to ERAB or HADH2, as the Kd for NAD⁺ was independently determined to be on the order of 500 uM. Therefore, a high cooperative binding of NAD⁺ and inhibitor must account for the observed reaction. No significant cooperativity was observed for NADH binding in the presence of inhibitor. The formation of a covalent adduct apparently occurs because the inhibitor closely mimics the reaction stereochemistry of the substrate. A proposed mechanism for the reaction of Compound I with NAD⁺ is shown in Figure 4C.

Despite a lack of structural similarity between Compound I and either of the ERAB or HADH2 substrates (wild-type or mutant ), the inhibitor is in intimate contact with the protein within the substrate-binding cleft. A schematic representation of the protein-ligand interactions is shown in Figure 4D. The inhibitor contacts nine protein residues, primarily through hydrophobic interactions. Two hydrogen bonds are formed between Compound I and ERAB or HADH2, one between the OH of Tyr 168 and N1 of Compound I and the other between N_{ε} of Gln 165 and the carbonyl oxygen of Compound I. As mentioned previously, human 17β-hydroxysteroid dehydrogenase is a human protein closely related to ERAB or HADH2 whose structure has been reported (Ghosh et al., *Structure,* Vol. 3, pp. 503-513 (1995)). Likewise, 15-PGD and CAA20237.1, identified using the BLAST search engine (Altschul et al., *Nucleic Acids Research,* Vol. 25, pp. 3389-3402 (1997)), are two reported human sequences closely related to ERAB or HADH2 (see Figure 2). At most three of the nine residues that interact with the inhibitor are found in any of these three related proteins. This uniqueness suggests that a high level of specificity may be attainable for ERAB or HADH2 inhibitors. Consistent with this, Compound I showed no detectable inhibition of two closely related members of the SDR family, *E. coli* 17β-hydroxysteroid dehydrogenase and *Streptomycetes hydrogenans* 3α-20β- hydroxysteroid dchydrogenase, at concentrations as high as 100 µM.

### D. Crystallographic Analysis

The C214R mutant of ERAB or HADH2, made by the mutagenesis process described above, was engineered to avoid cysteine oxidation in the protein, with arginine selected as the replacement amino acid because of its occurrence at this position in other mammalian ERAB or HADH2 sequences (He et al., *Journal of Biochemistry* Vol. 273, pp. 10741-10746 (1998)). The C214R mutant of ERAB or HADH2 was concentrated to 20 mg/ml for crystallization. Five mM β-NAD and 5 mM Compound I were combined; and the precipitate removed by centrifugation. Initial thin plates were observed in Hampton Crystal Screen condition 40 (20% isopropanol, 20% PEG 4000, pH 5.6). The final conditions for crystal growth were 20% MME PEG 2000, 0.1 M Na citrate pH 6.0, 4% isopropanol. Nuclei were introduced to the drops by streak seeding [Stura et al., *Journal of Crystal Growth*, Vol.110, pp. 270 - 282 (1991)]. Single, thick plates grew over five days. Isopropanol, Tris, EDTA, glycerol and sodium citrate were all purchased from Fisher Scientific (Pittsburgh, PA). NaCl, β-mercaptoethanol, acetyl CoA, β-NAD⁺ and MME PEG 2000 were from Sigma. PEG 400 was from BDH Laboratory Supplies (Poole, England). Crystal Screen and VDX plates were purchased from Hampton Research (Laguna Niguel, CA).

The results from the crystallographic analysis are shown in Table 1 below. Crystal coordinates are set forth in Table II.

**Table I.**

| Data Collection, Molecular Replacement, and Refinement Statistics | |
|---|---|
| Crystal Information | |
| Space group | C2 |
| Unit cell parameters c = 110.0 Å, β = 105.6° | a = 122.0 Å, b = 80.8 Å, |
| Estimated solvent content | 50% |

| Data Collection | |
|---|---|
| Resolution | 30.0-2.0 Å |
| Total observations | 221,823 |
| Unique reflections (completeness) | 69,169 (99.4%) |
| Rsym | 0.066 |

| Molecular Replacement | |
|---|---|
| Program | EPMR |
| Data resolution range | 15.0-4.0 Å |
| Solution correlation coefficient/R-factor | 0.682/0.361 |

| Refinement | |
|---|---|
| Resolution range | 25.0-2.0 Å |
| Number of reflections | 68595 |
| Final R-factor (R-free) | 0.215 (0.263) |
| Number of non-hydrogen atoms | |
| Protein | 7224 |
| NAD | 176 |
| Compound I | 78 |
| Water | 282 |
| Rms deviations from ideal geometry | |
| Bond lengths | 0.005 Å |
| Bond angles | 1.5° |
| Residues within most favored regions of Ramachandran plot | 91.9% |

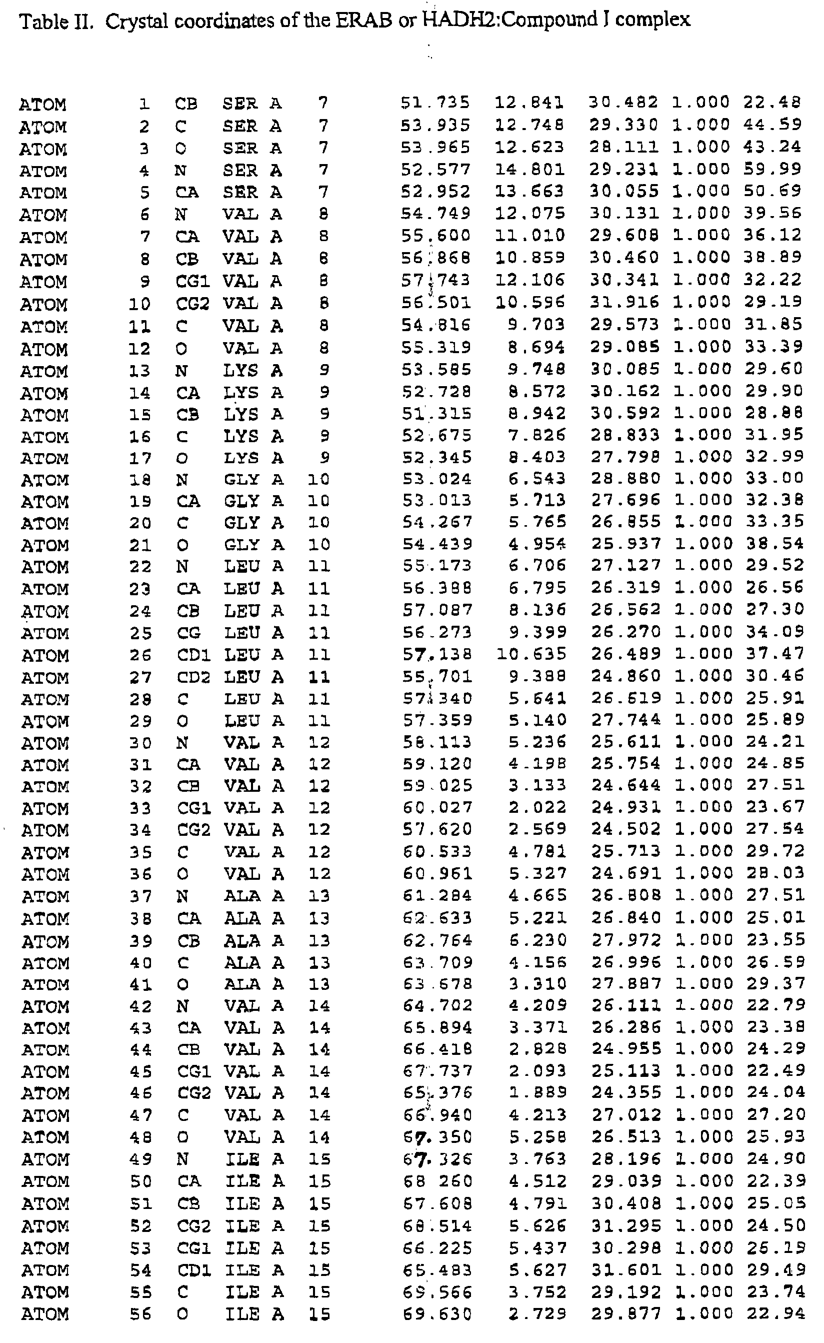

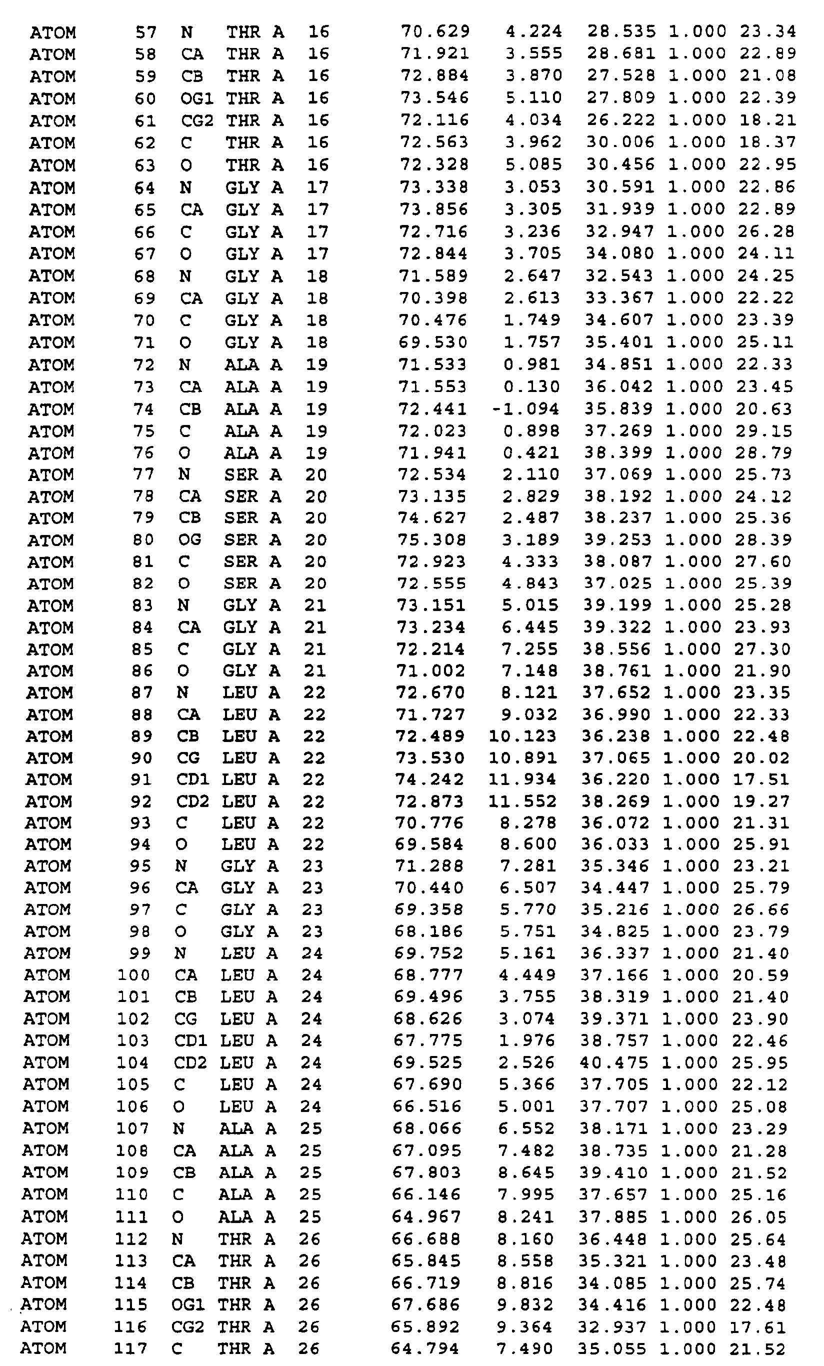

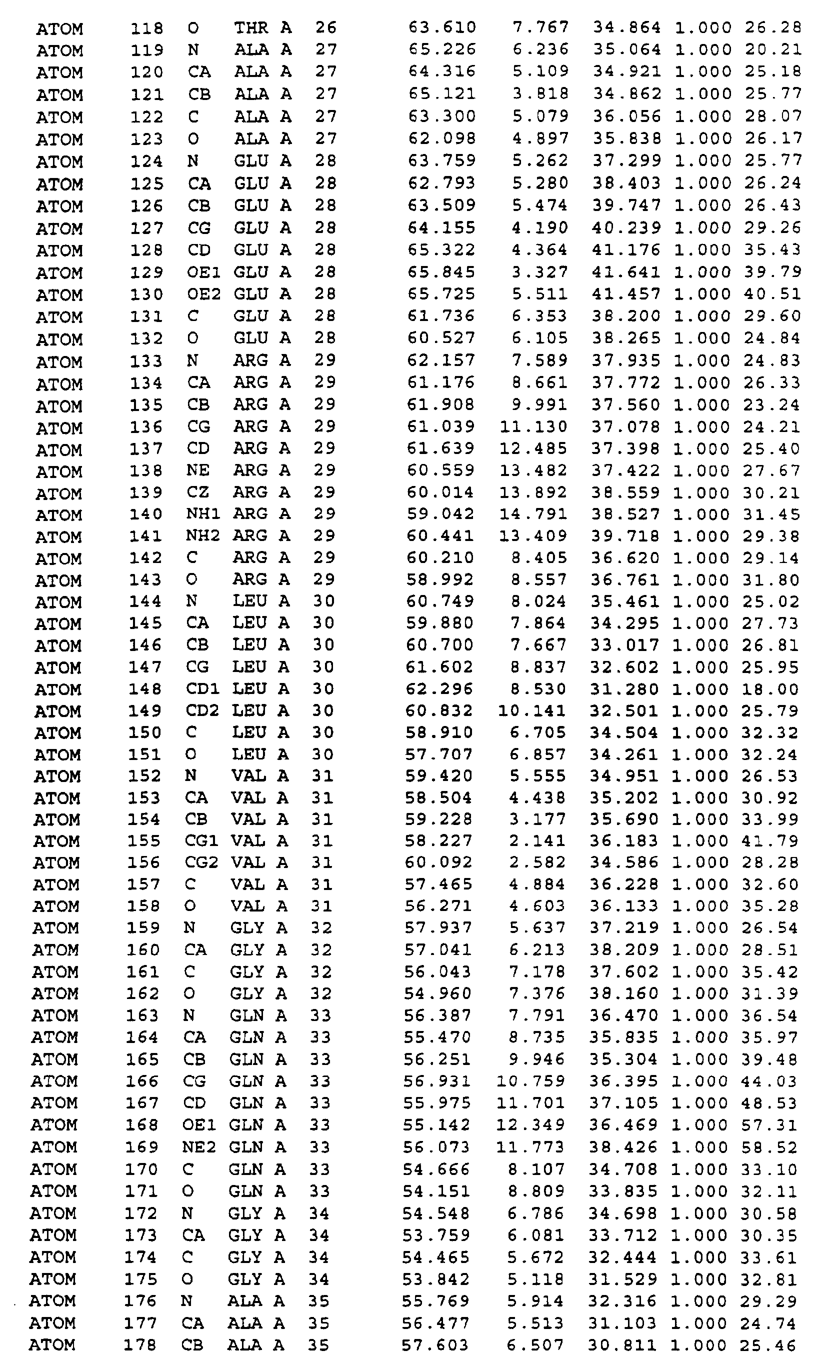

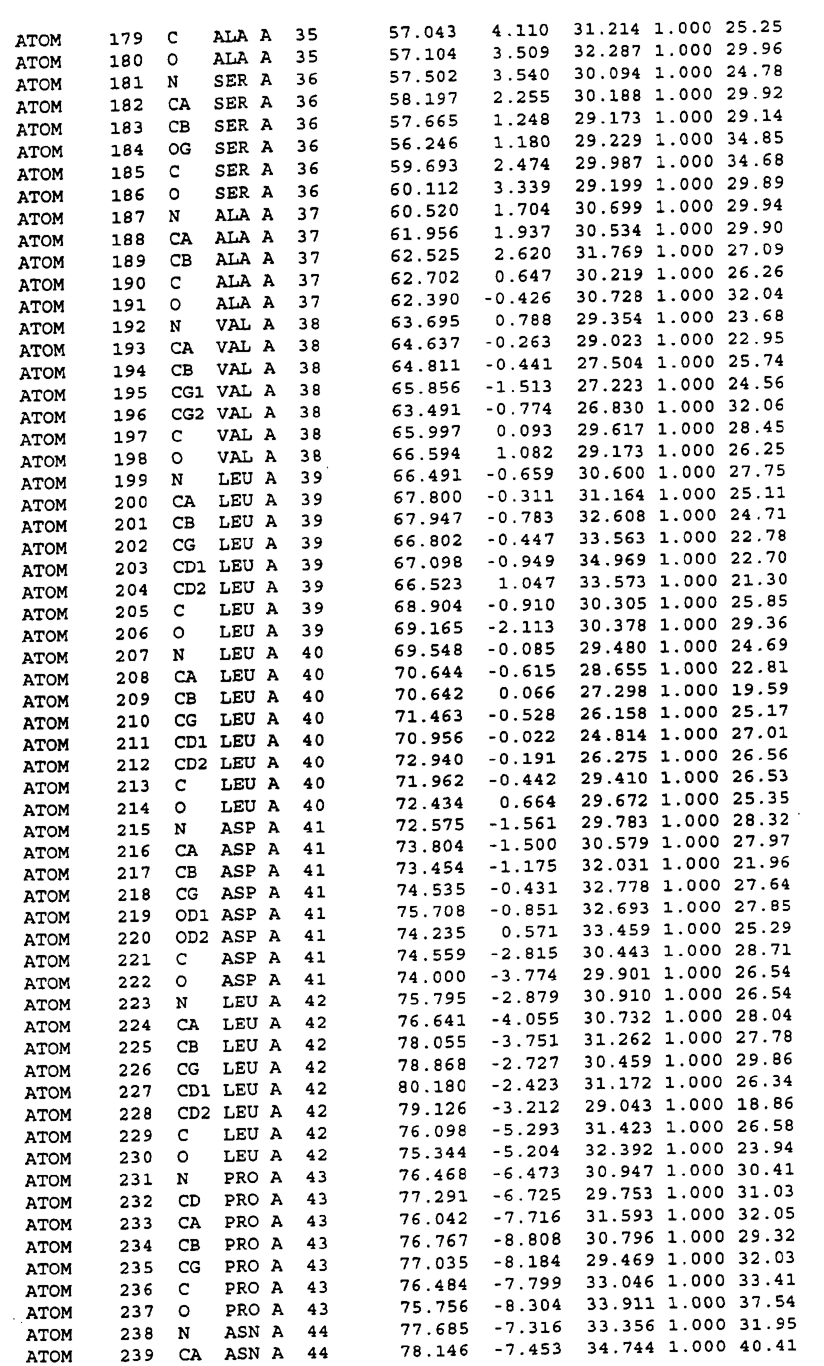

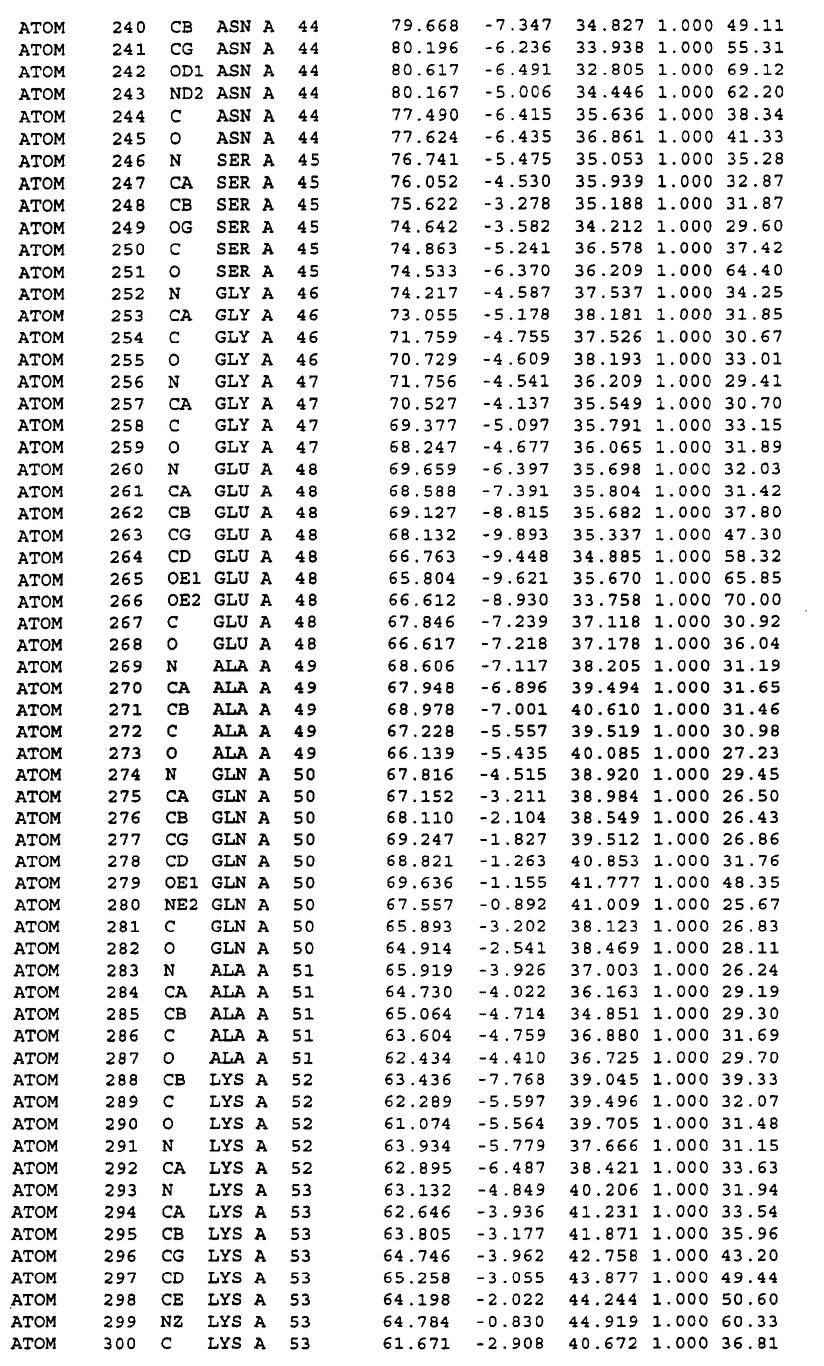

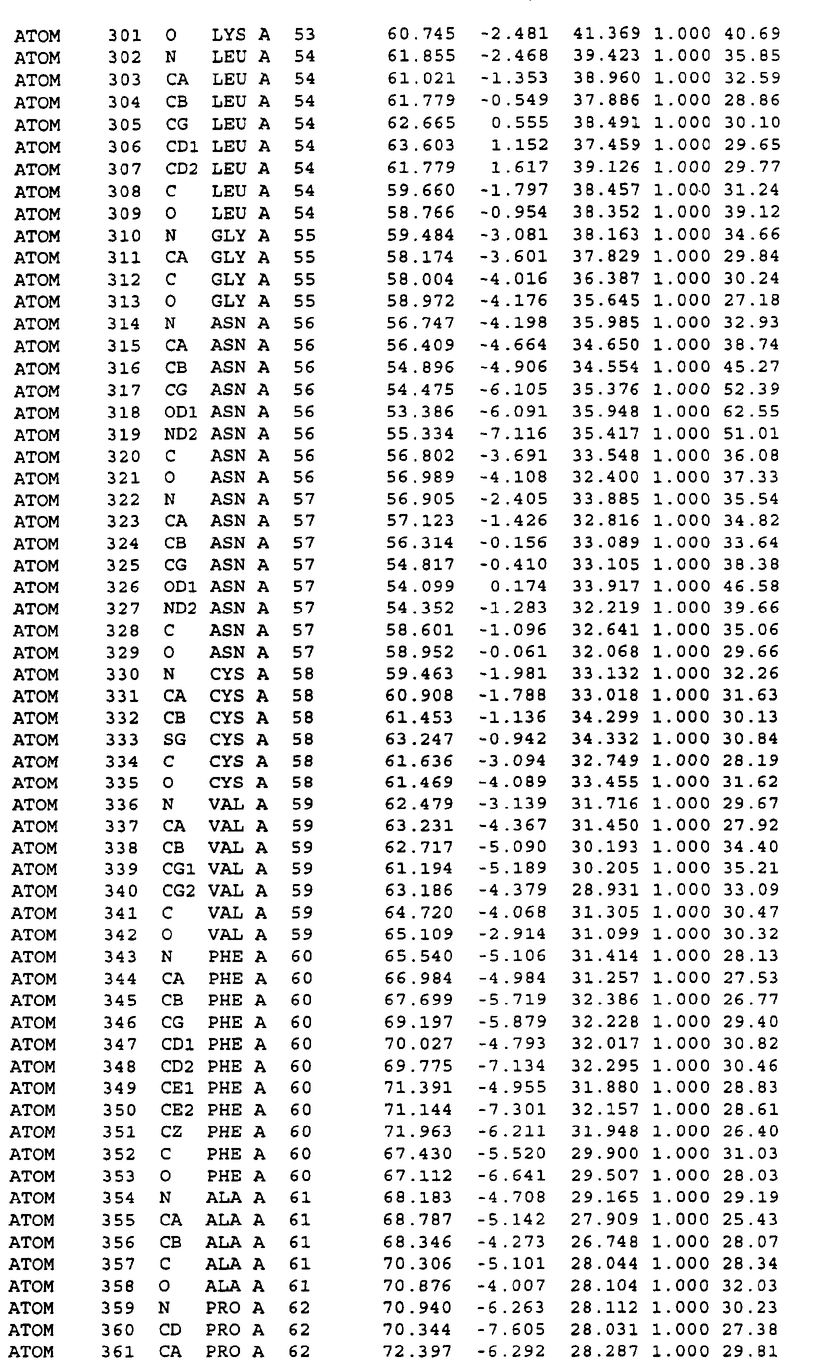

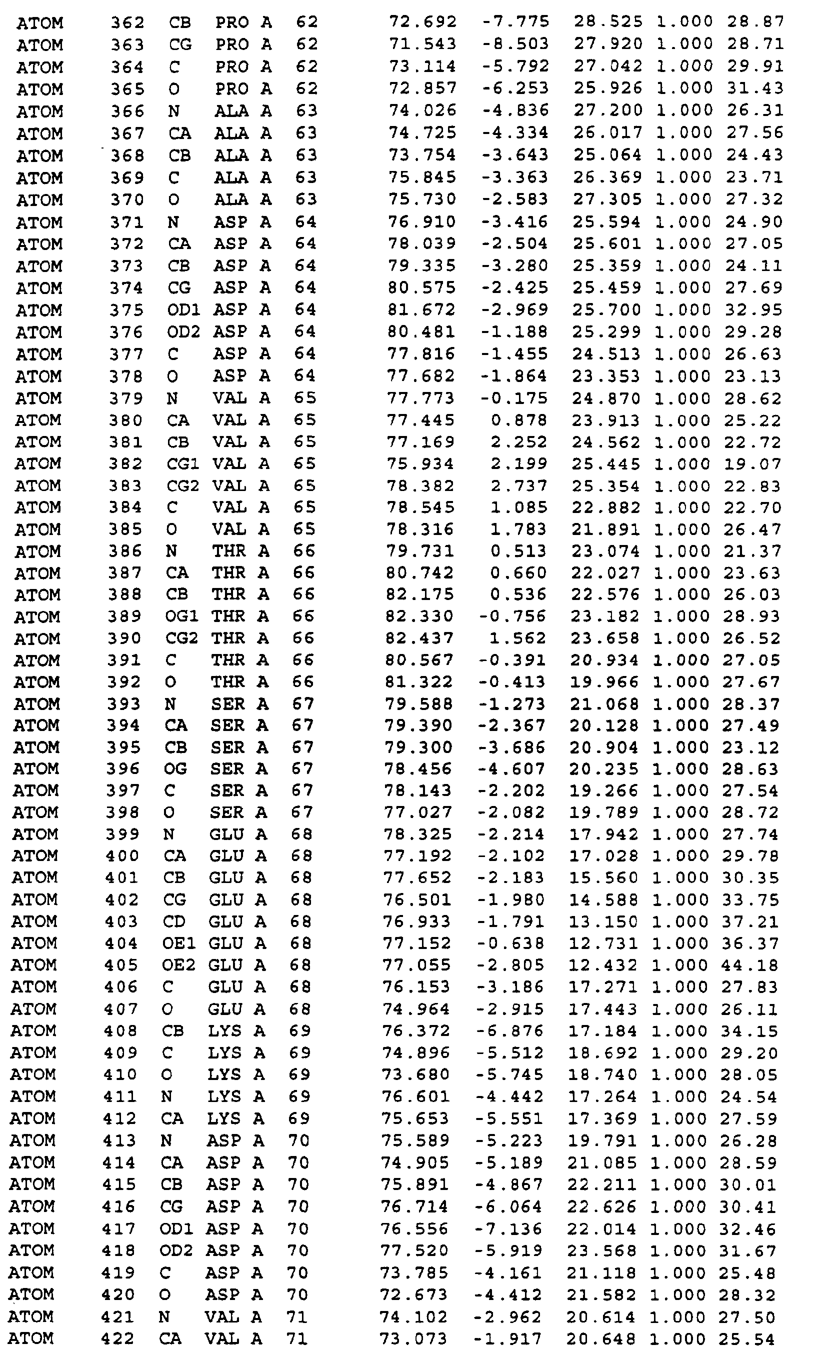

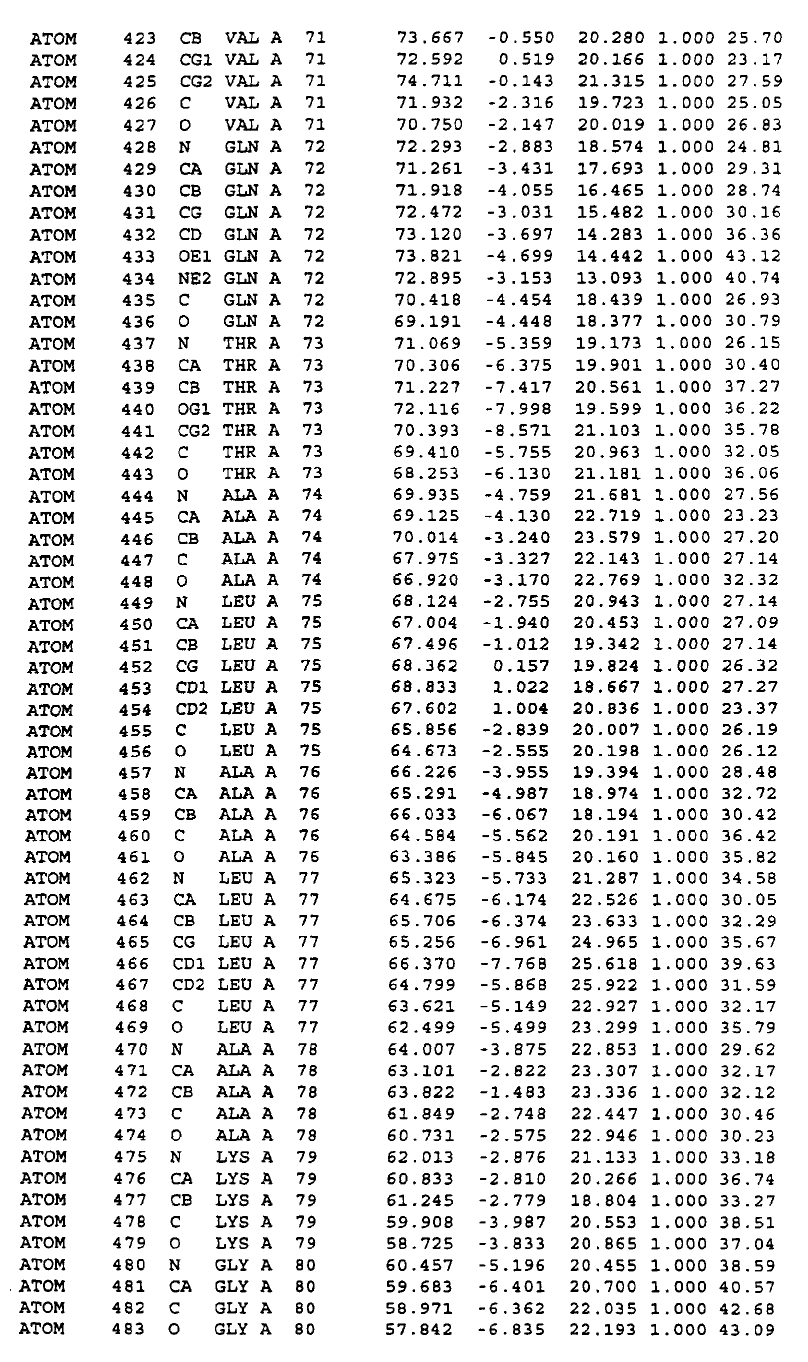

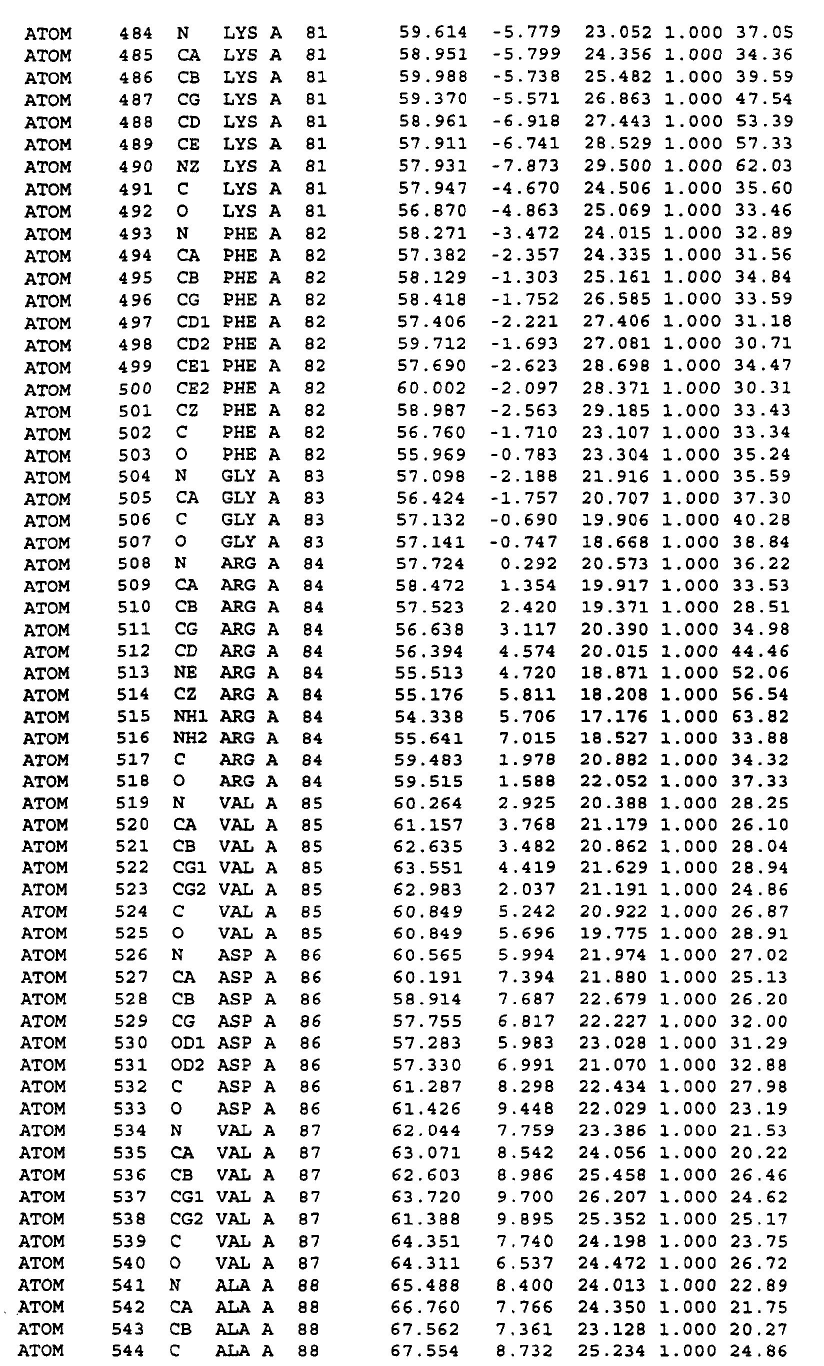

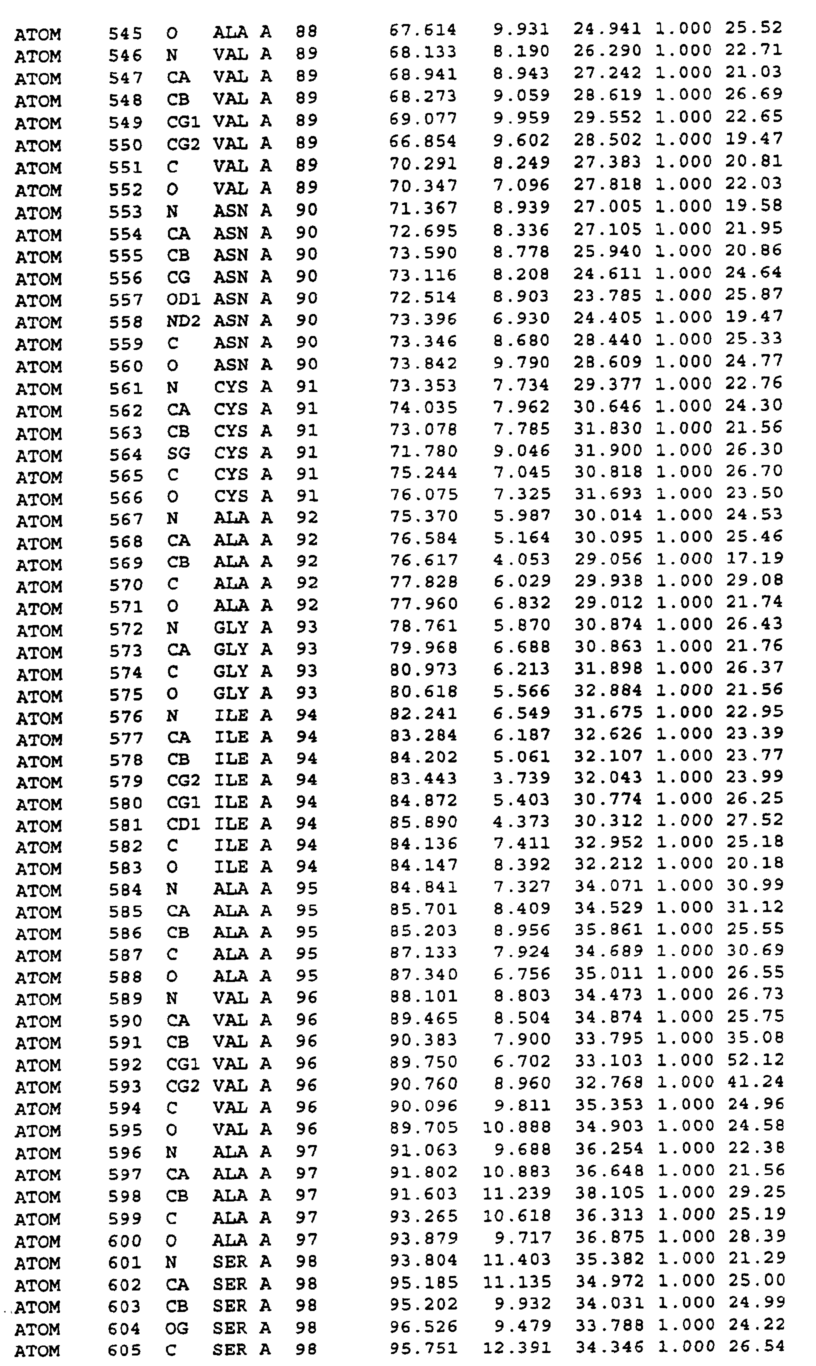

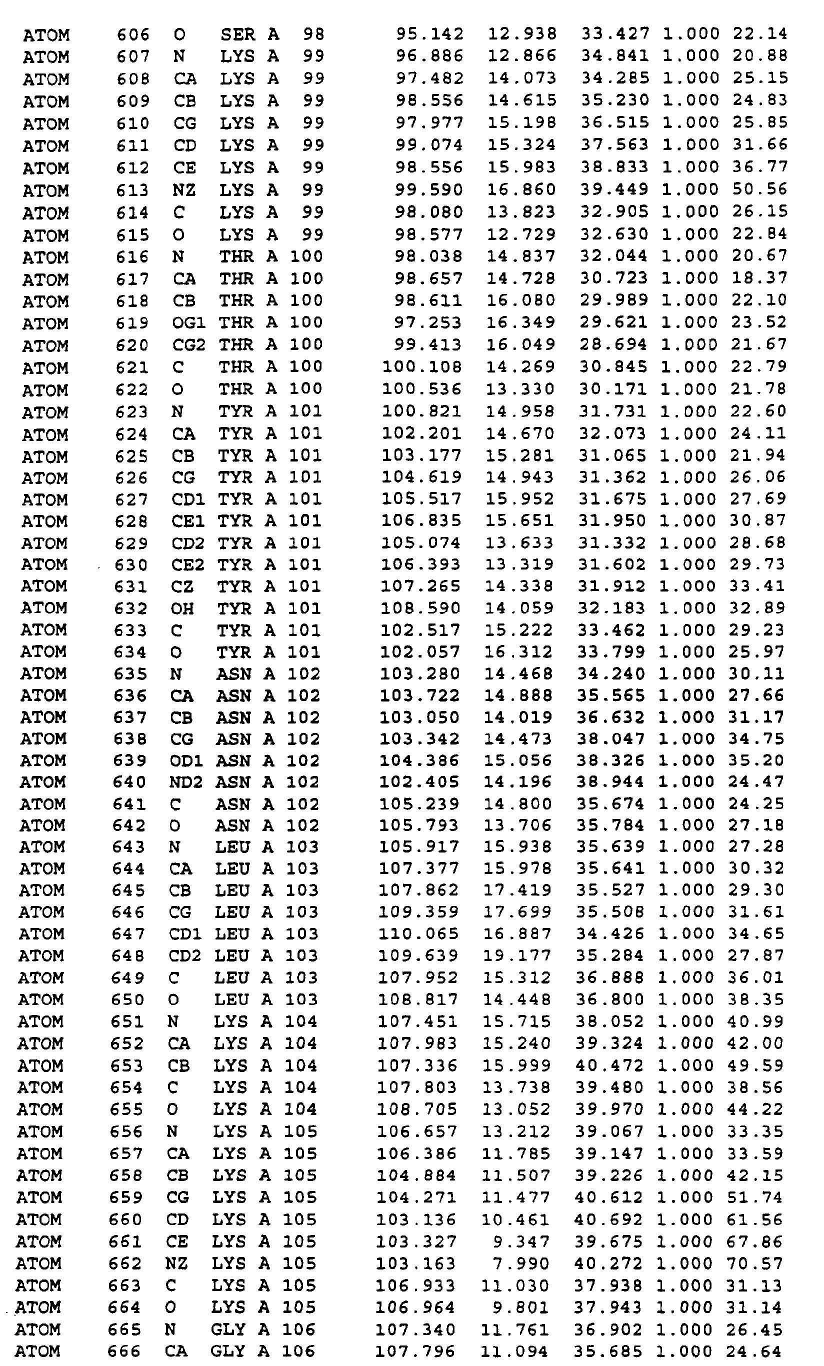

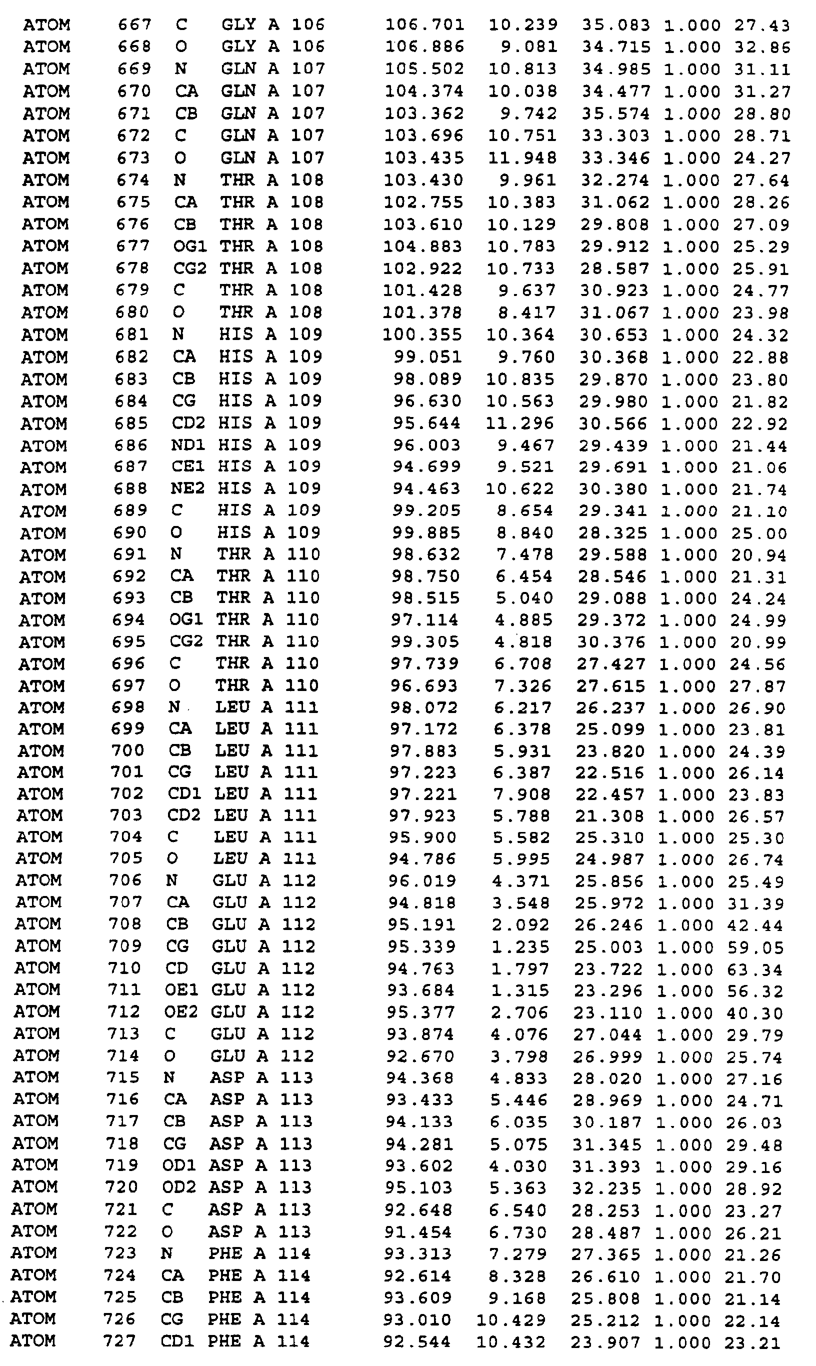

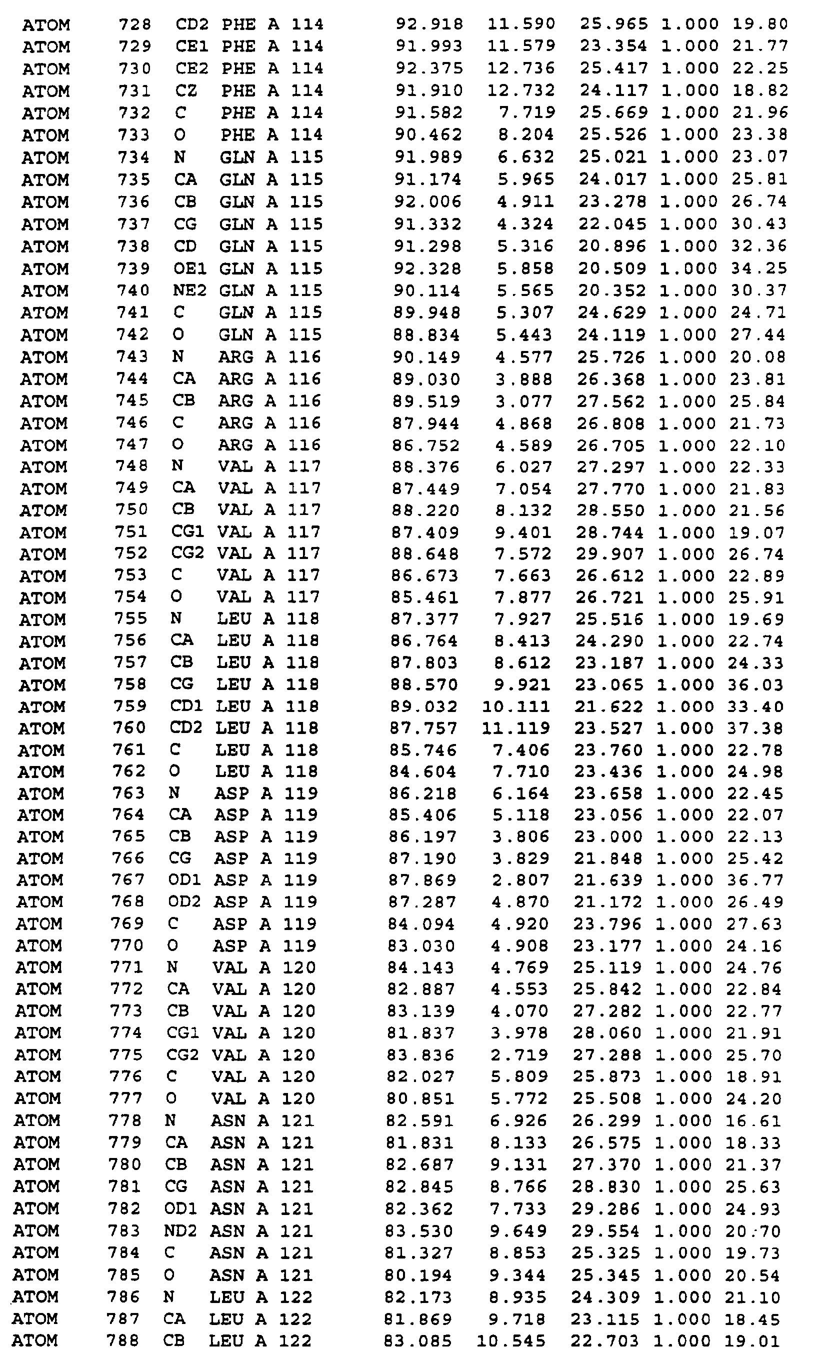

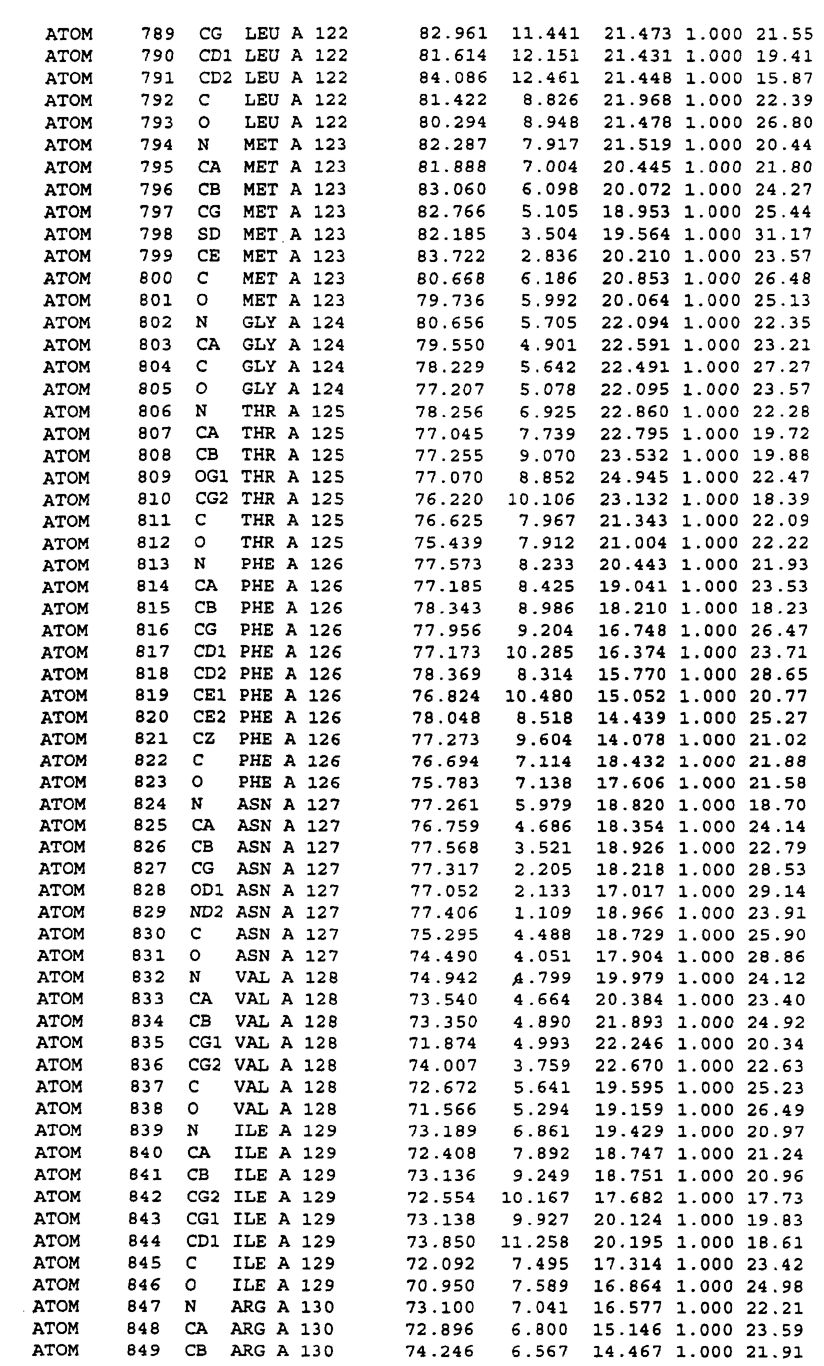

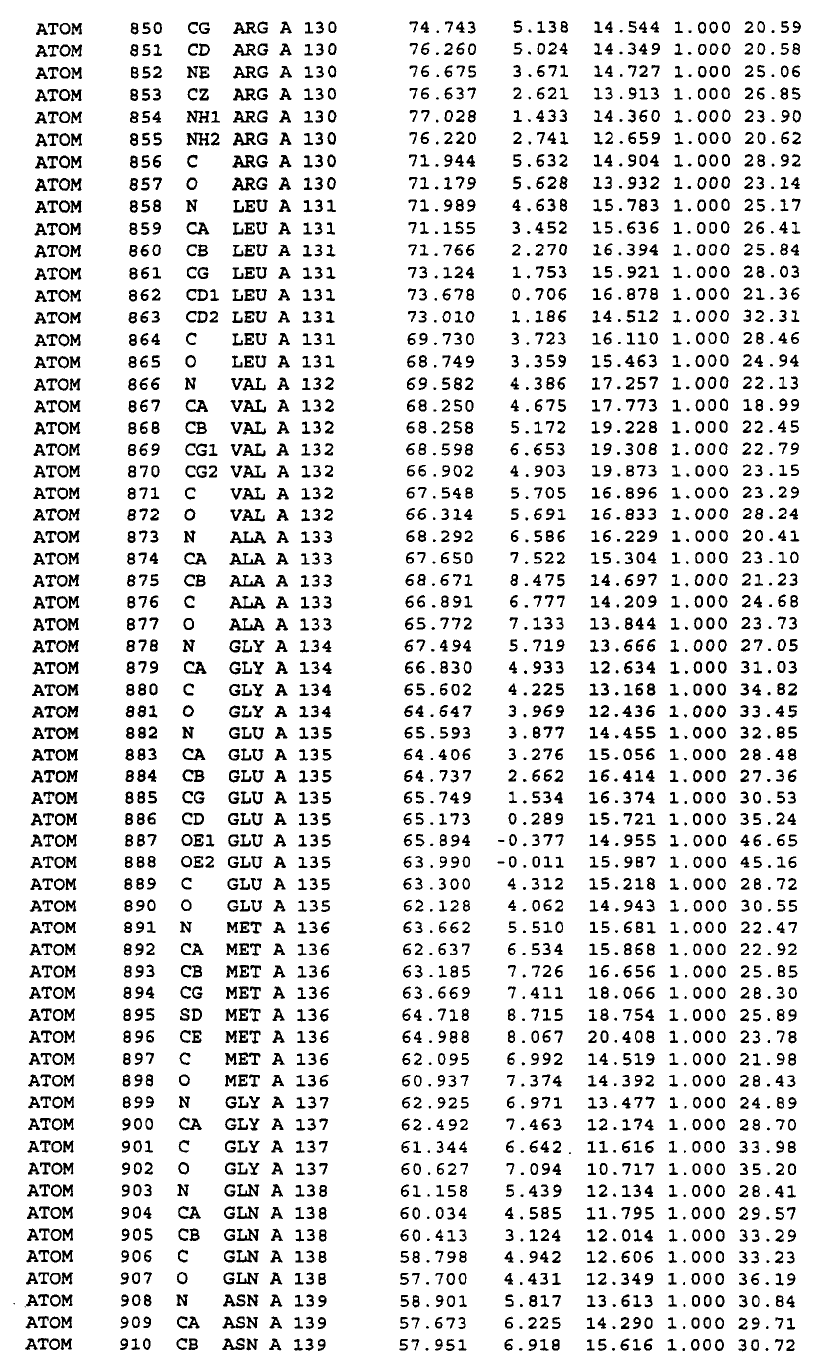

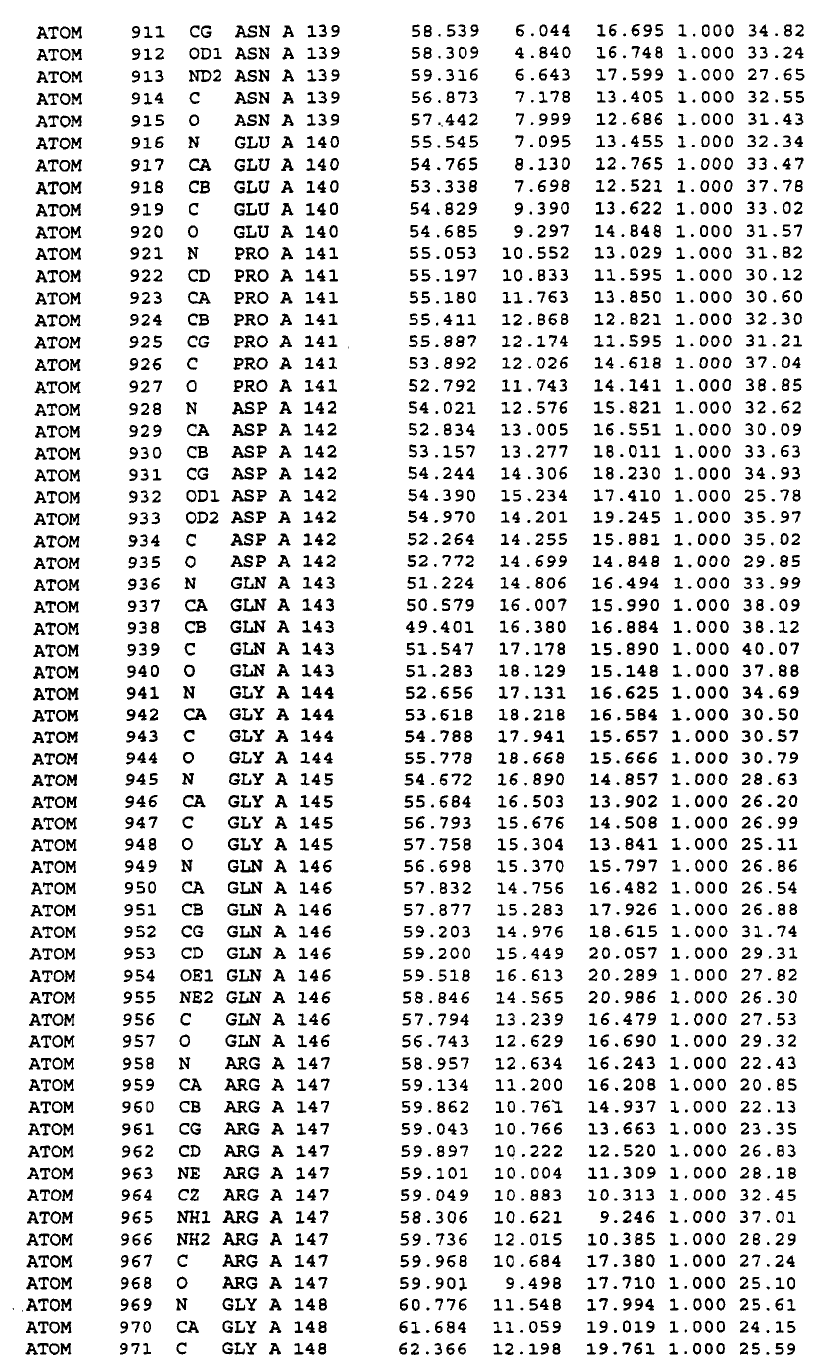

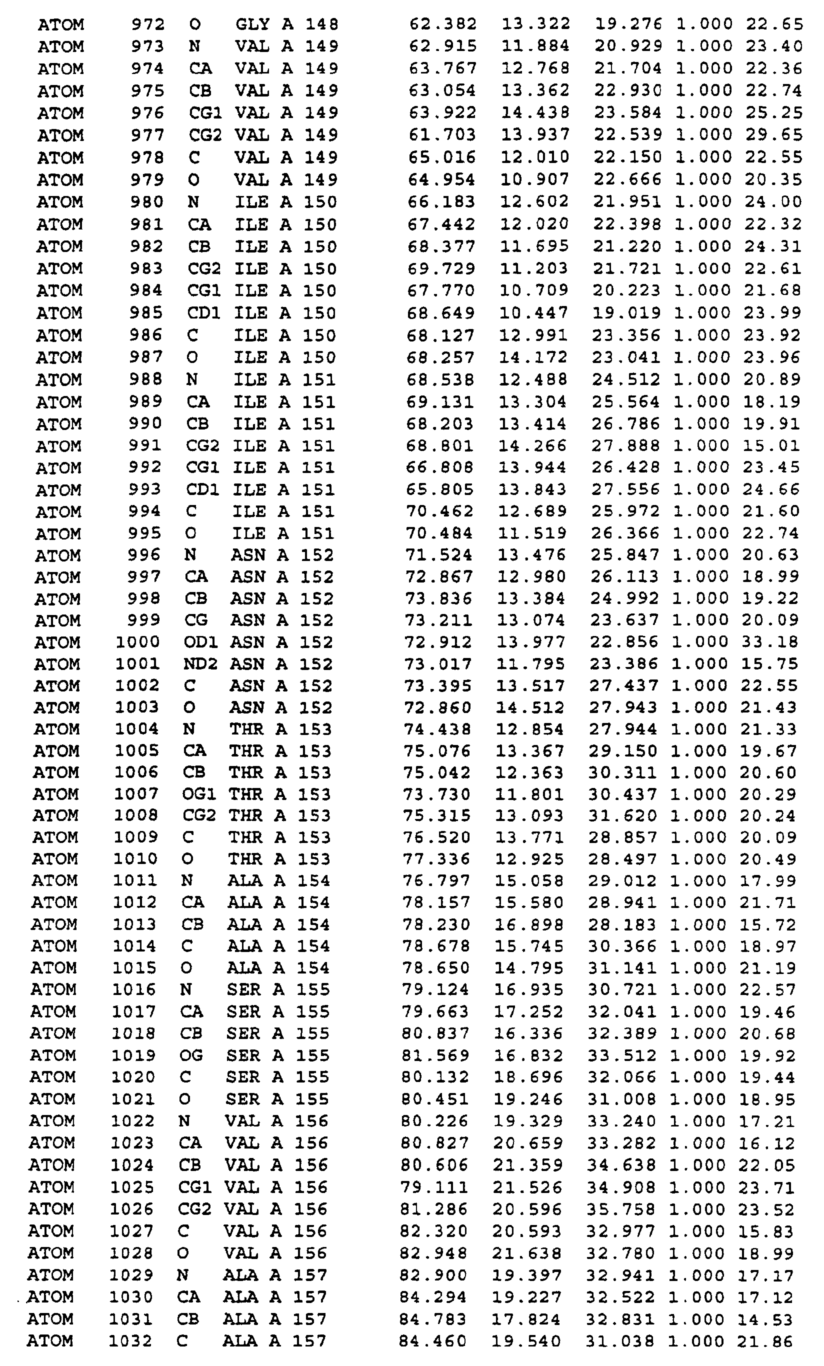

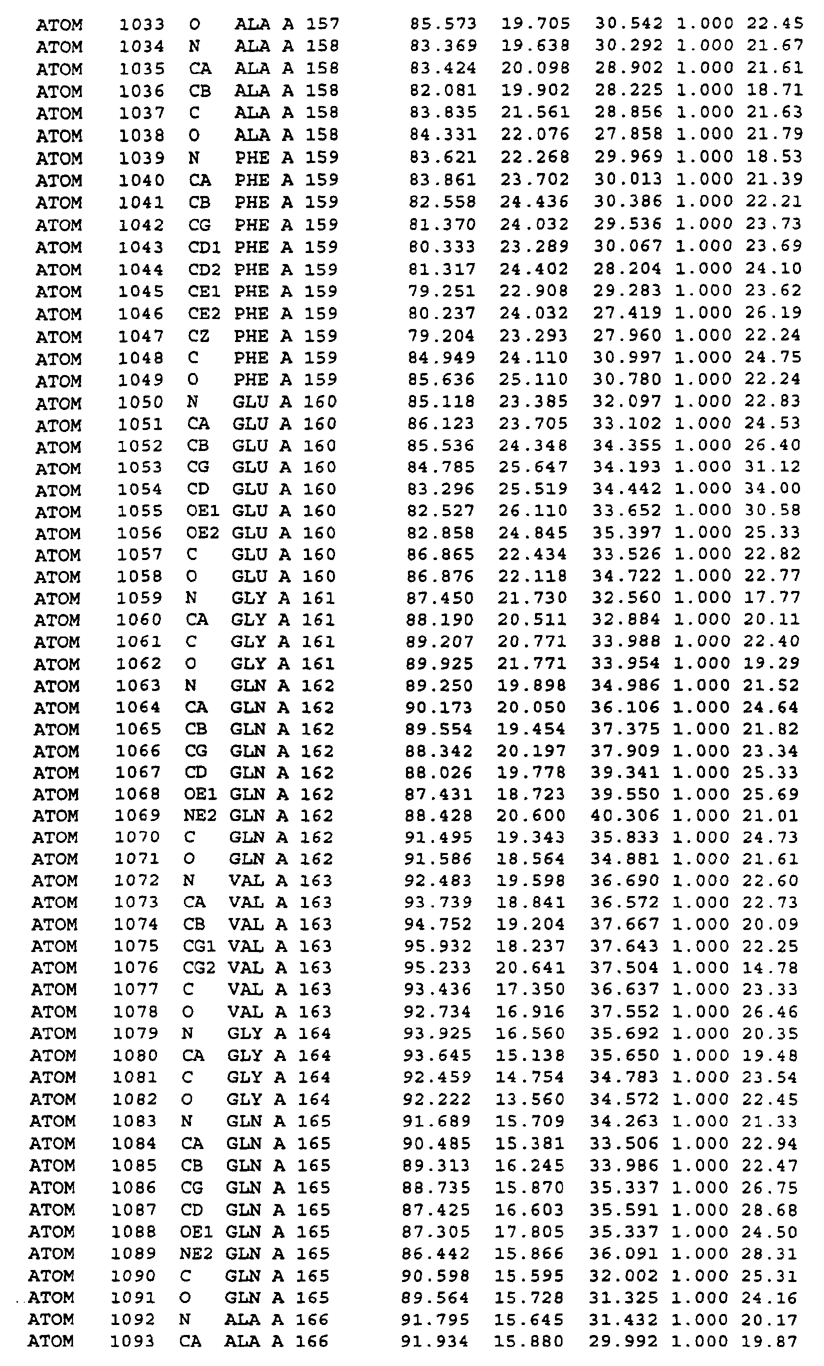

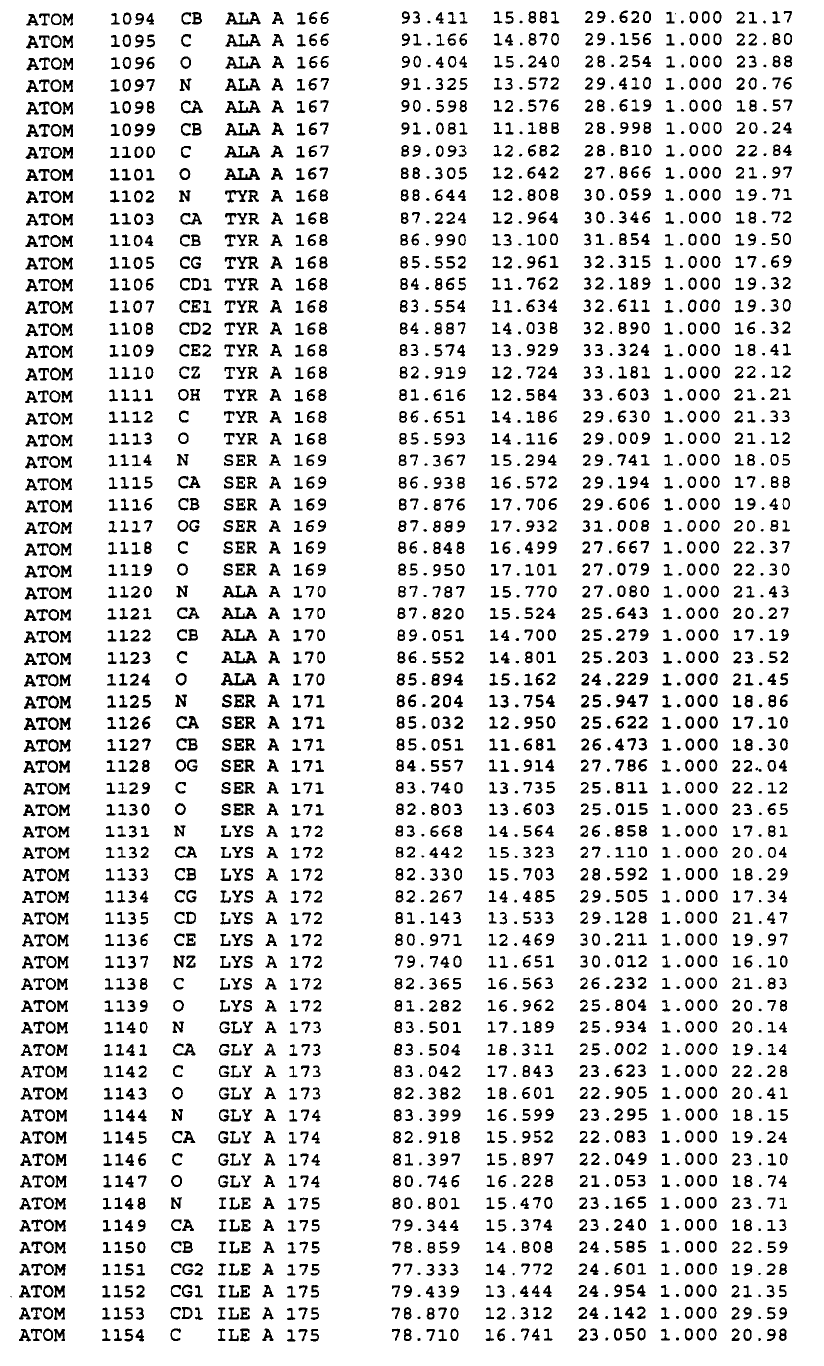

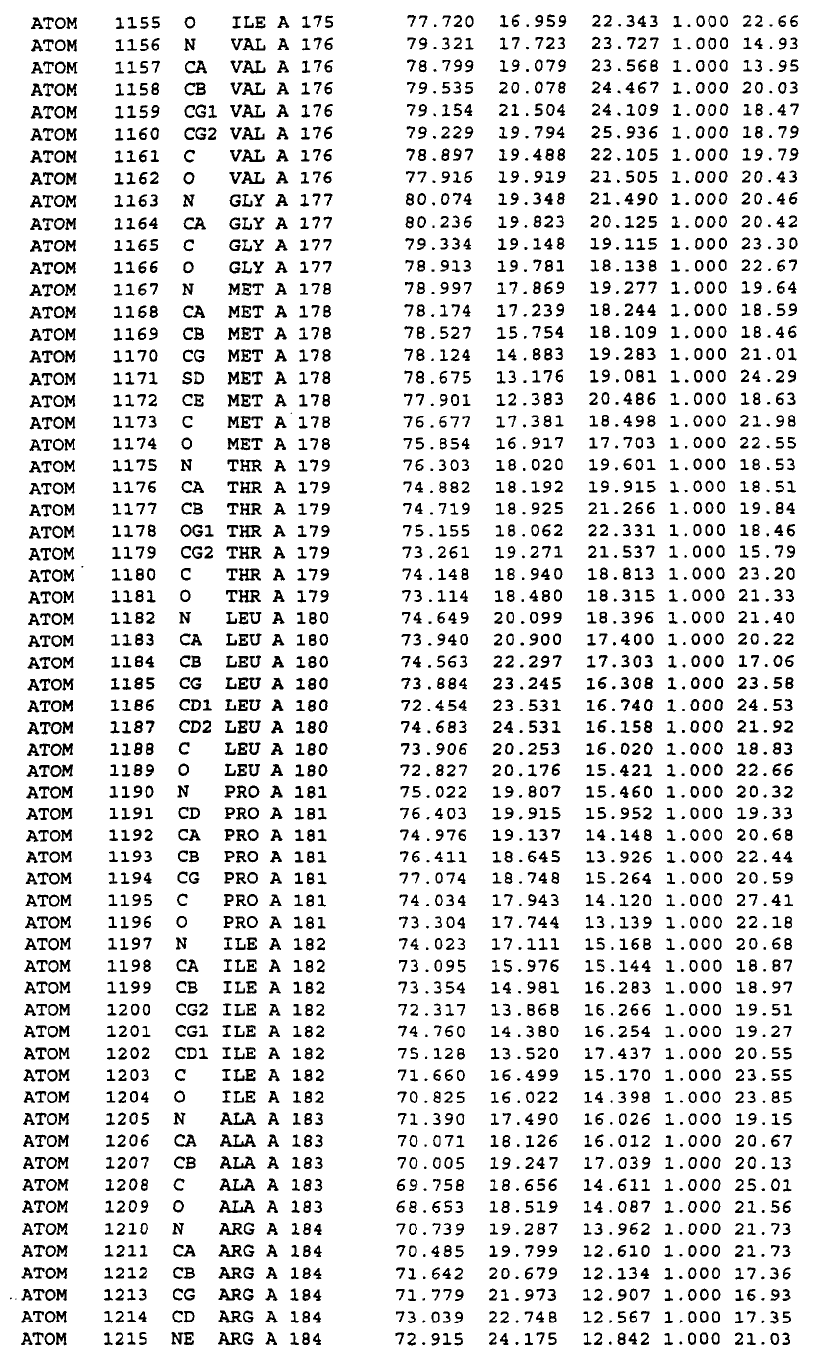

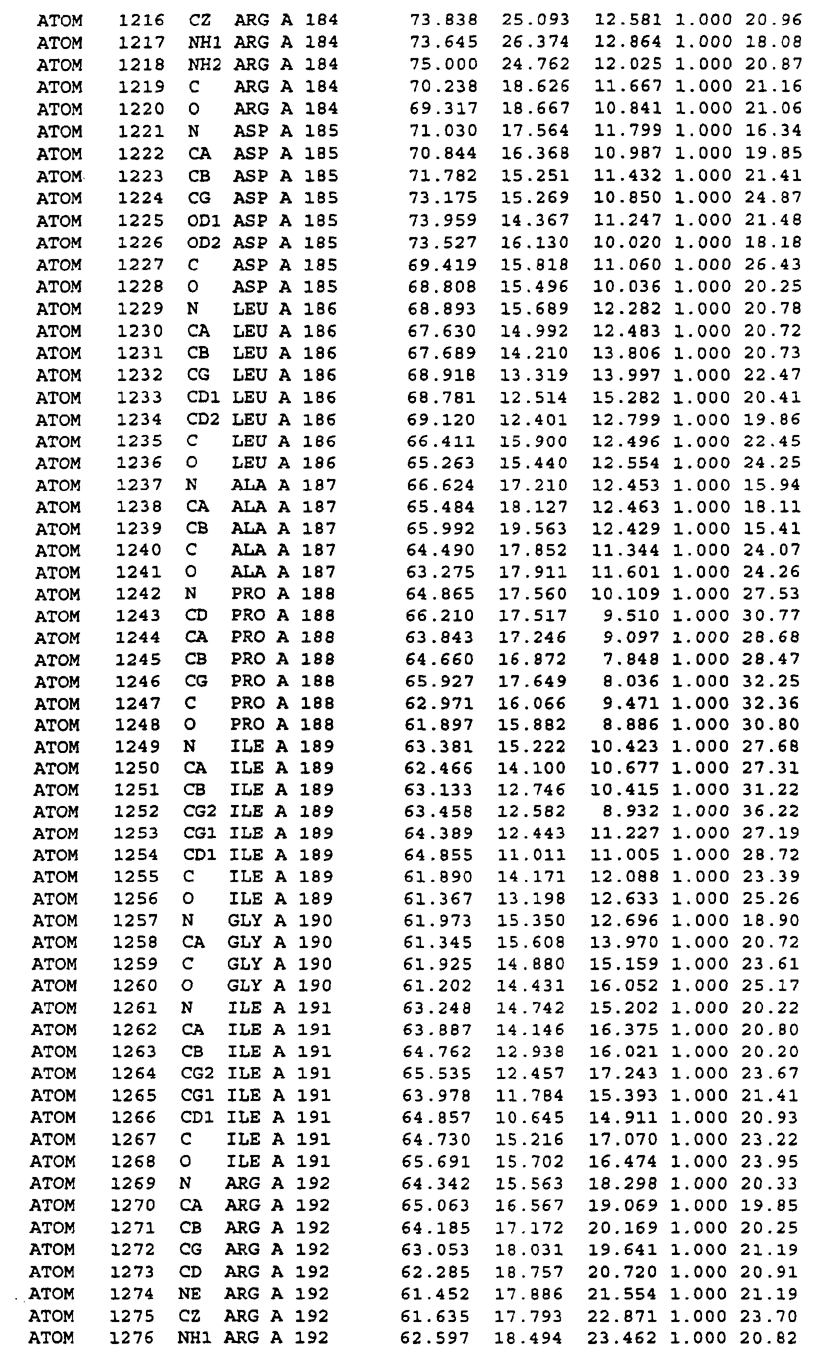

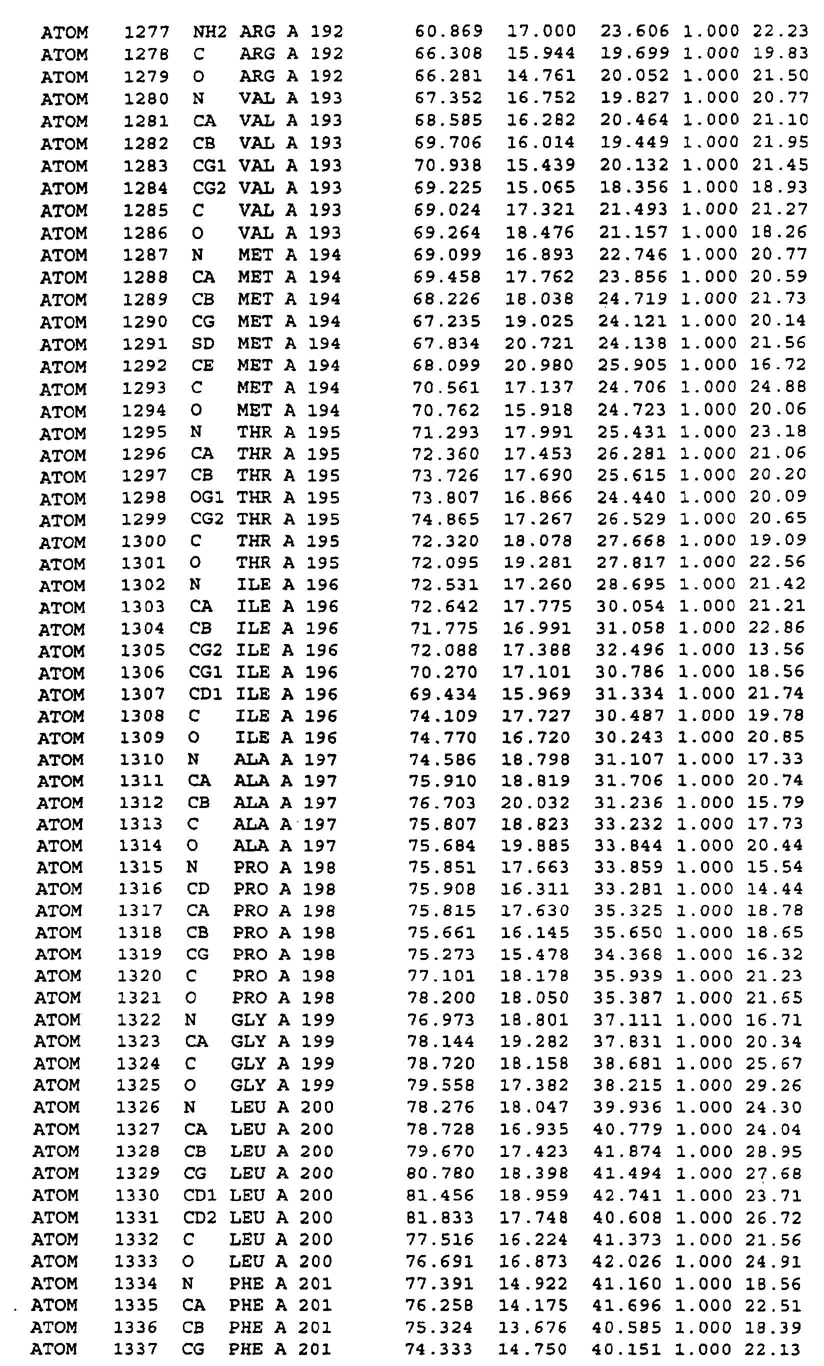

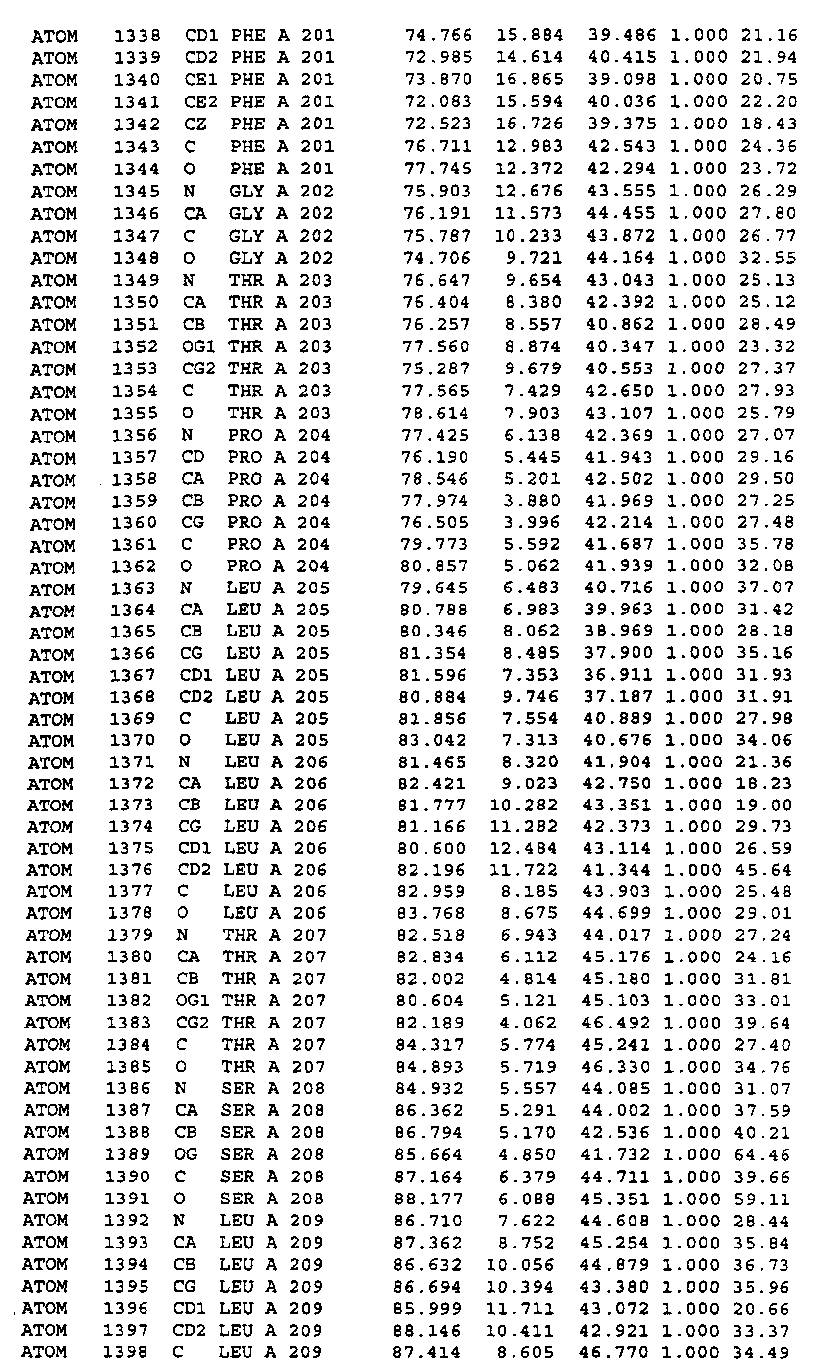

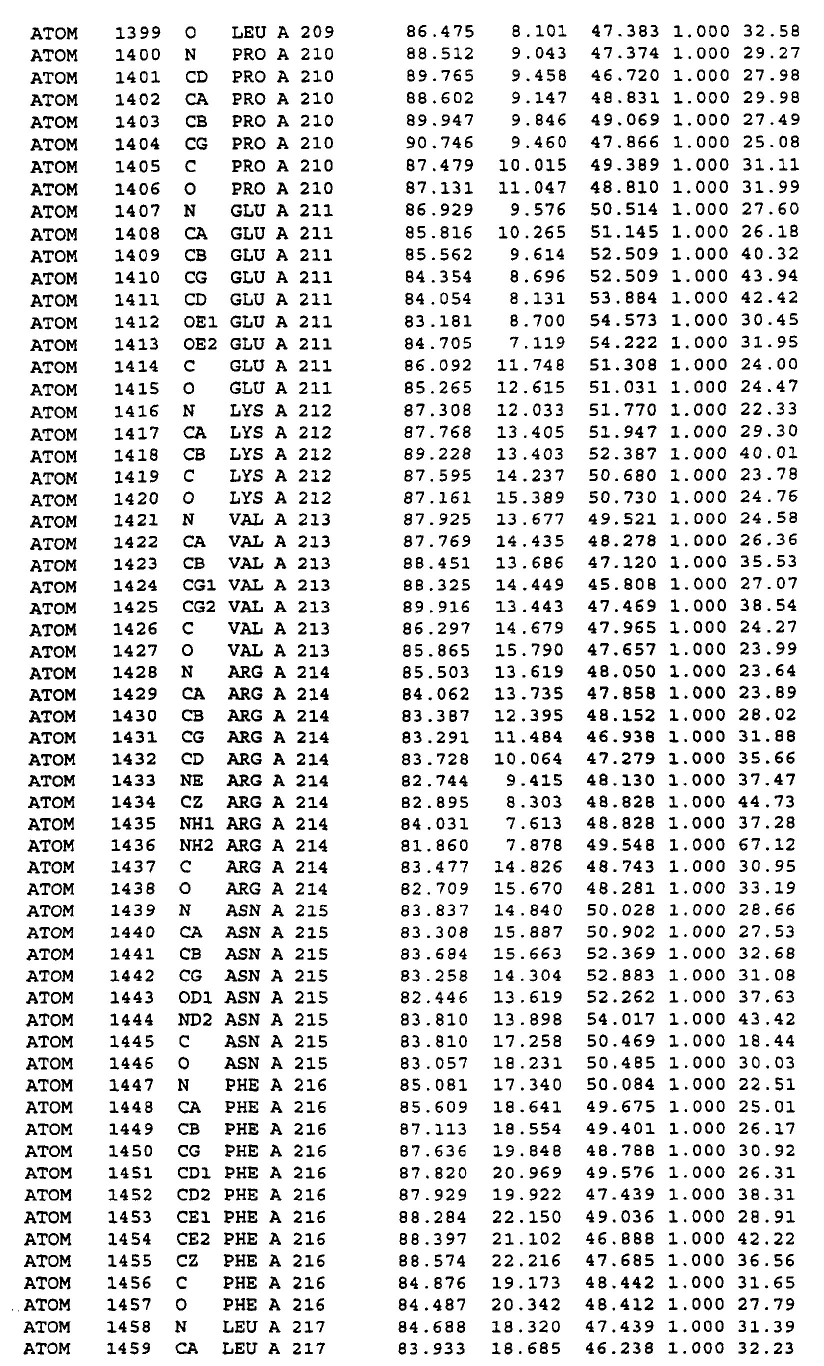

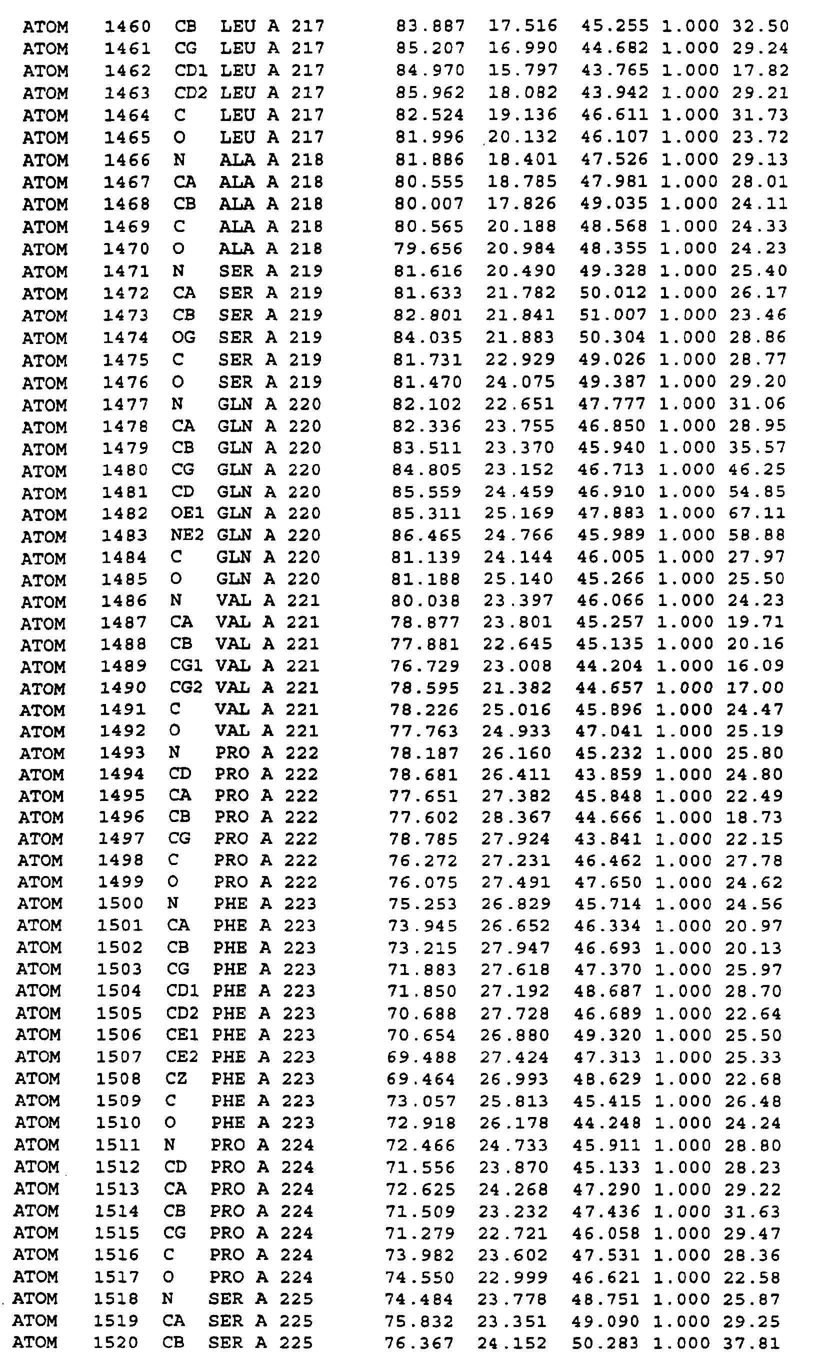

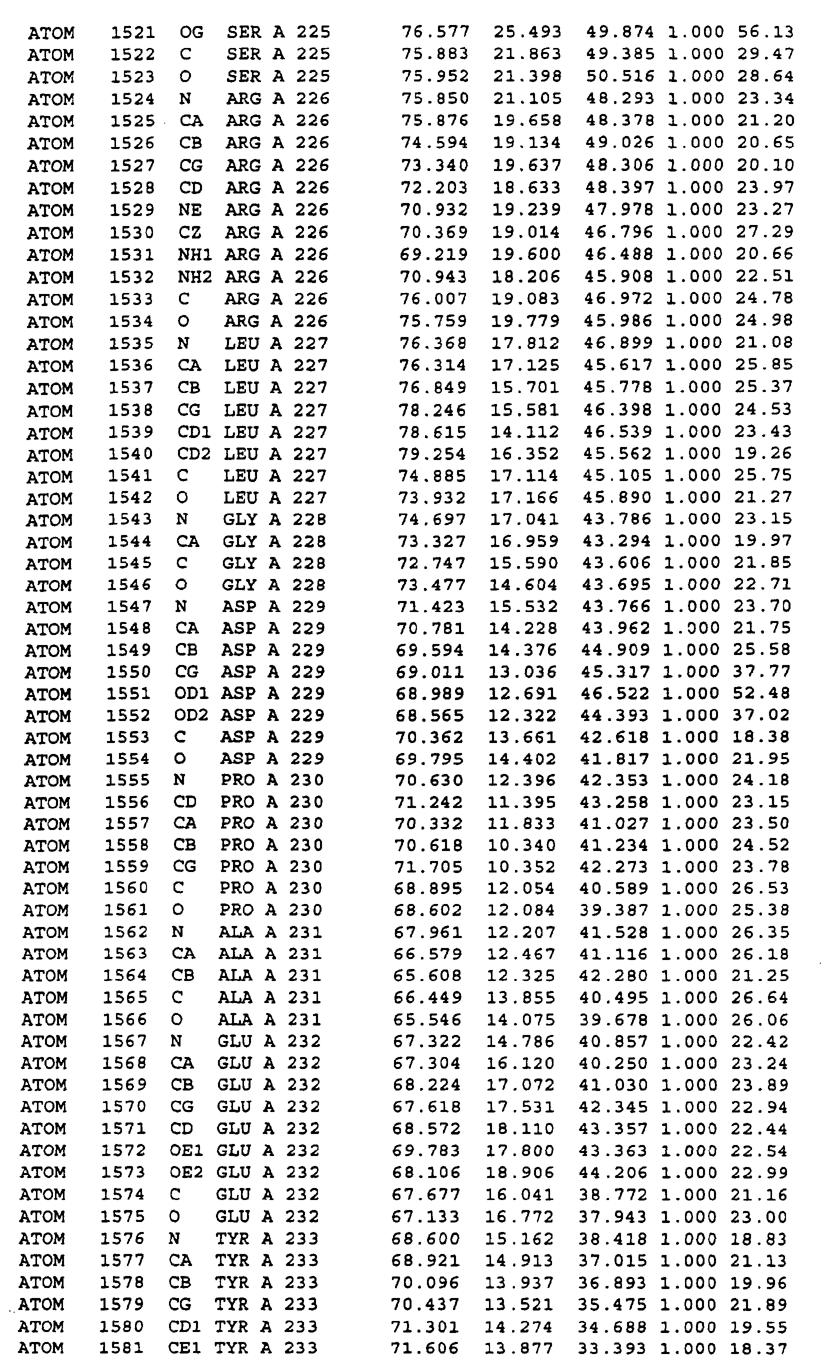

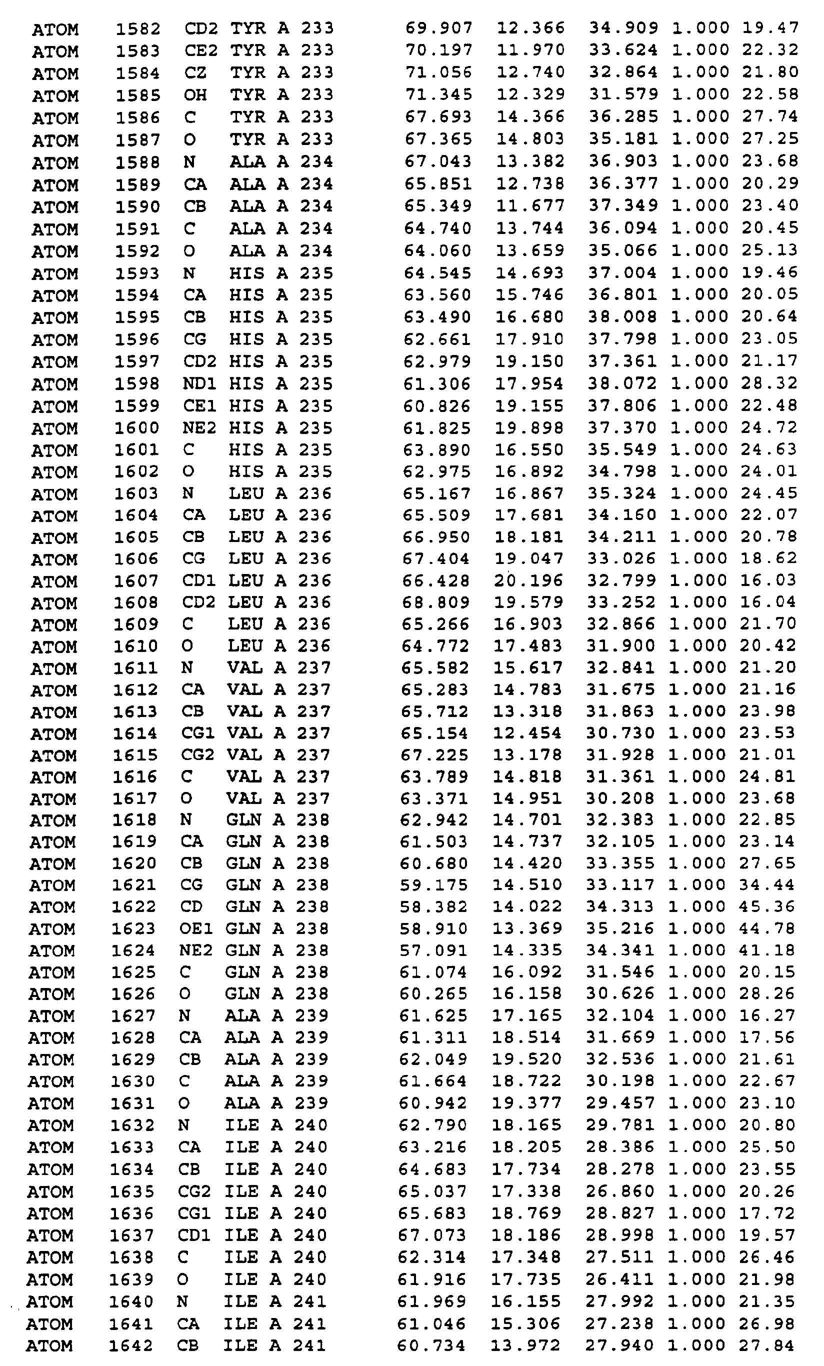

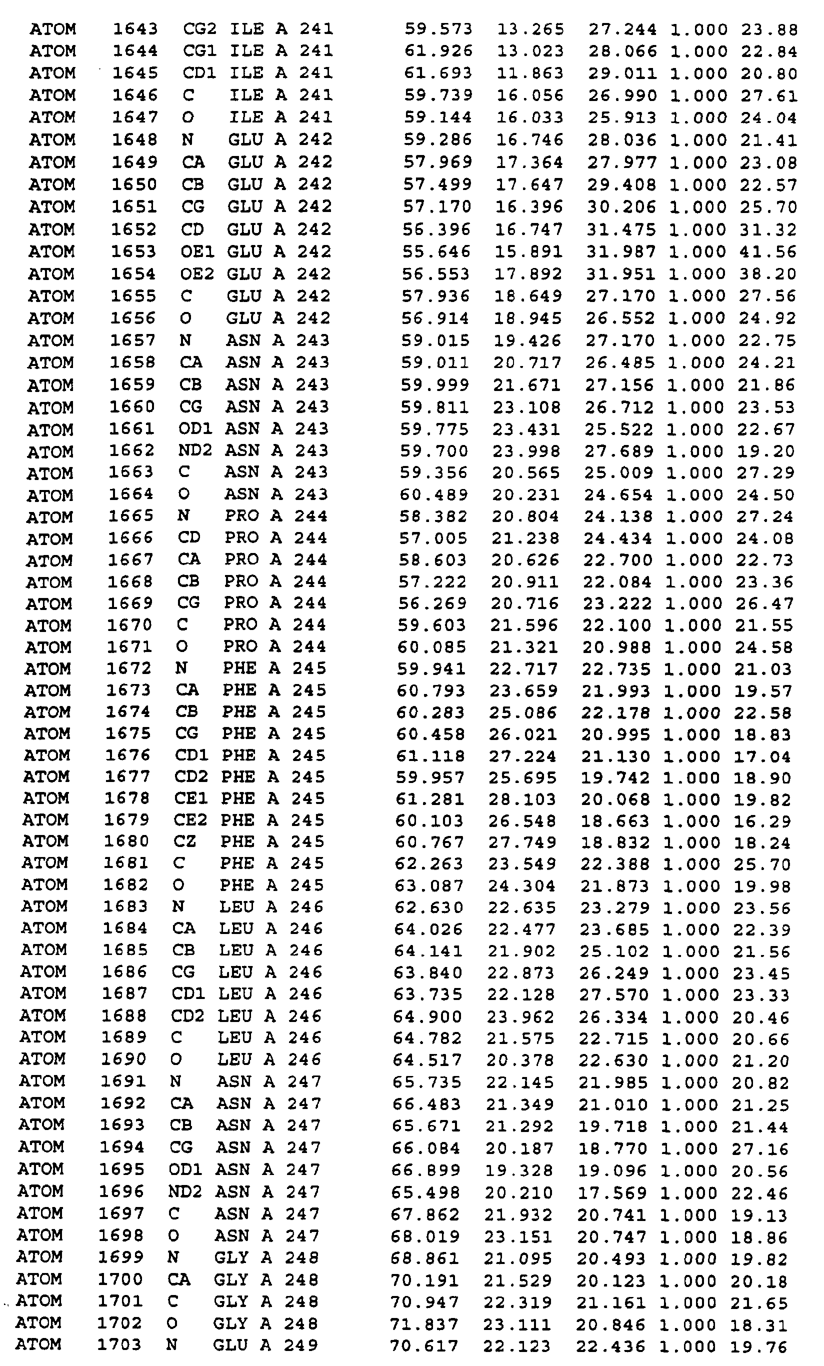

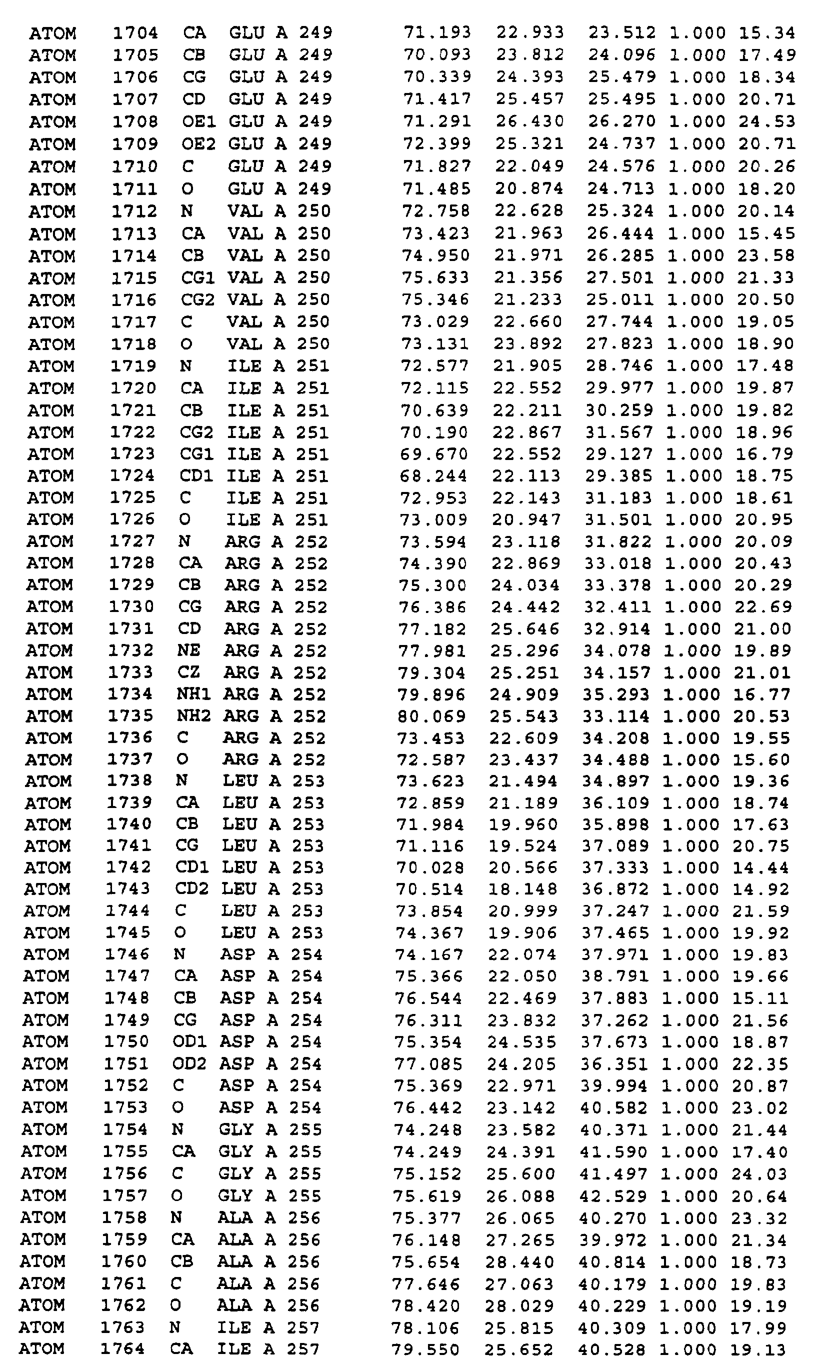

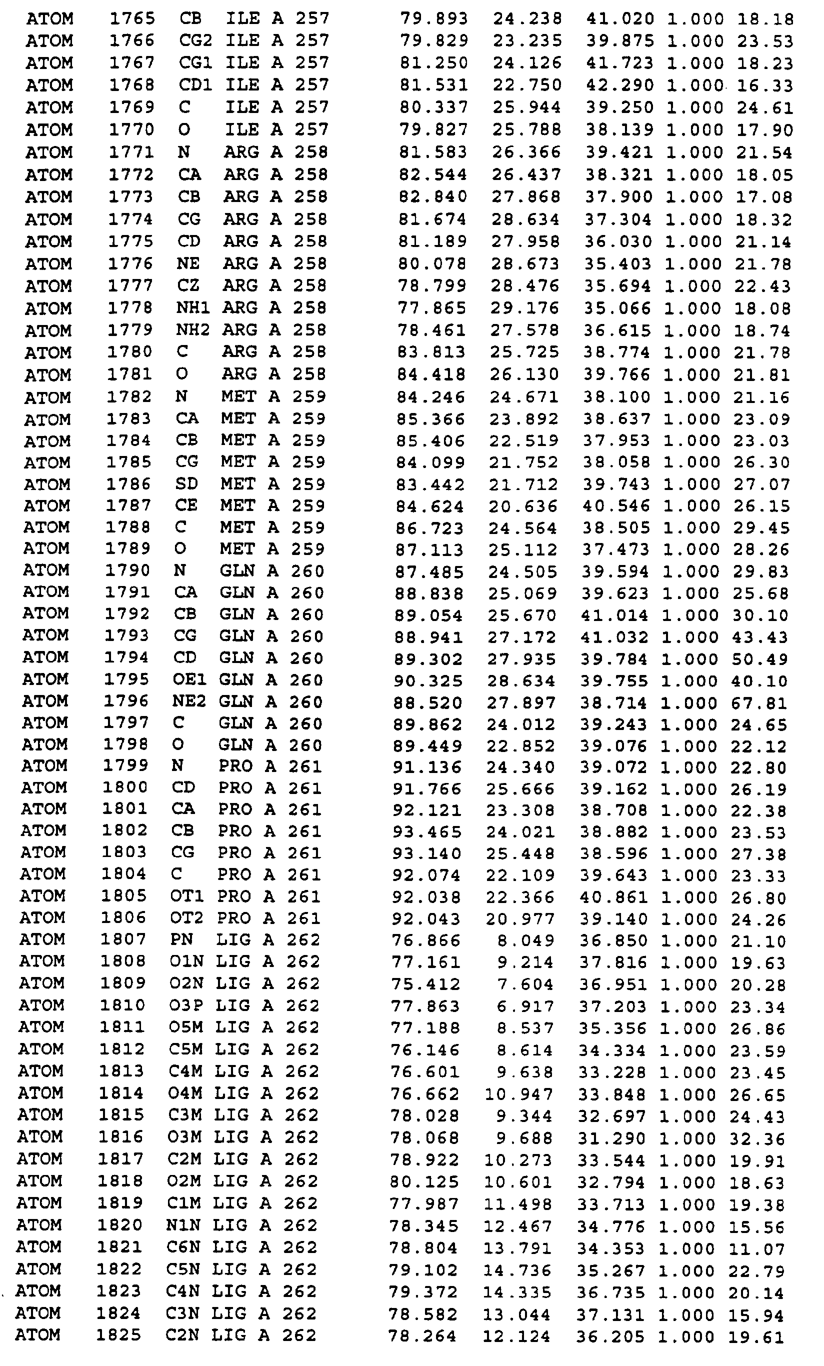

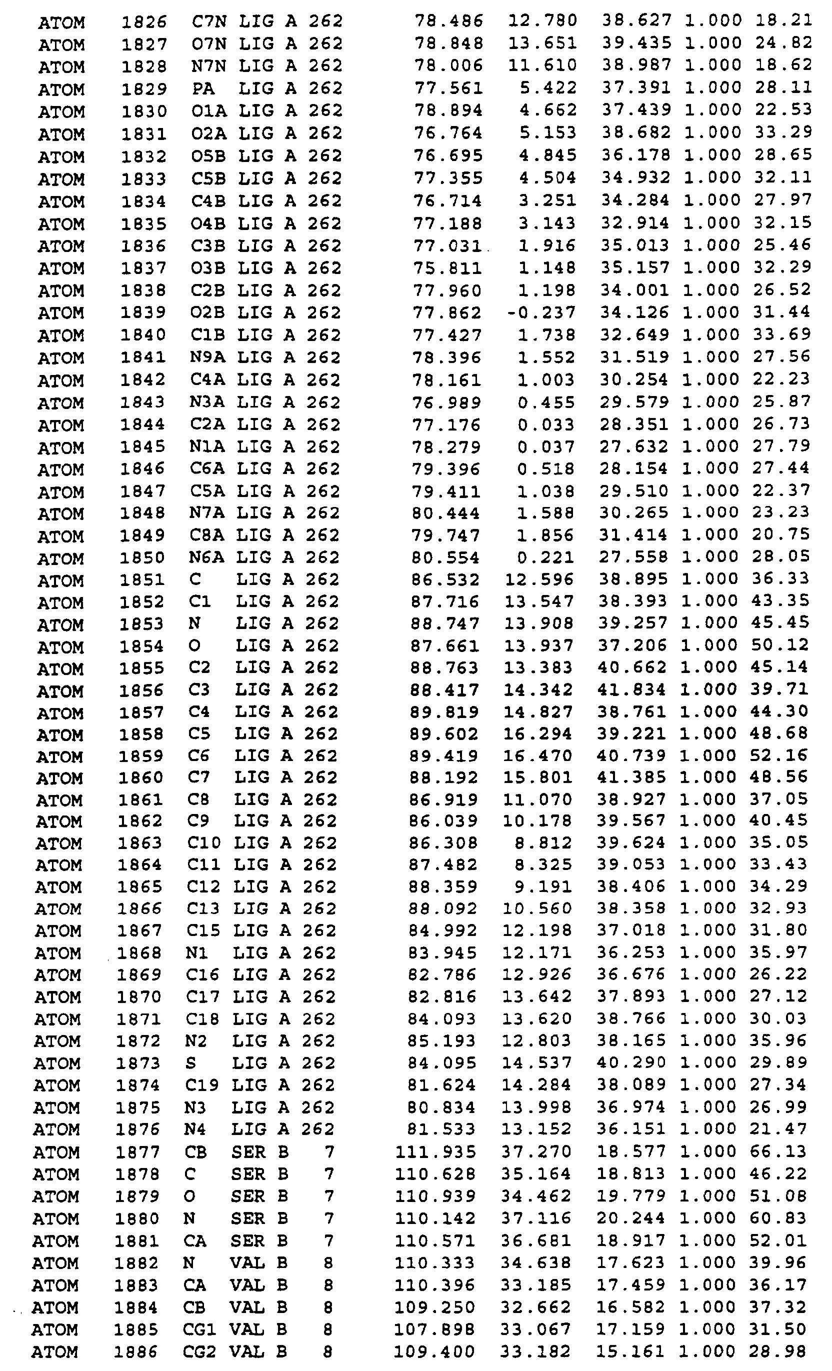

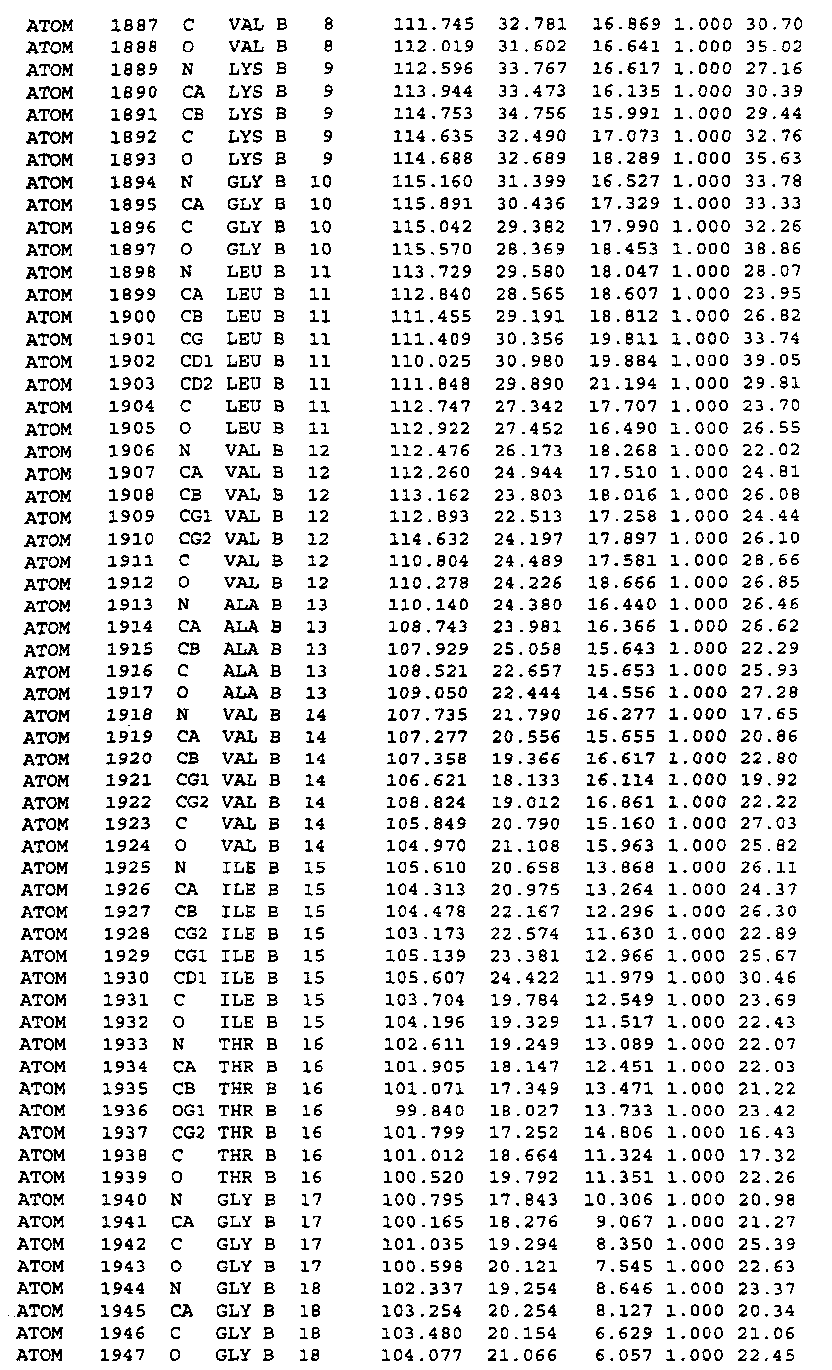

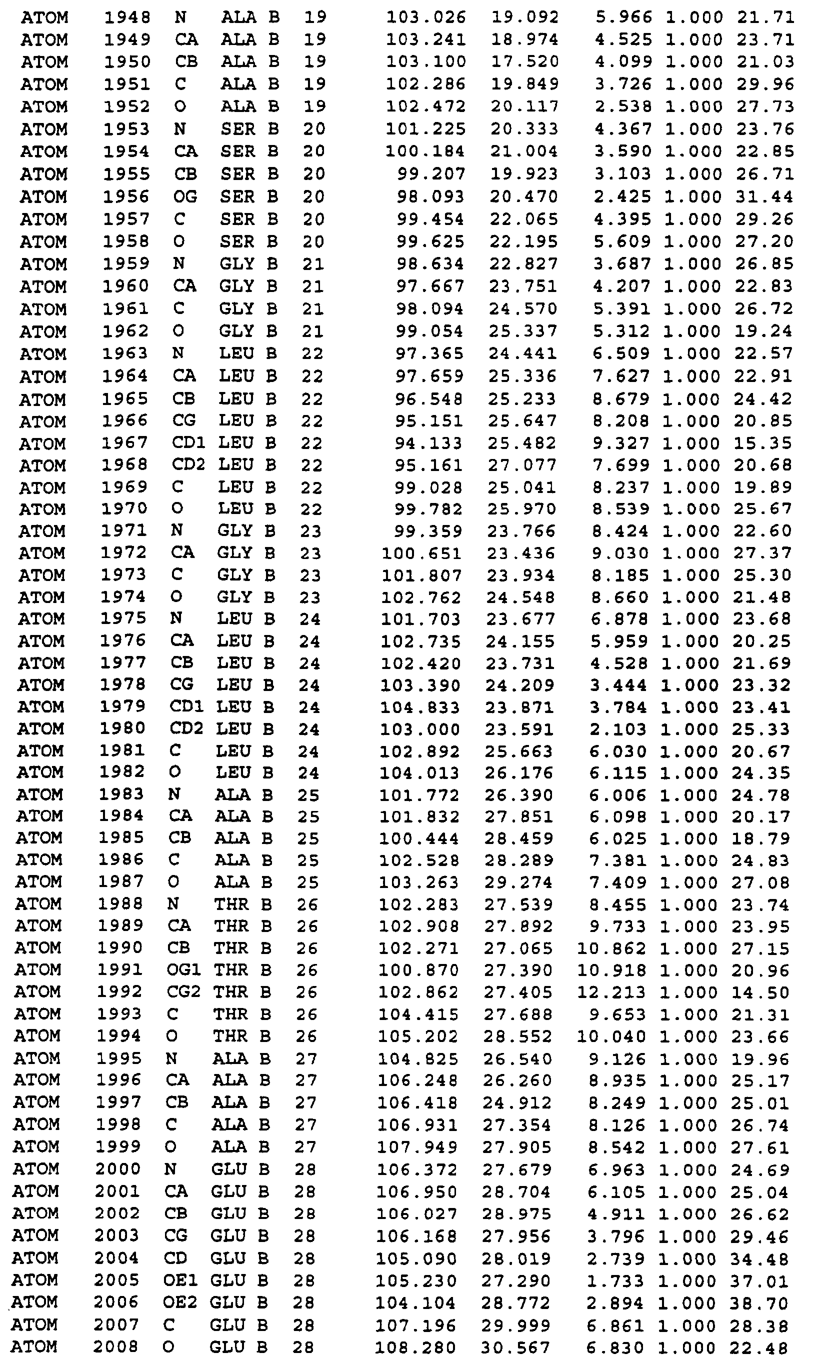

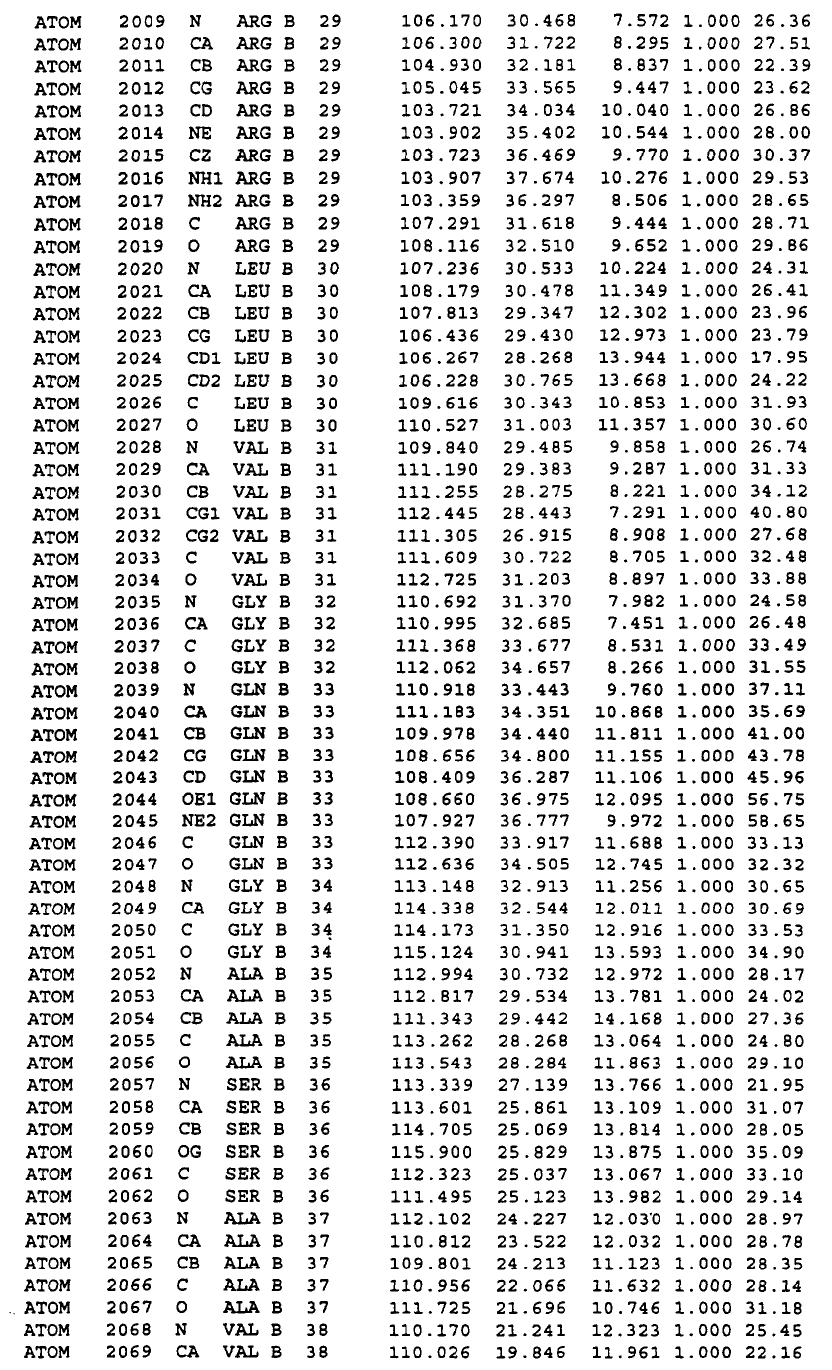

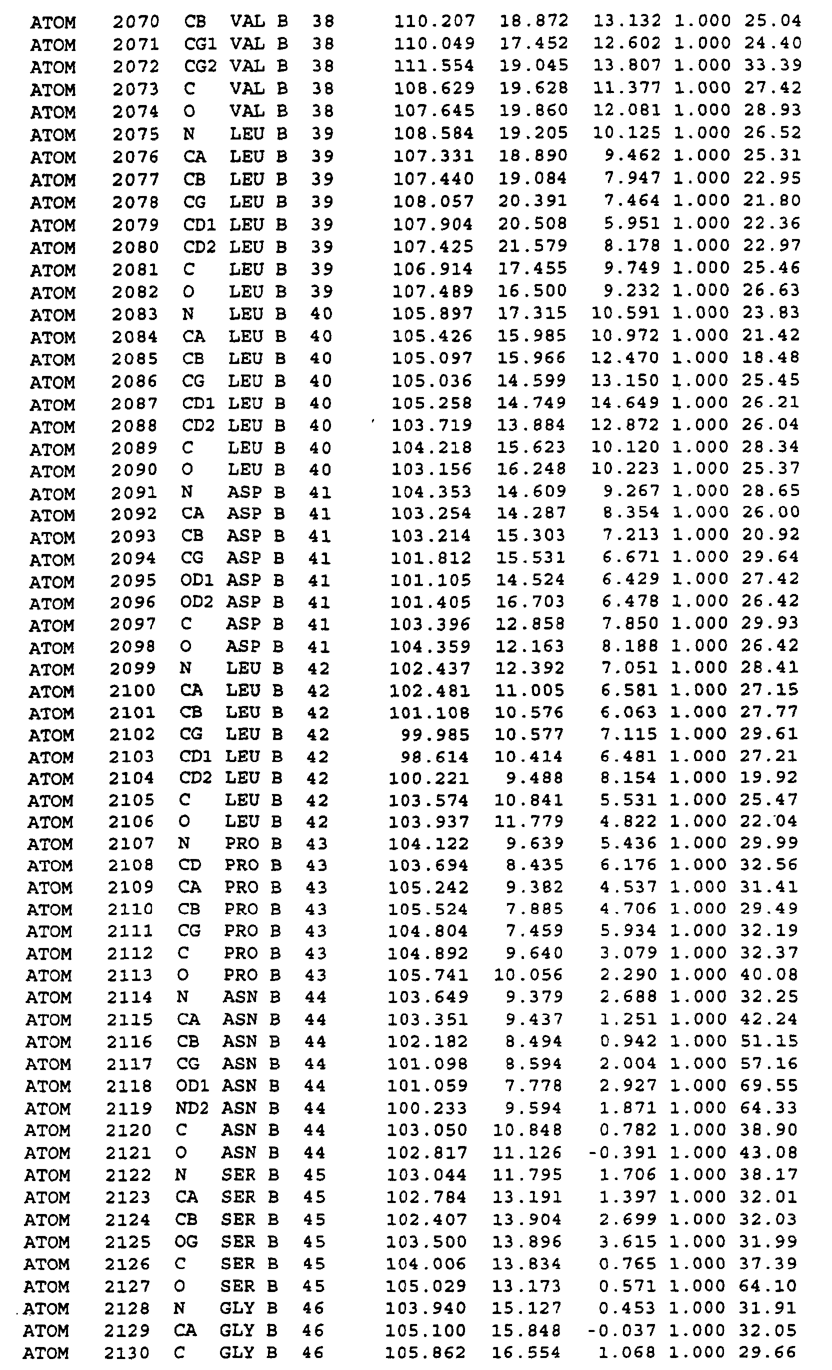

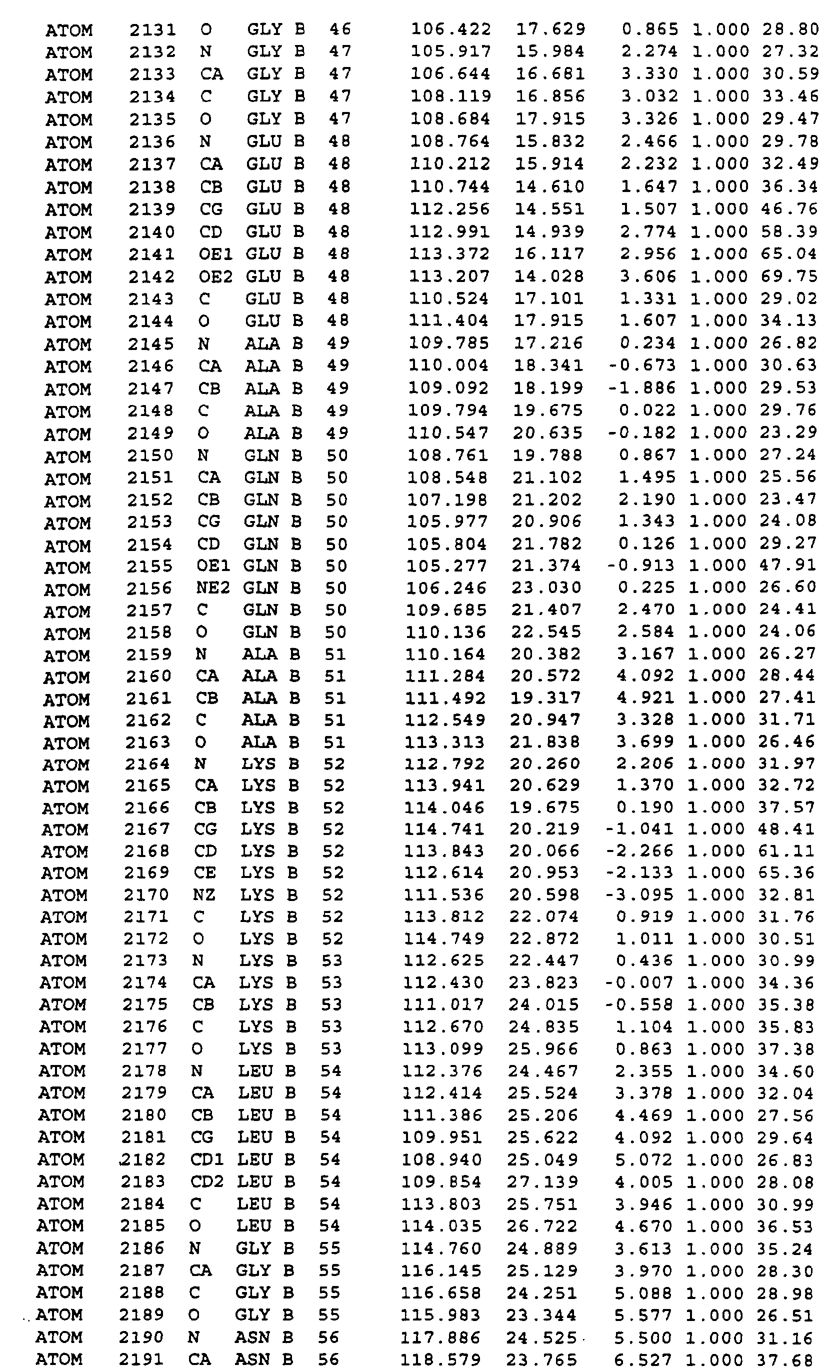

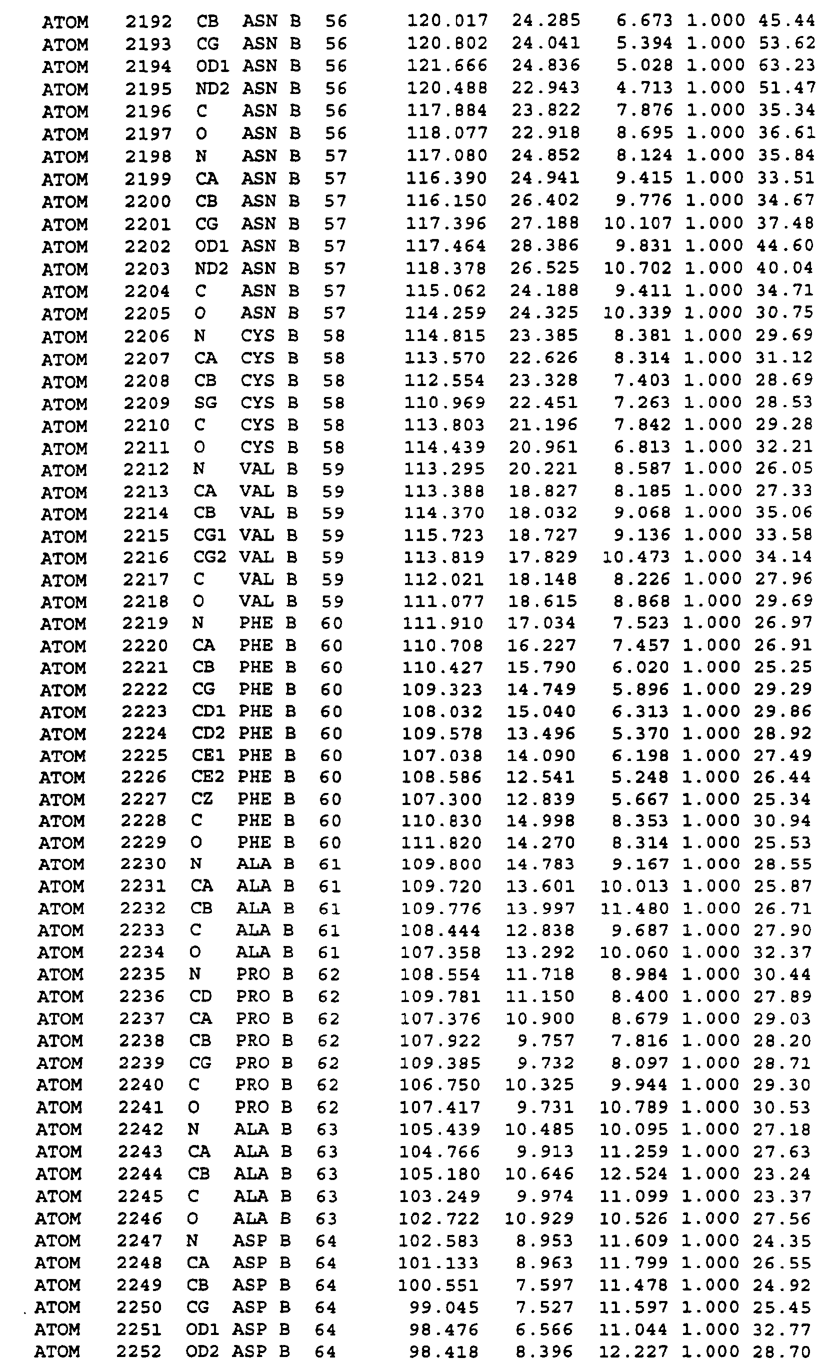

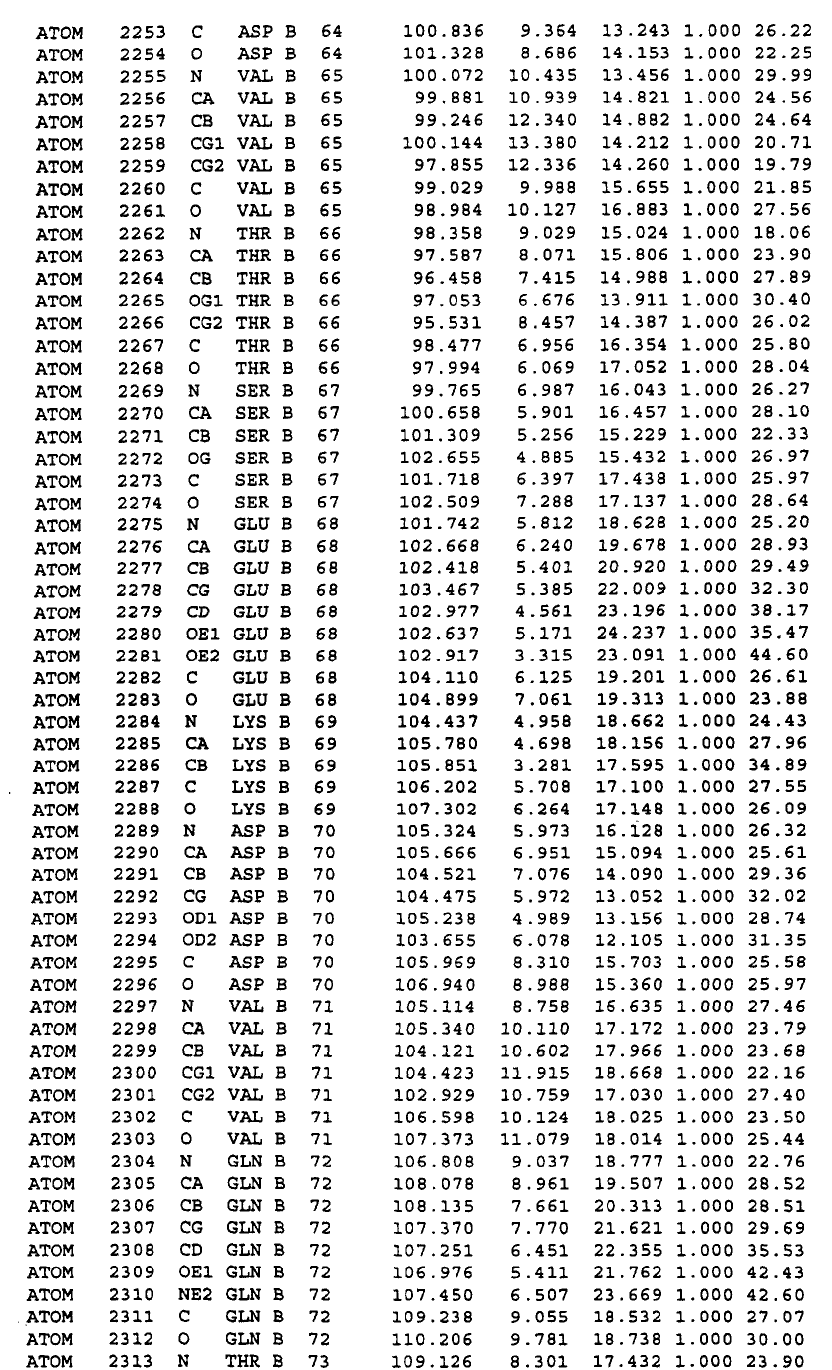

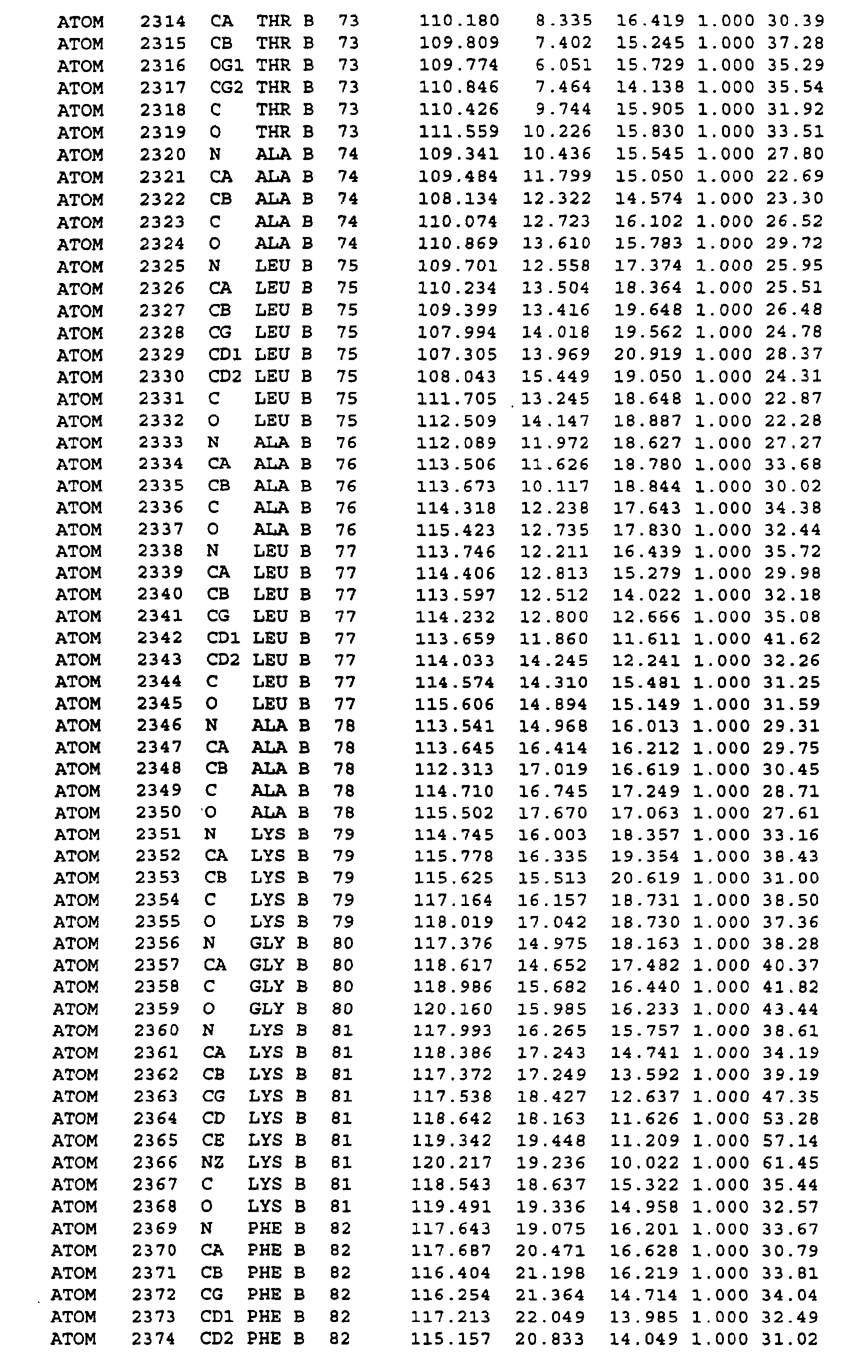

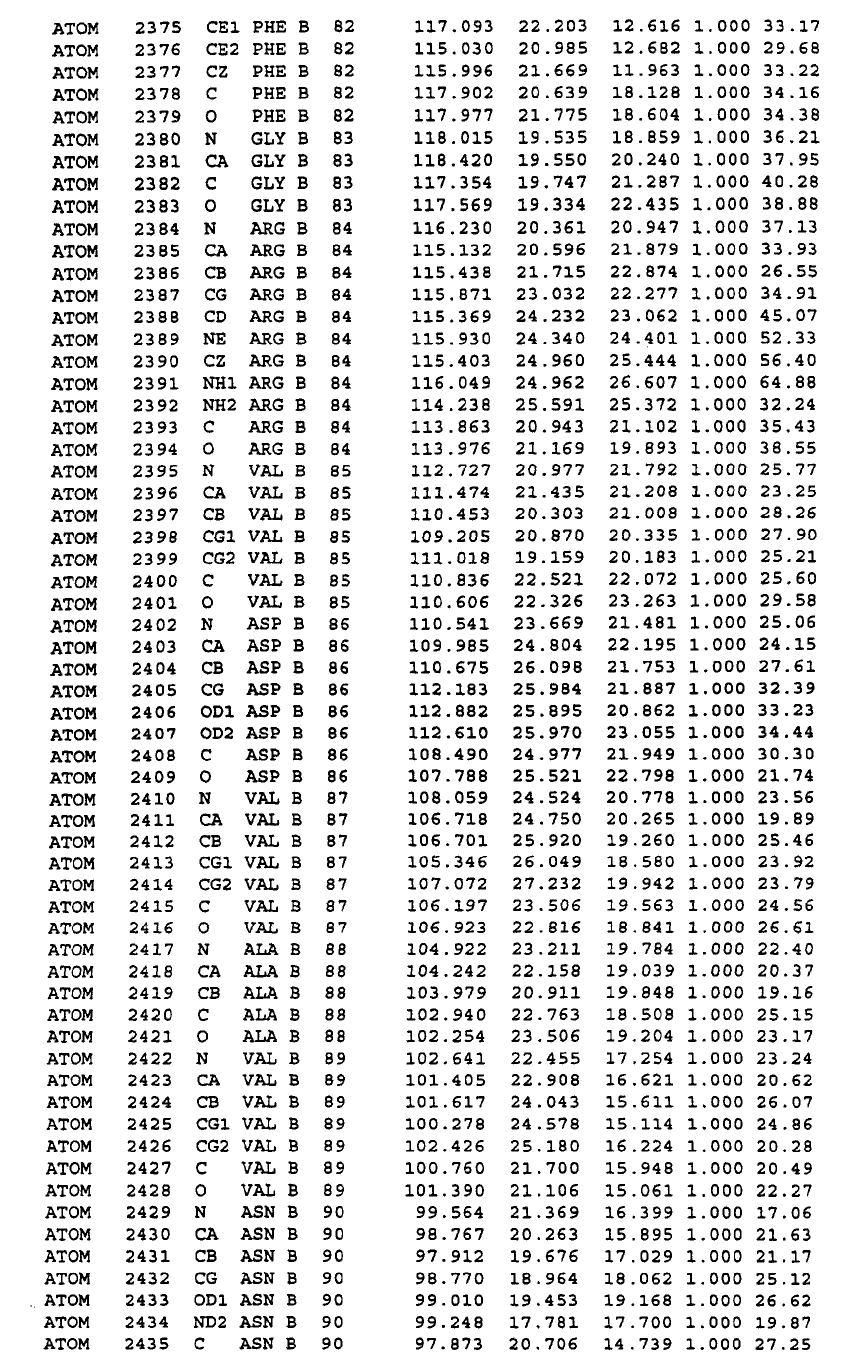

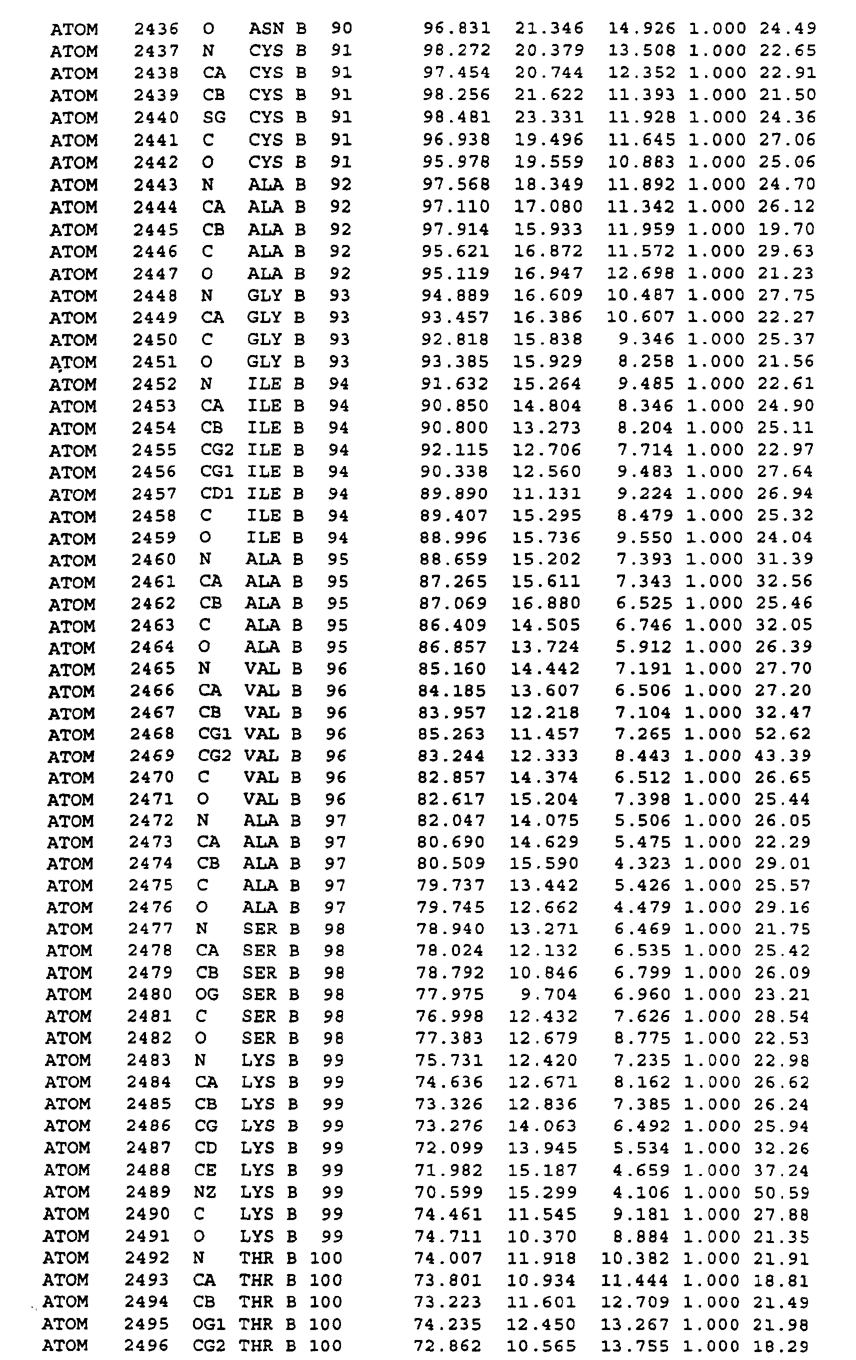

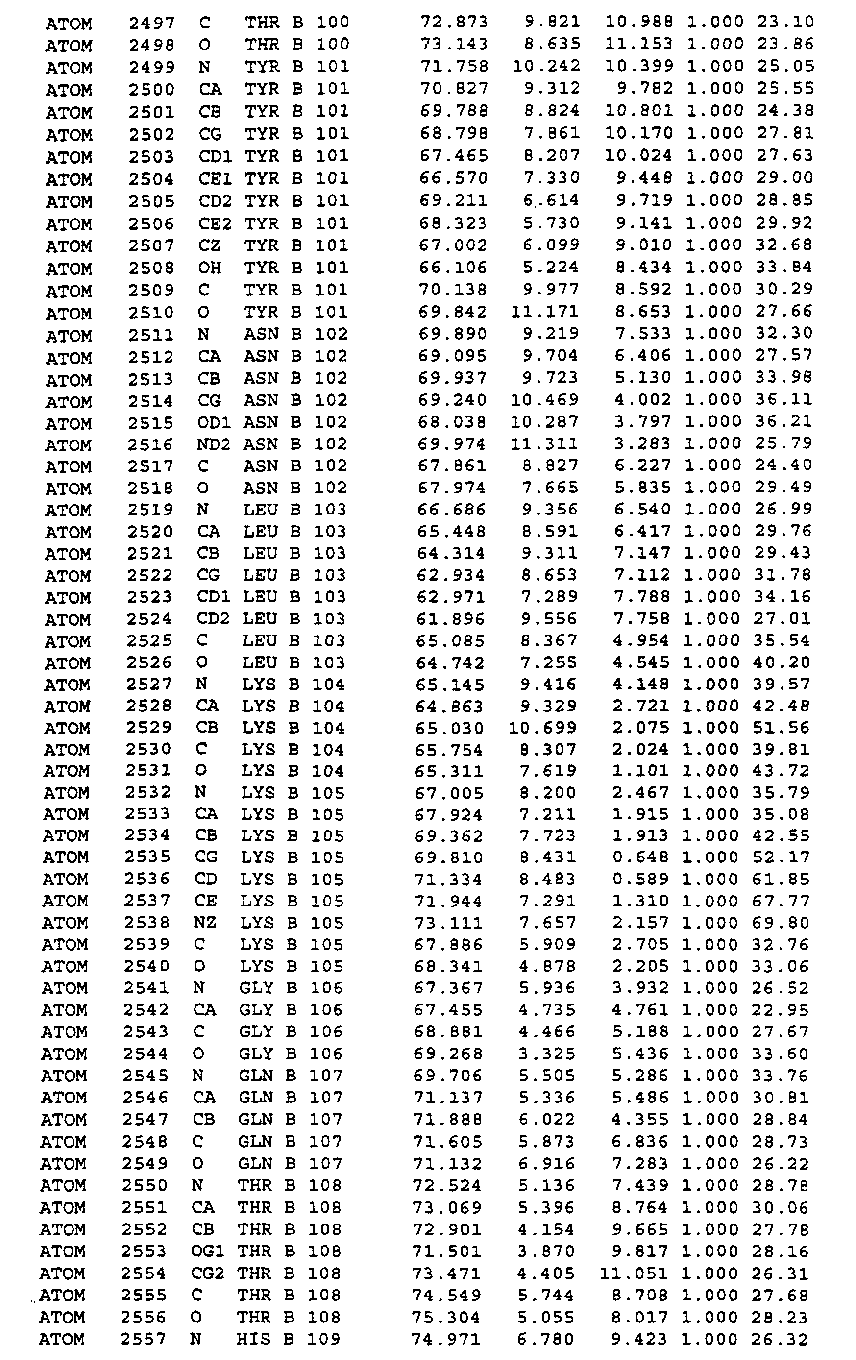

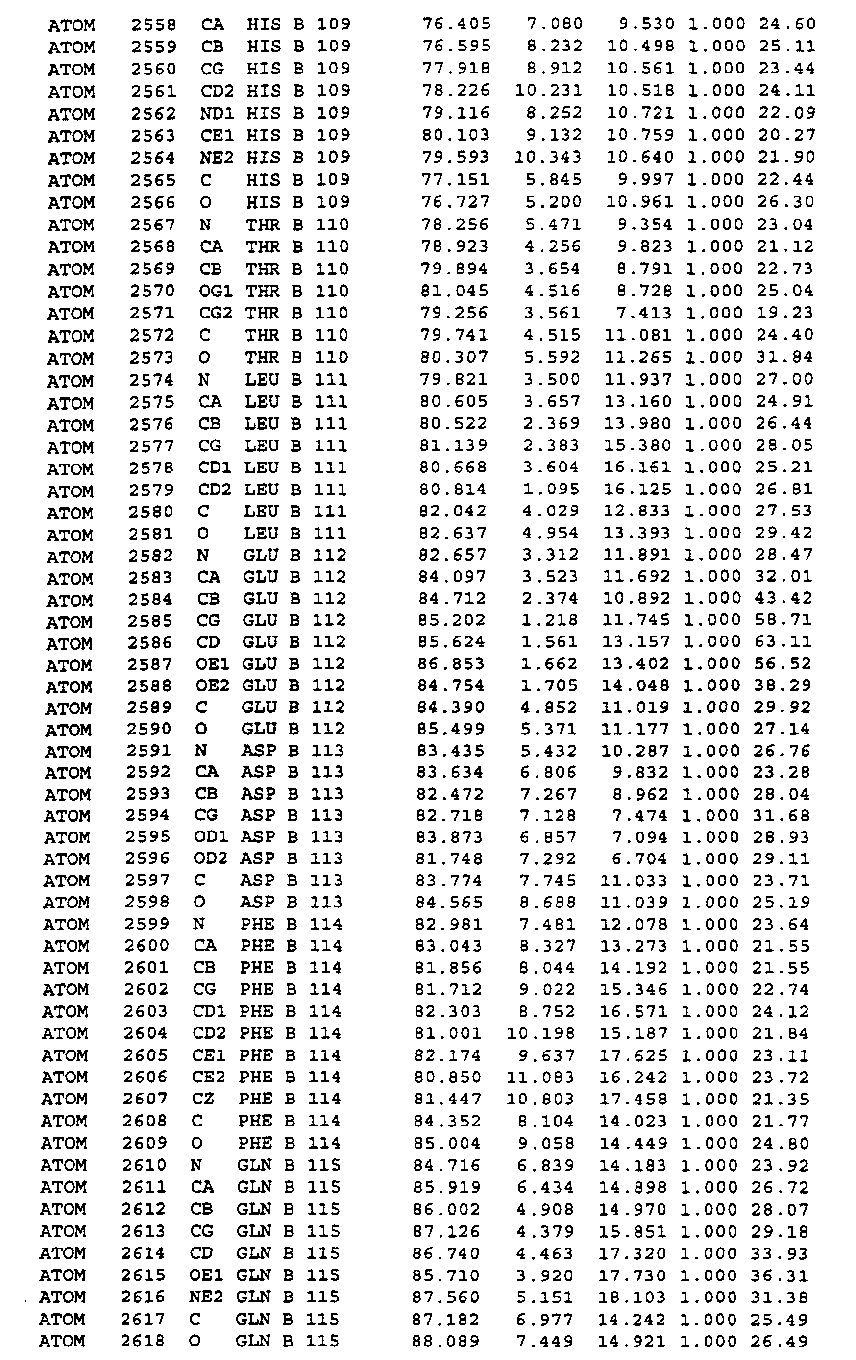

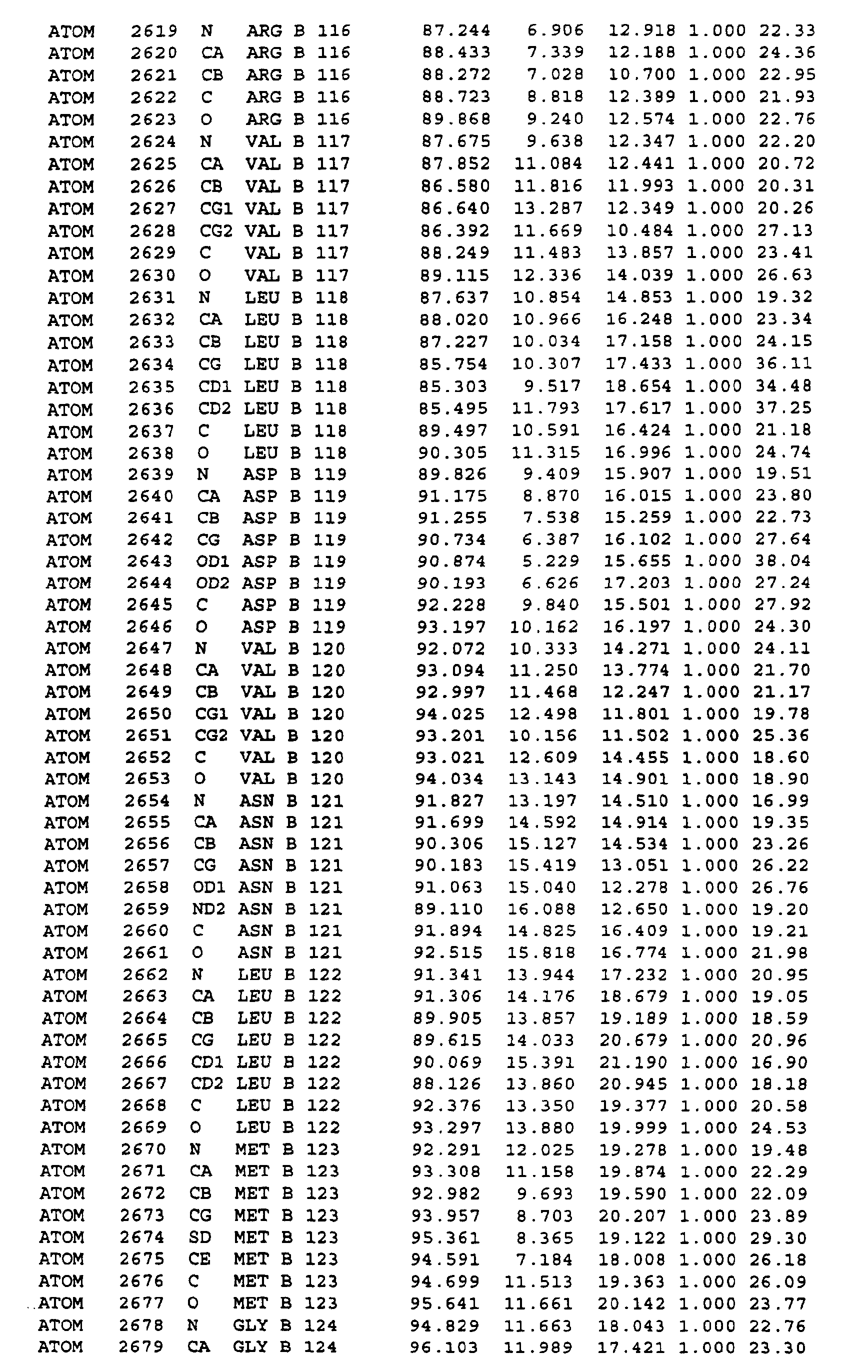

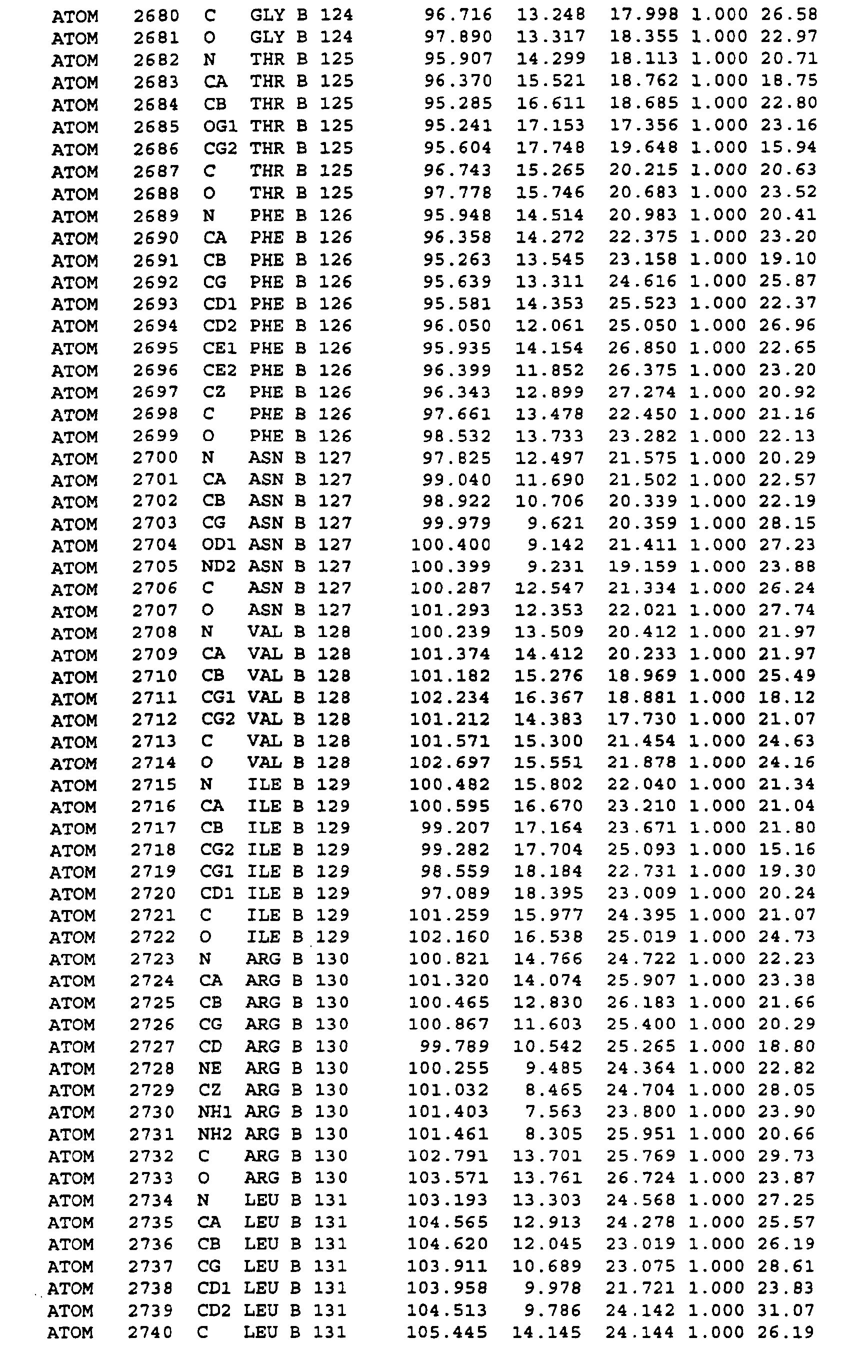

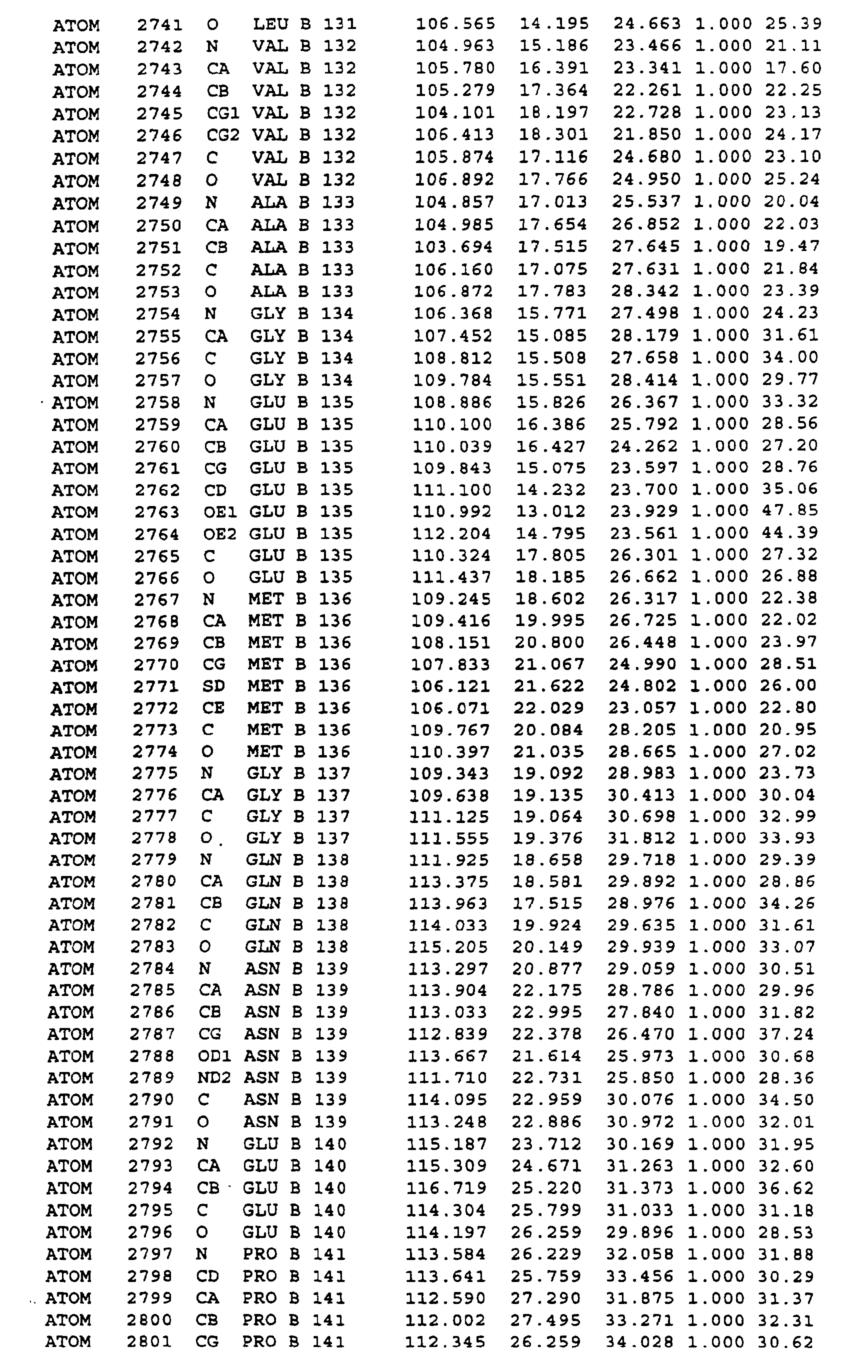

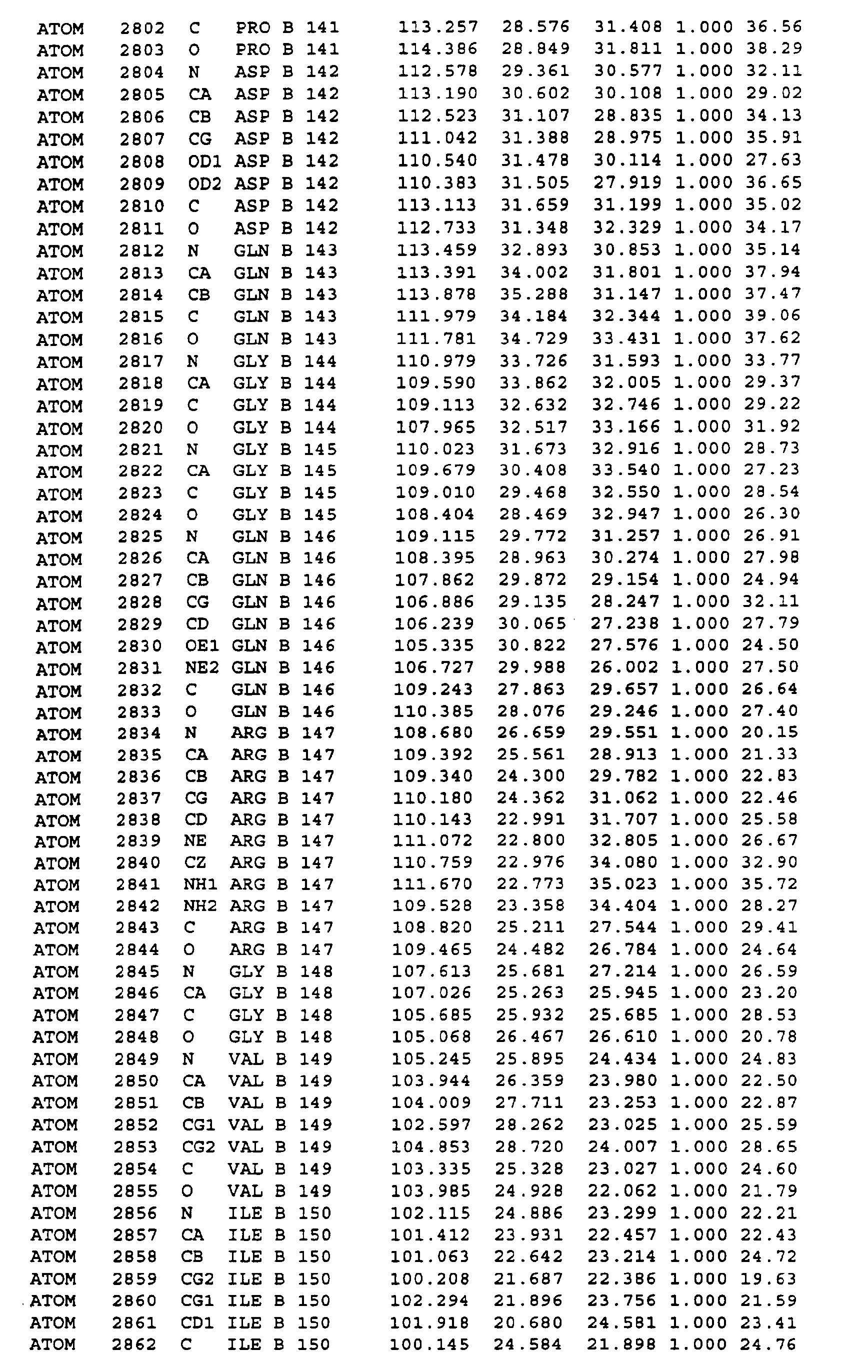

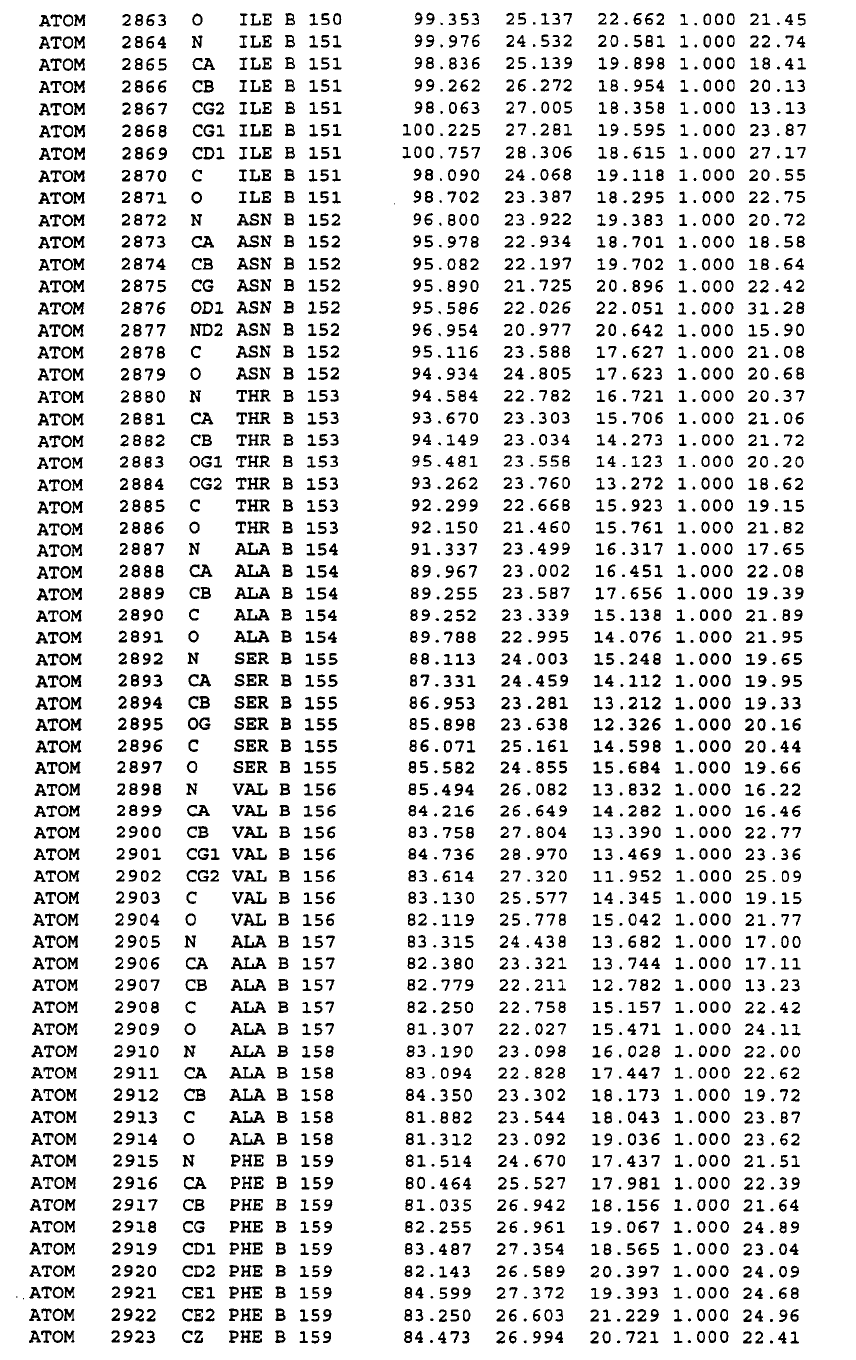

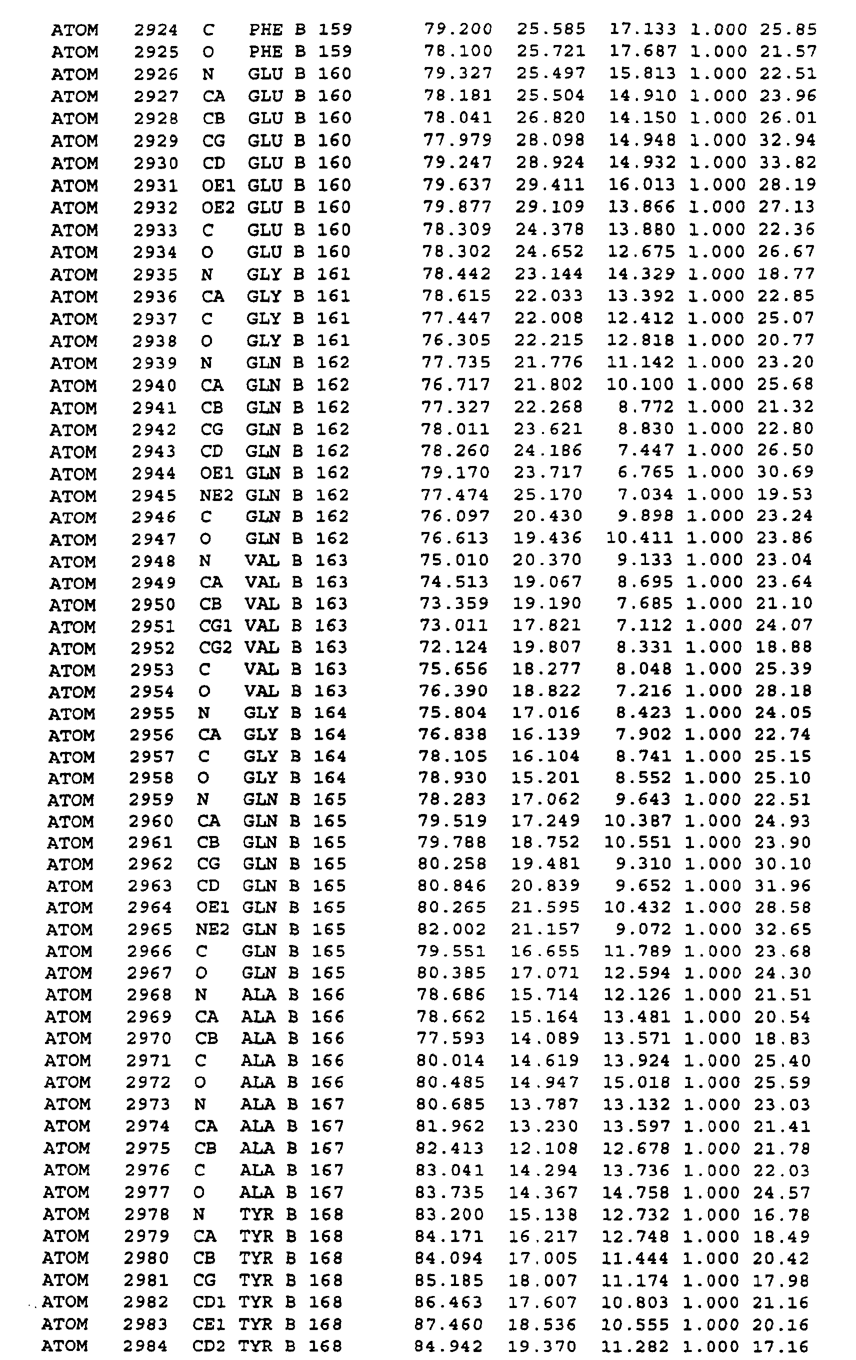

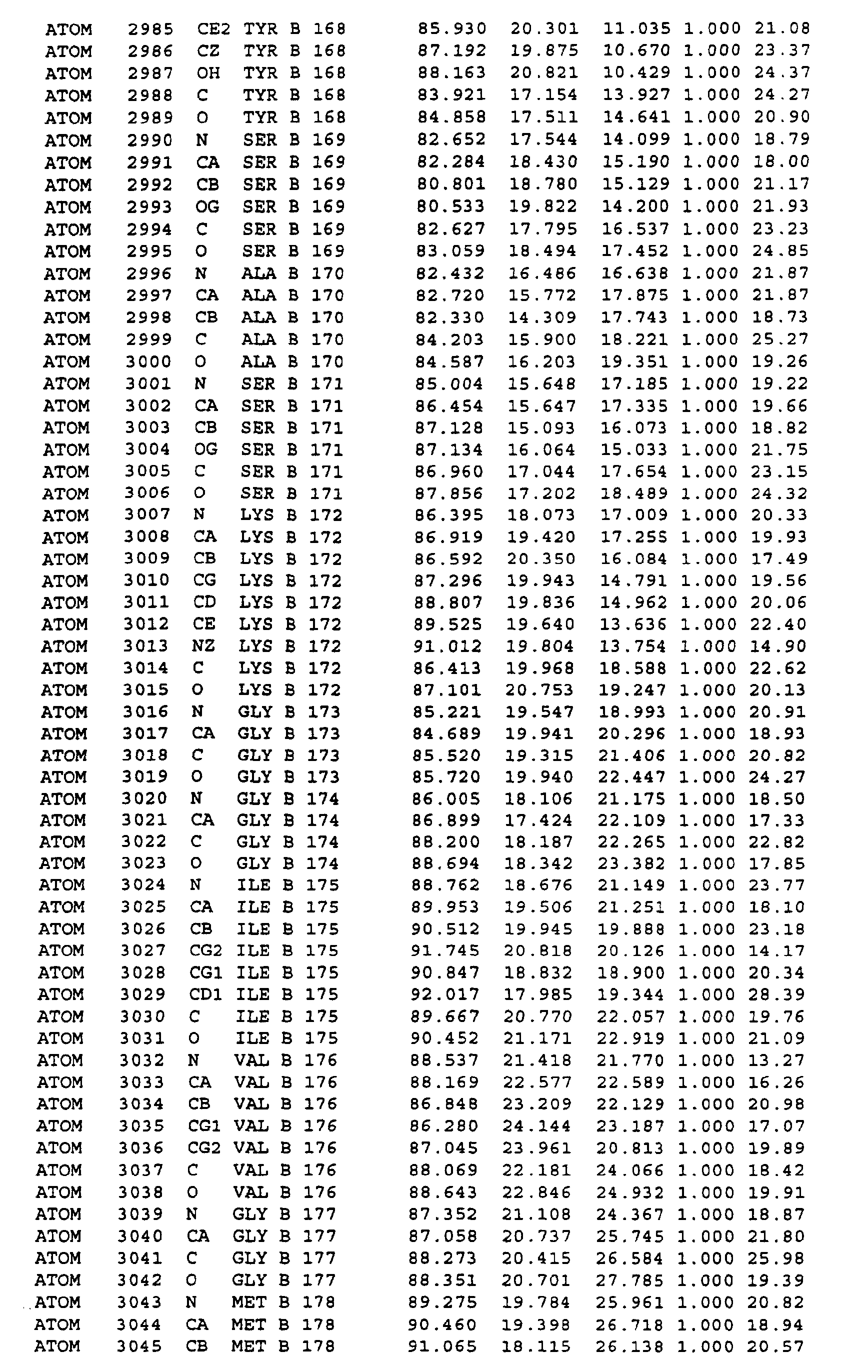

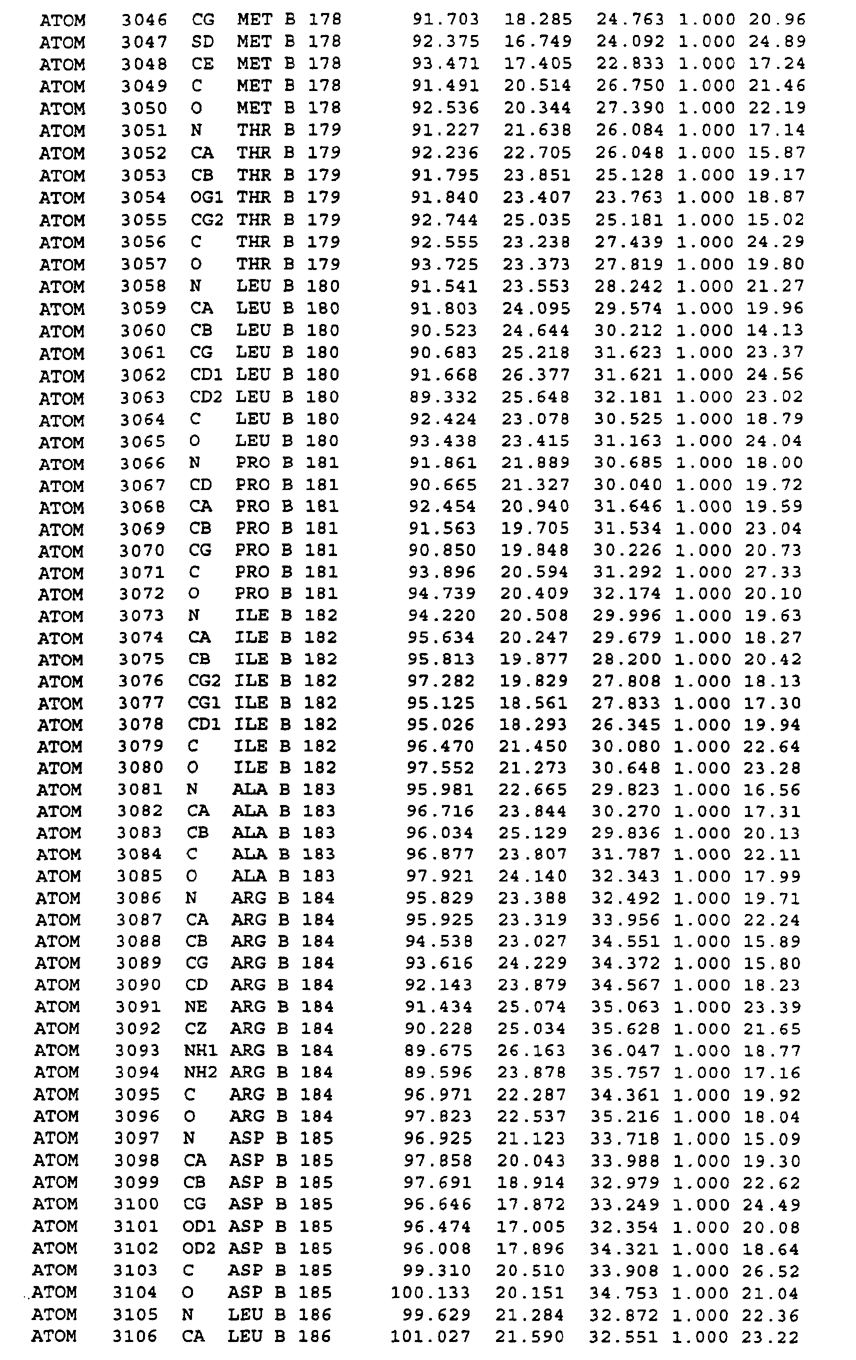

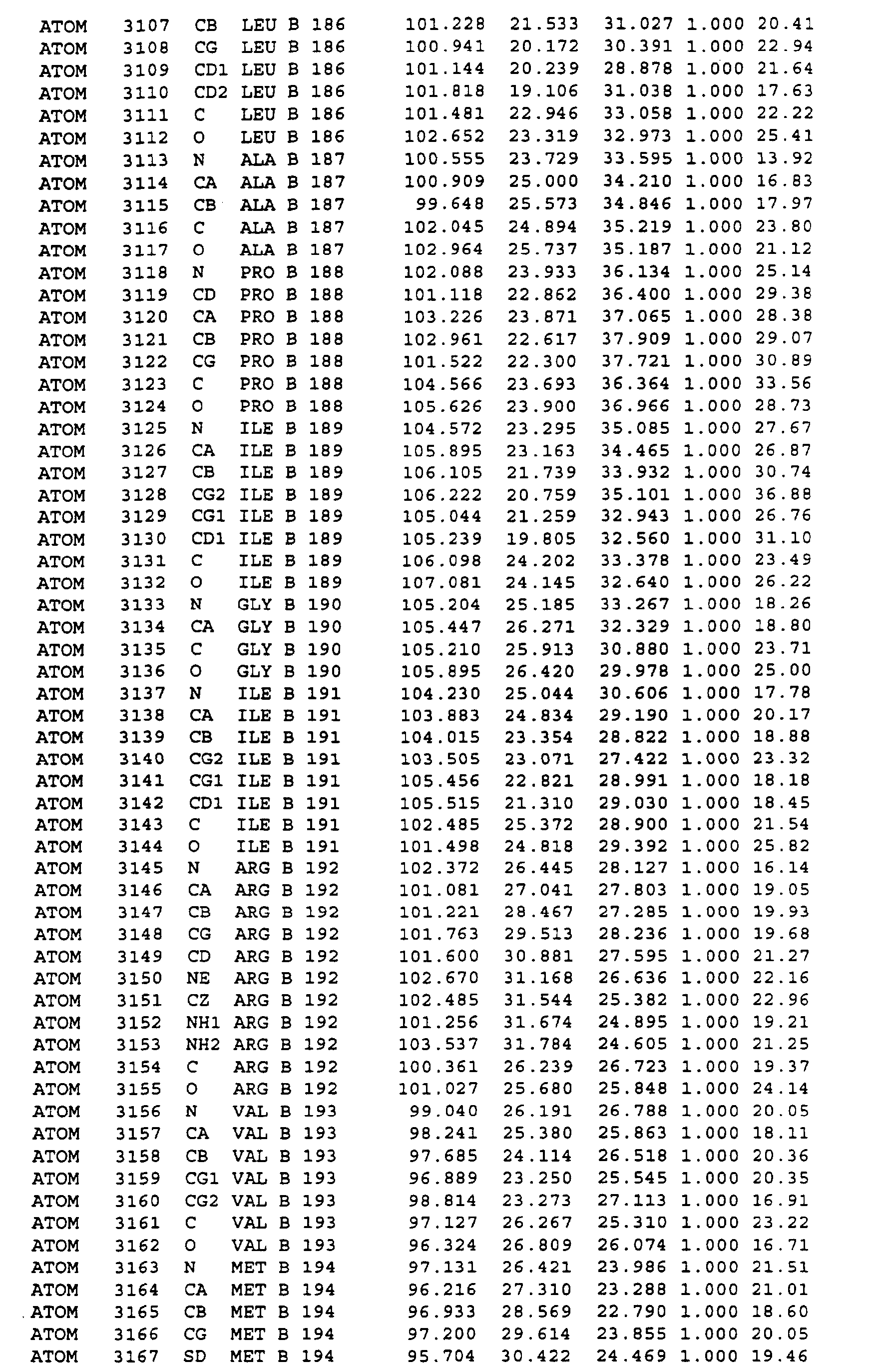

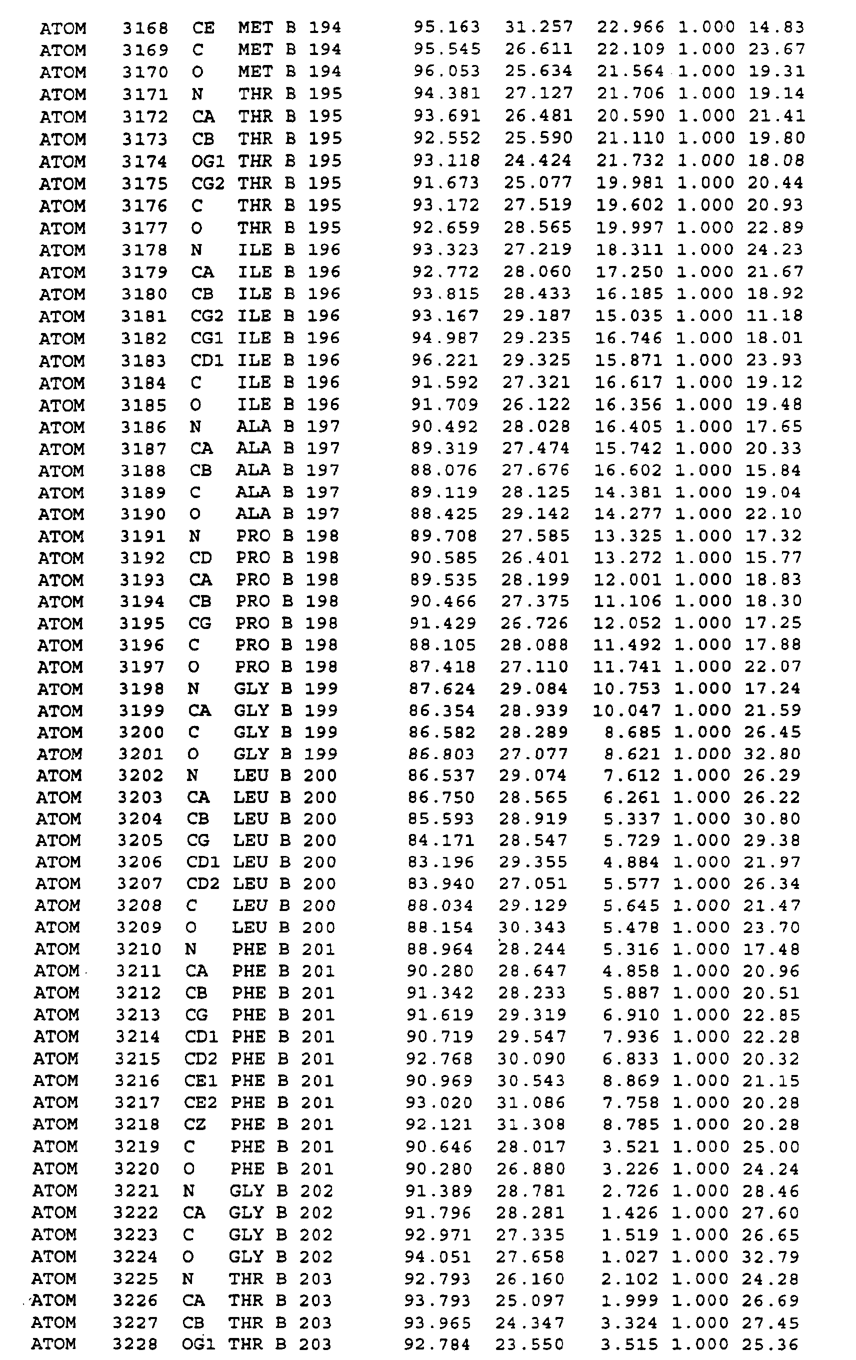

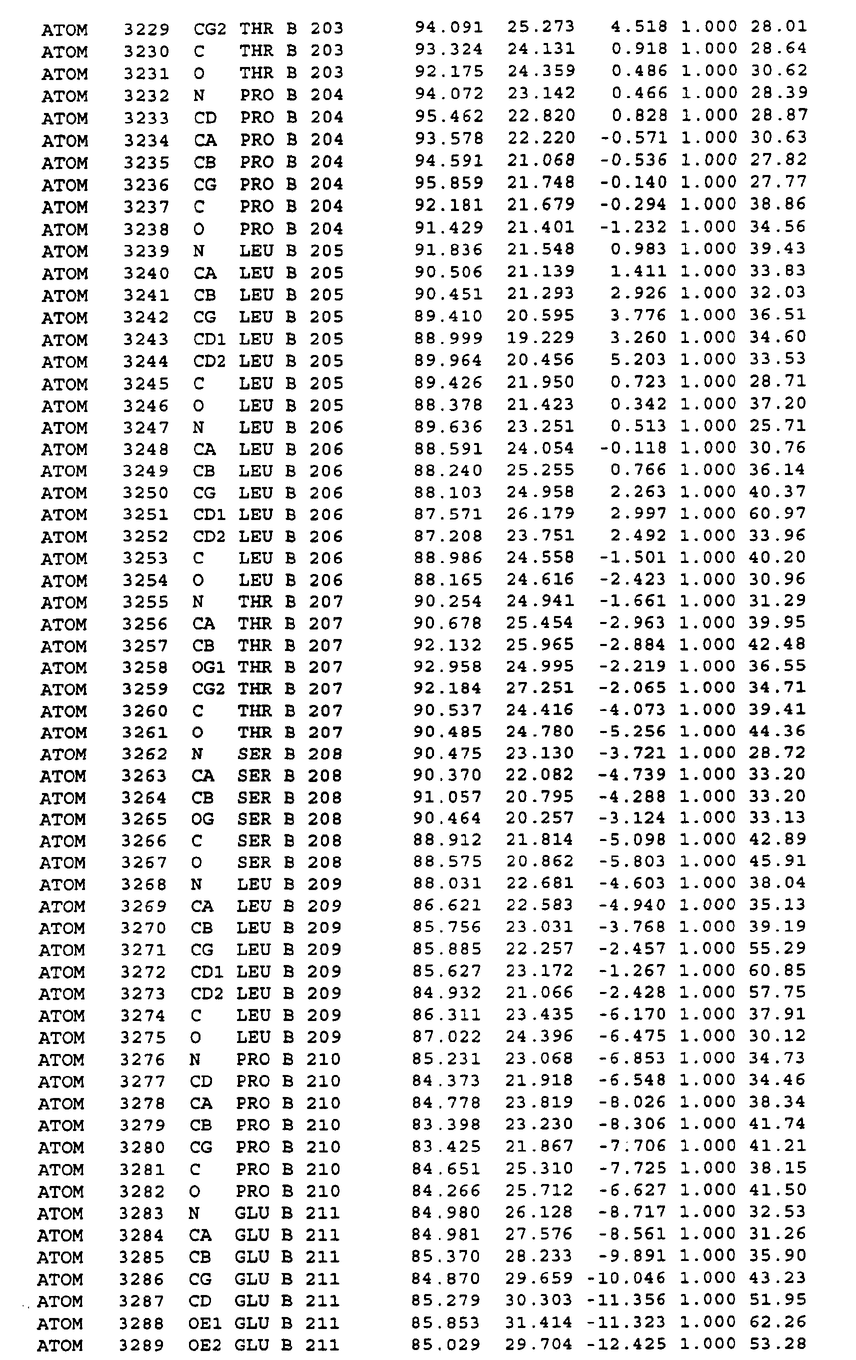

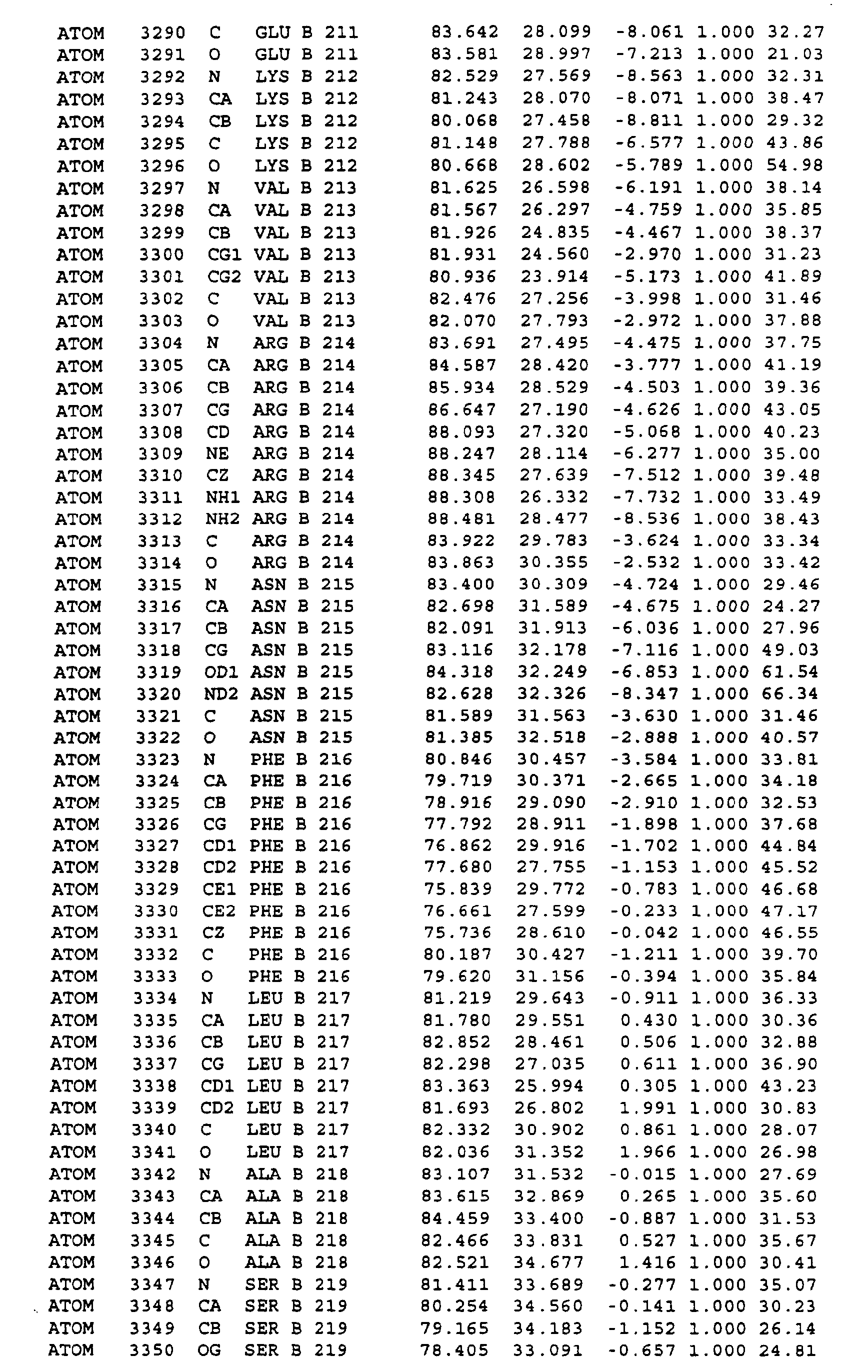

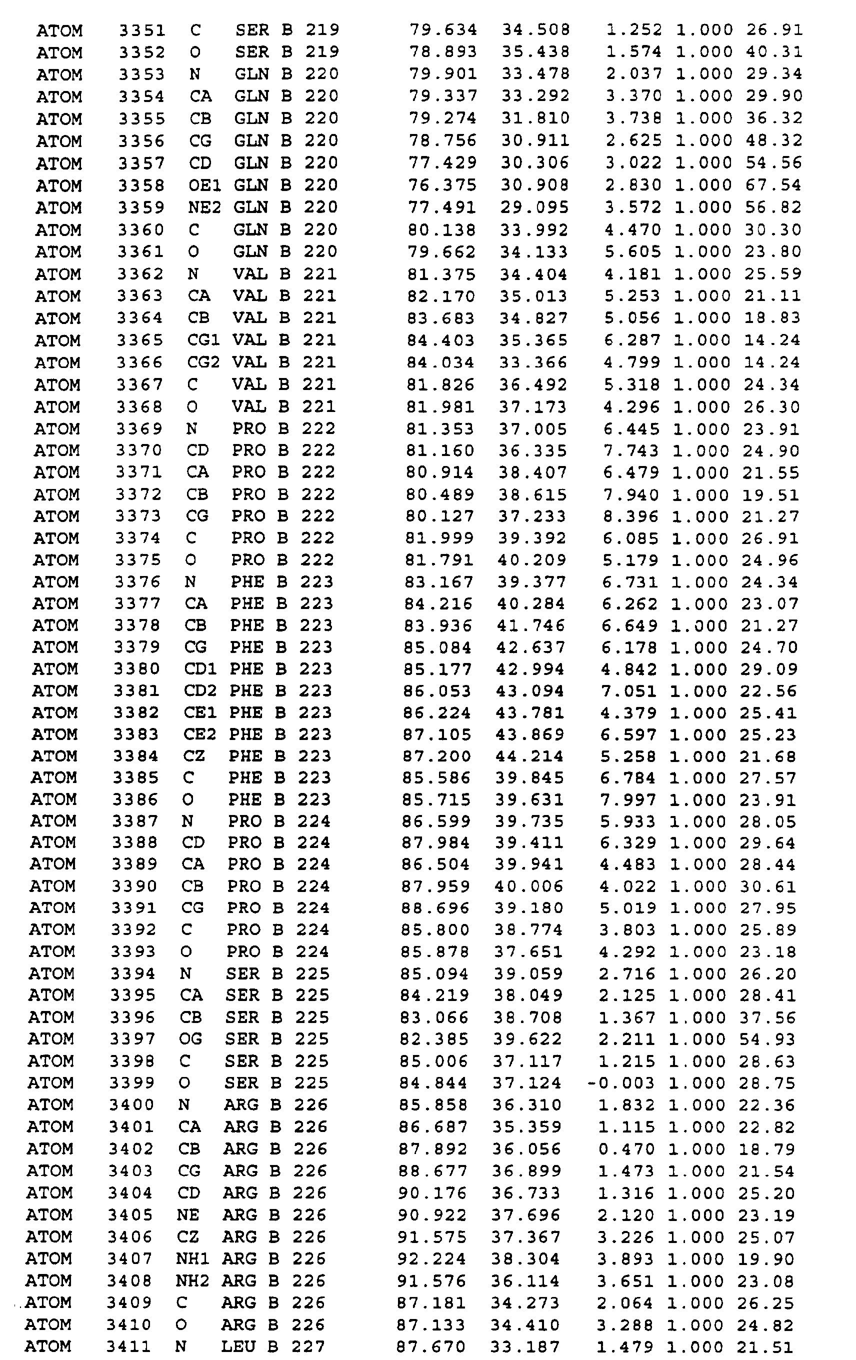

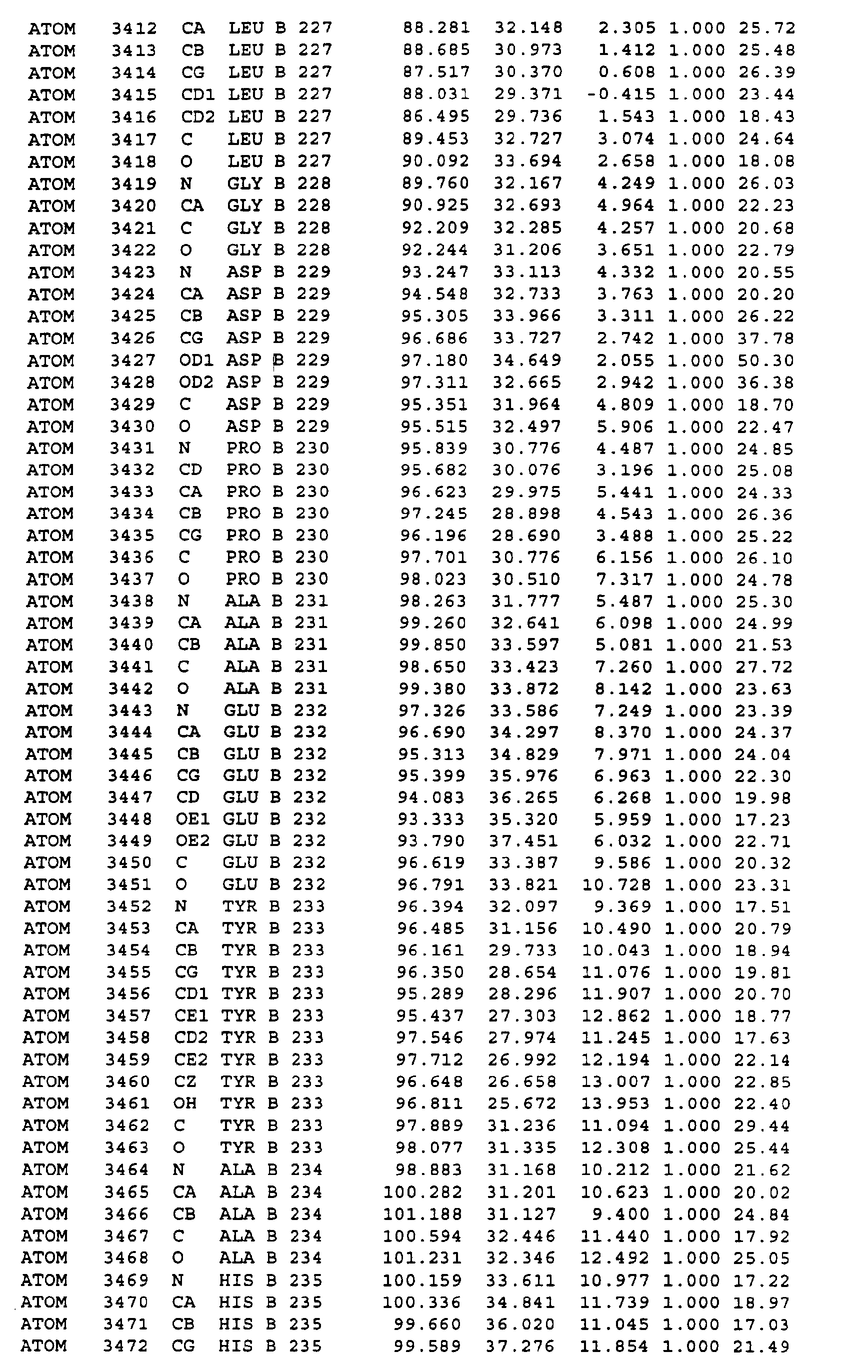

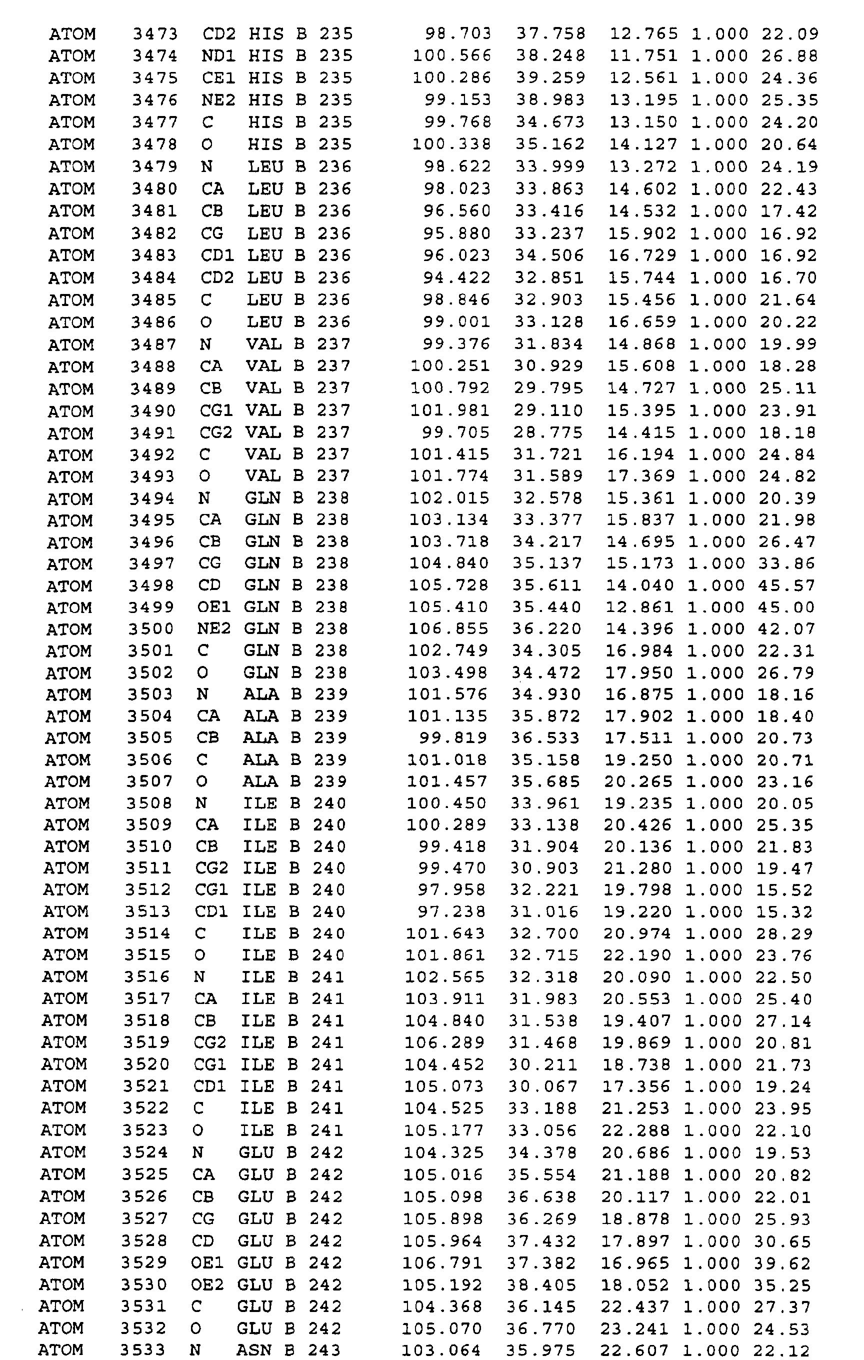

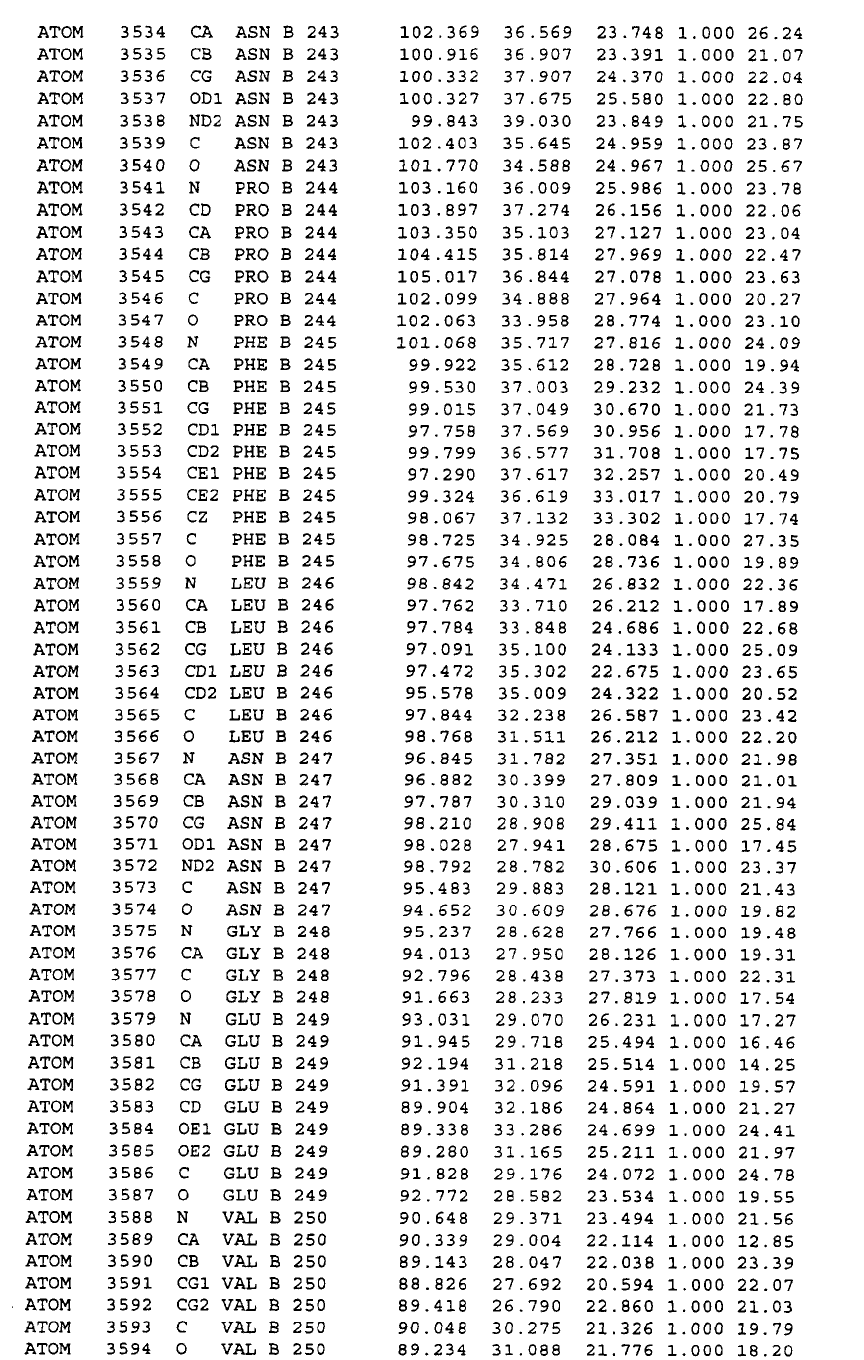

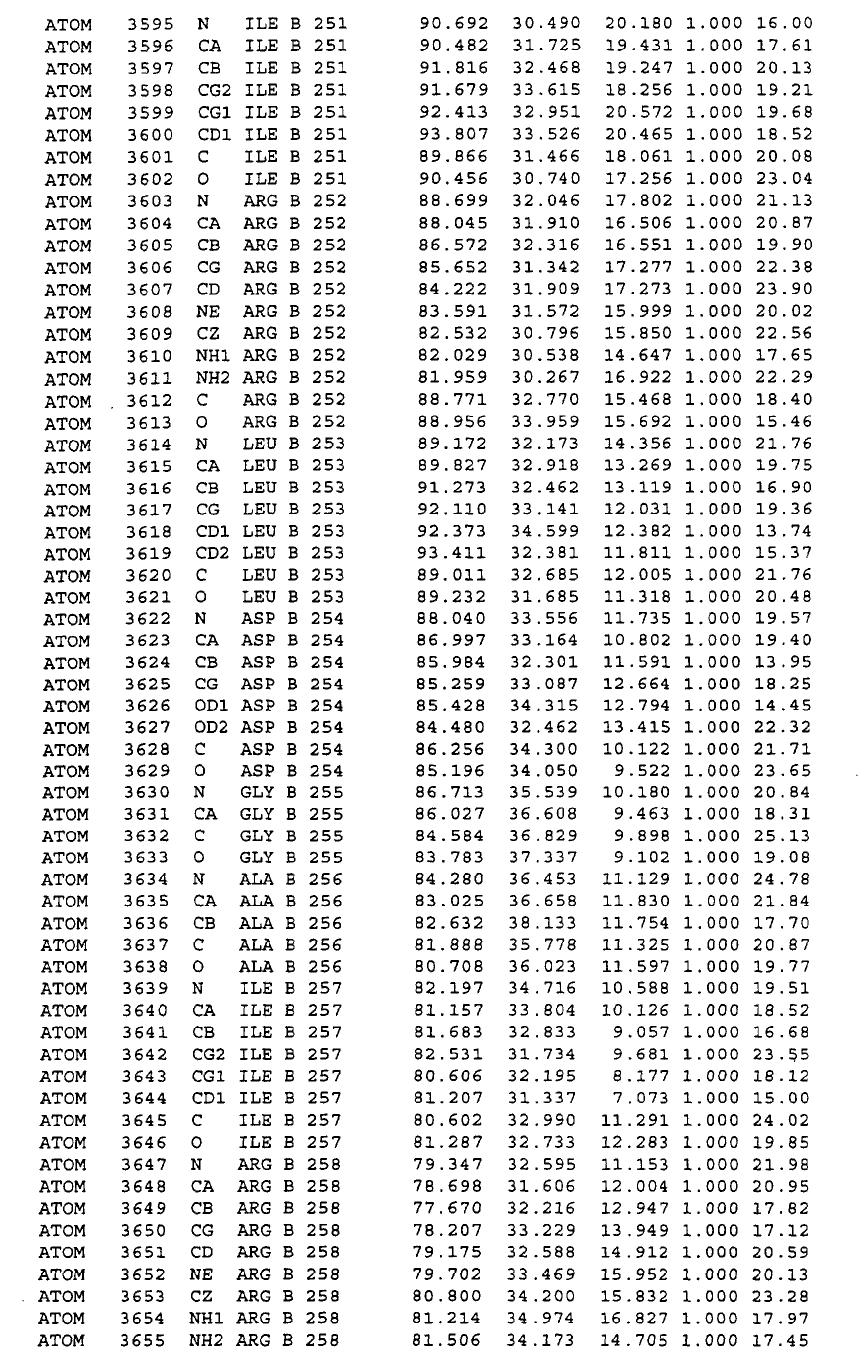

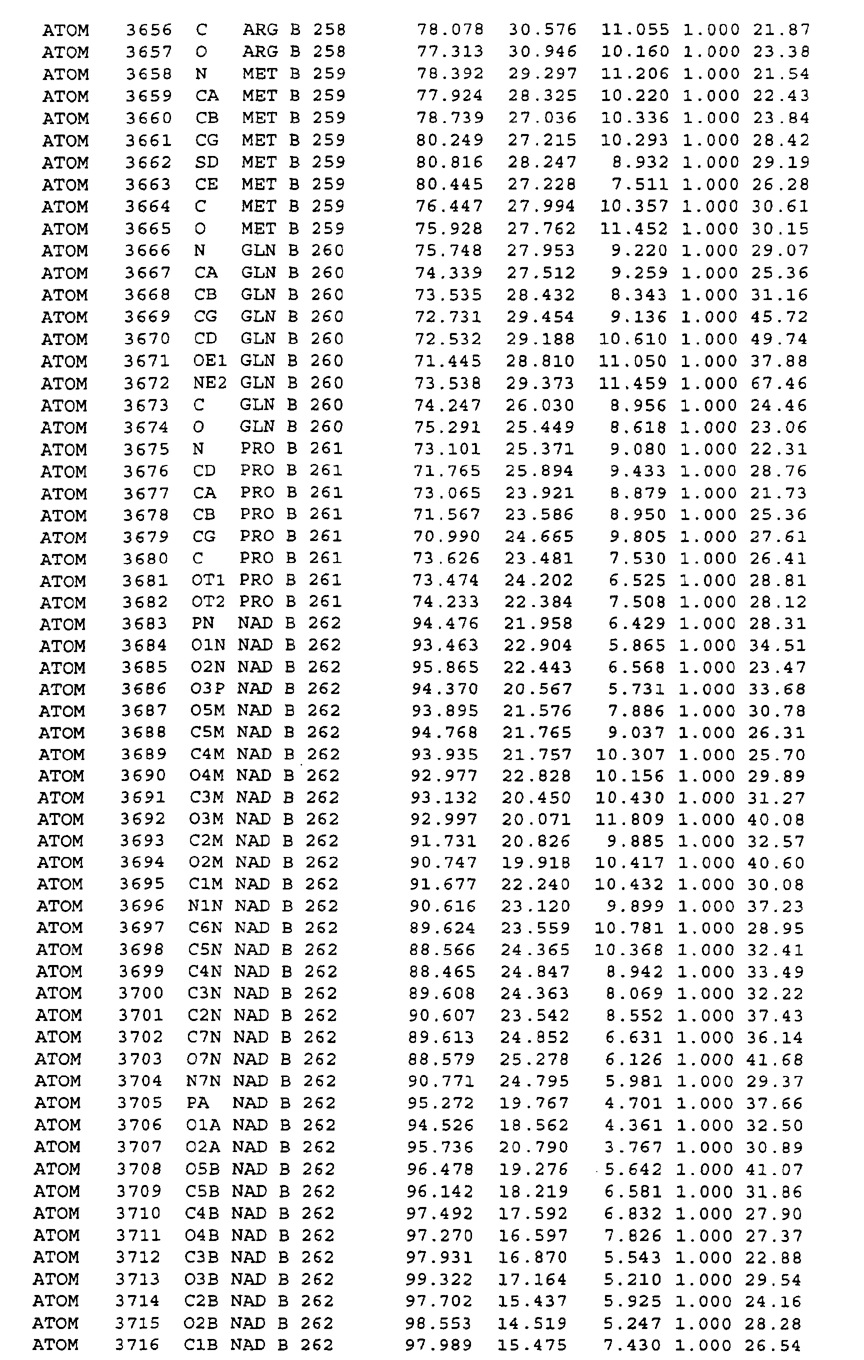

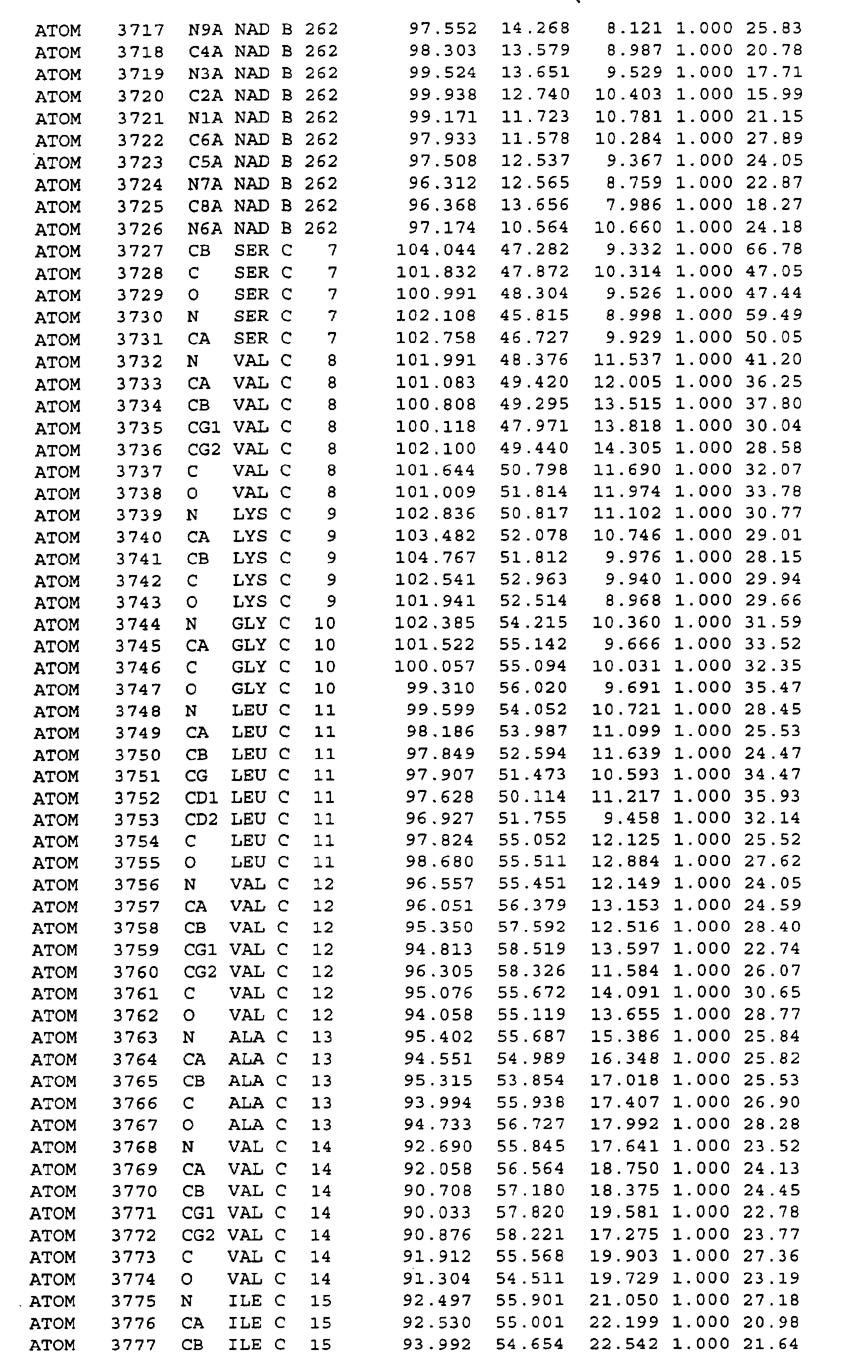

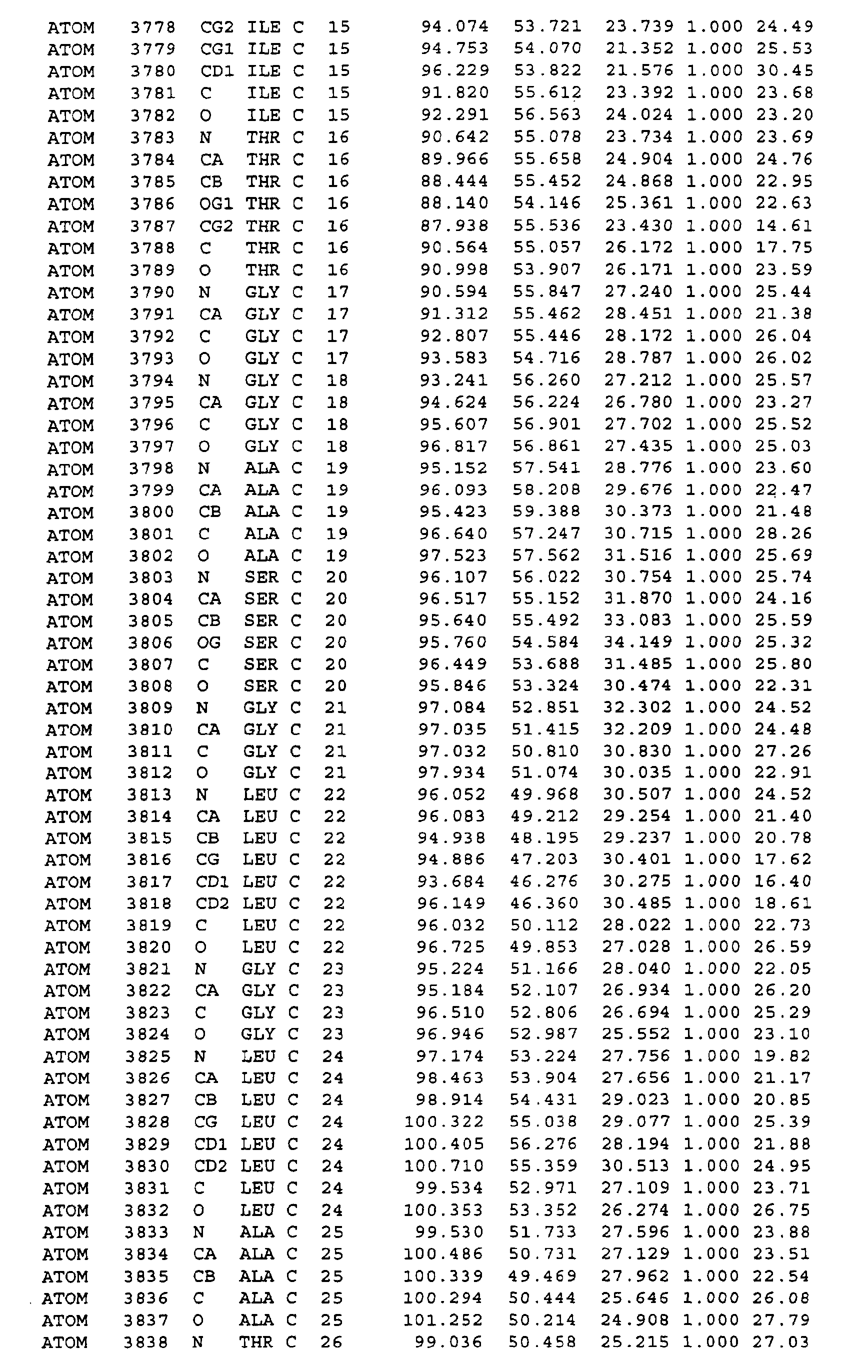

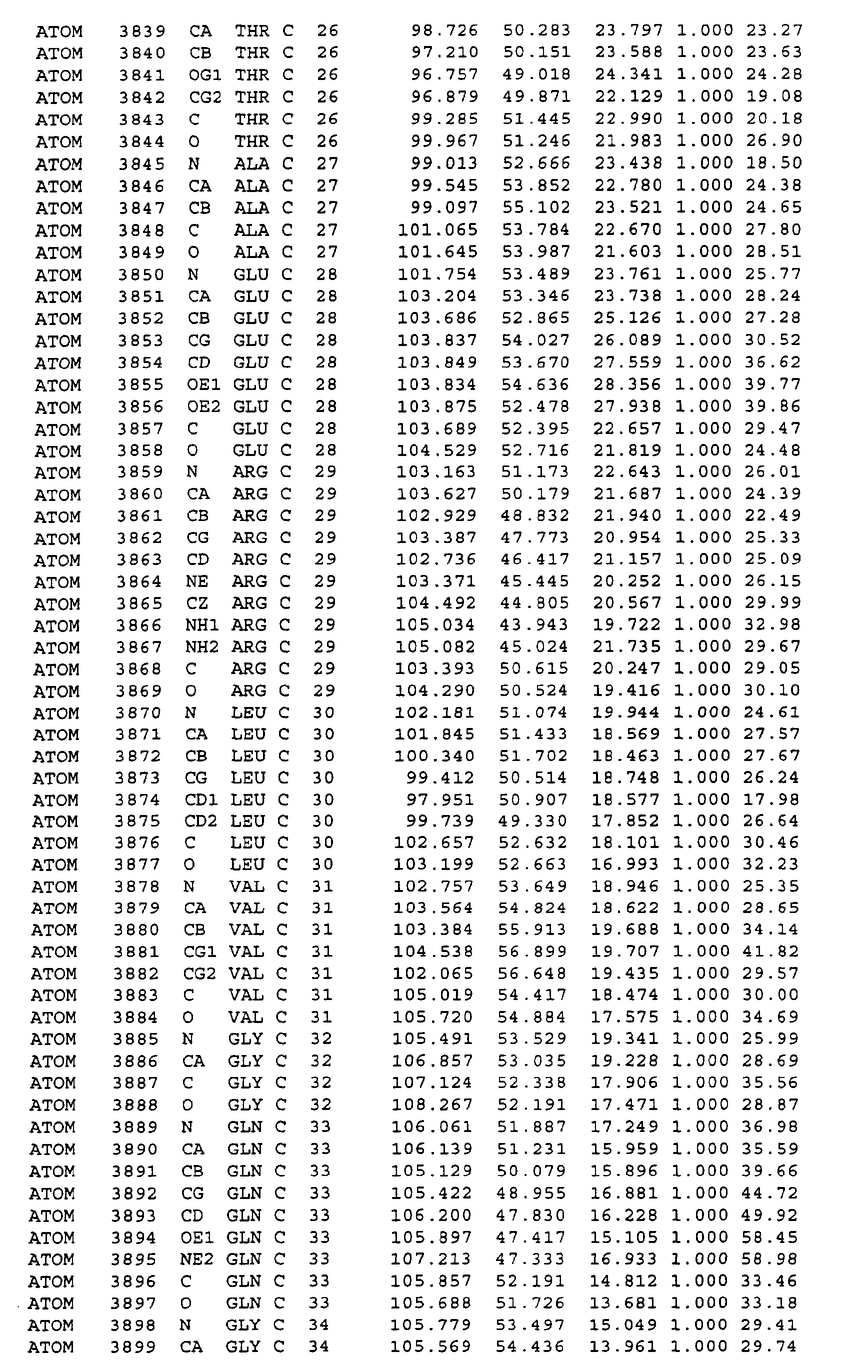

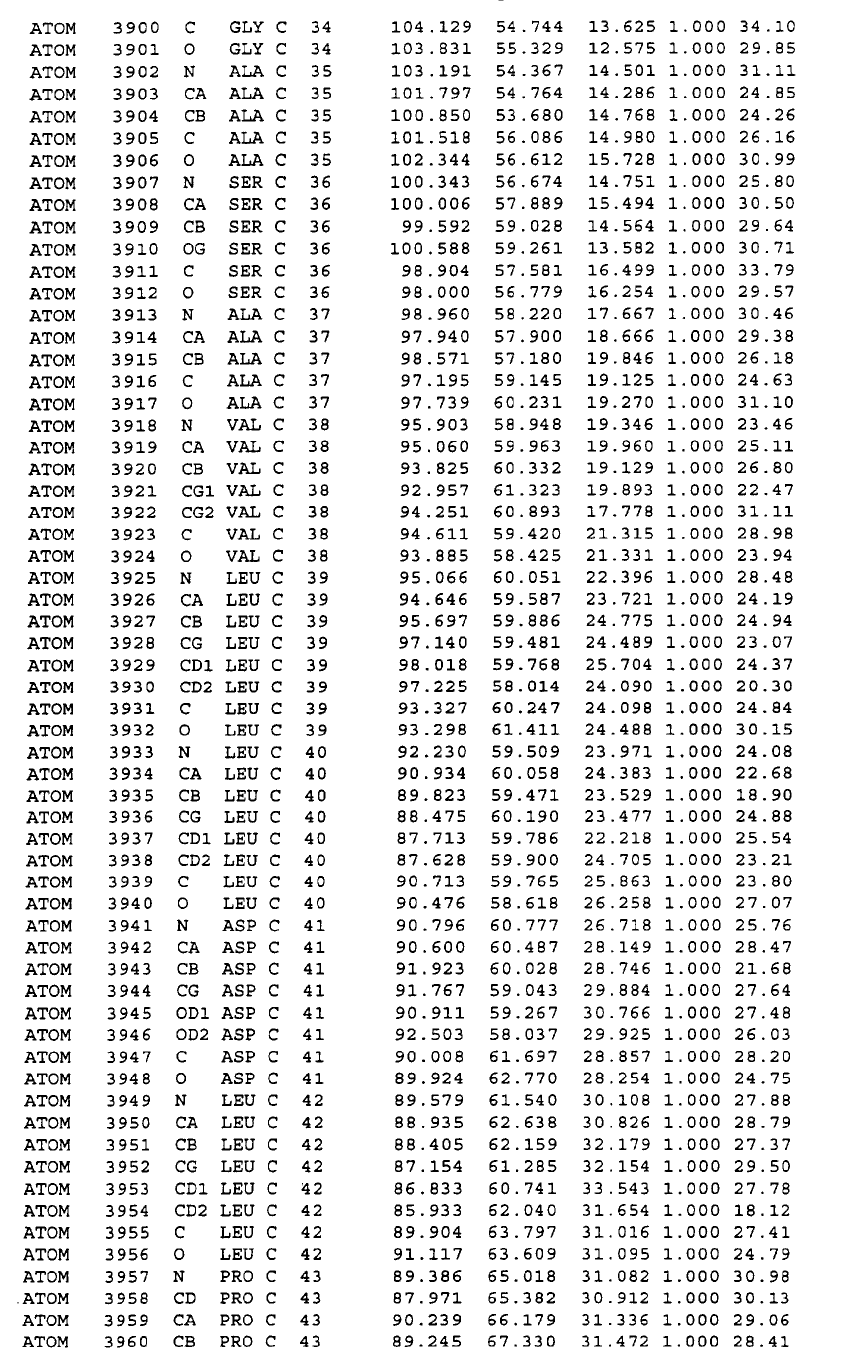

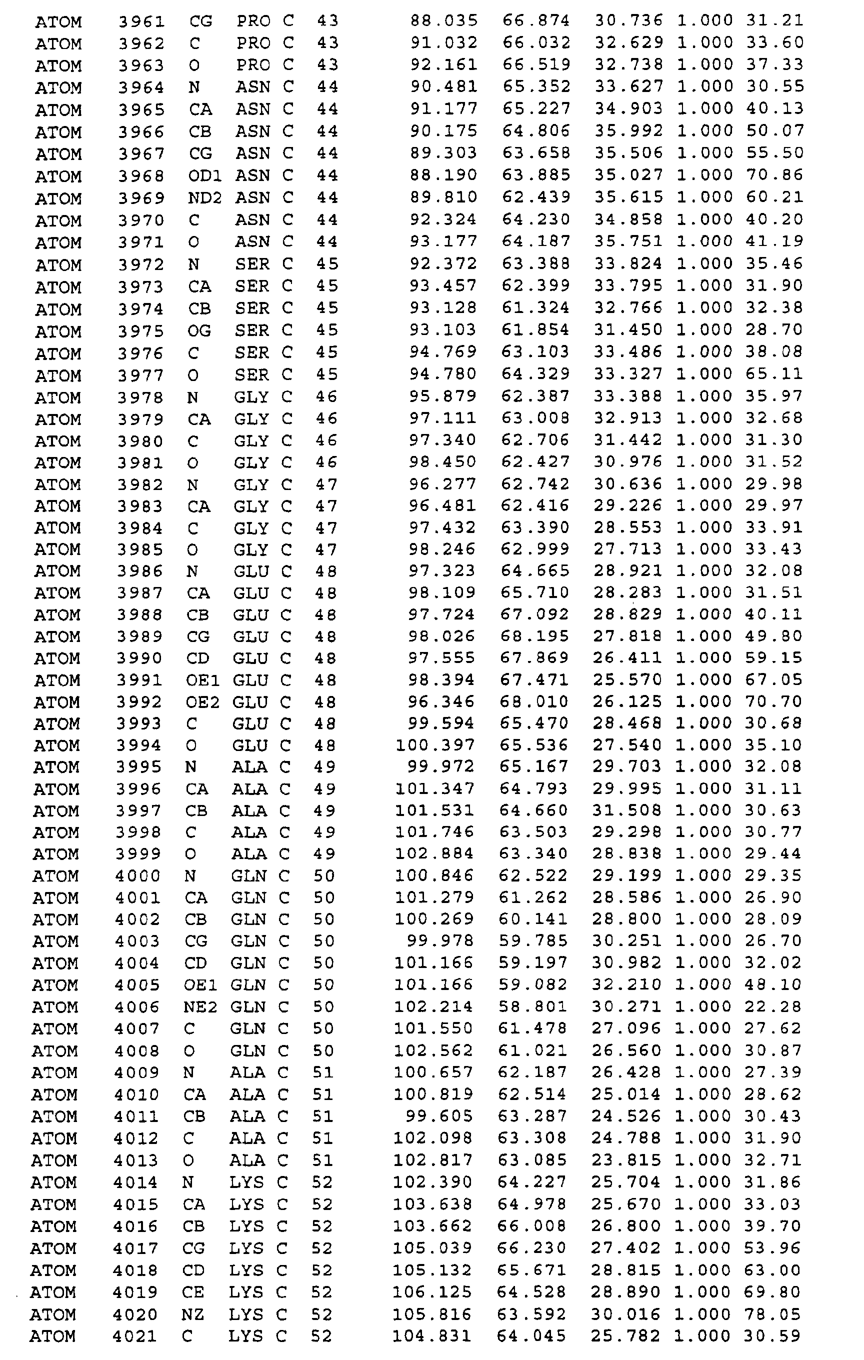

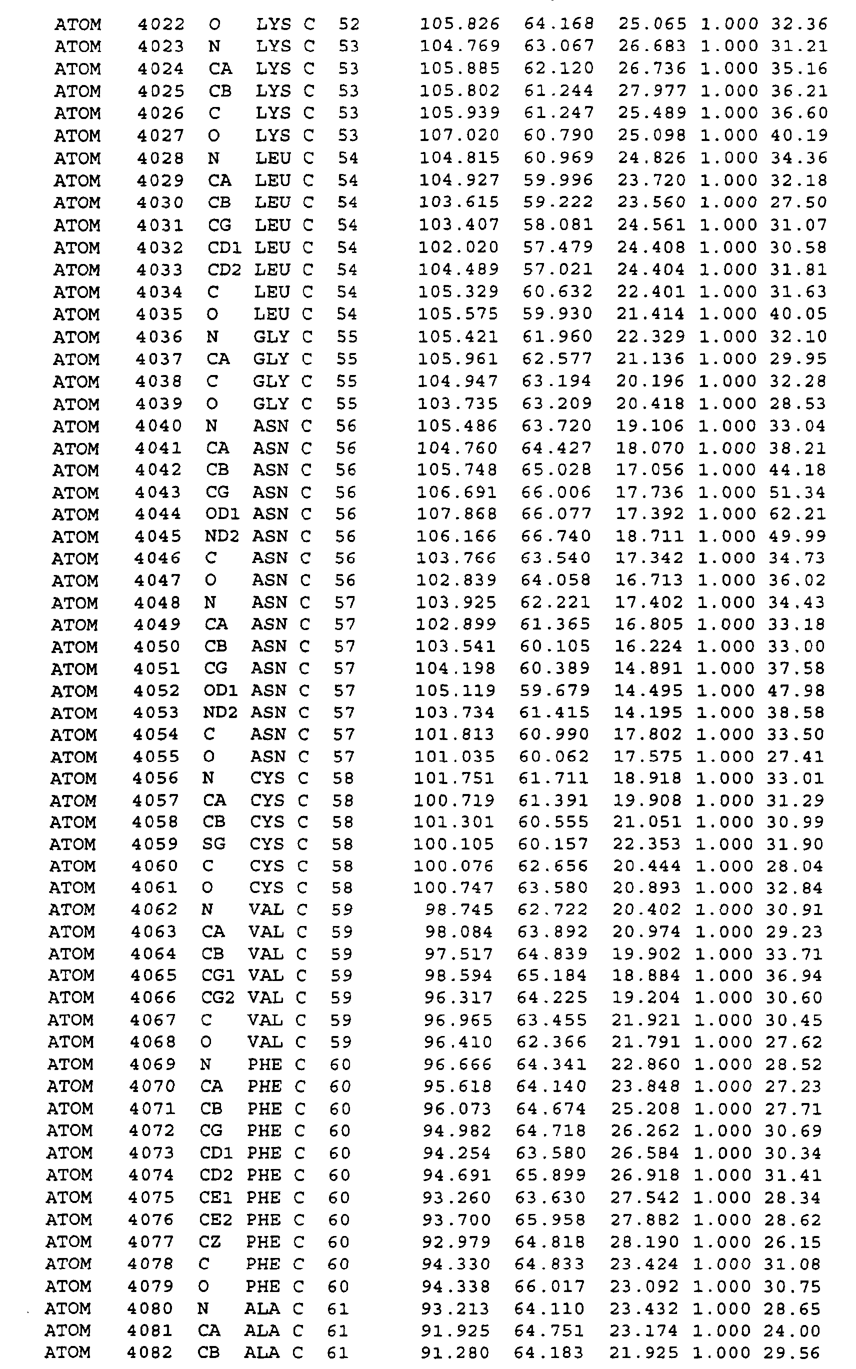

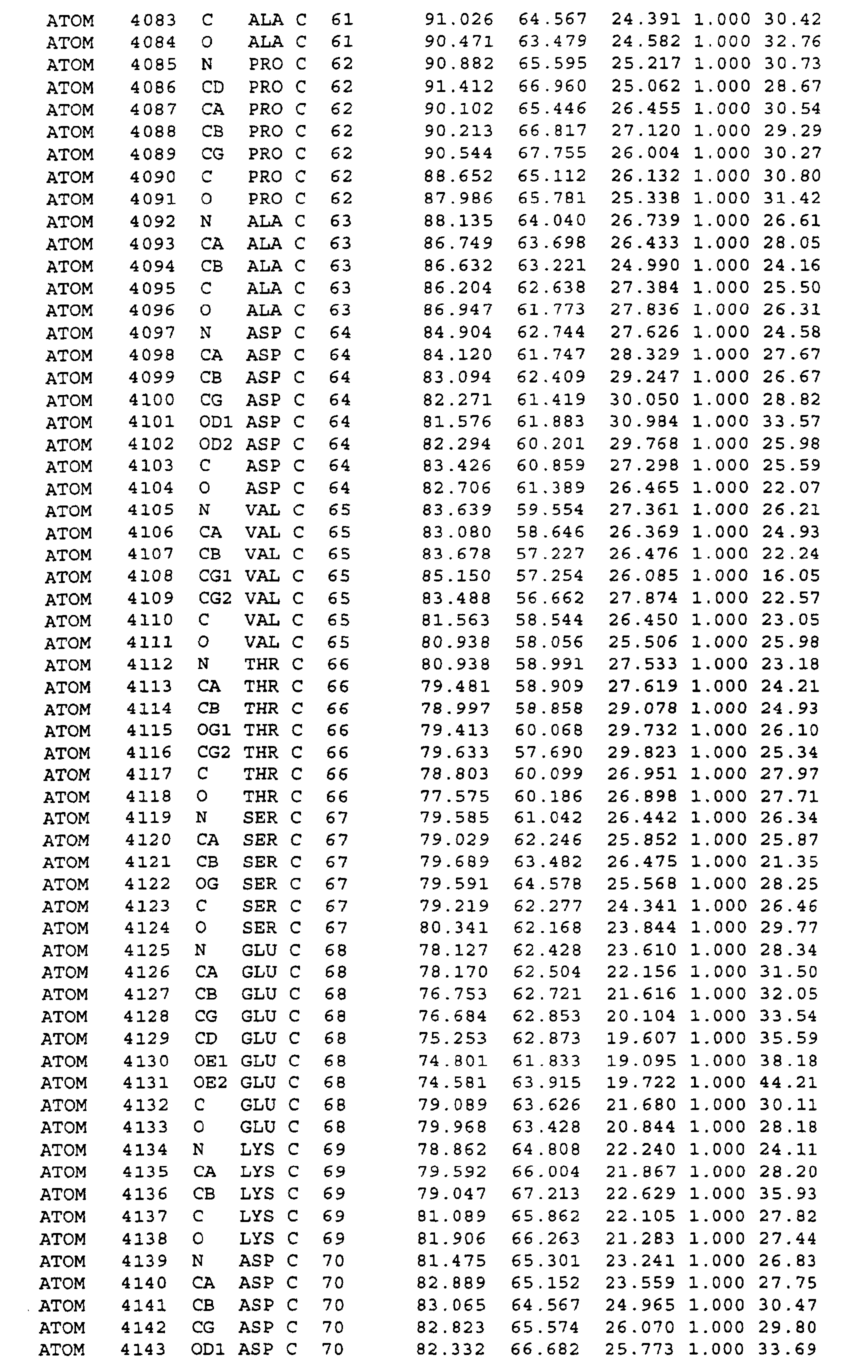

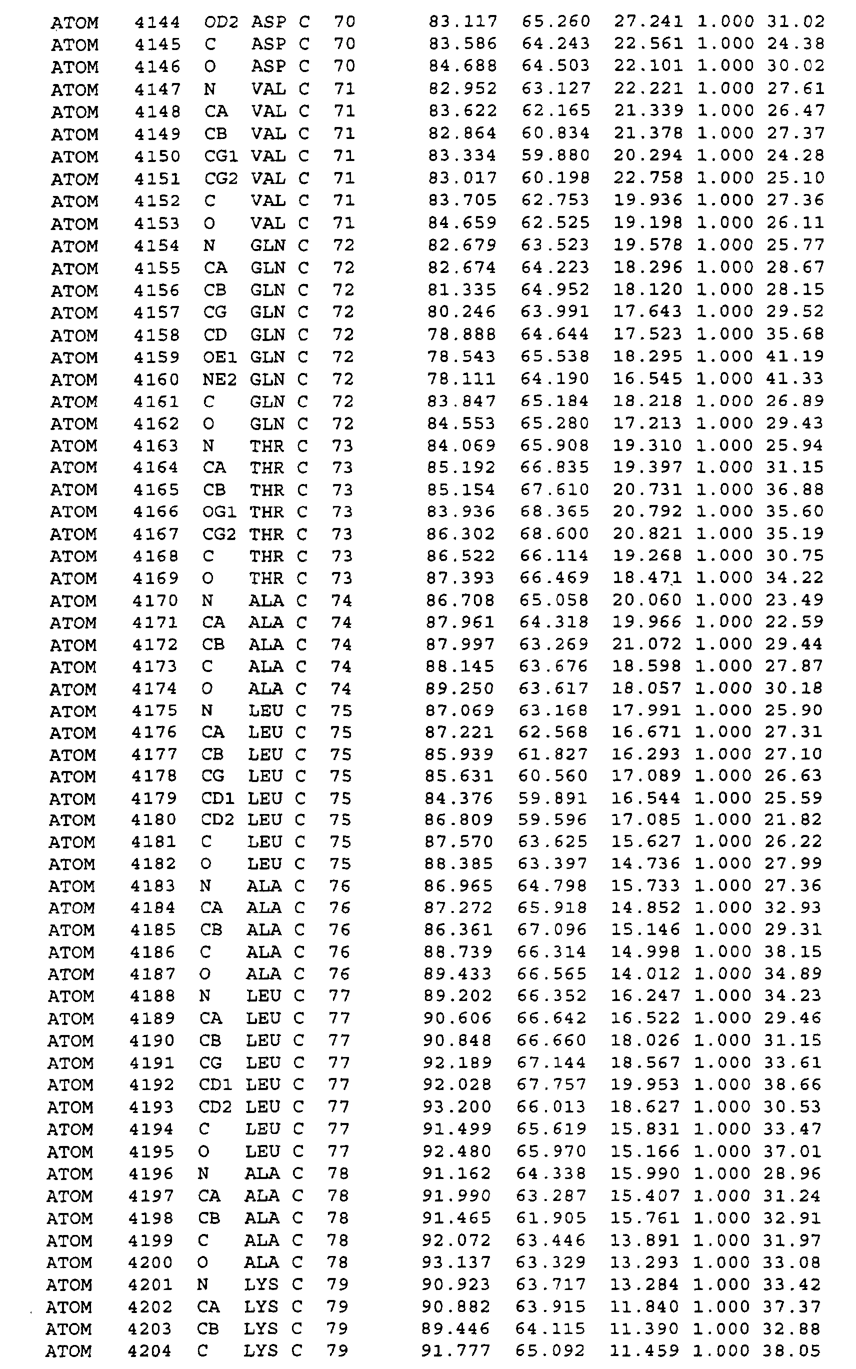

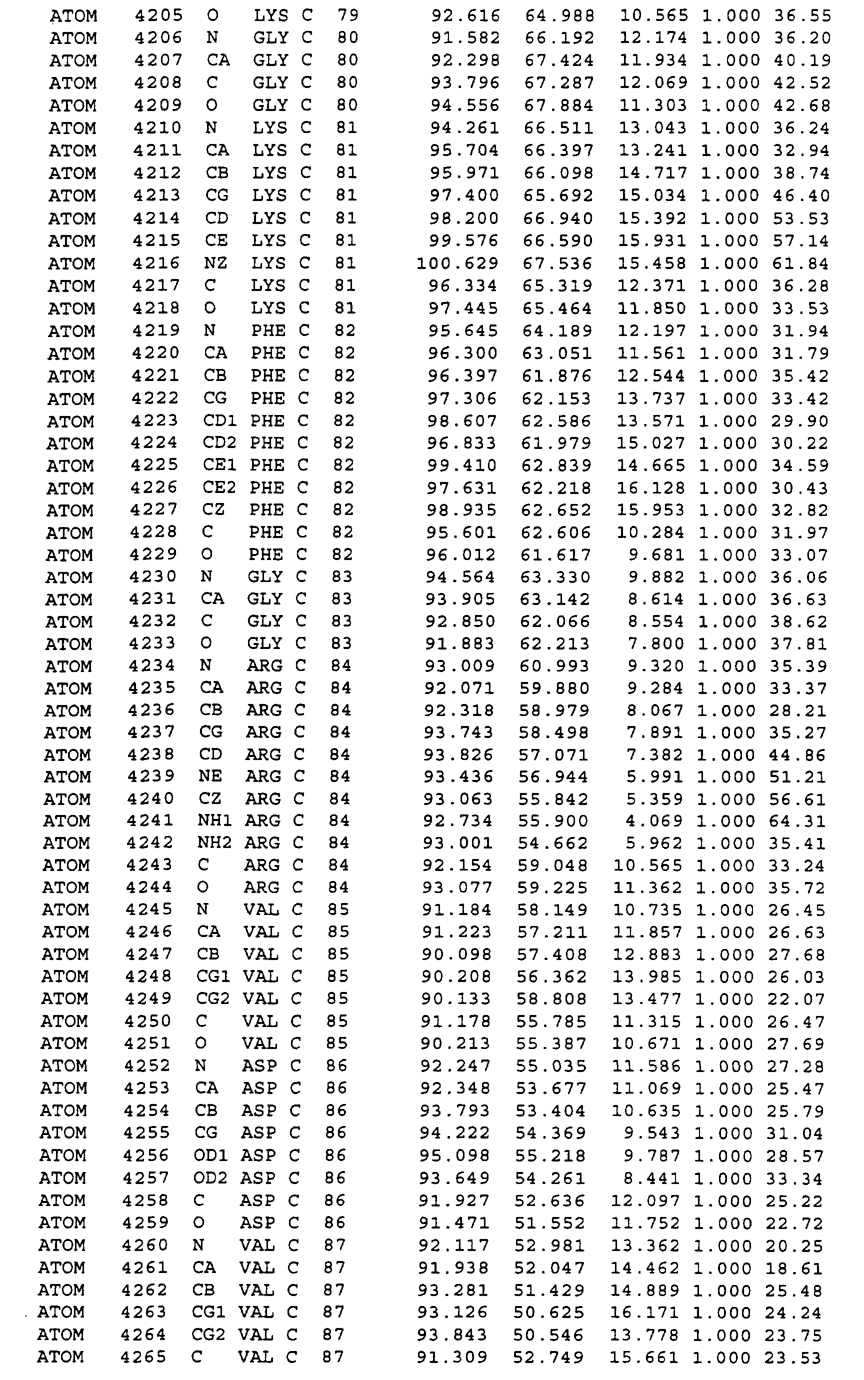

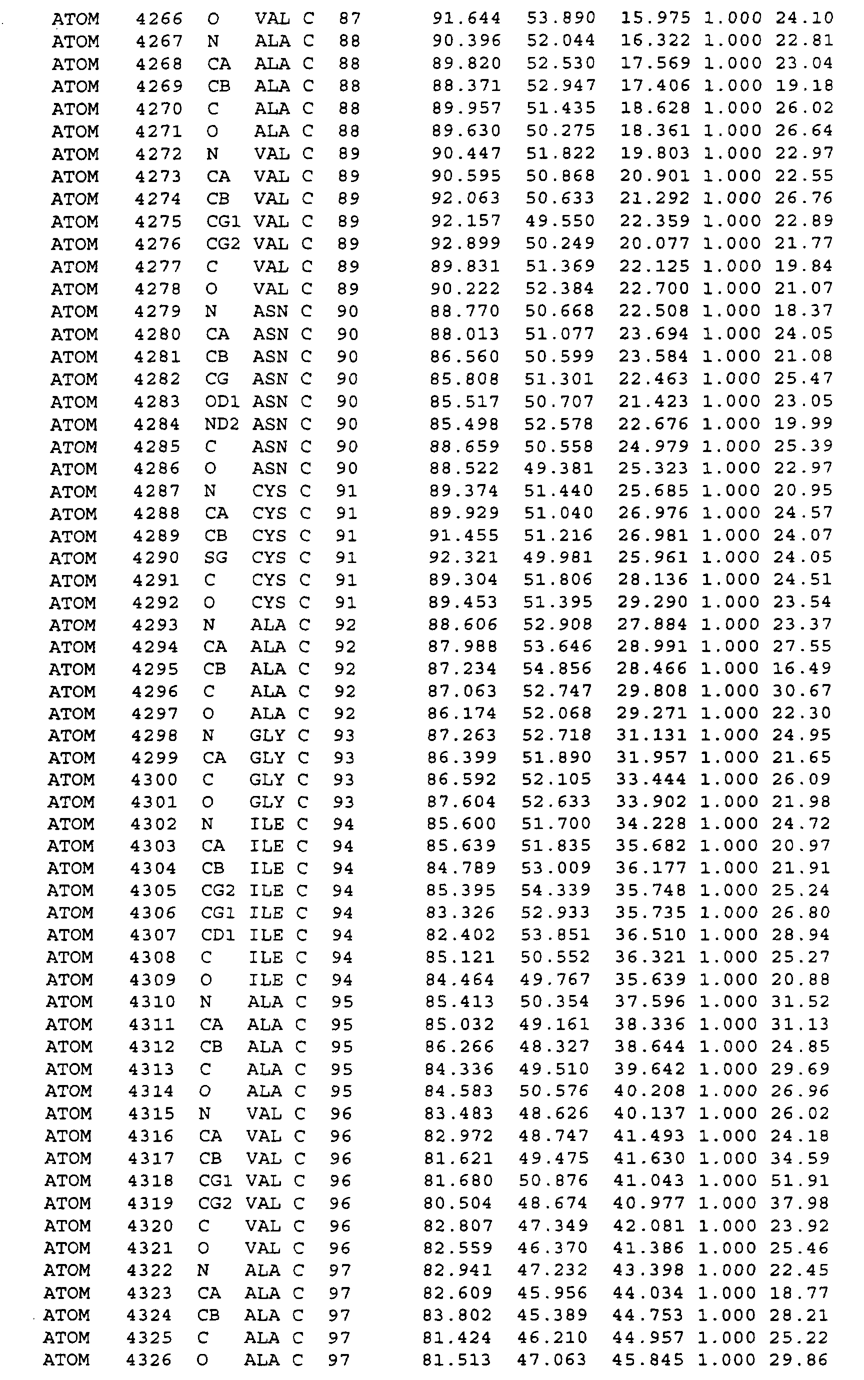

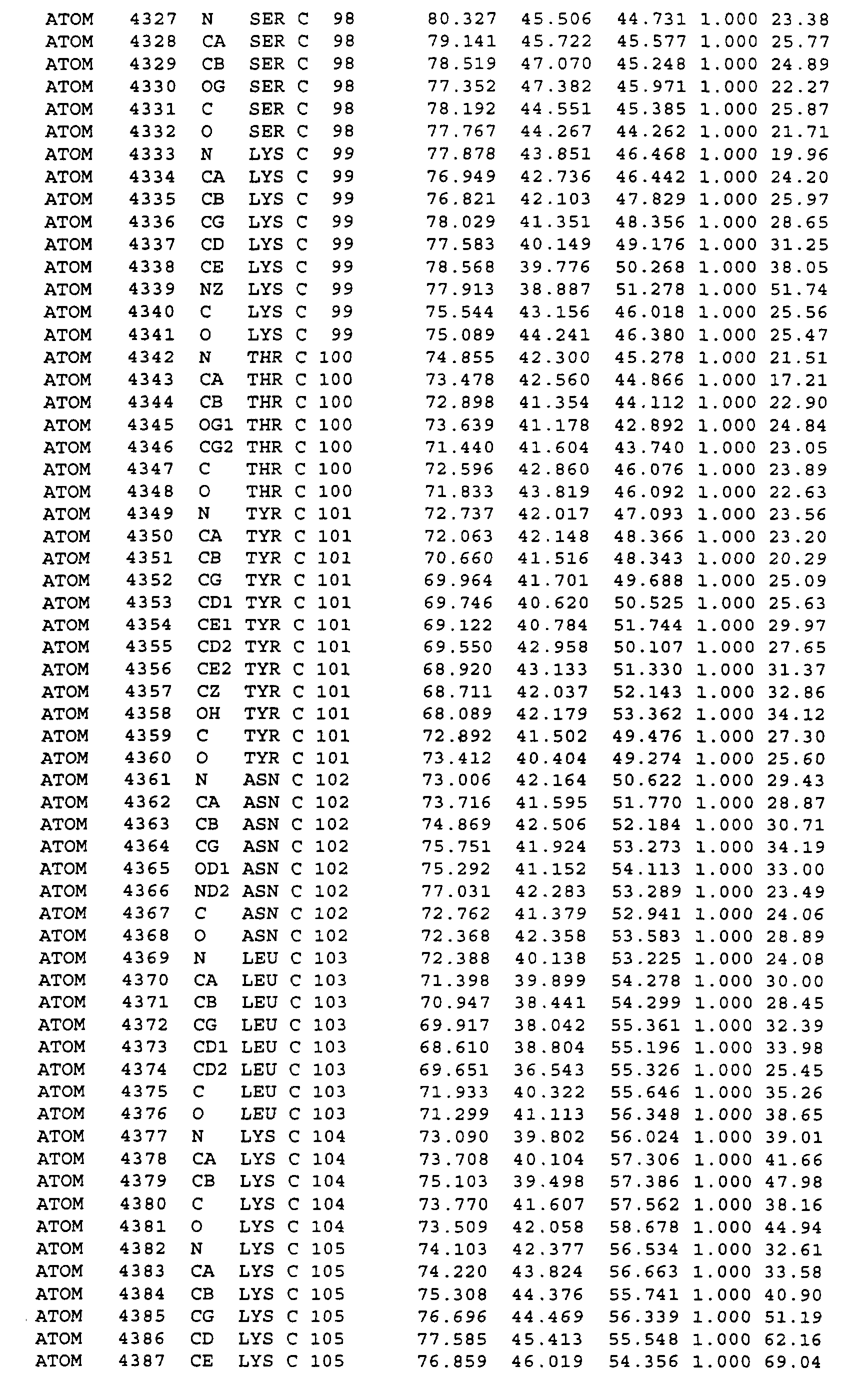

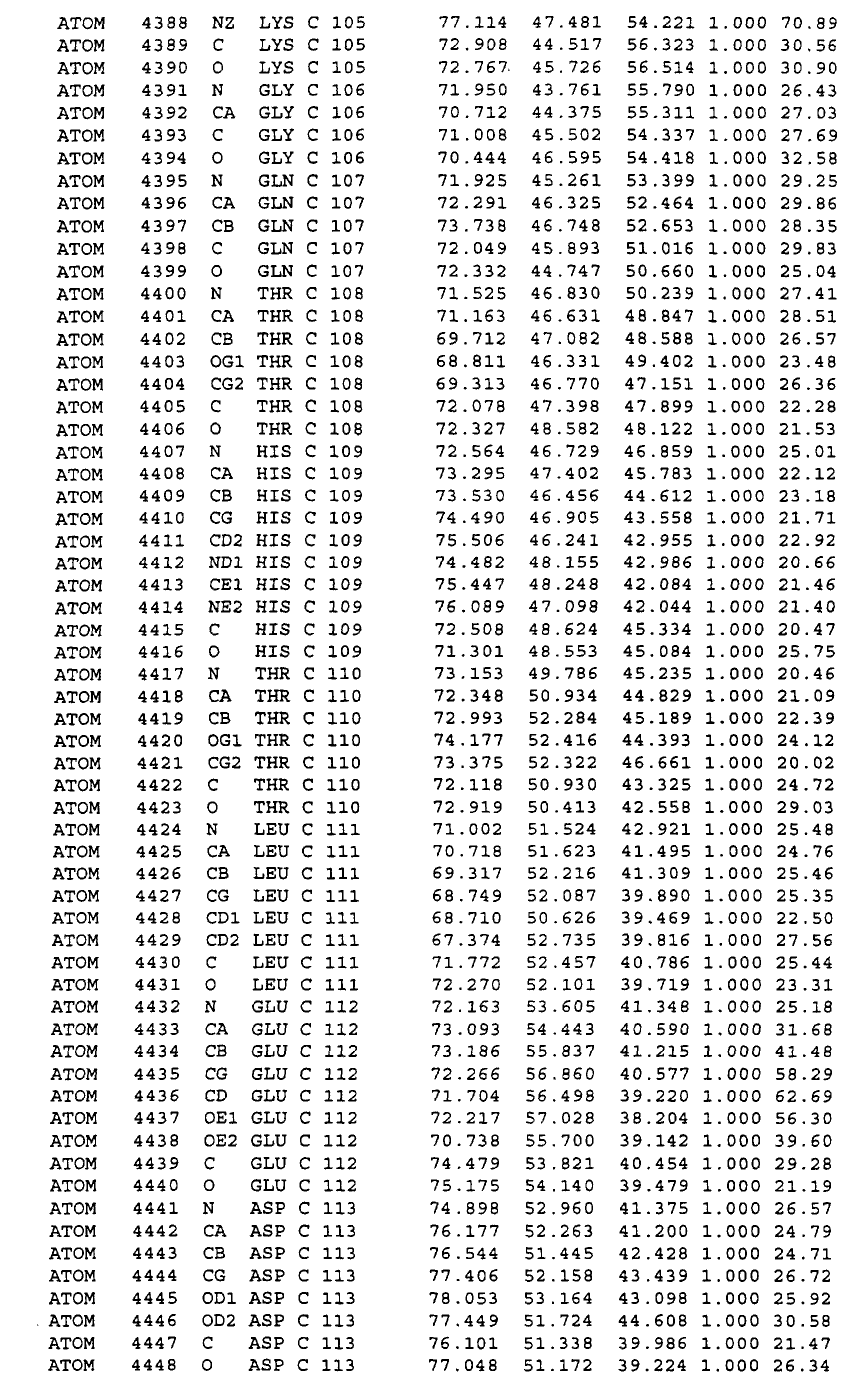

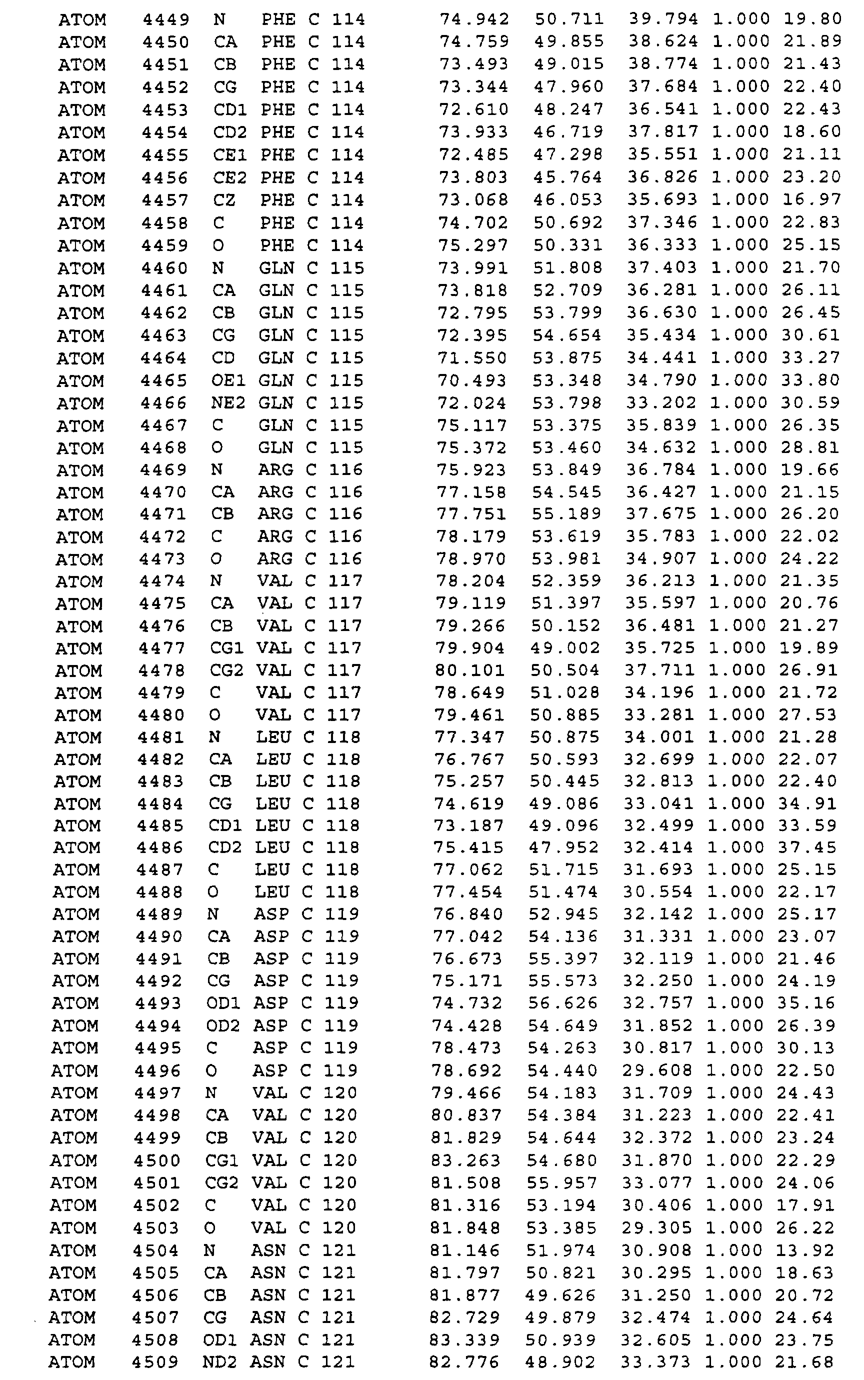

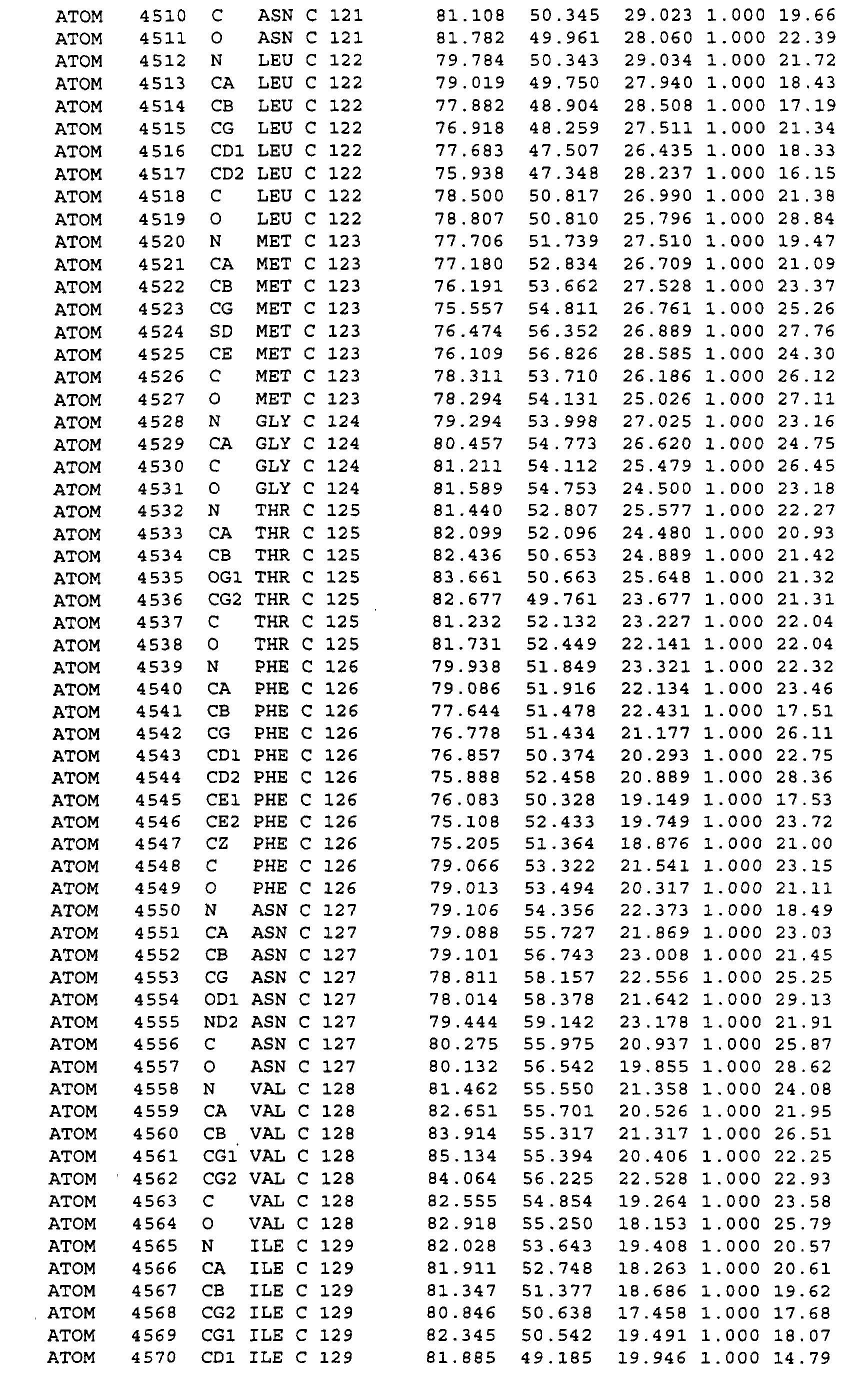

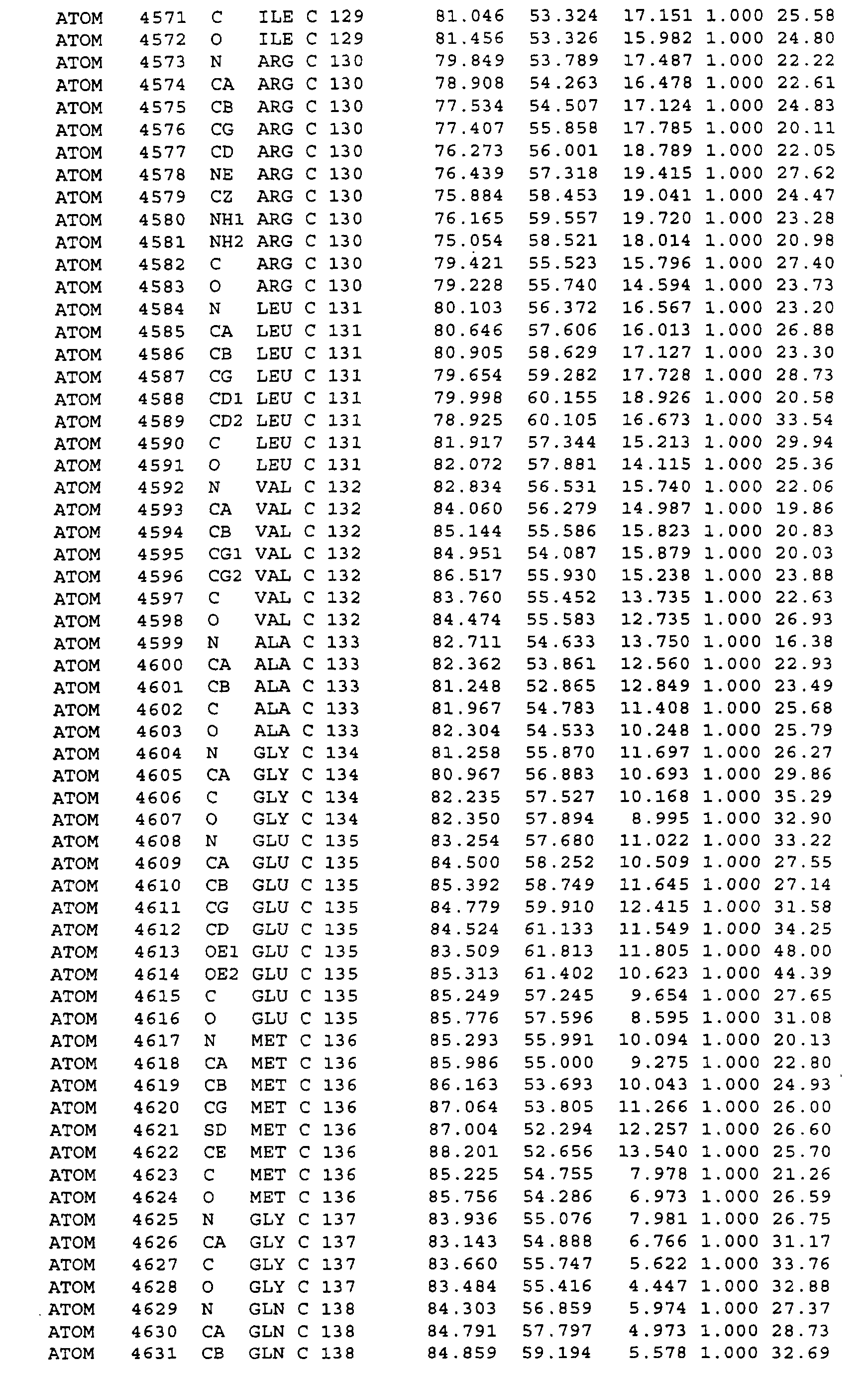

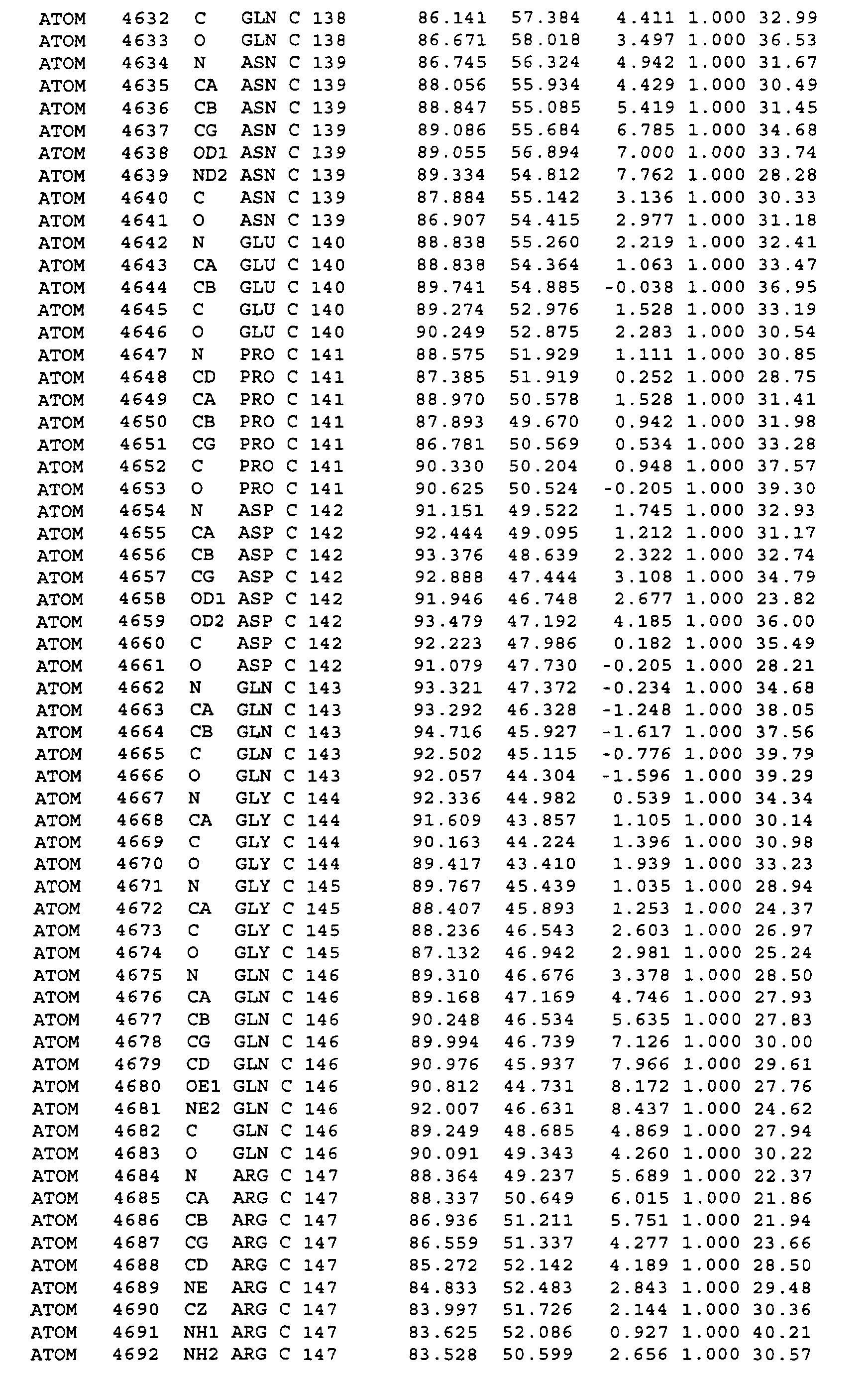

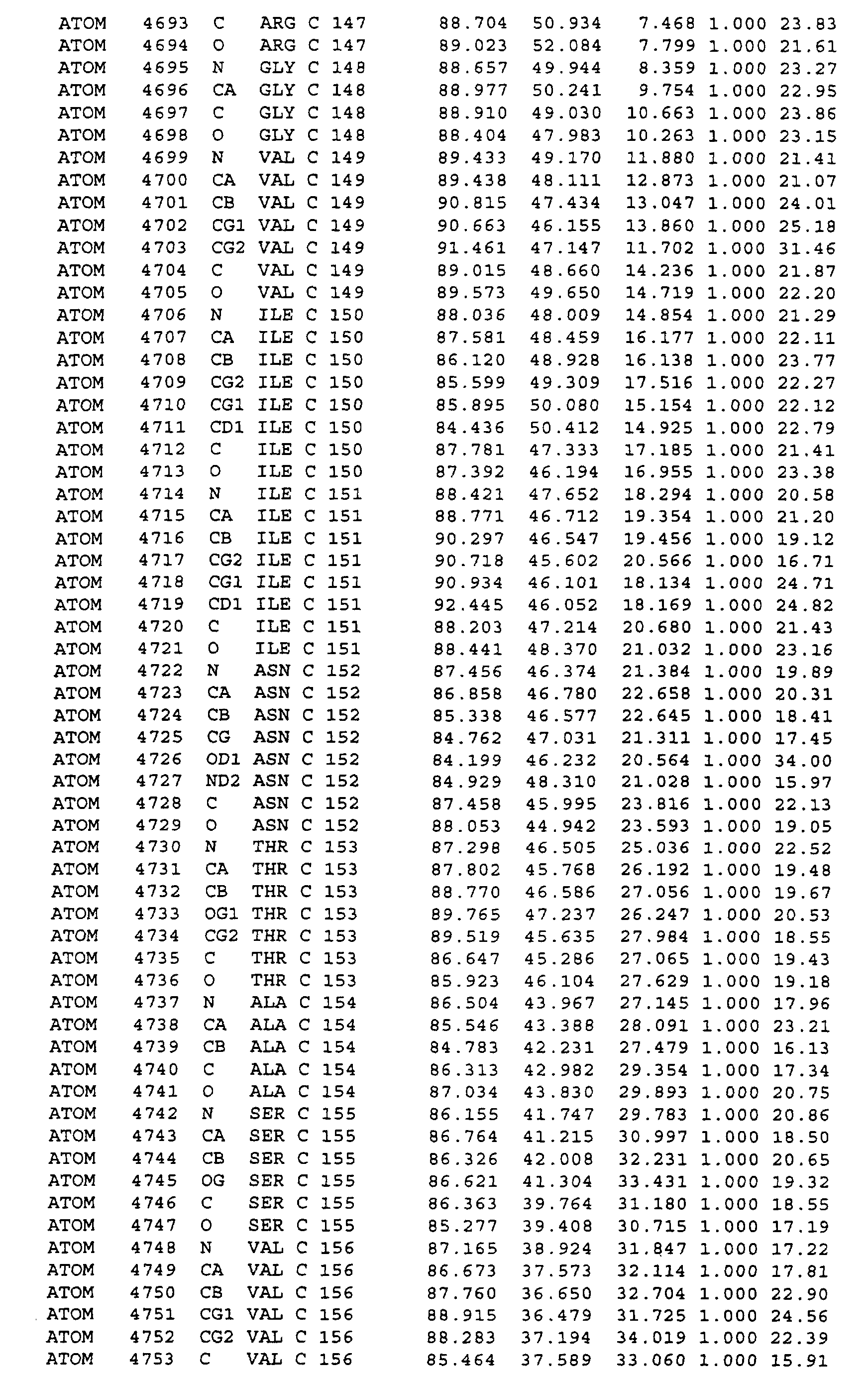

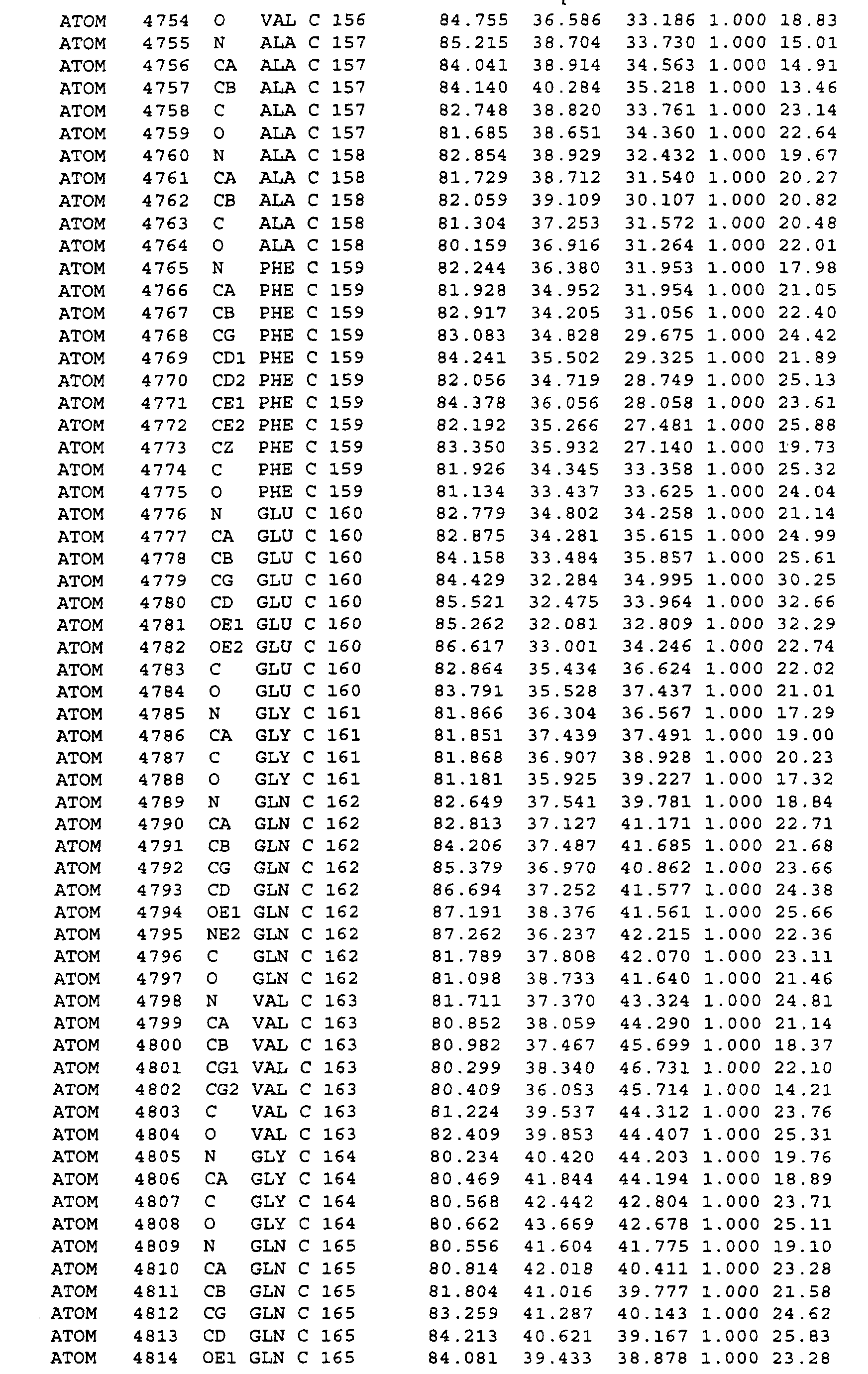

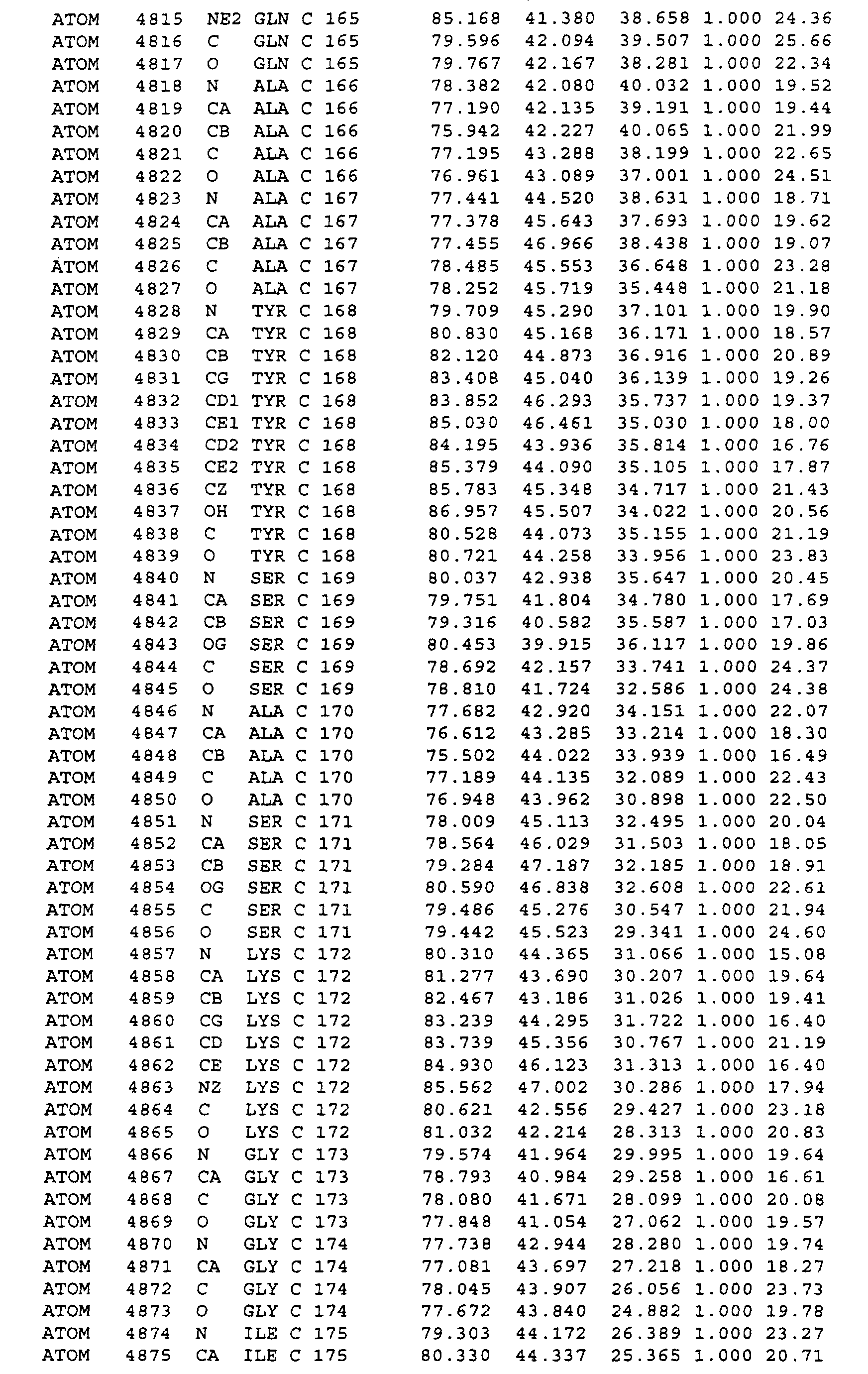

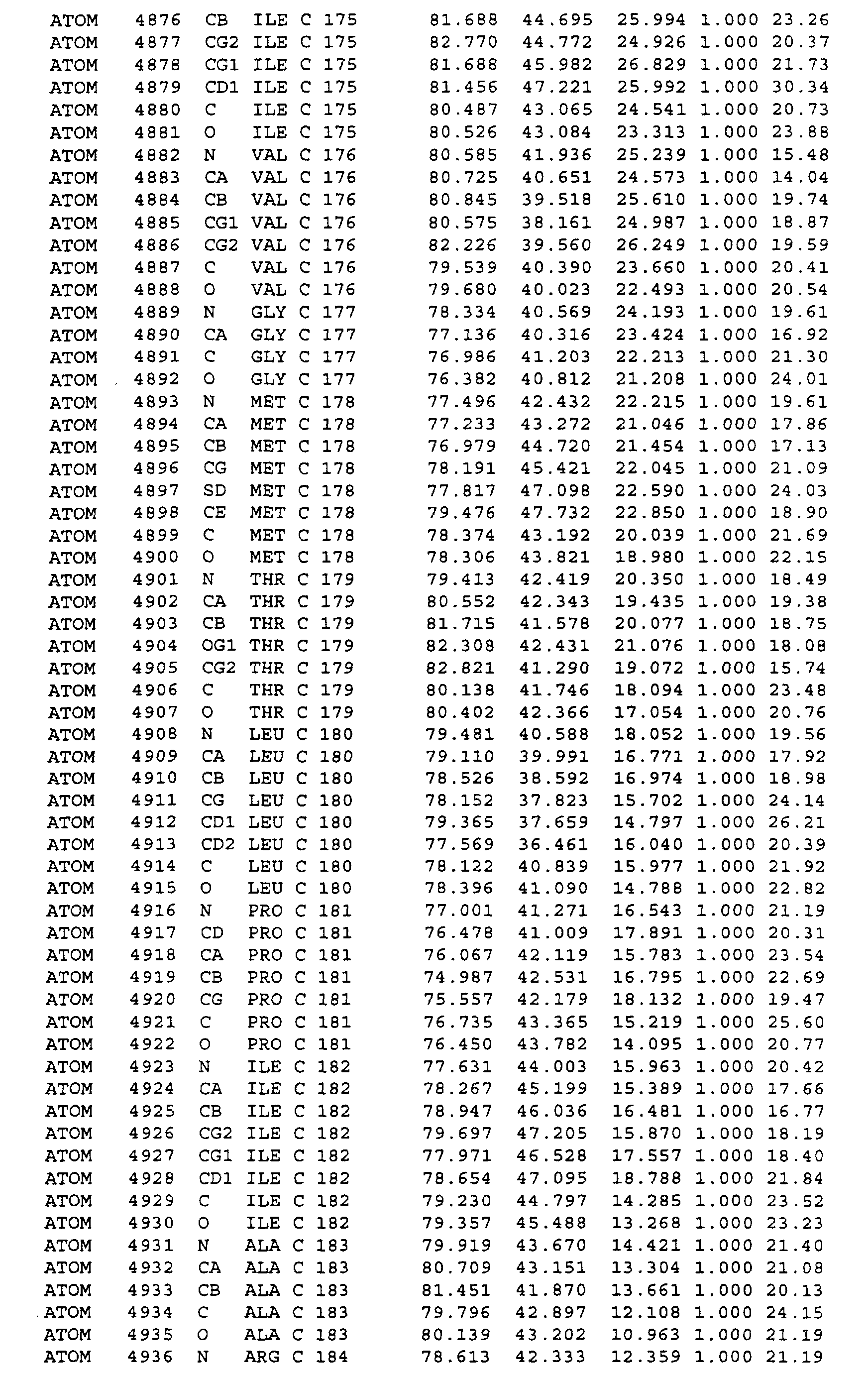

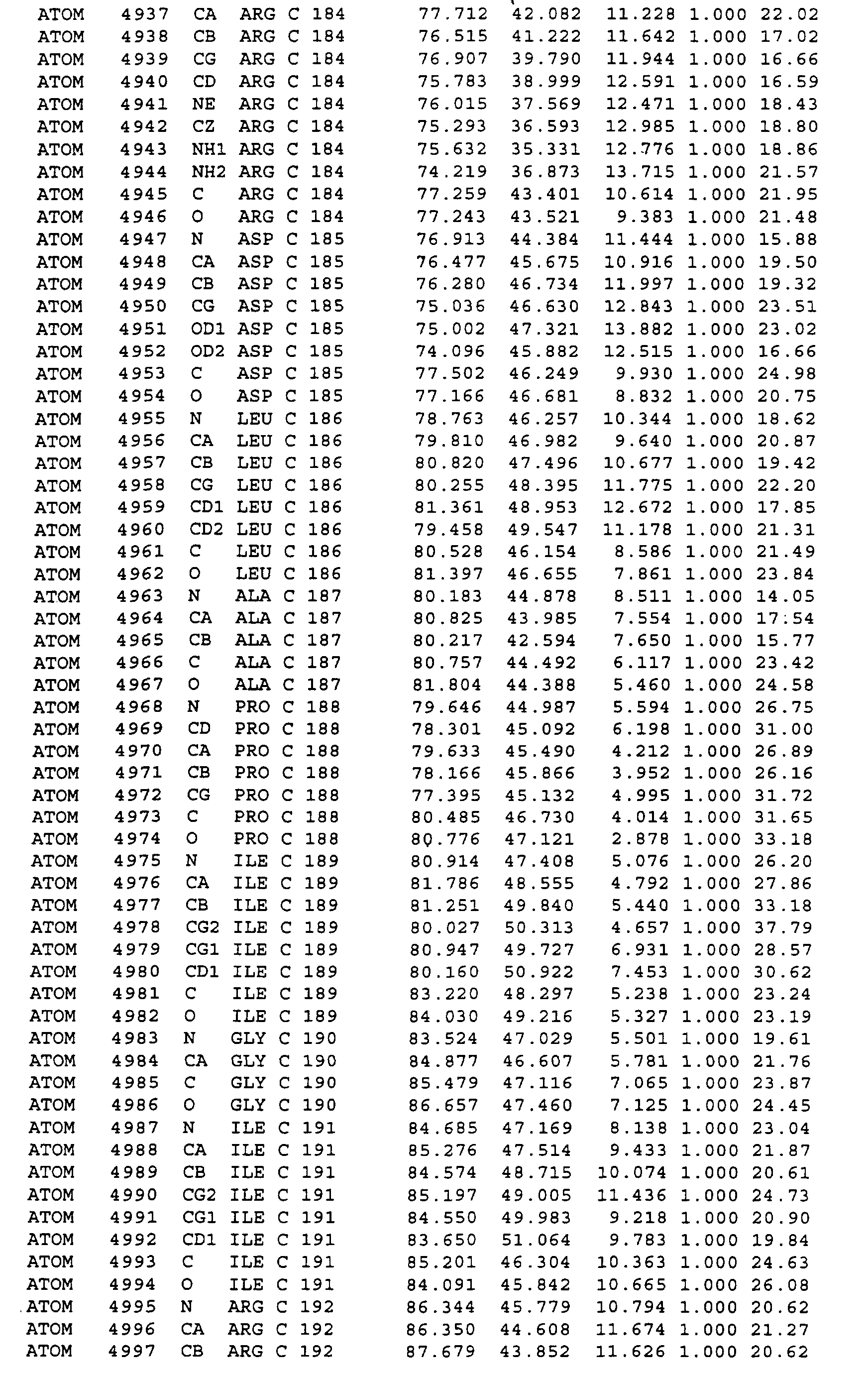

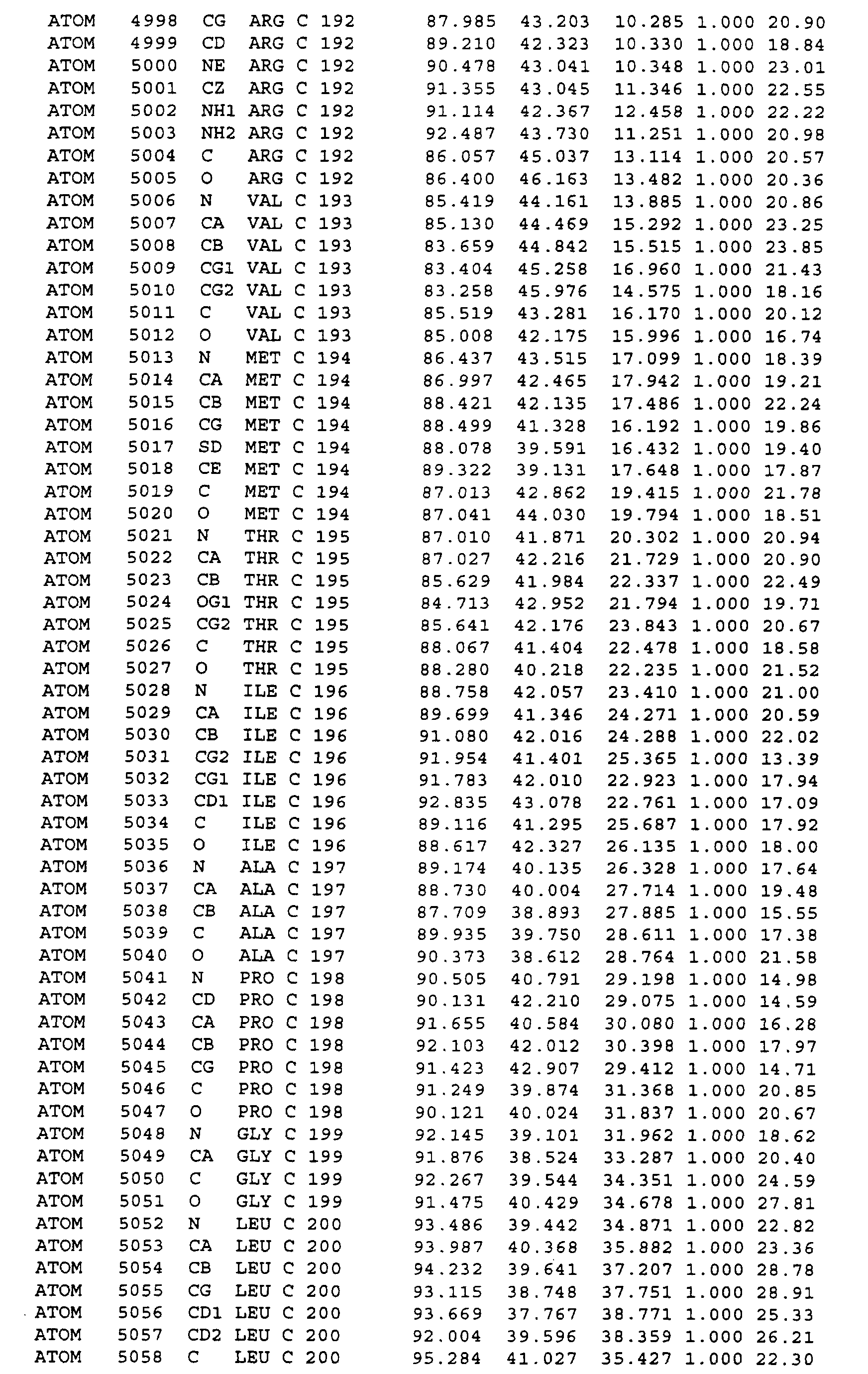

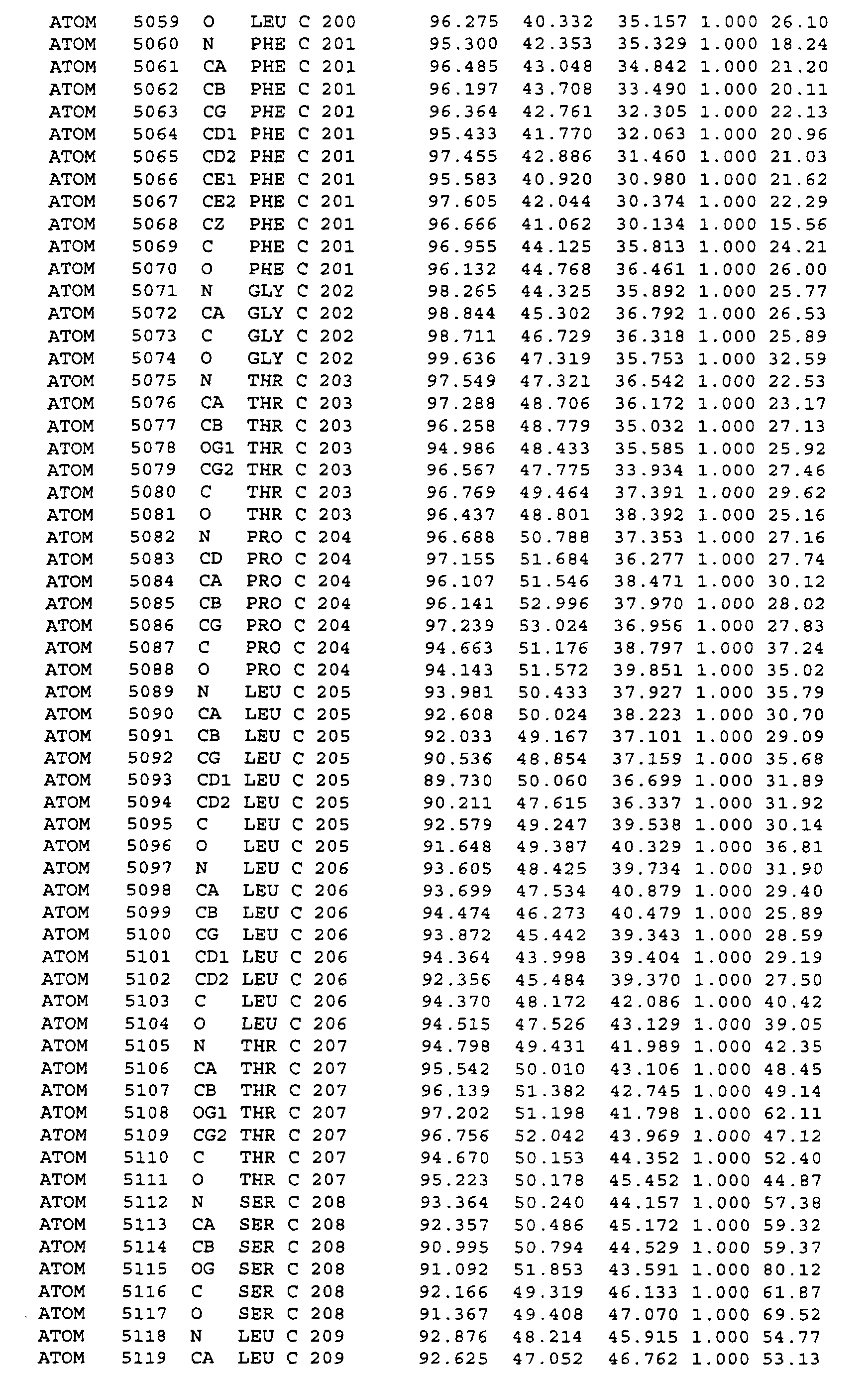

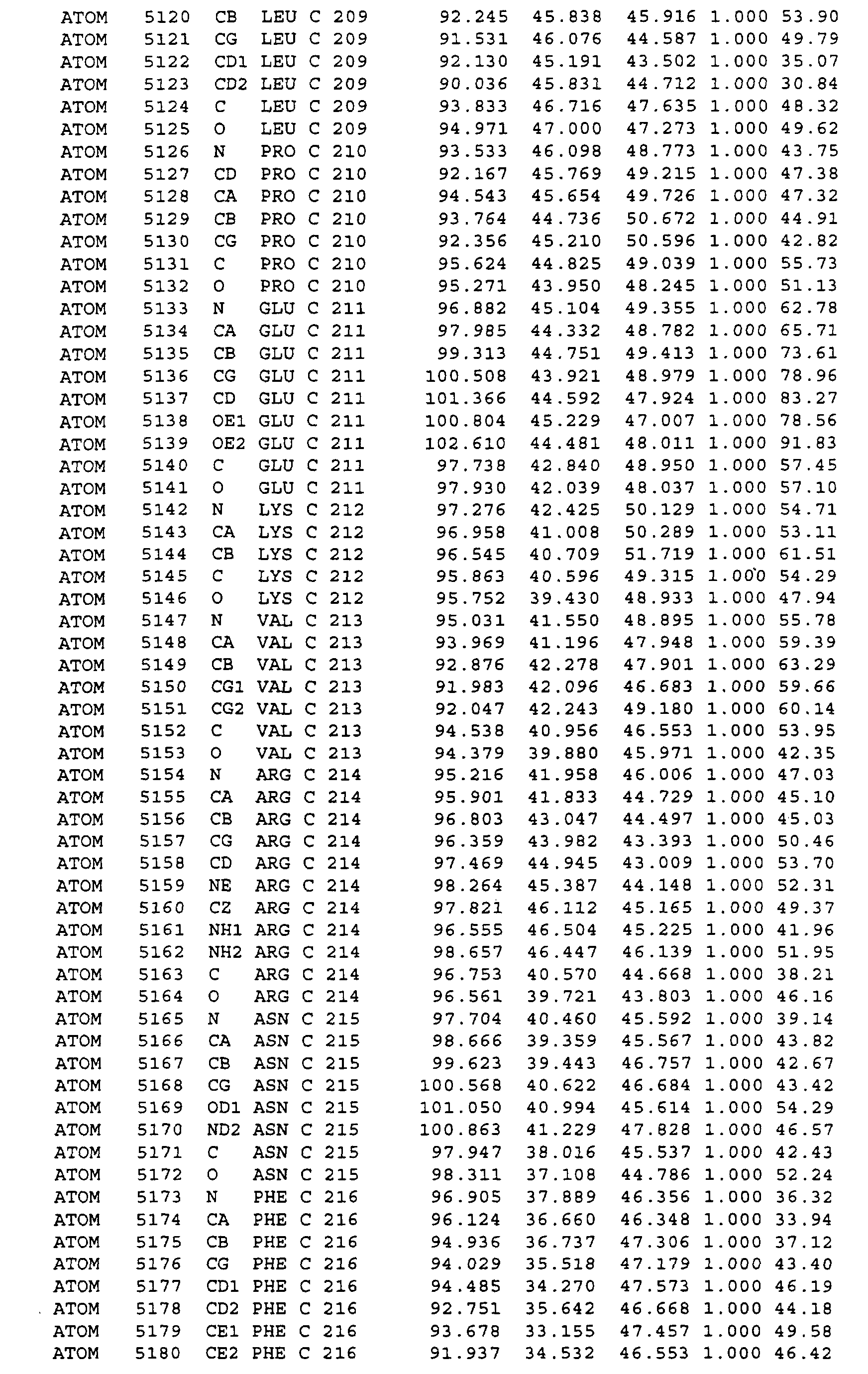

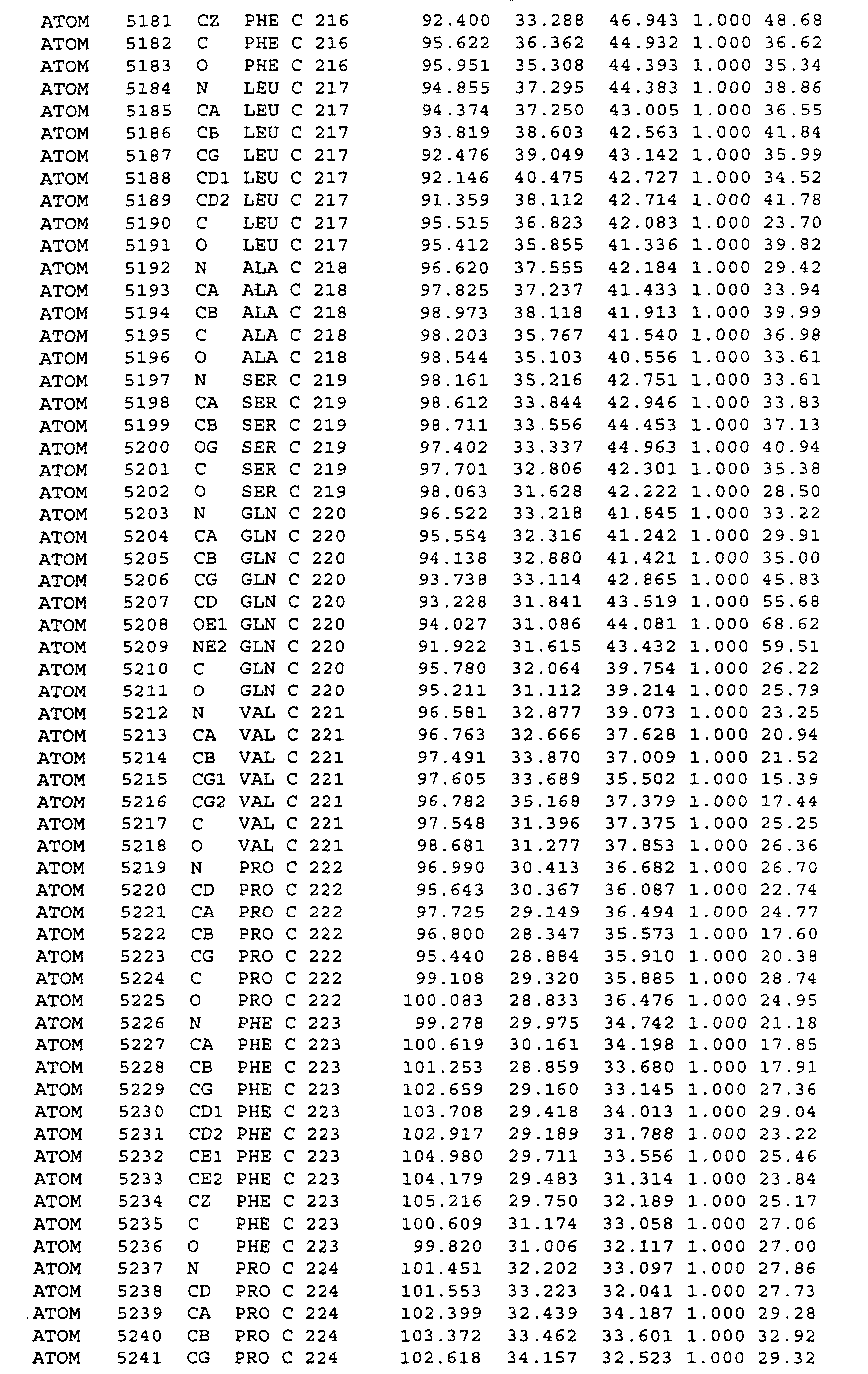

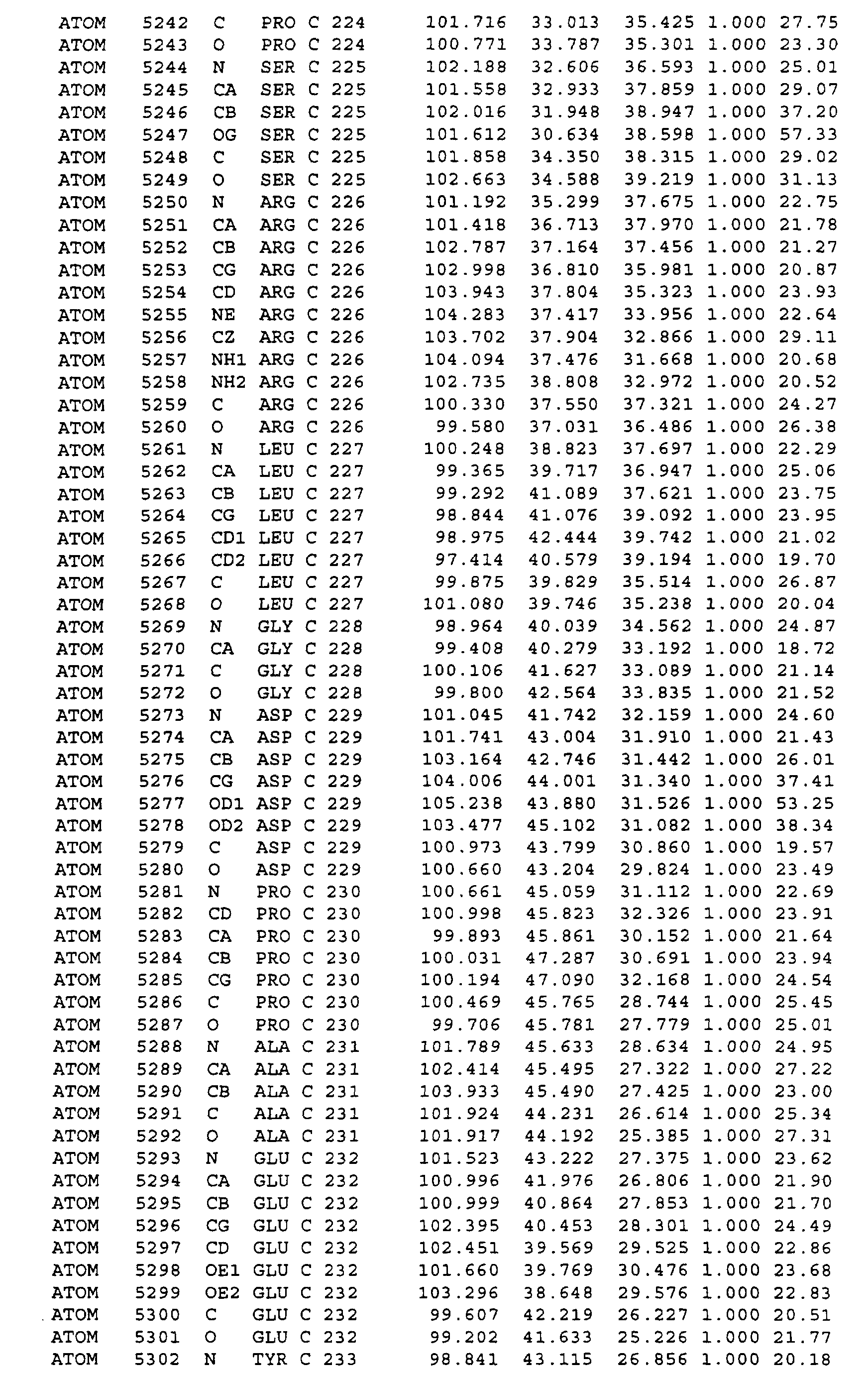

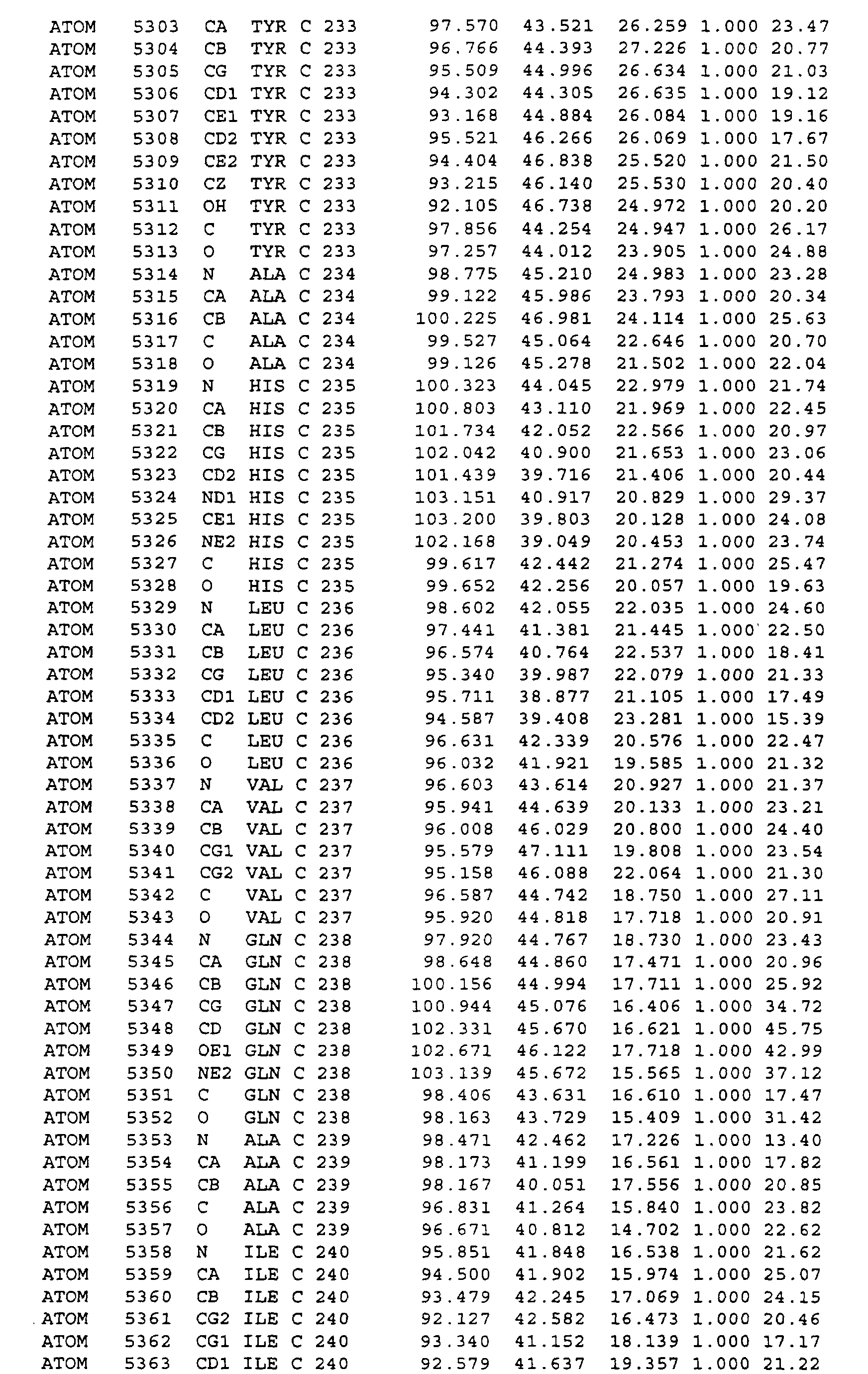

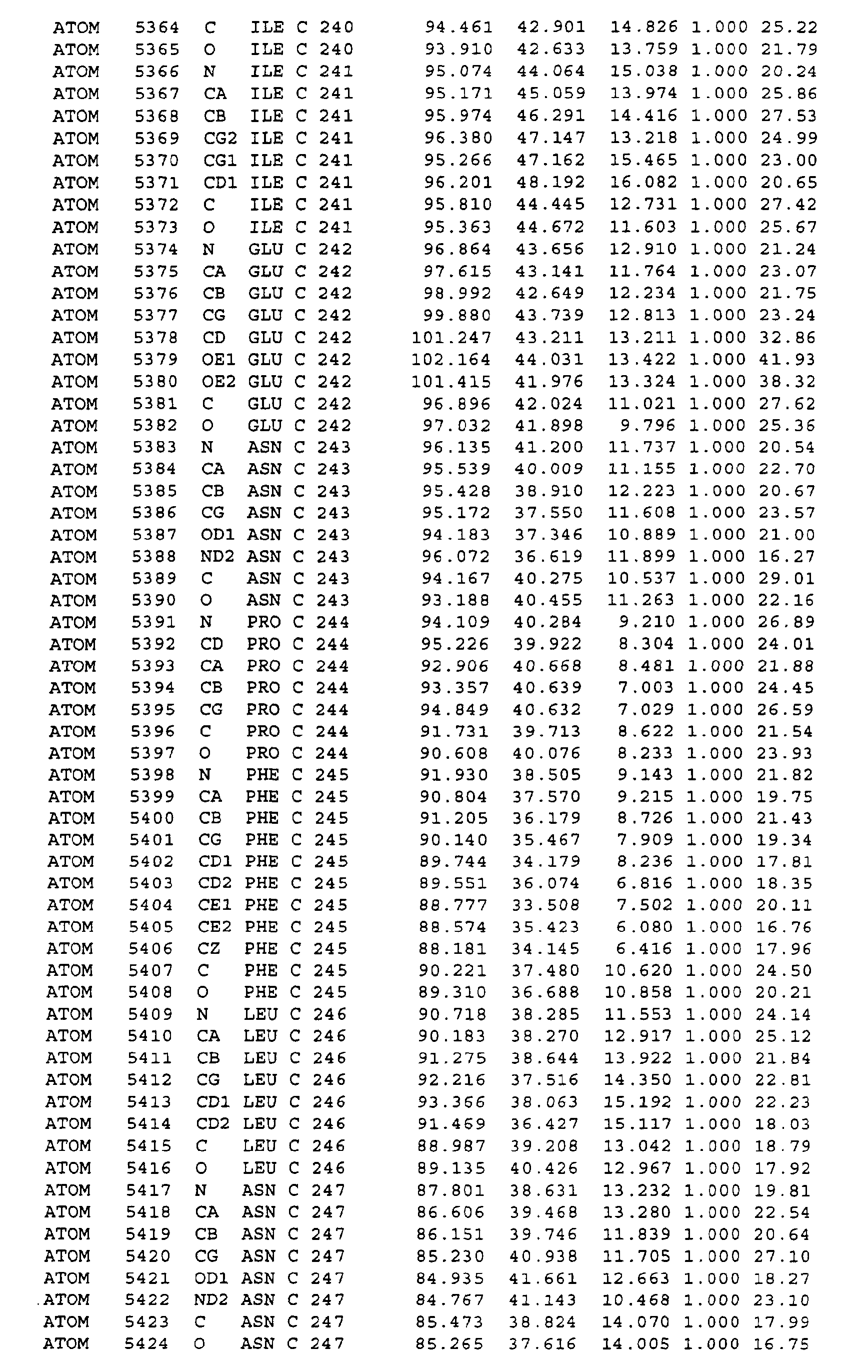

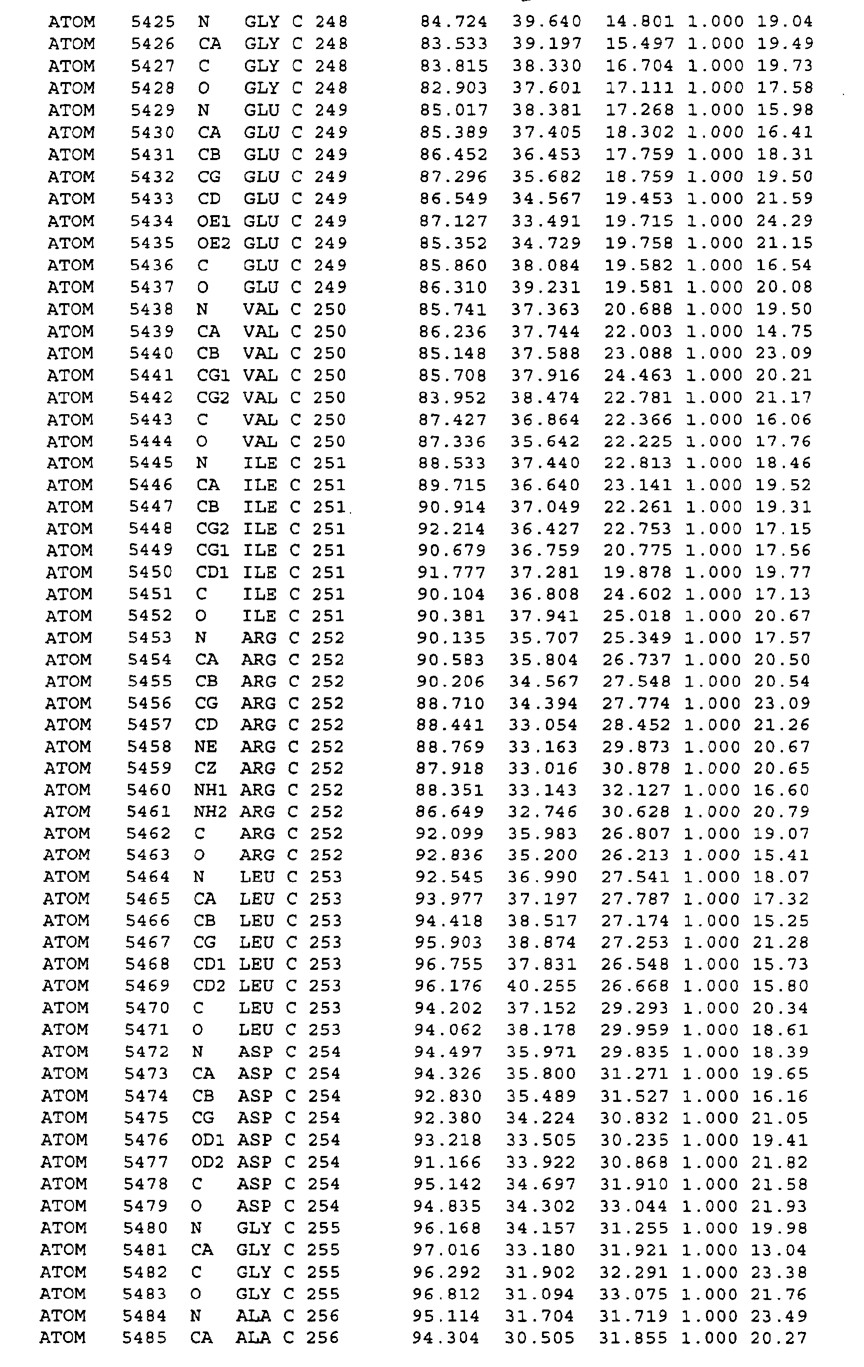

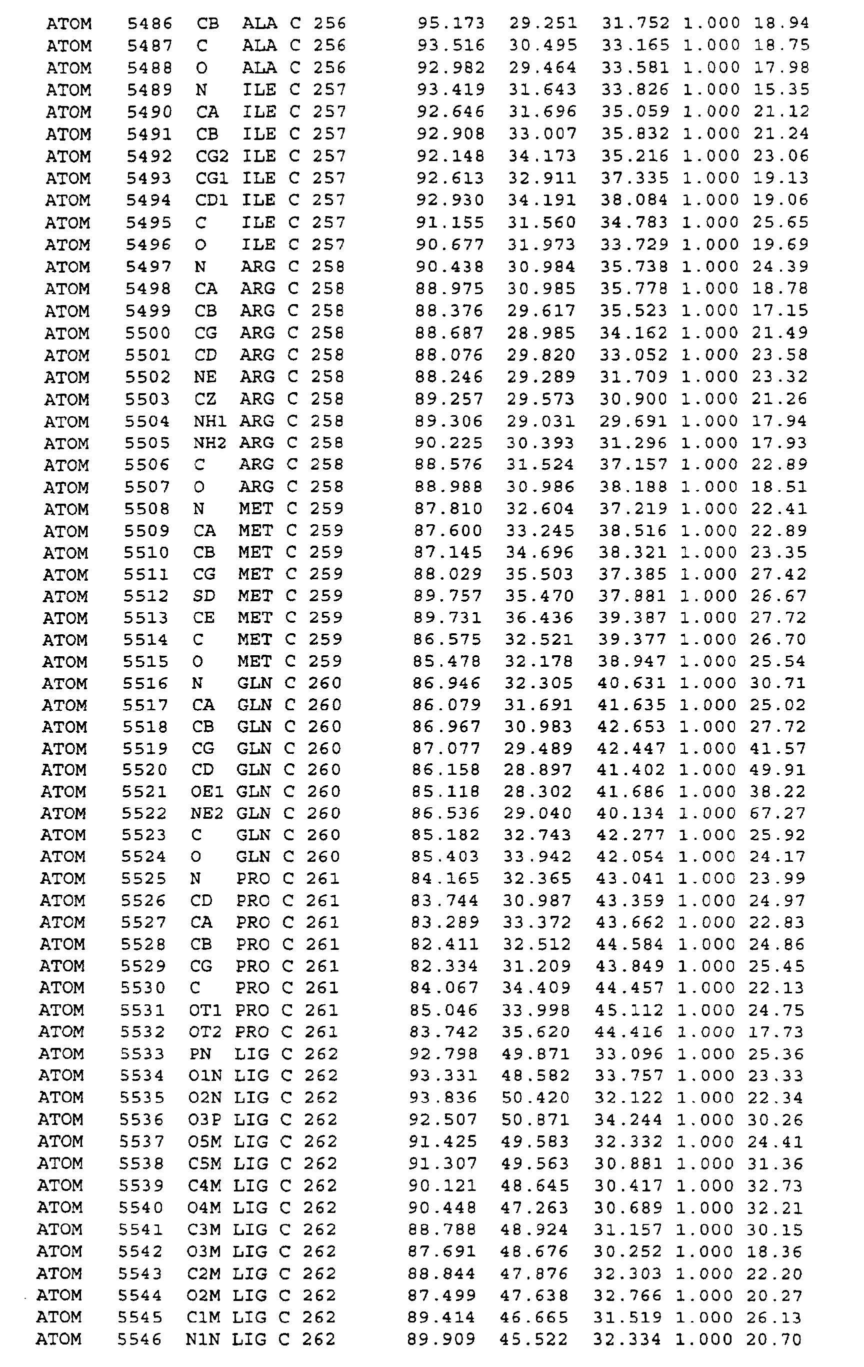

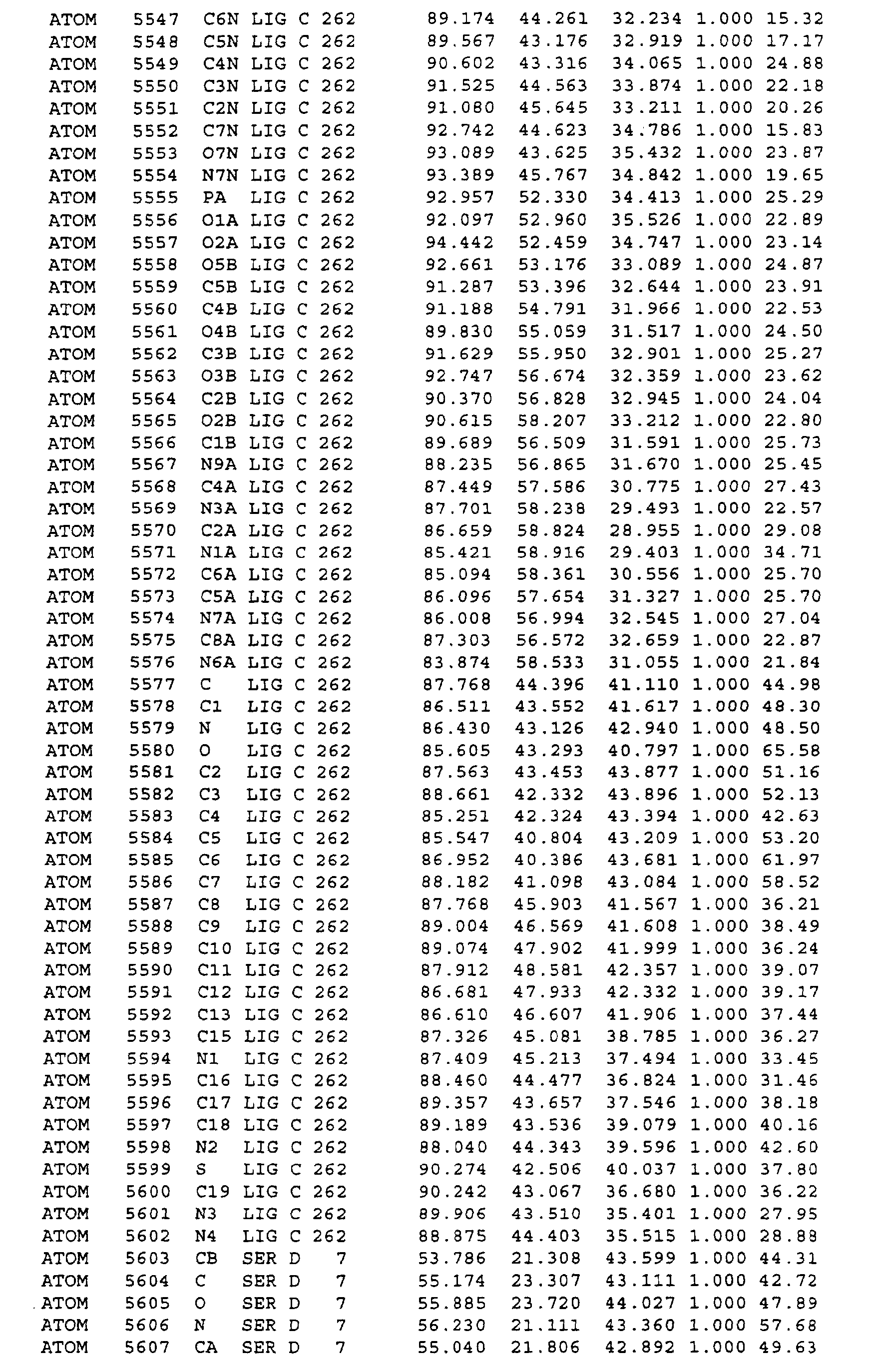

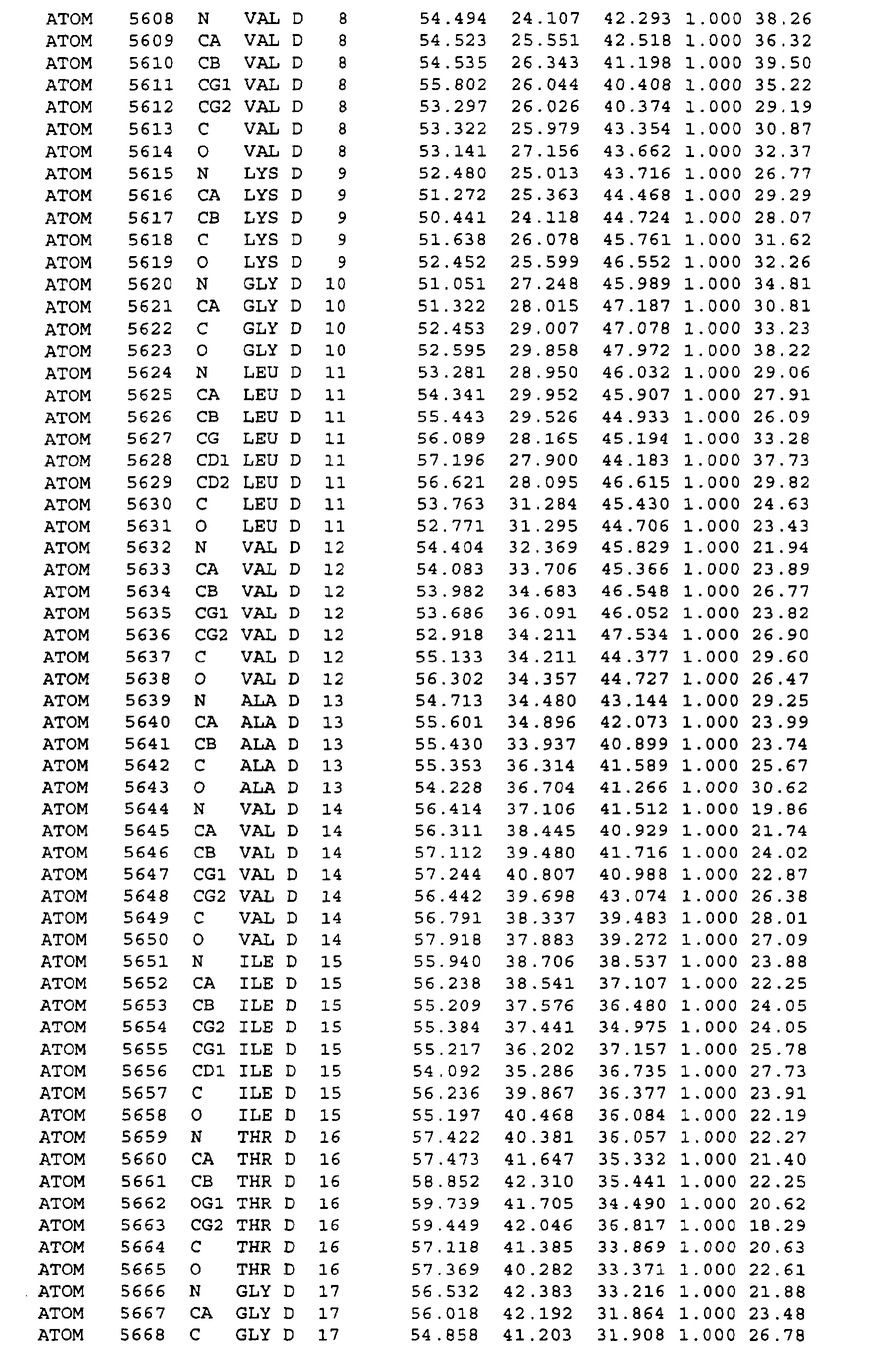

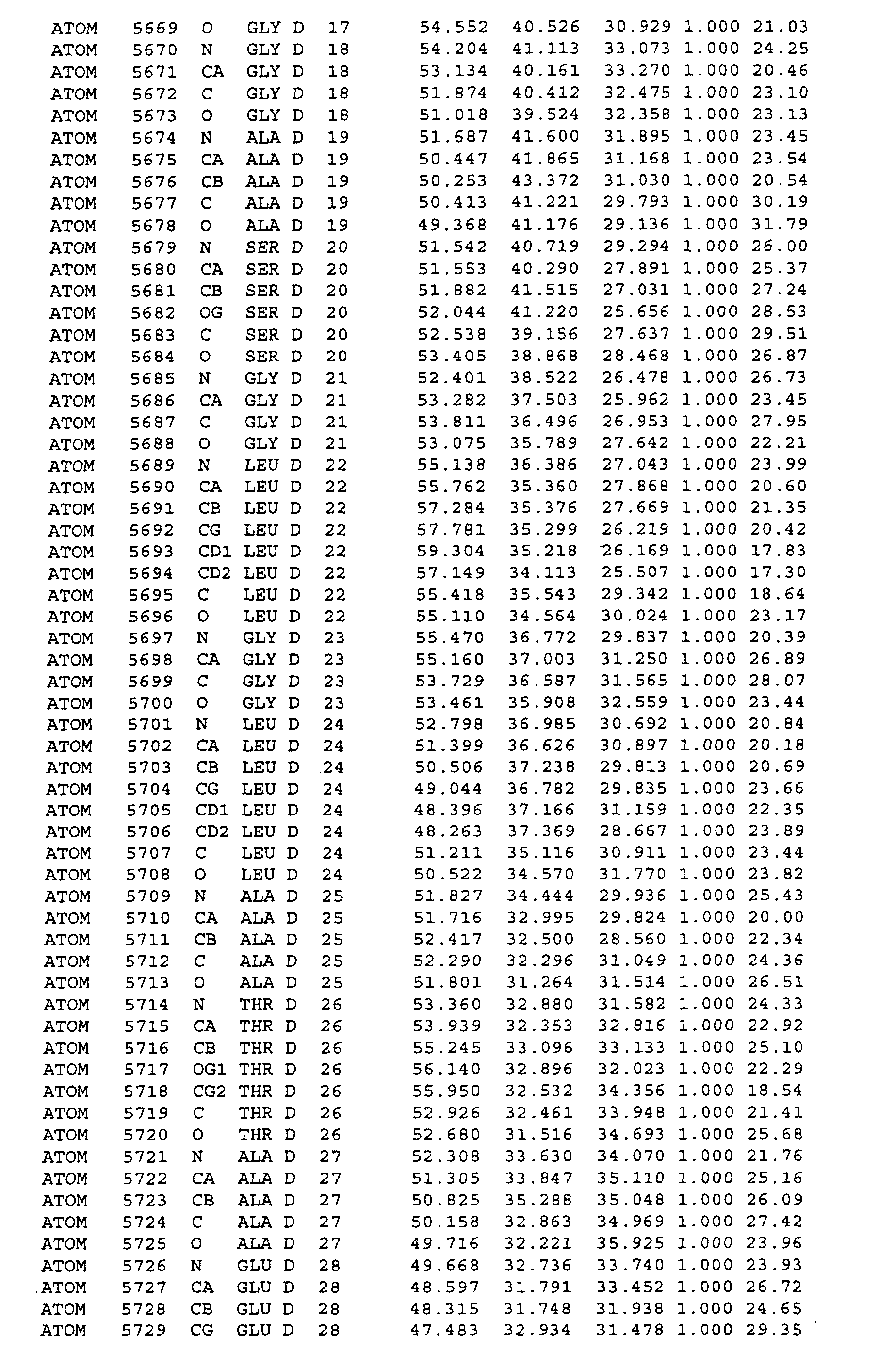

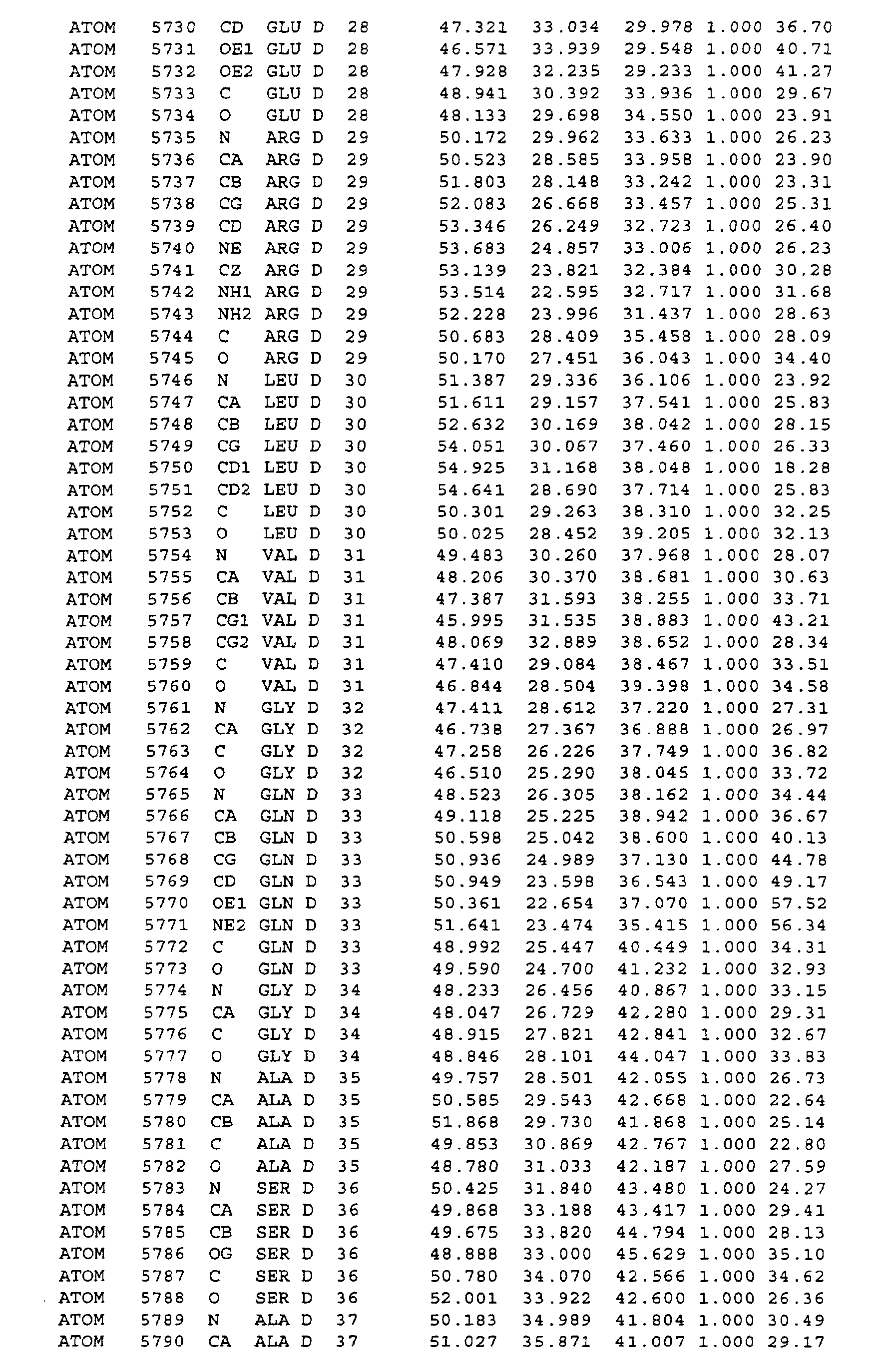

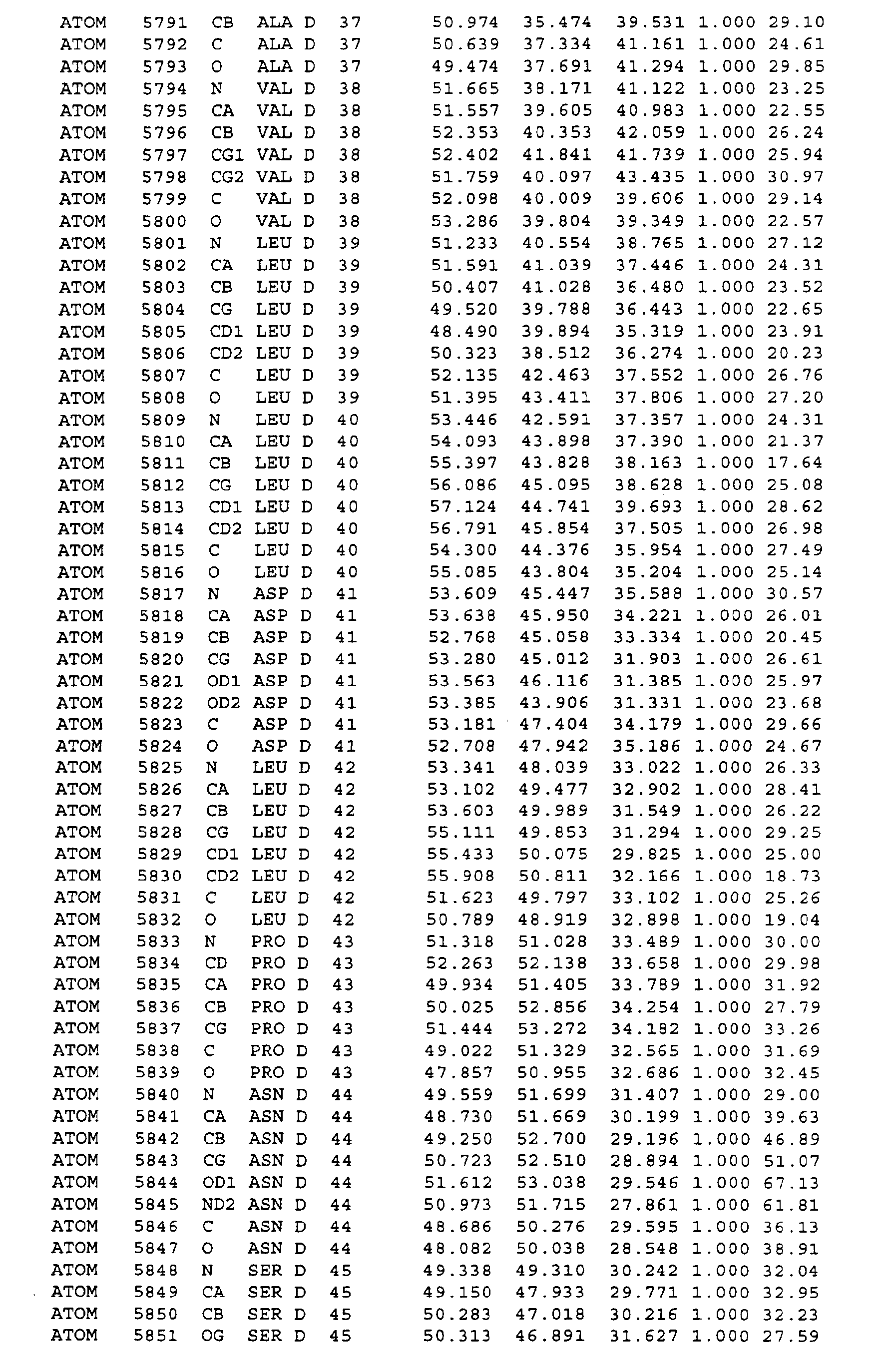

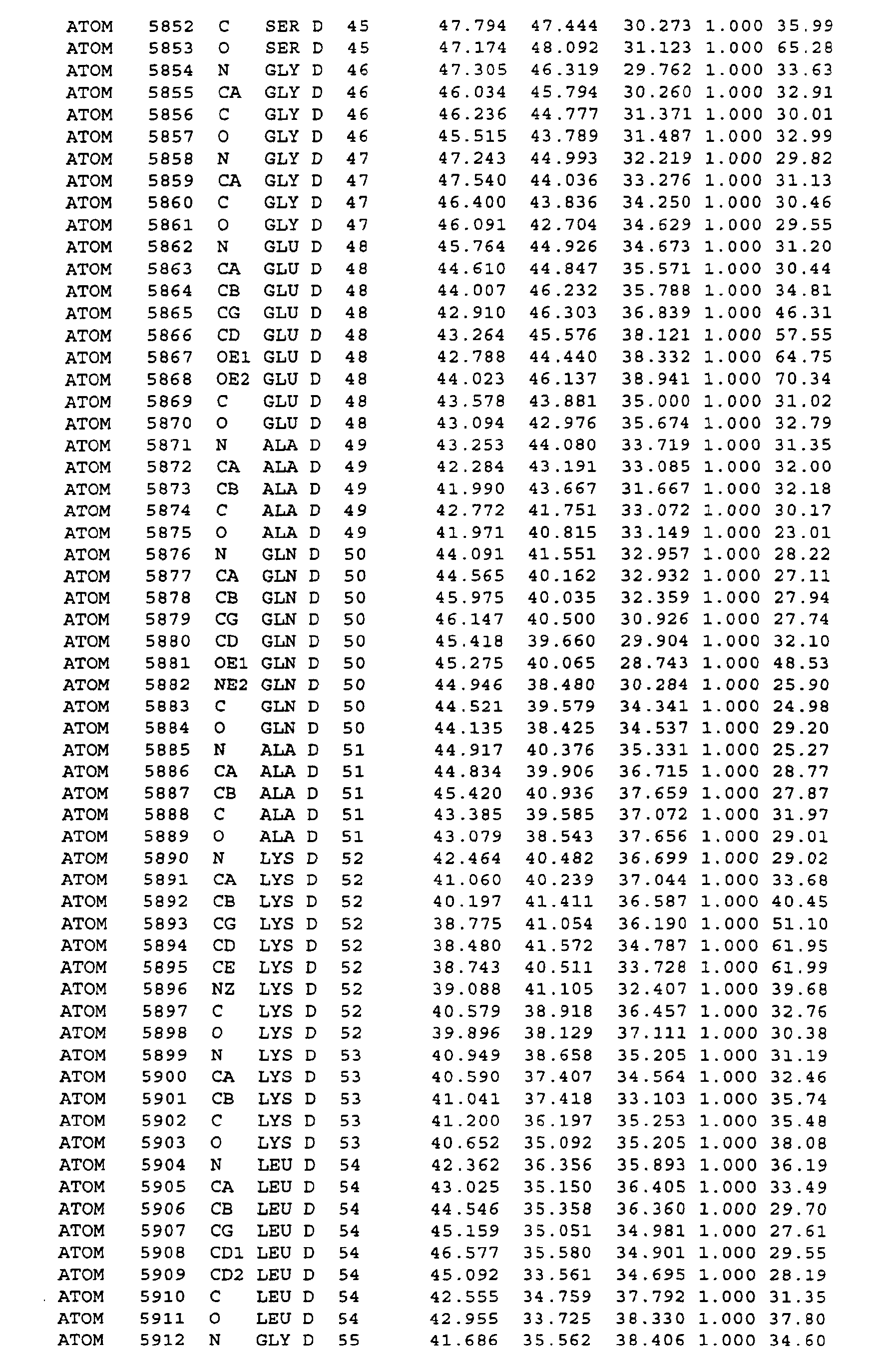

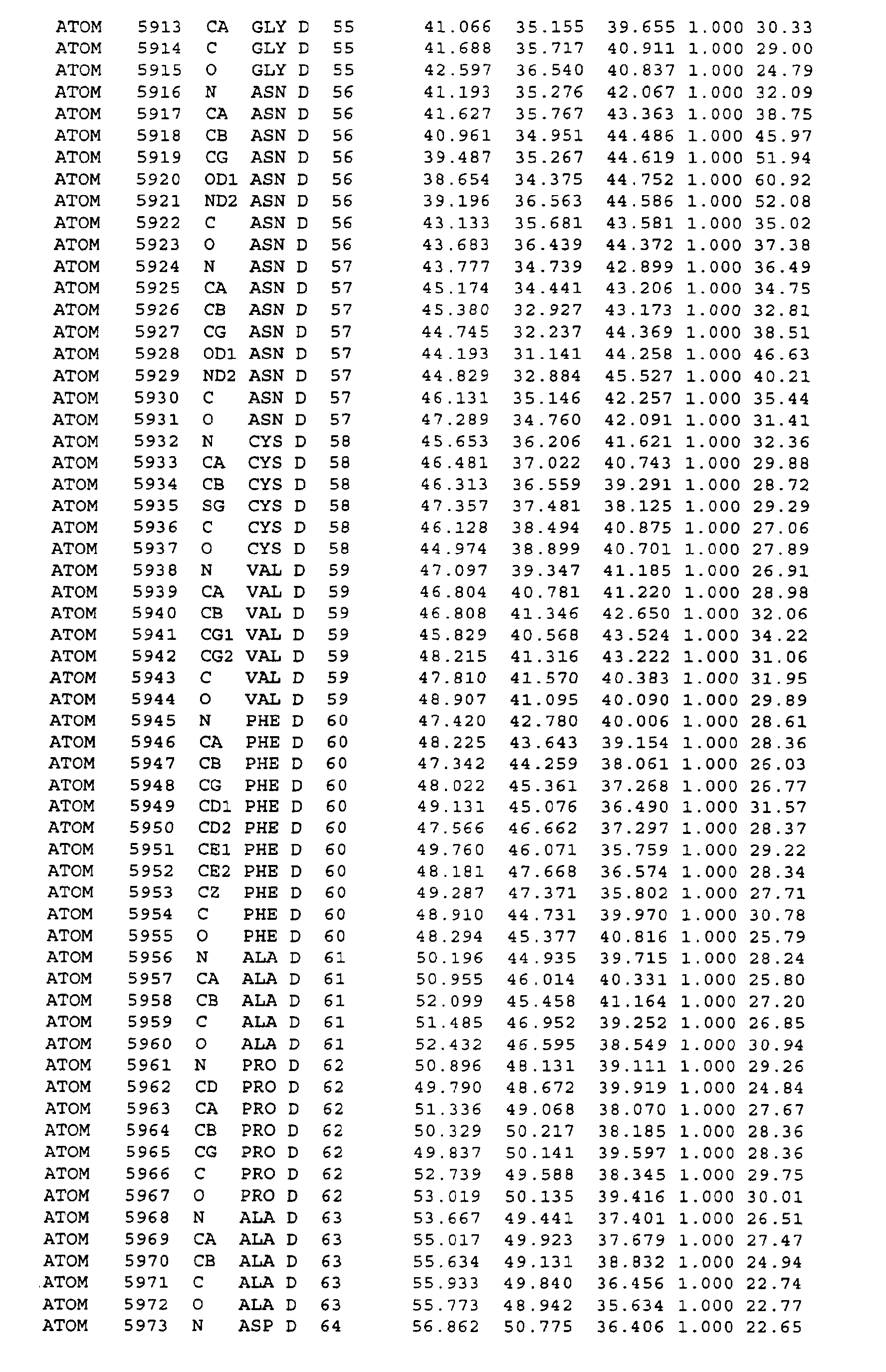

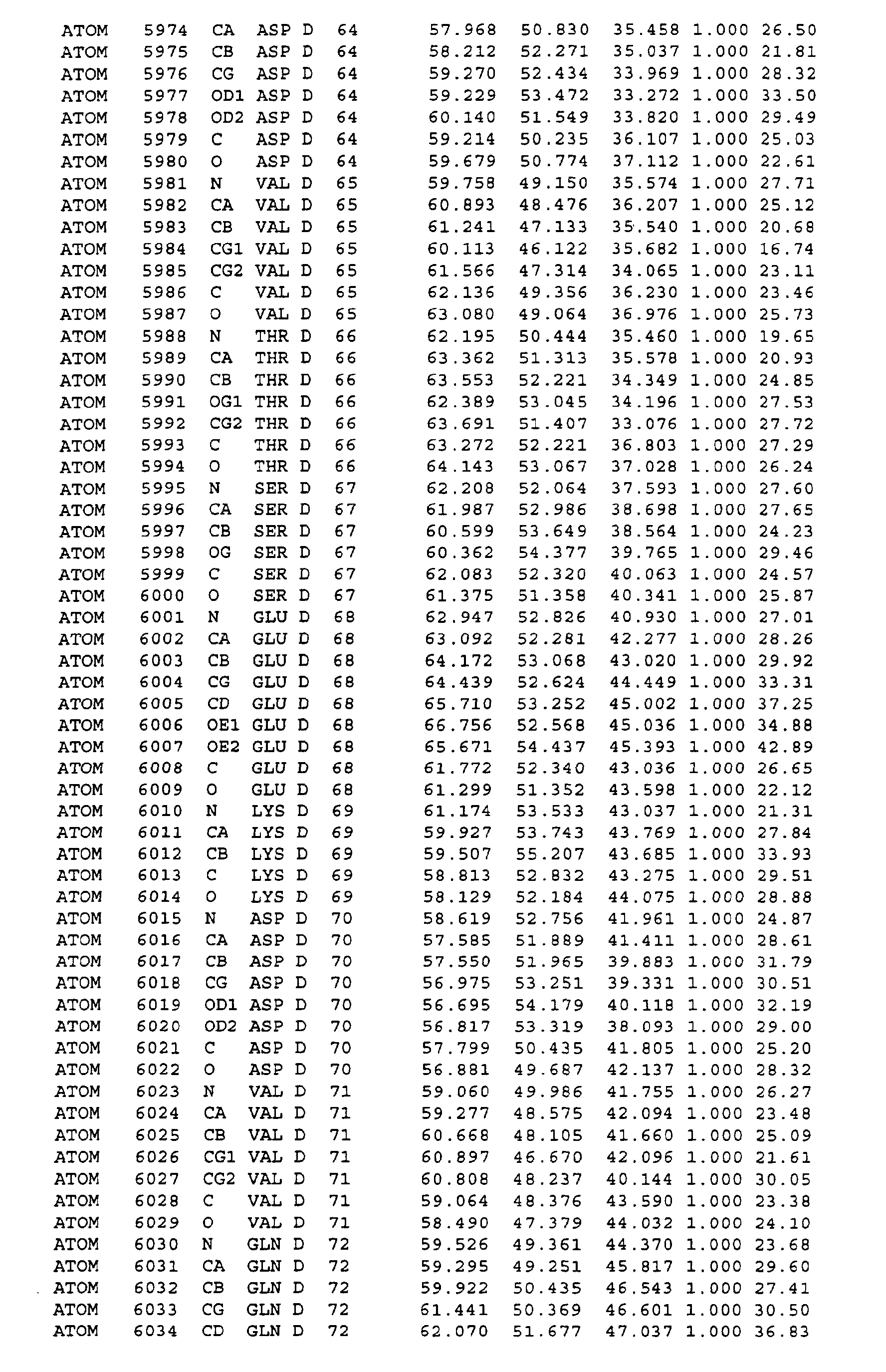

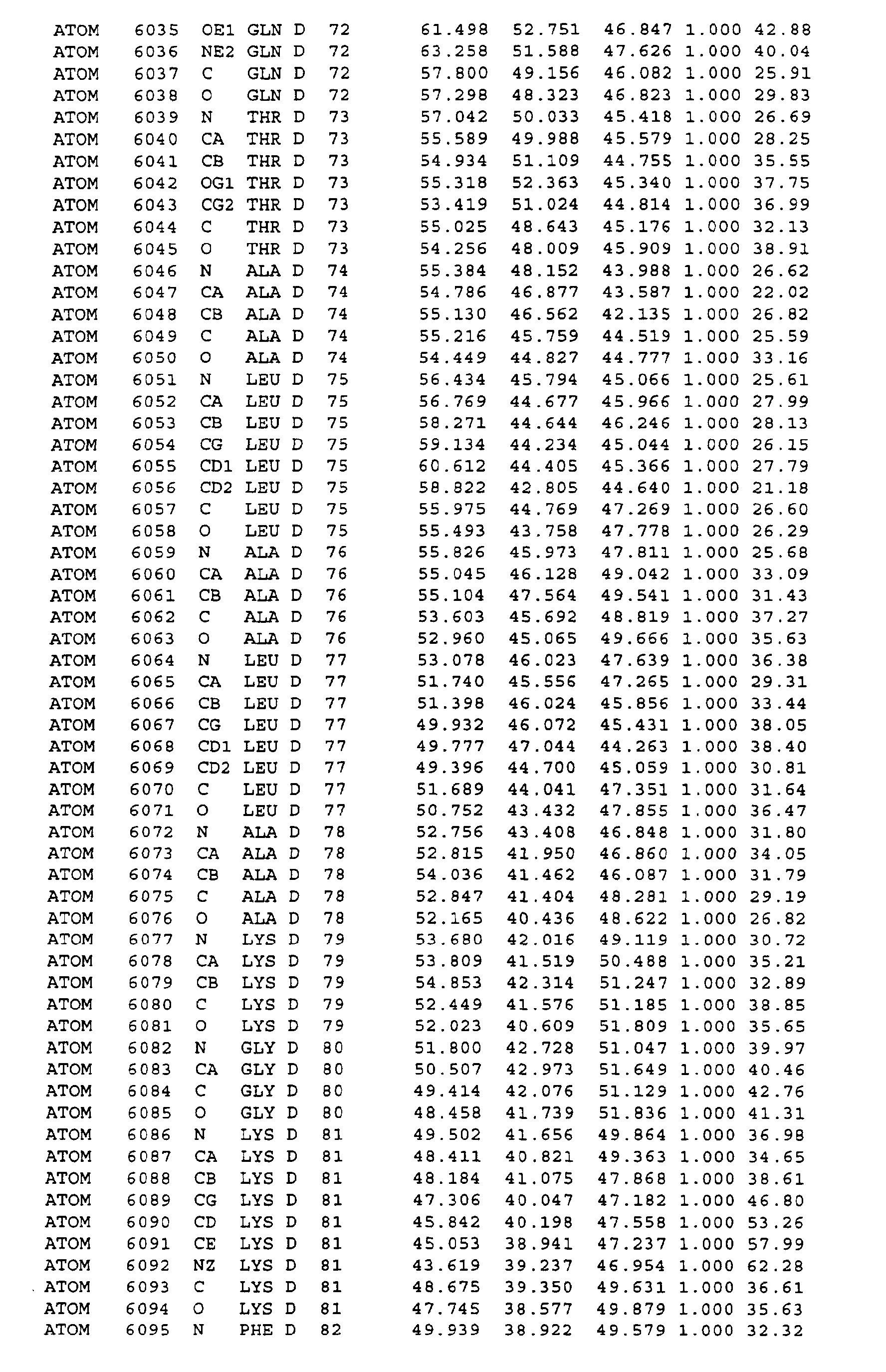

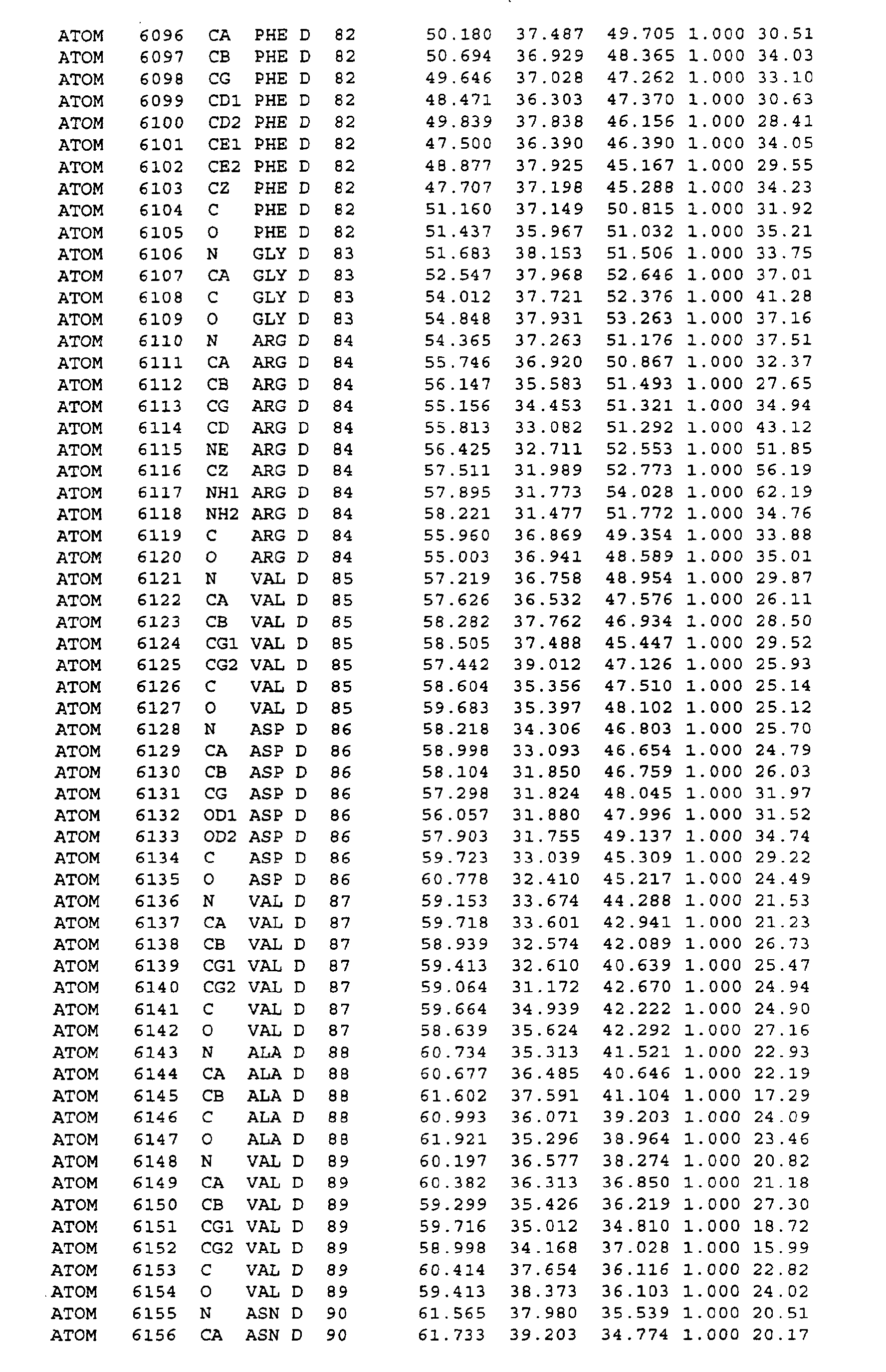

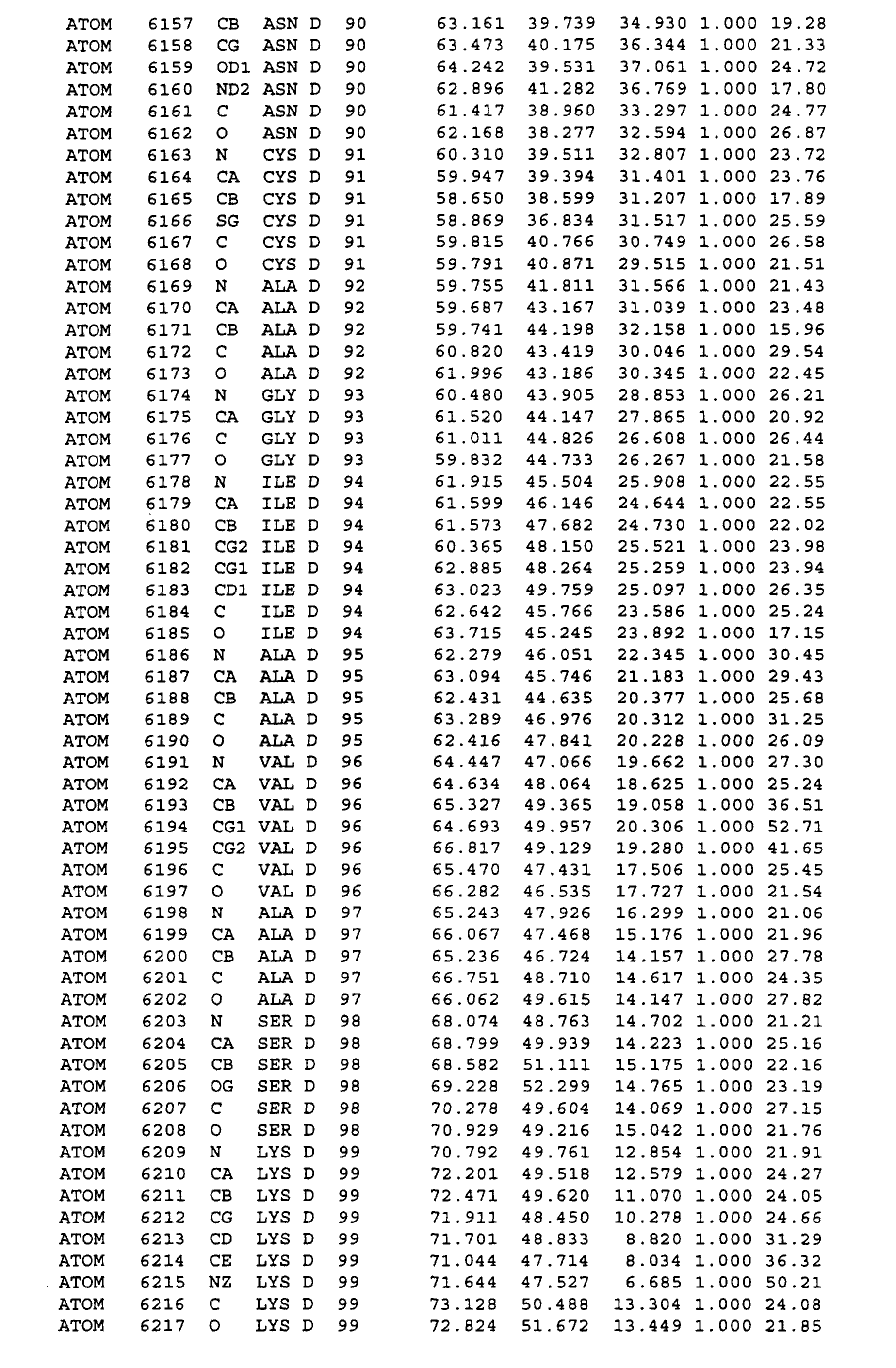

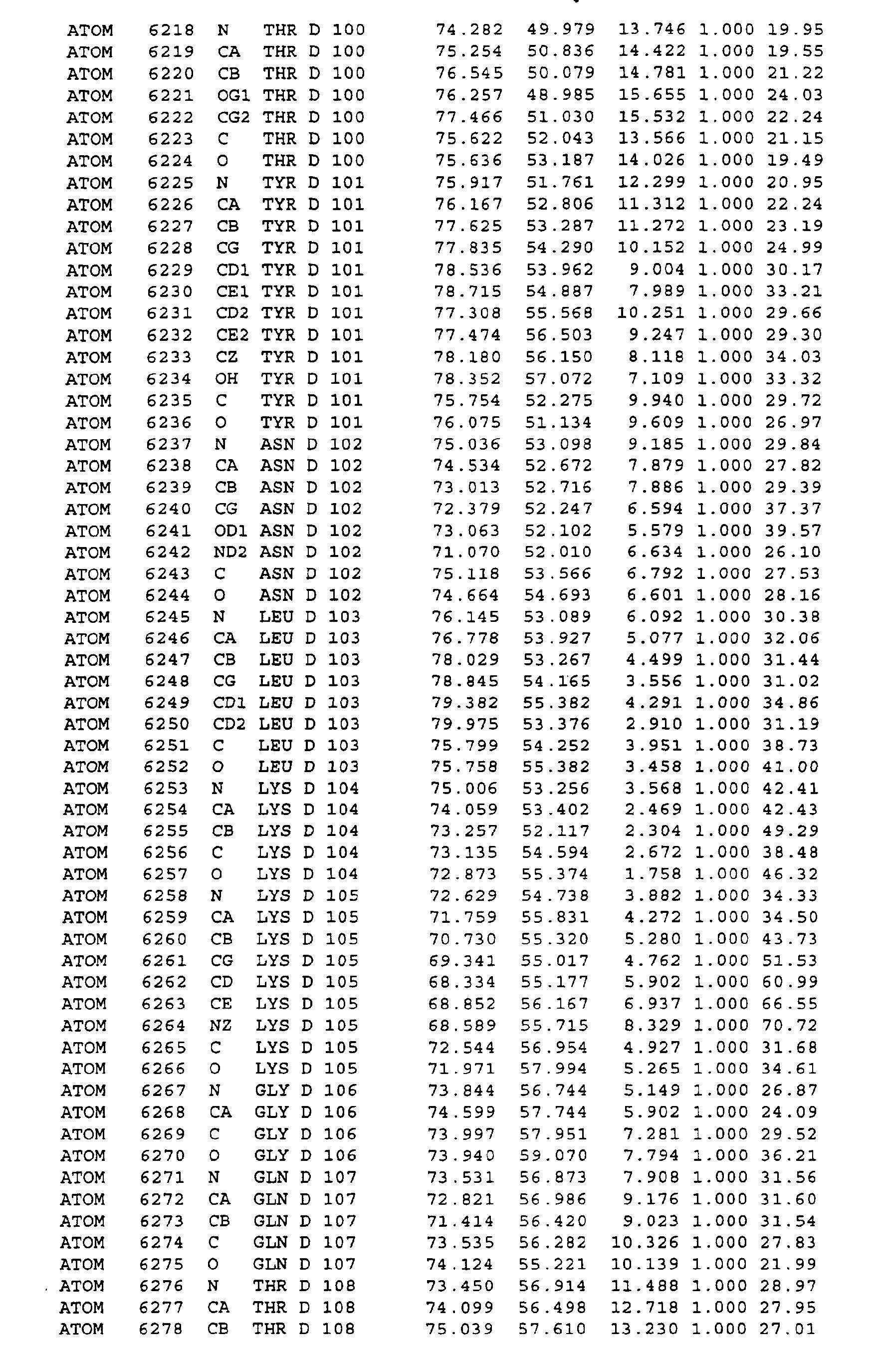

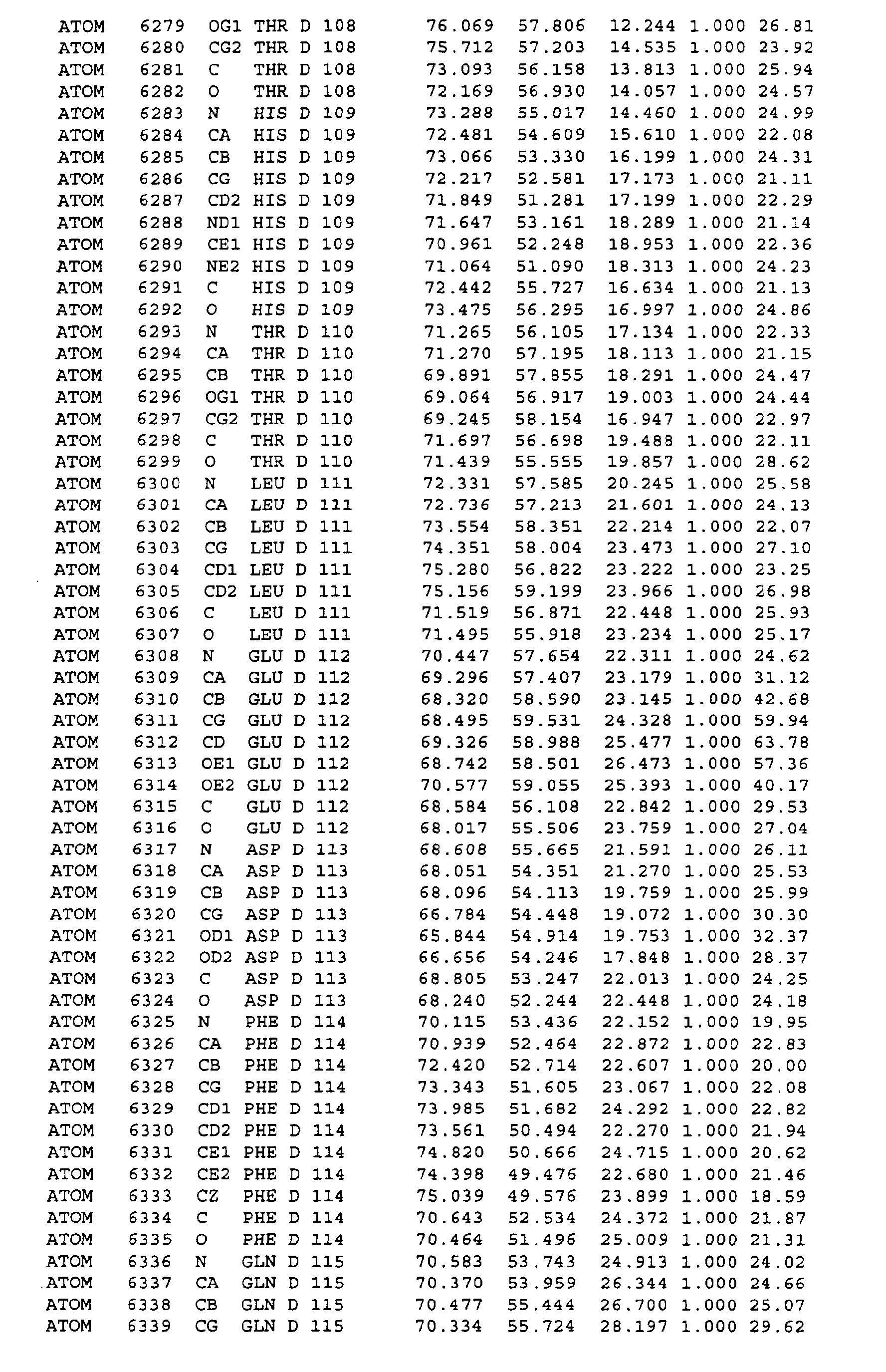

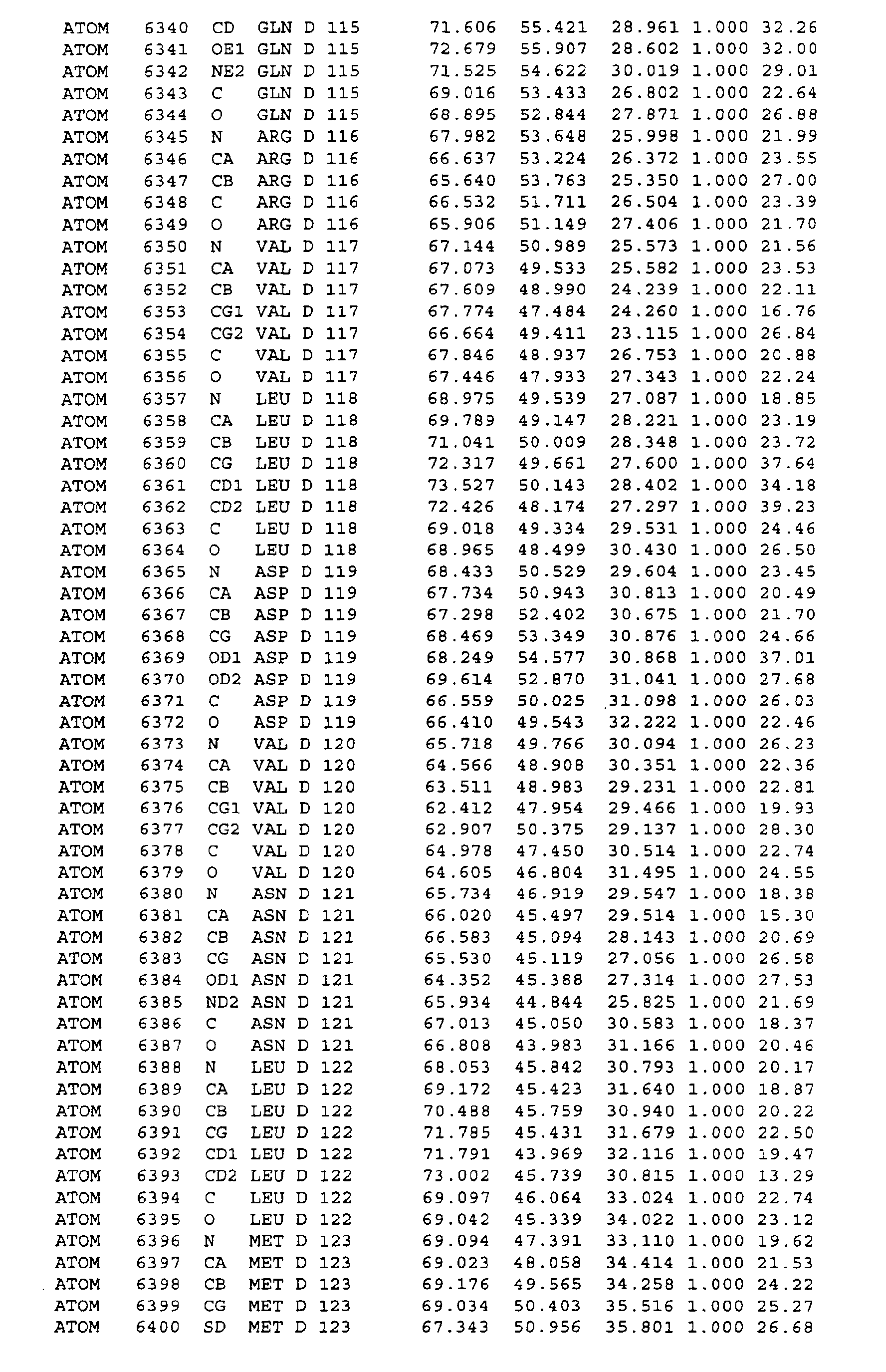

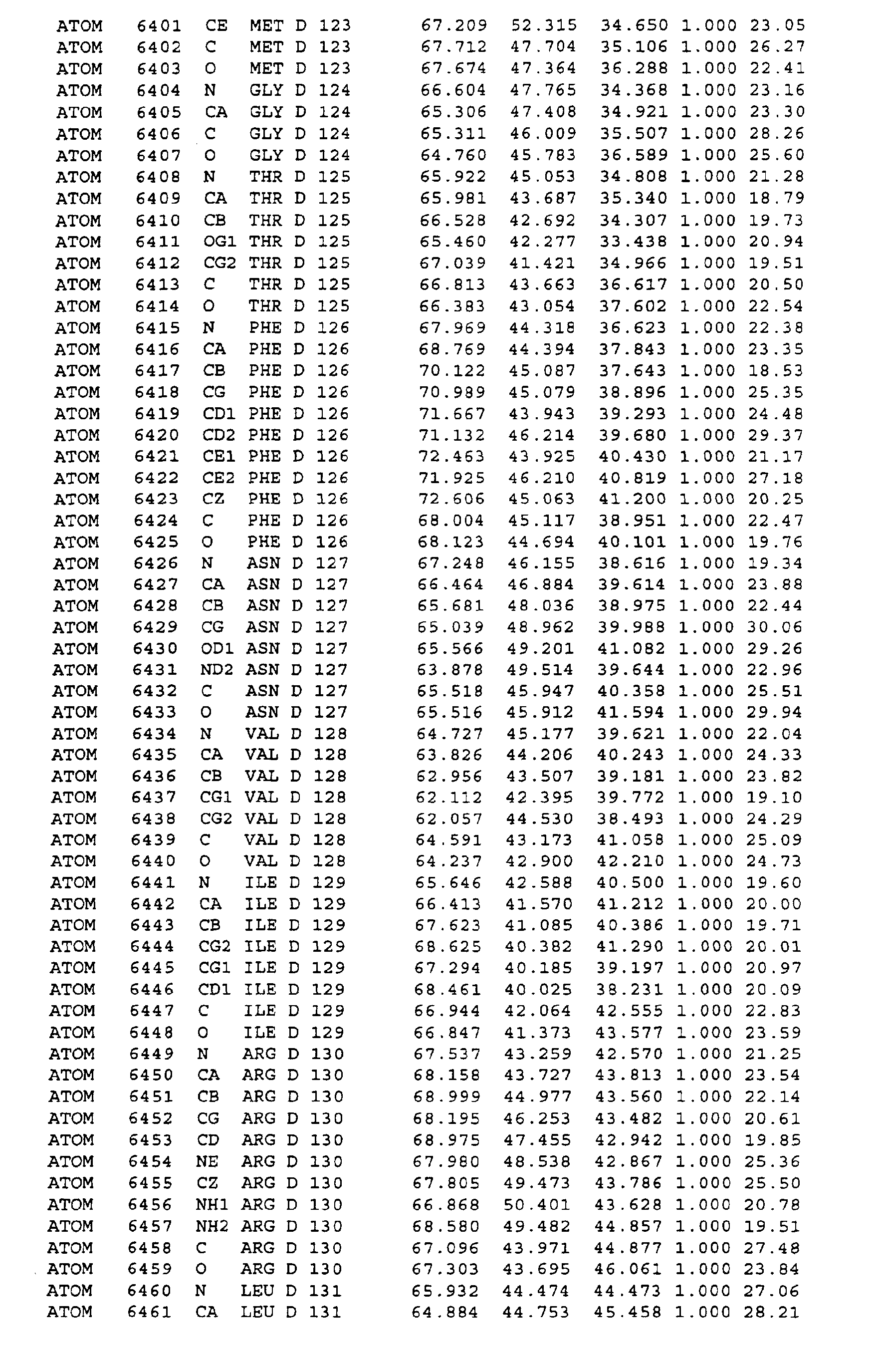

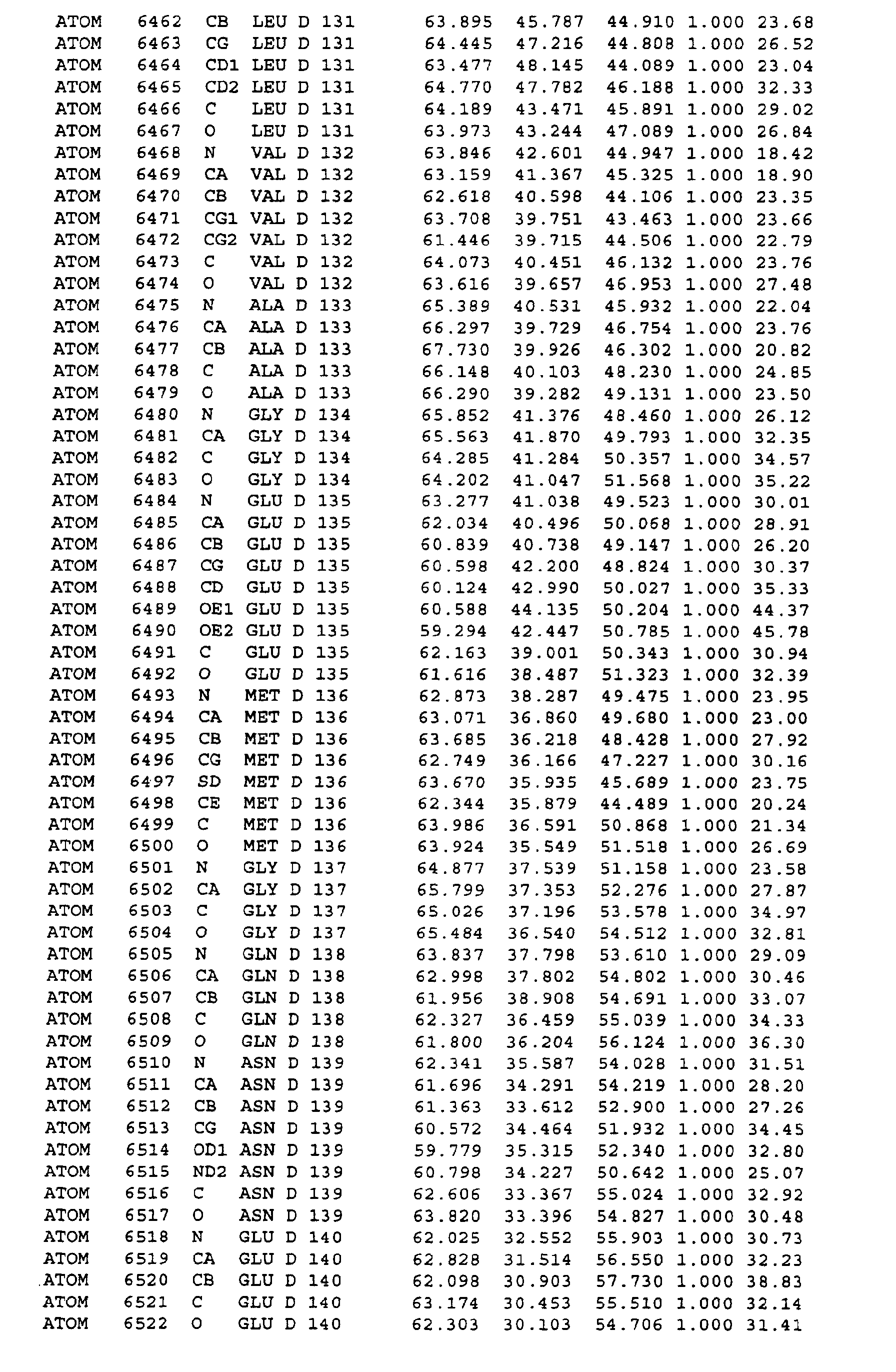

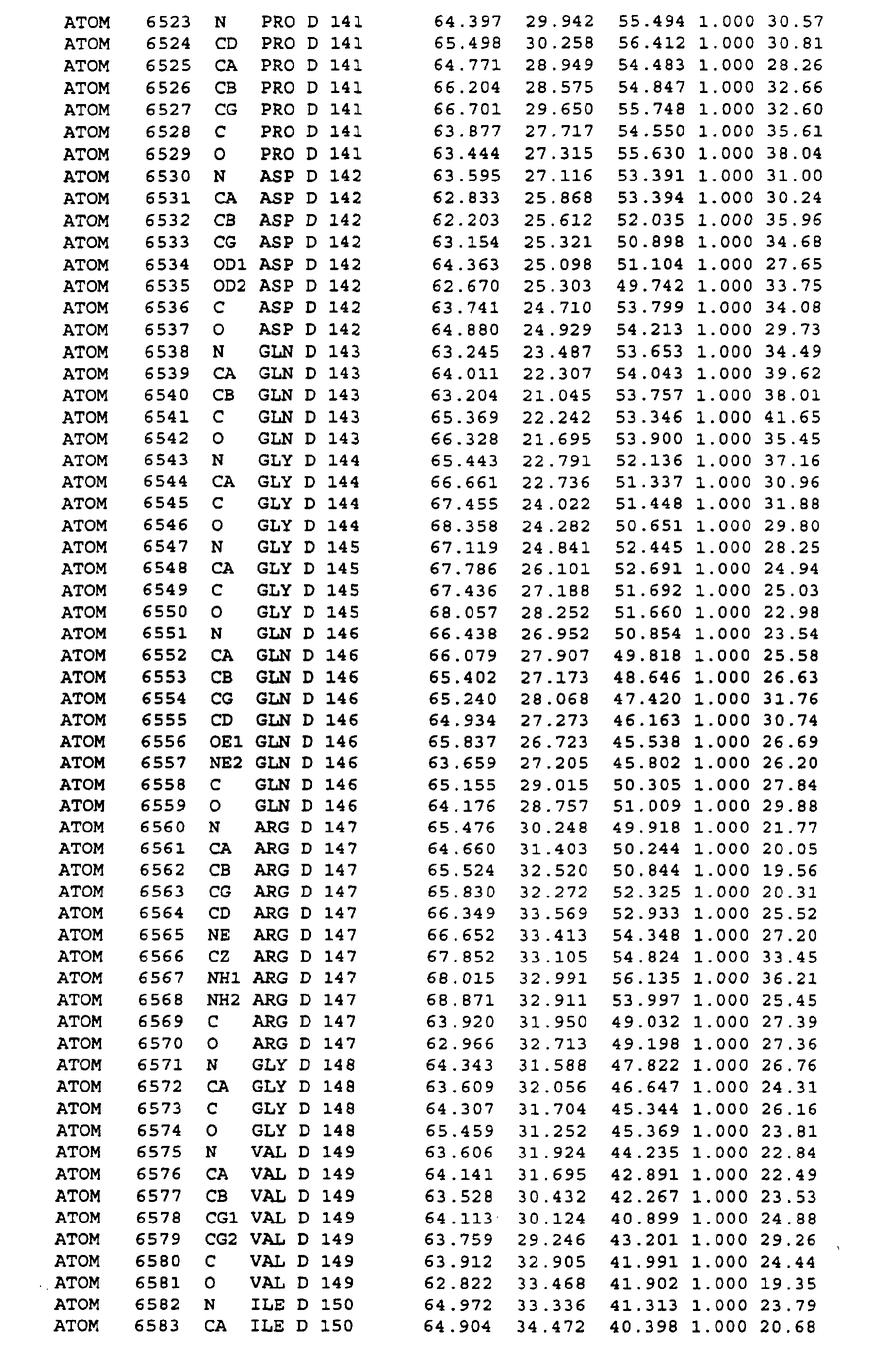

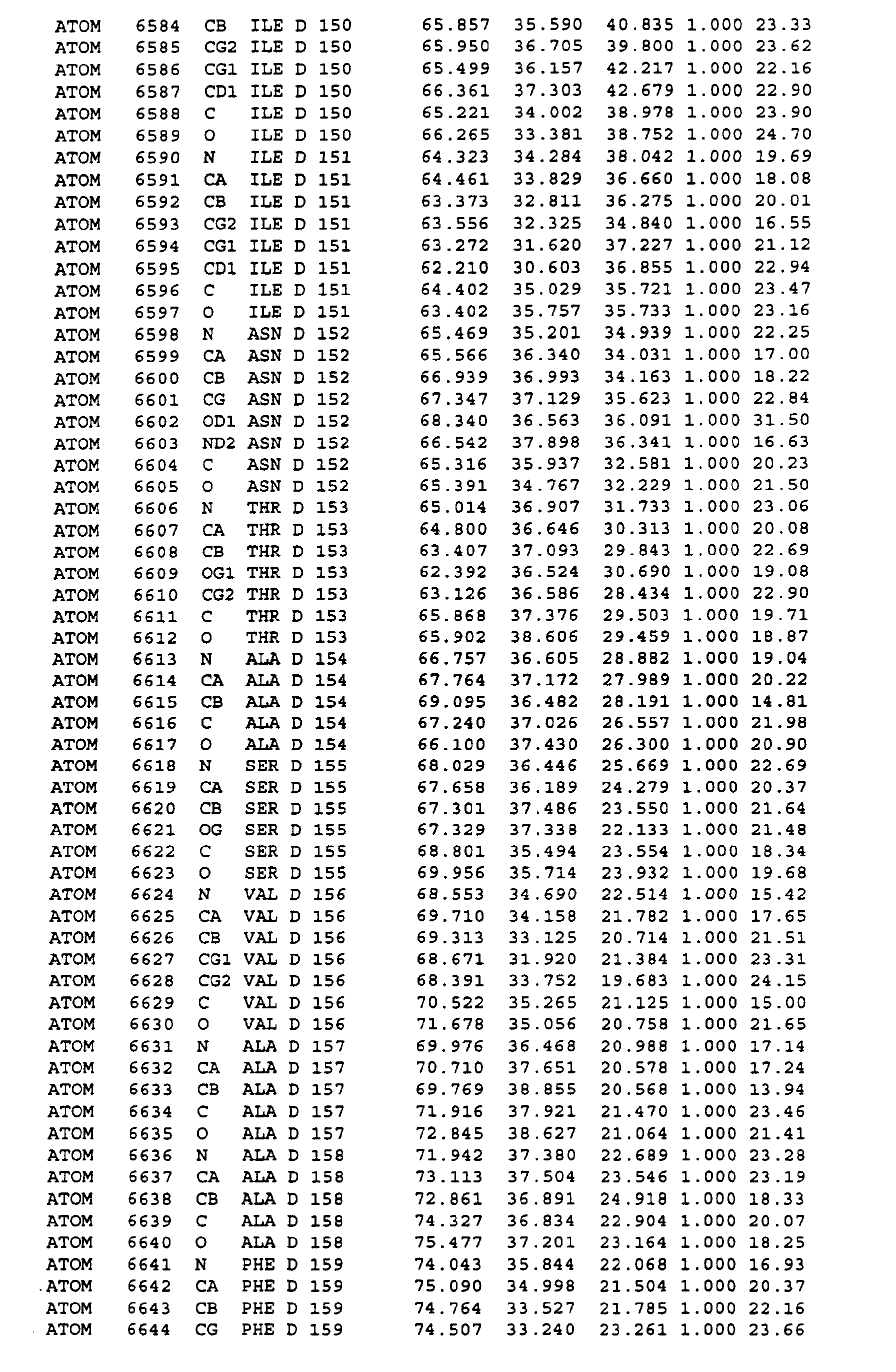

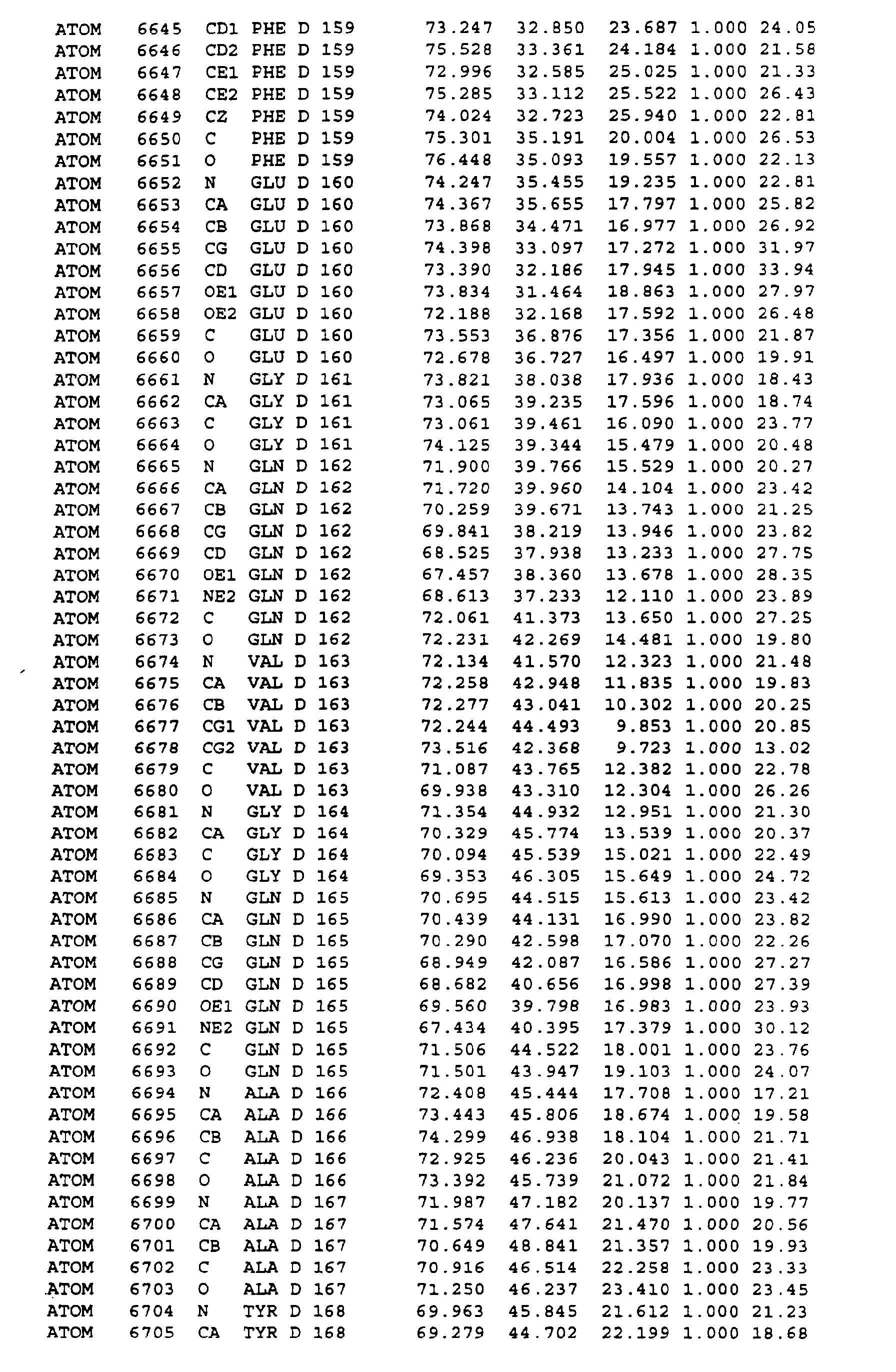

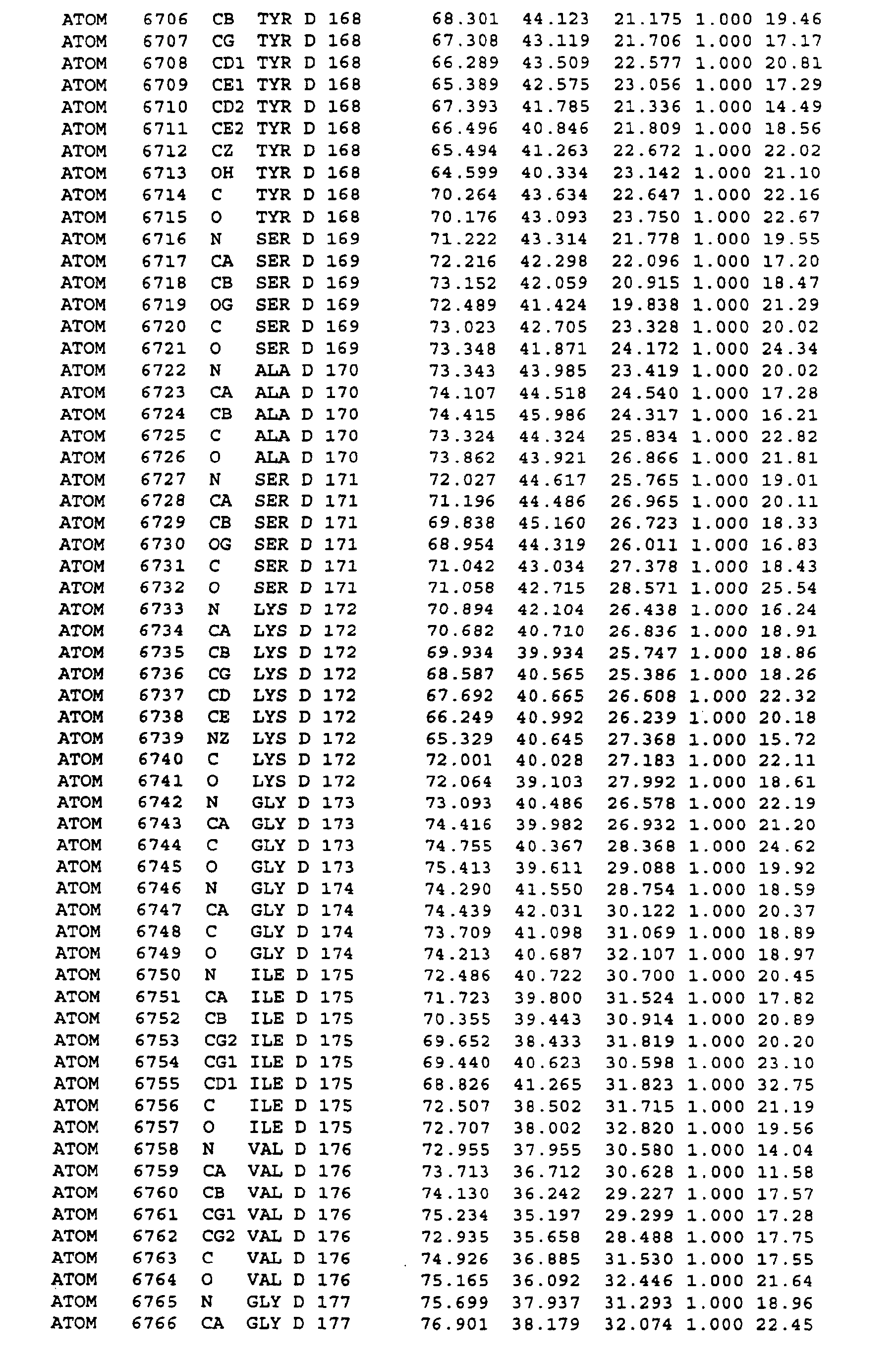

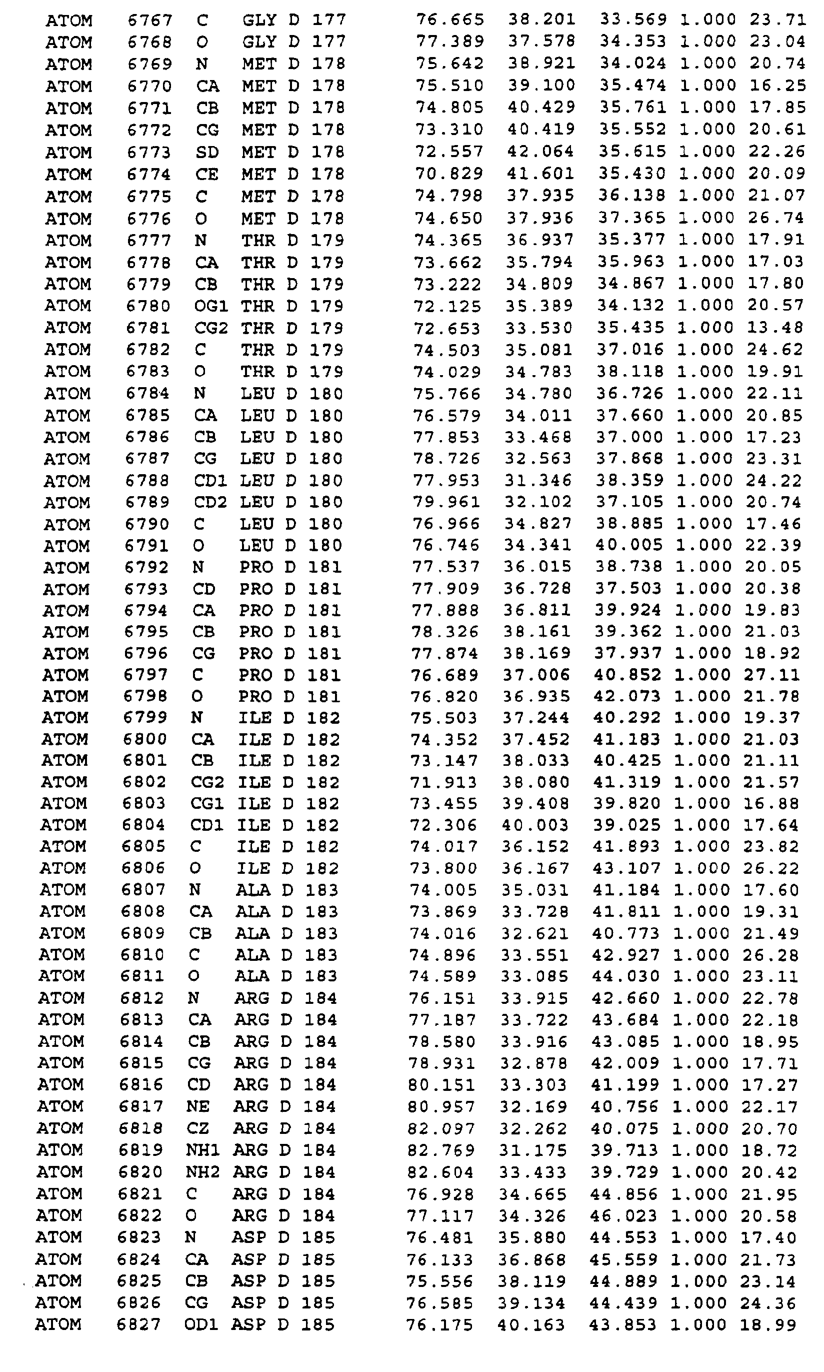

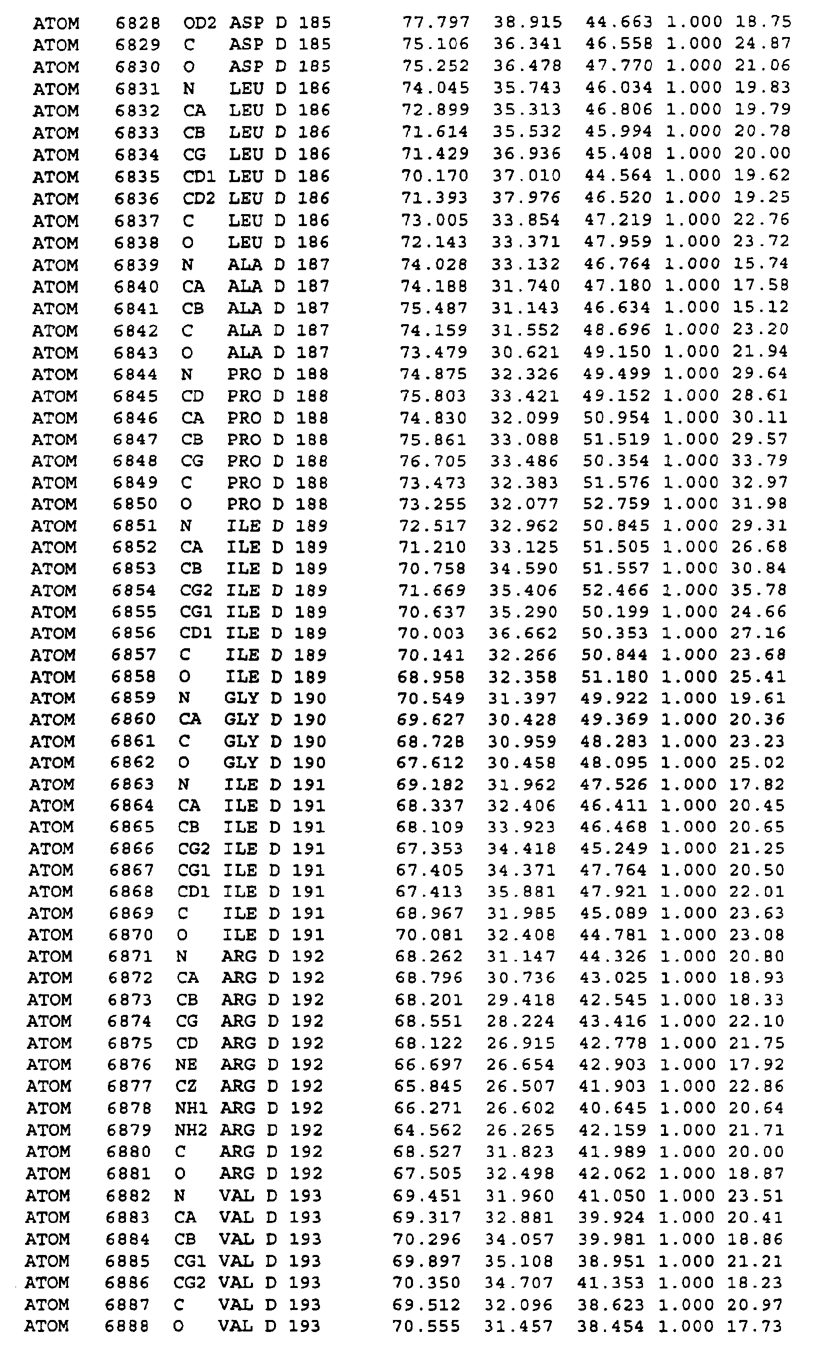

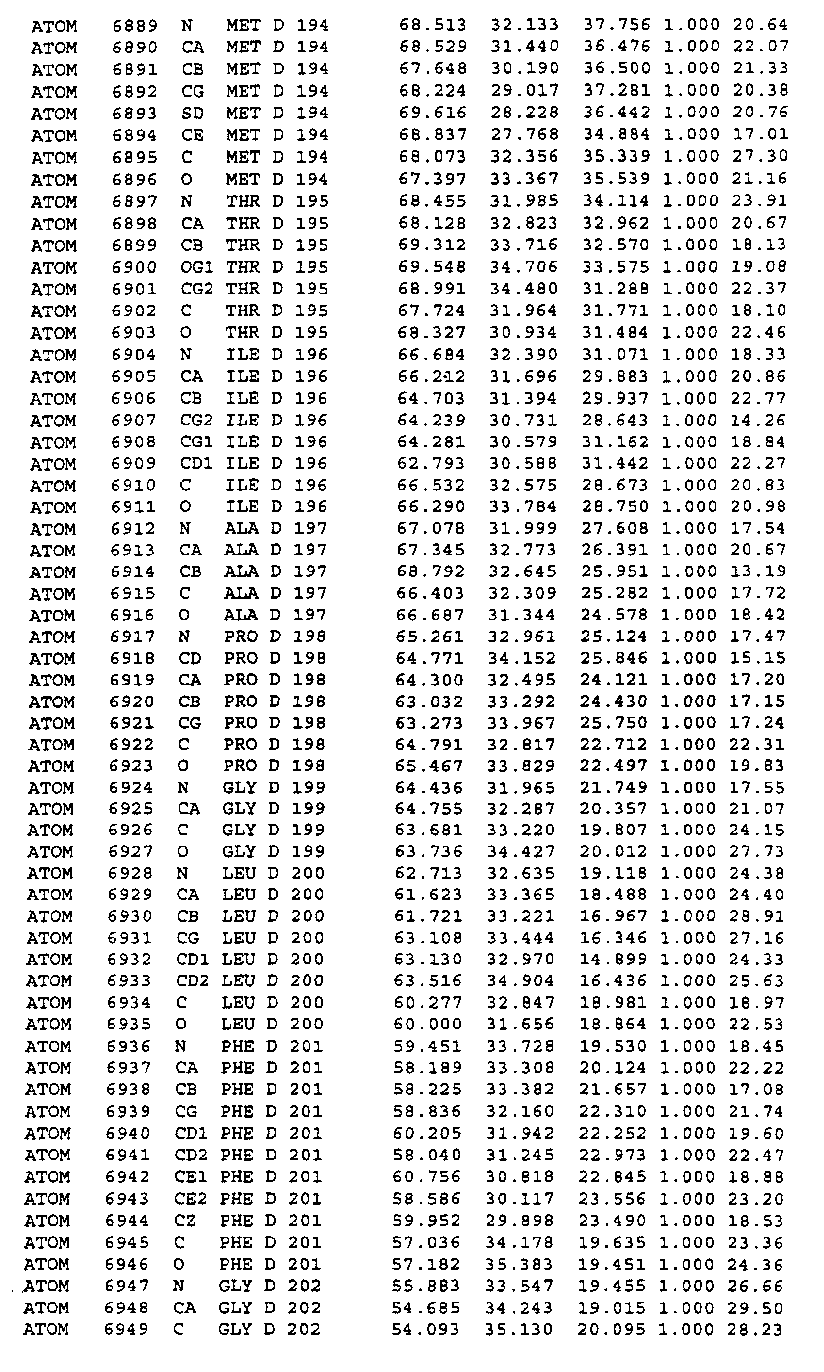

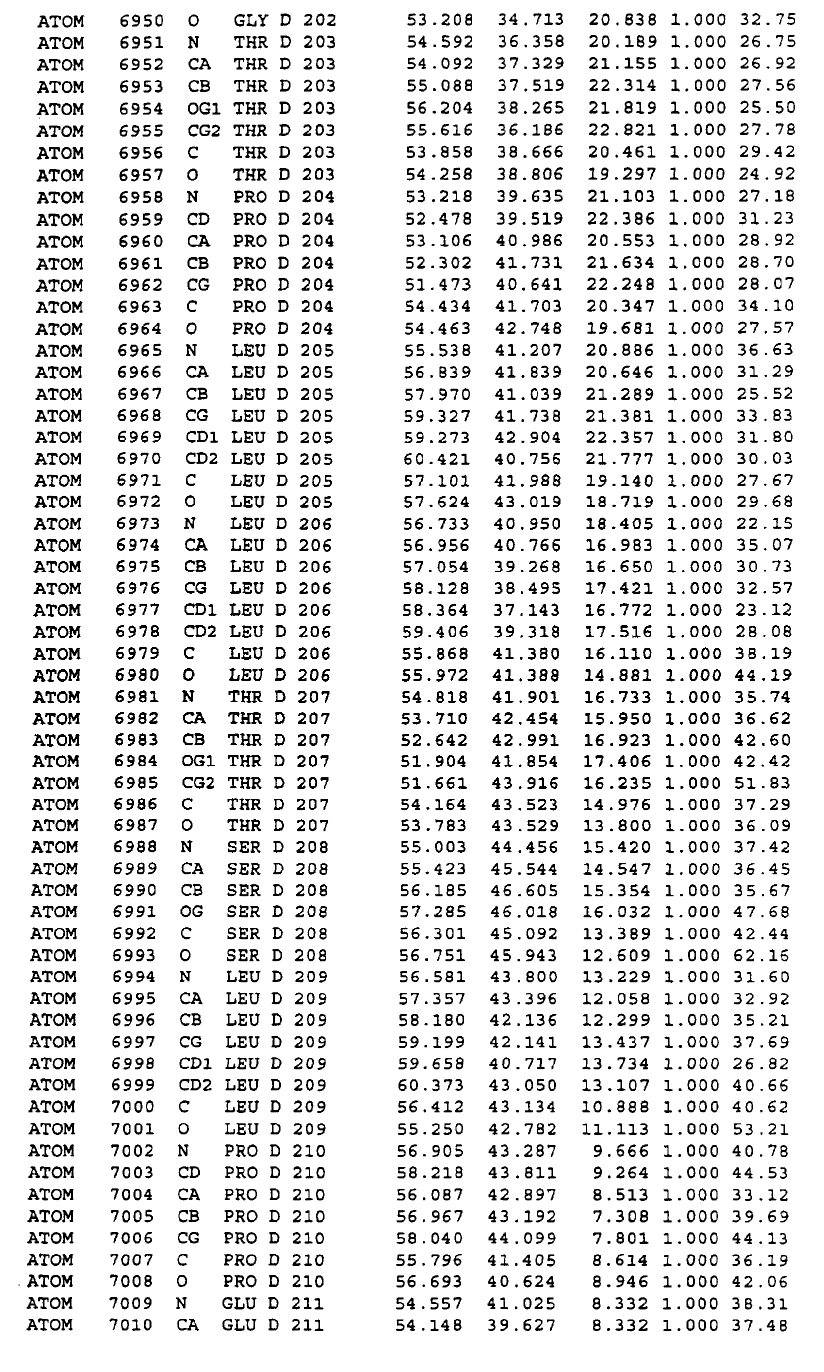

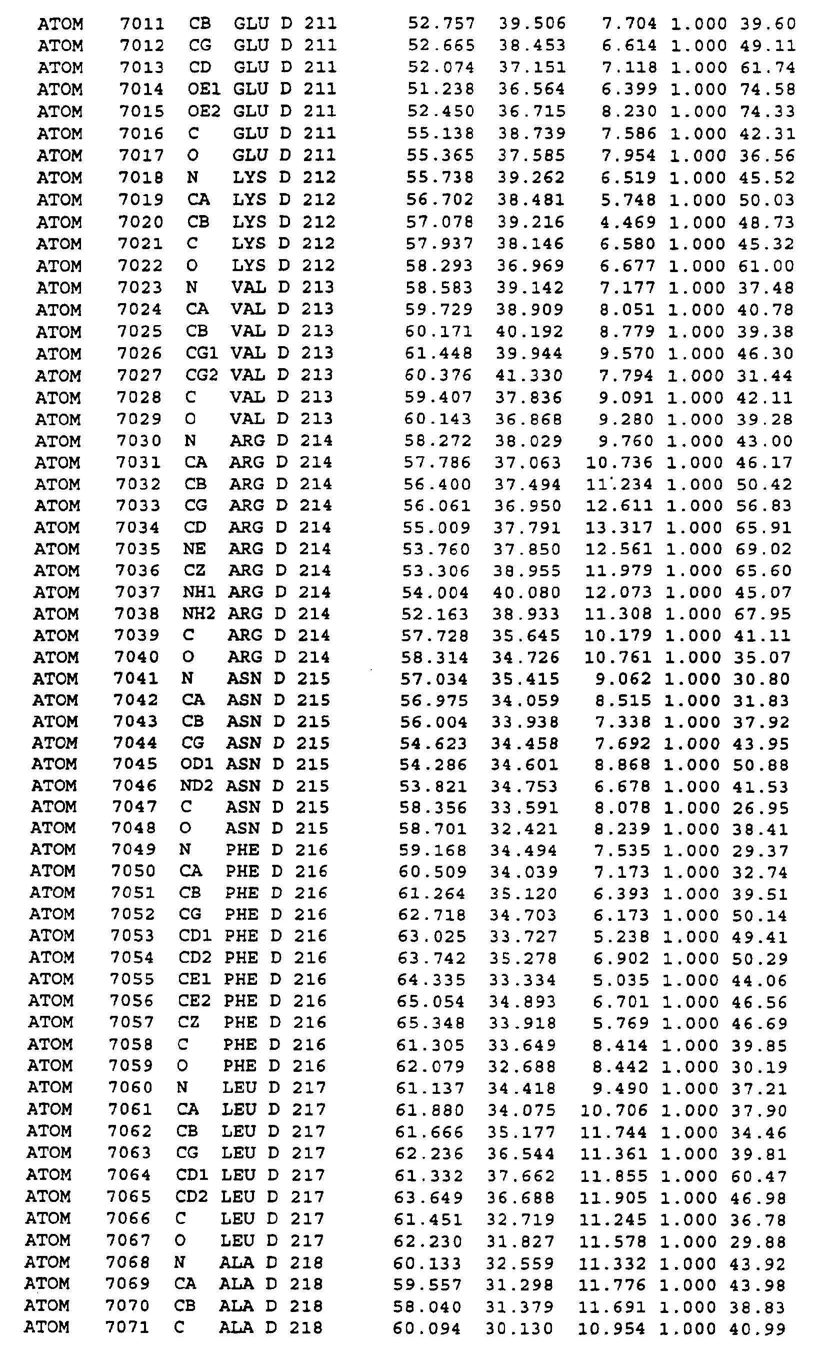

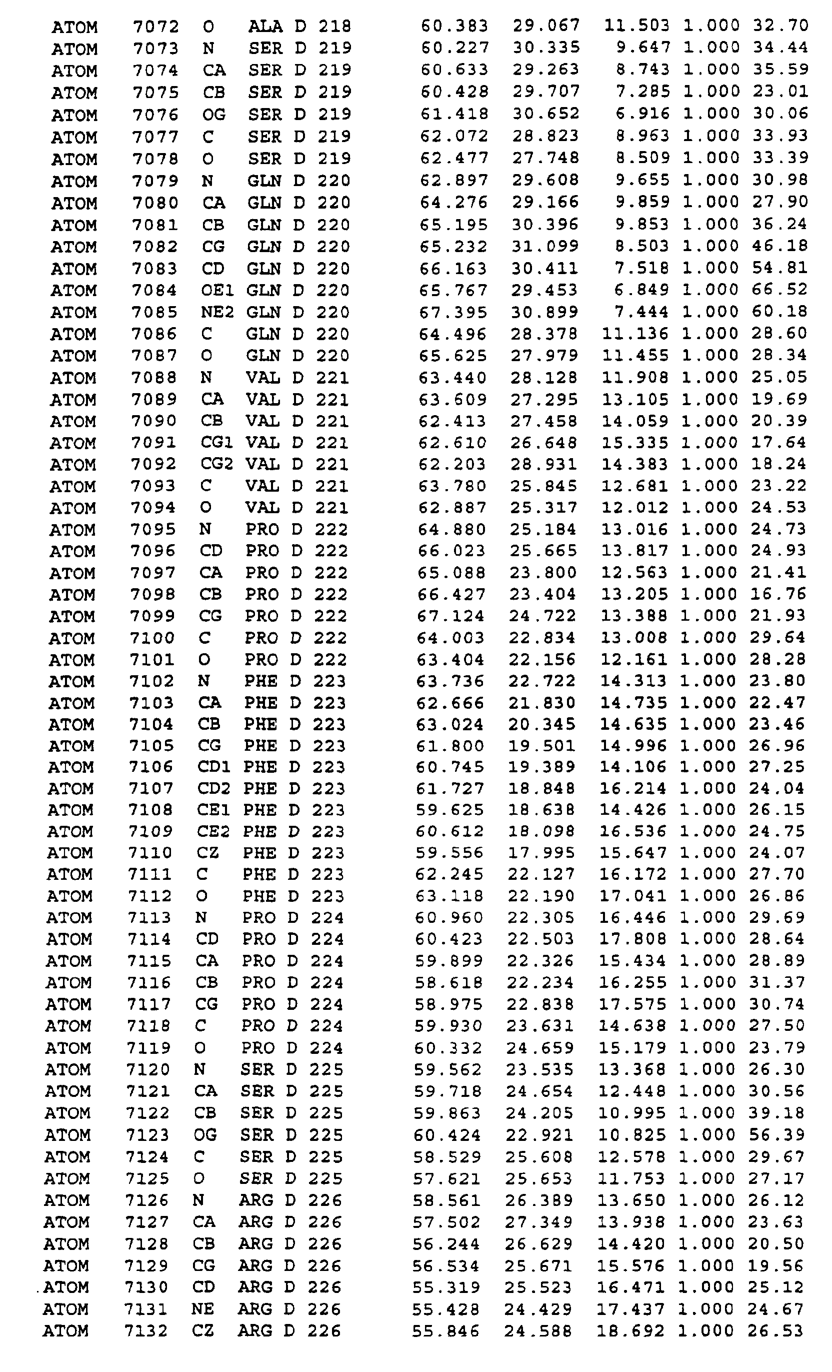

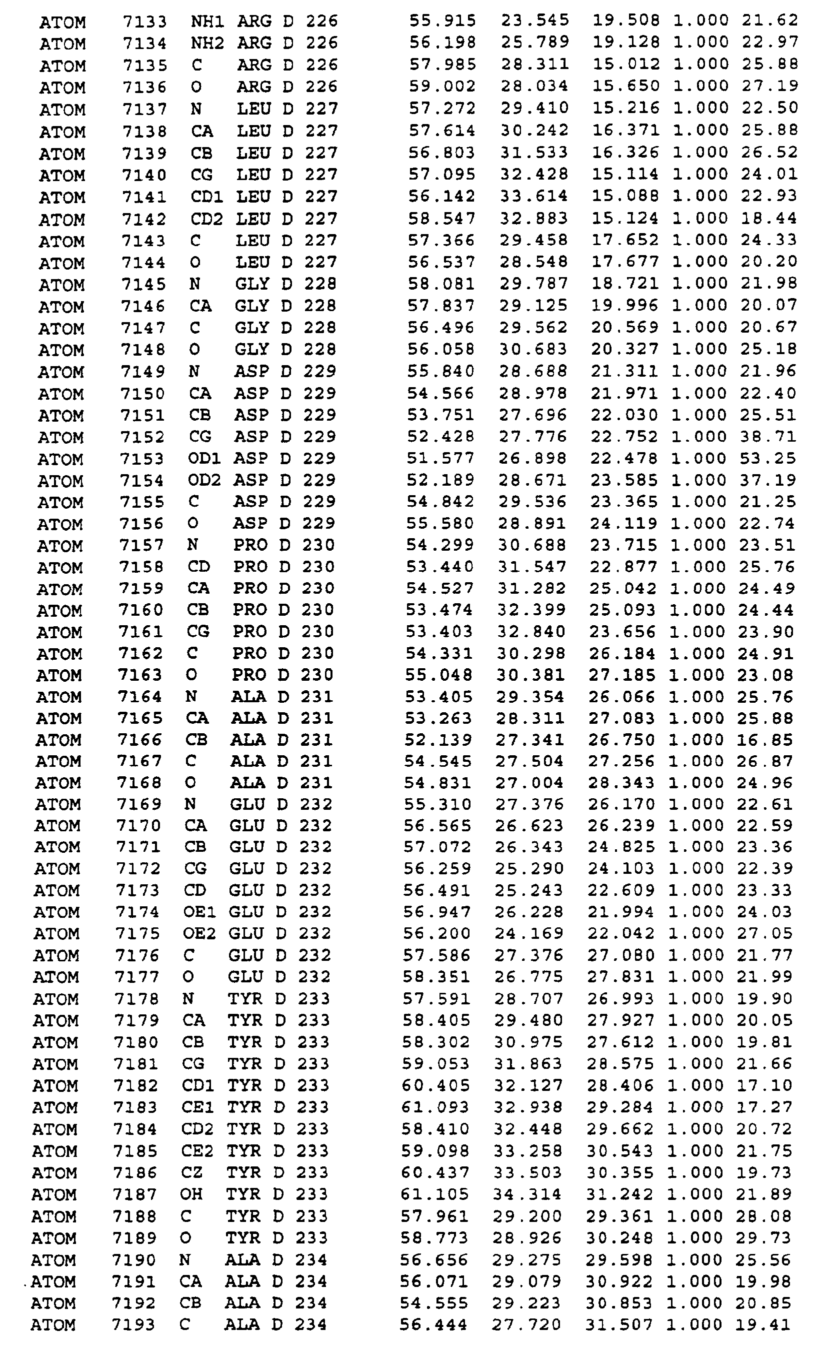

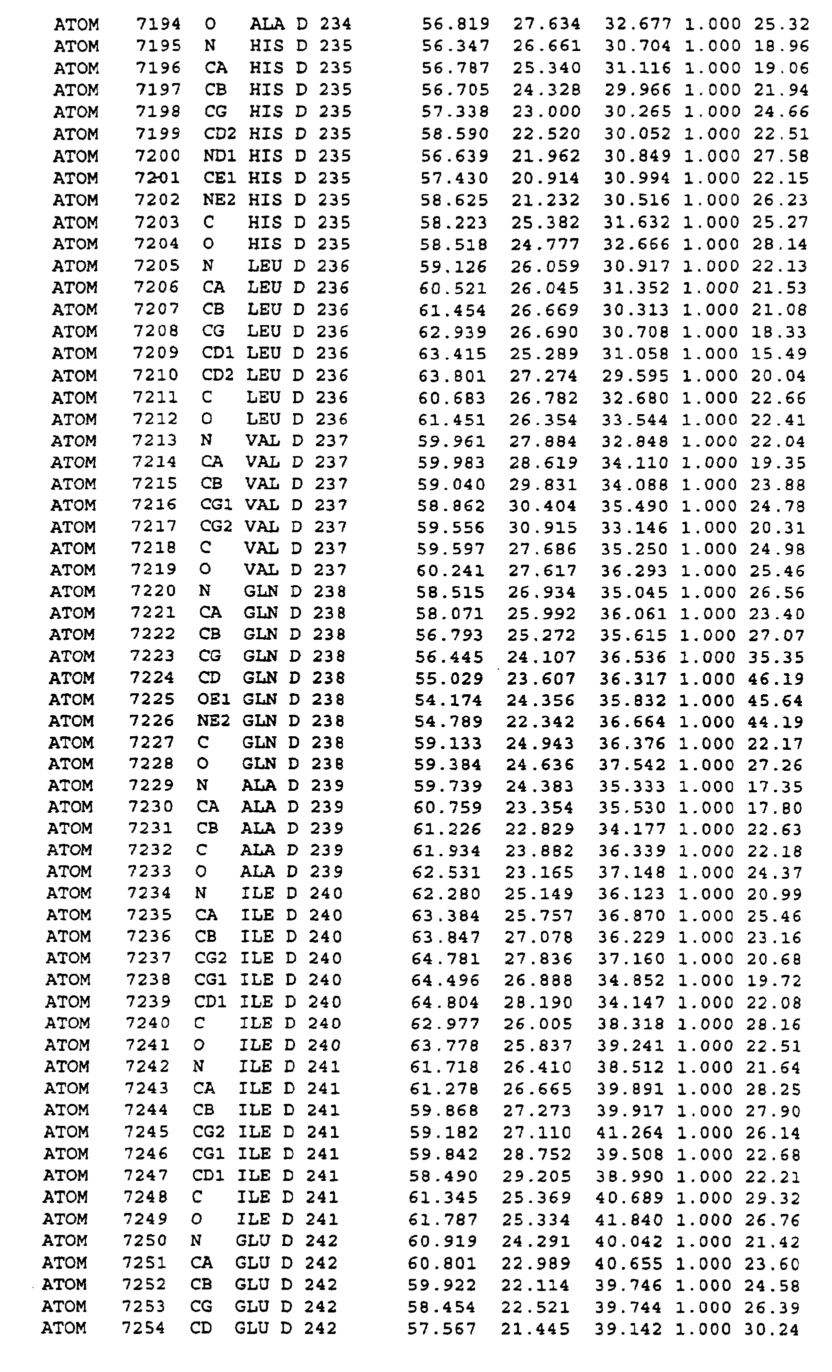

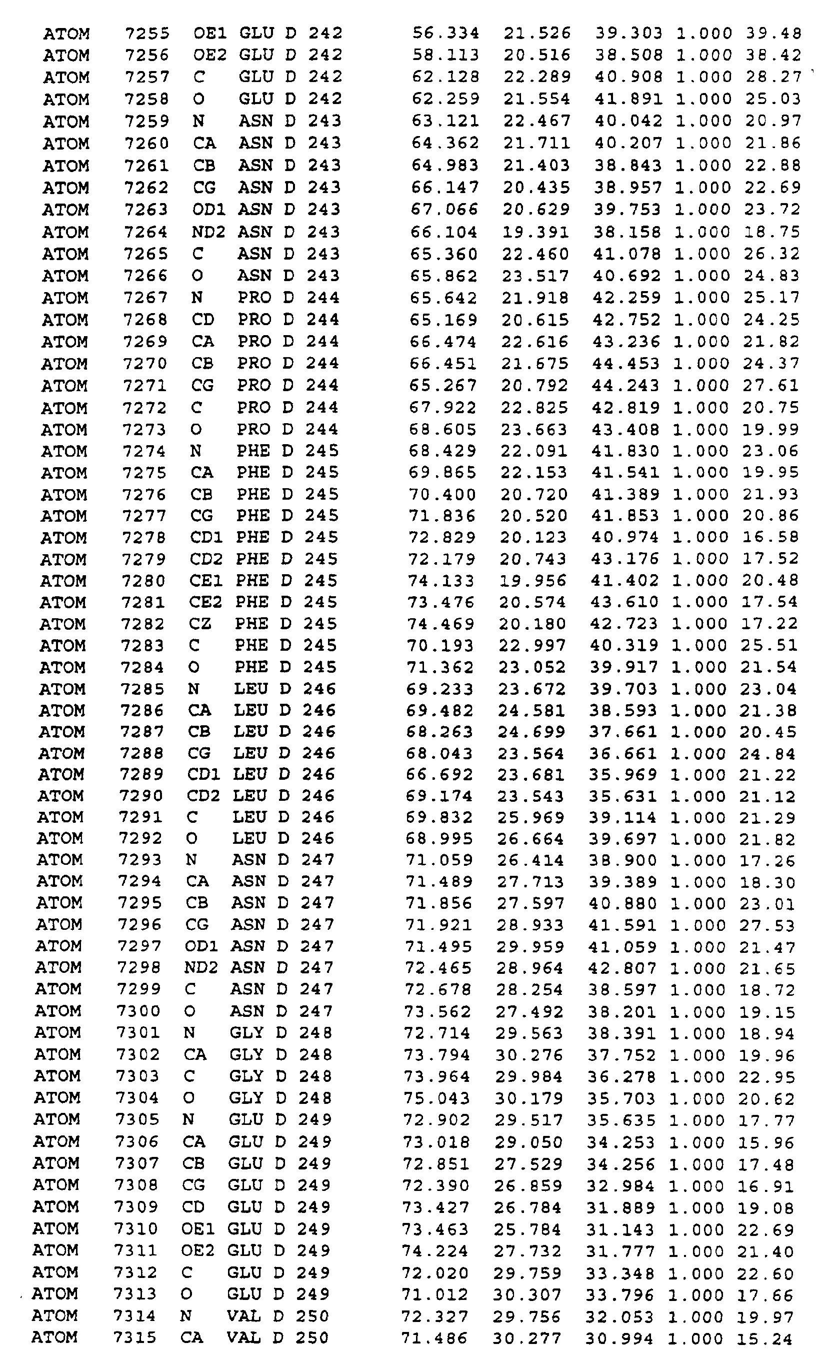

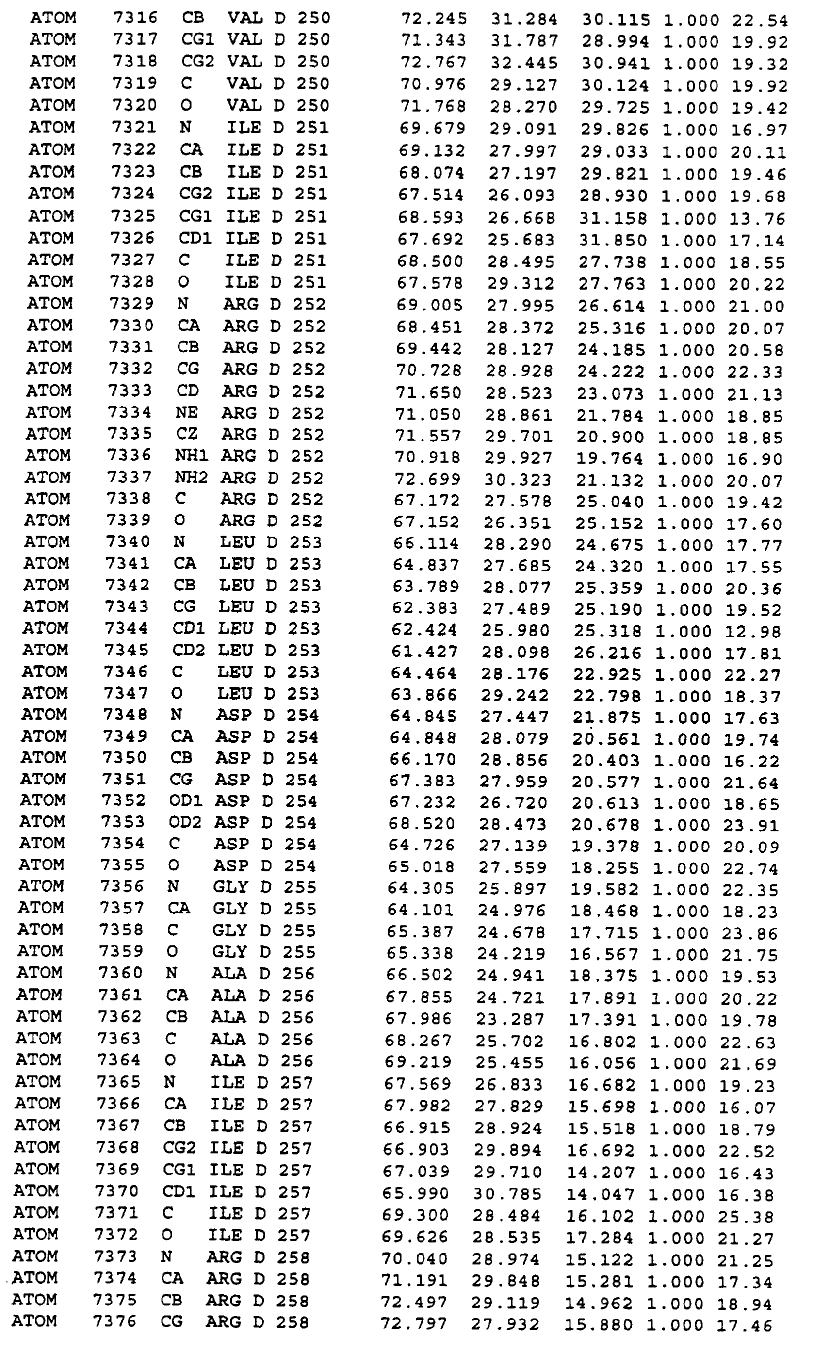

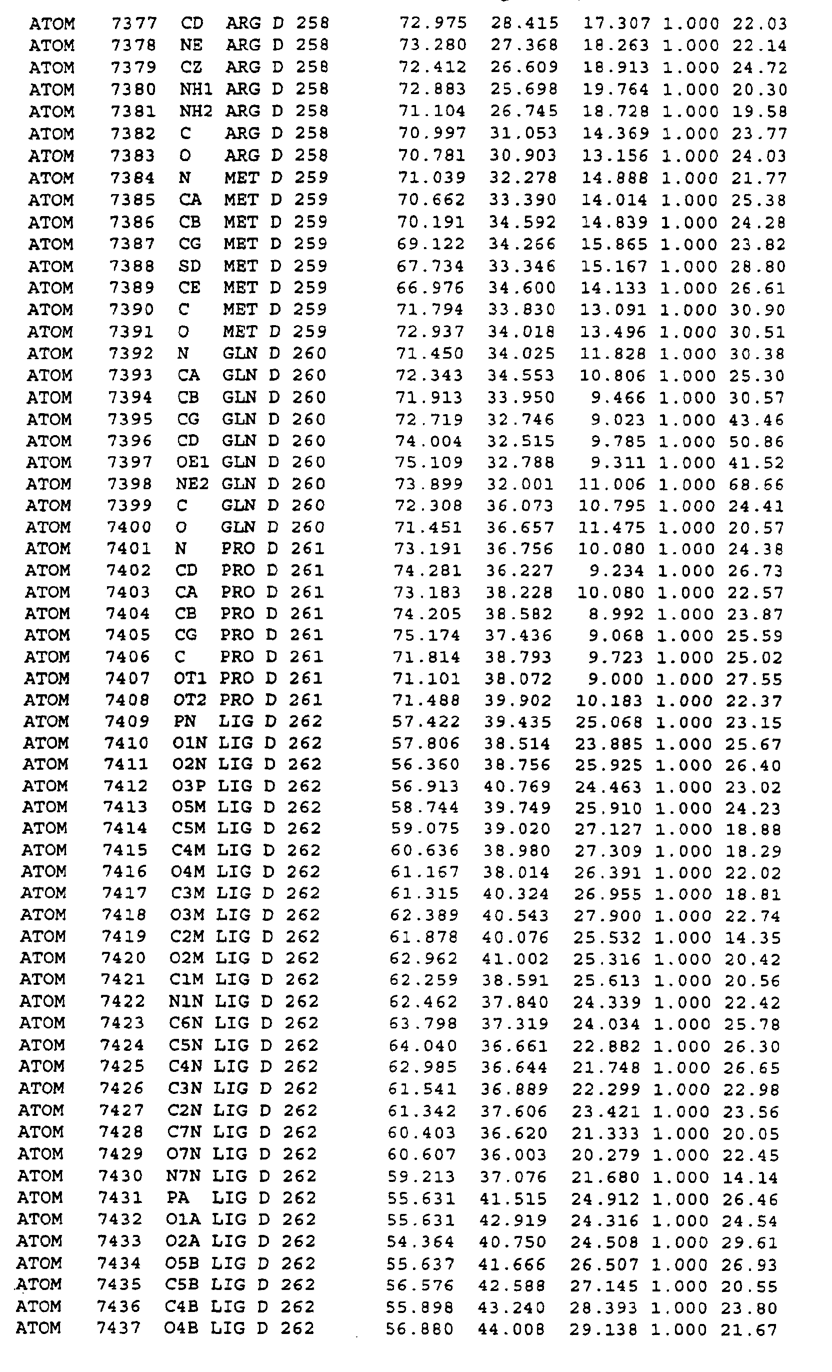

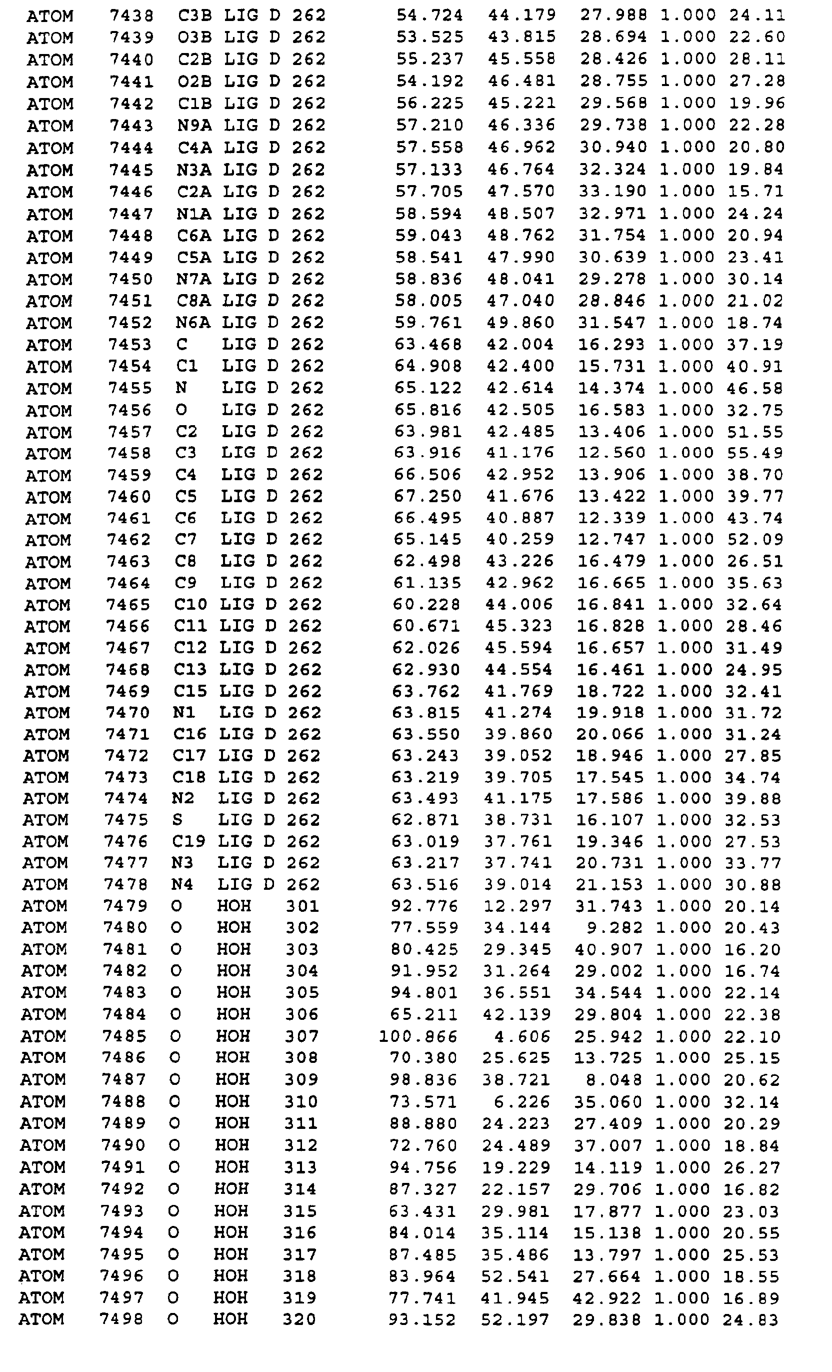

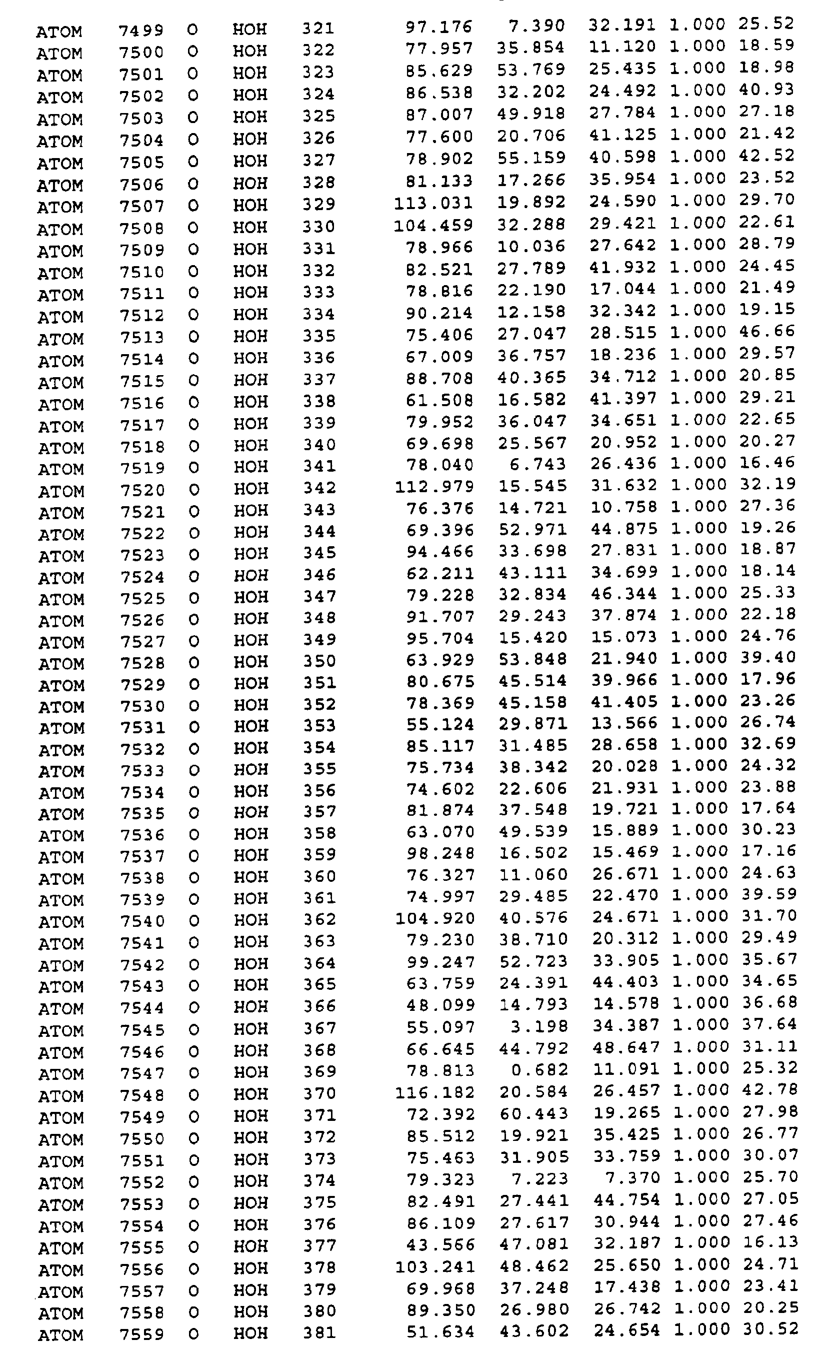

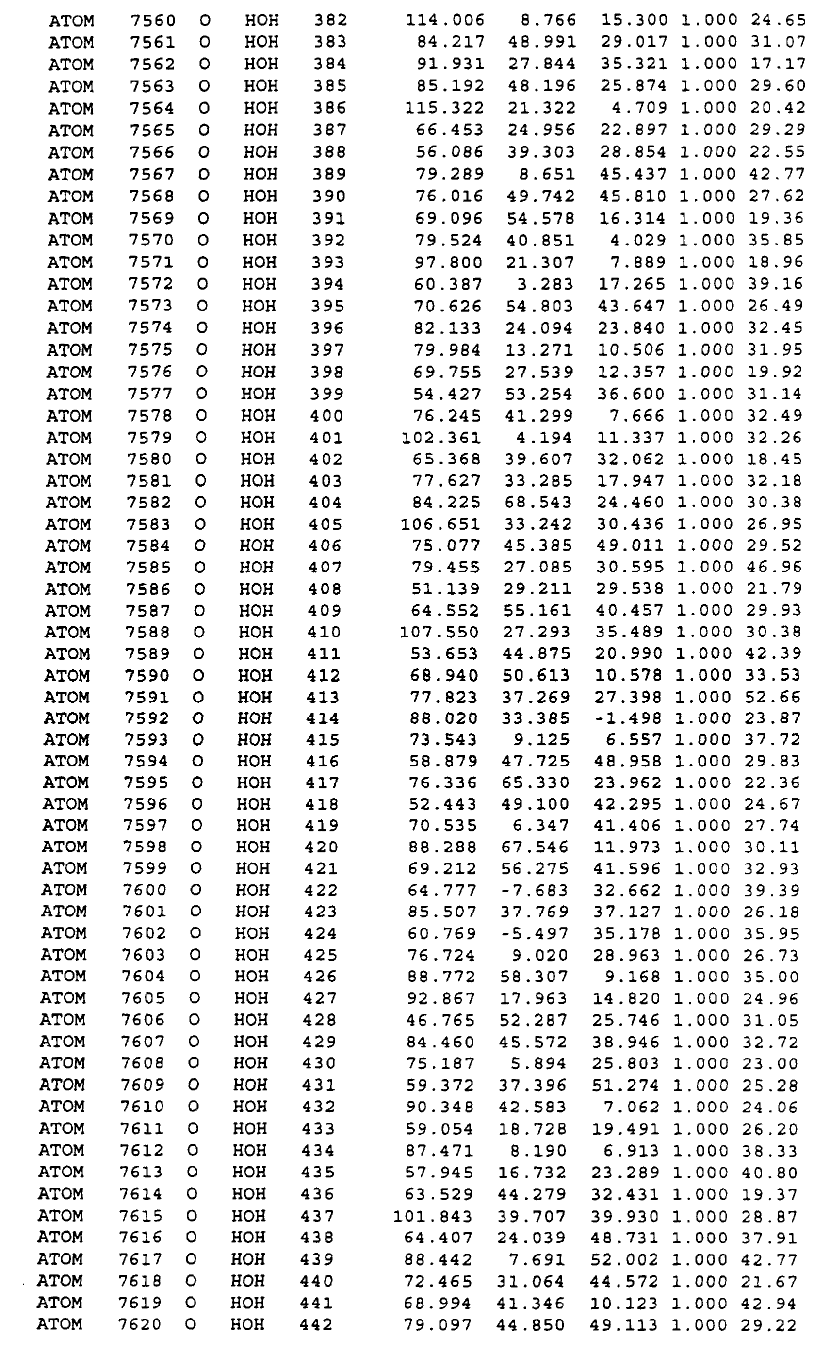

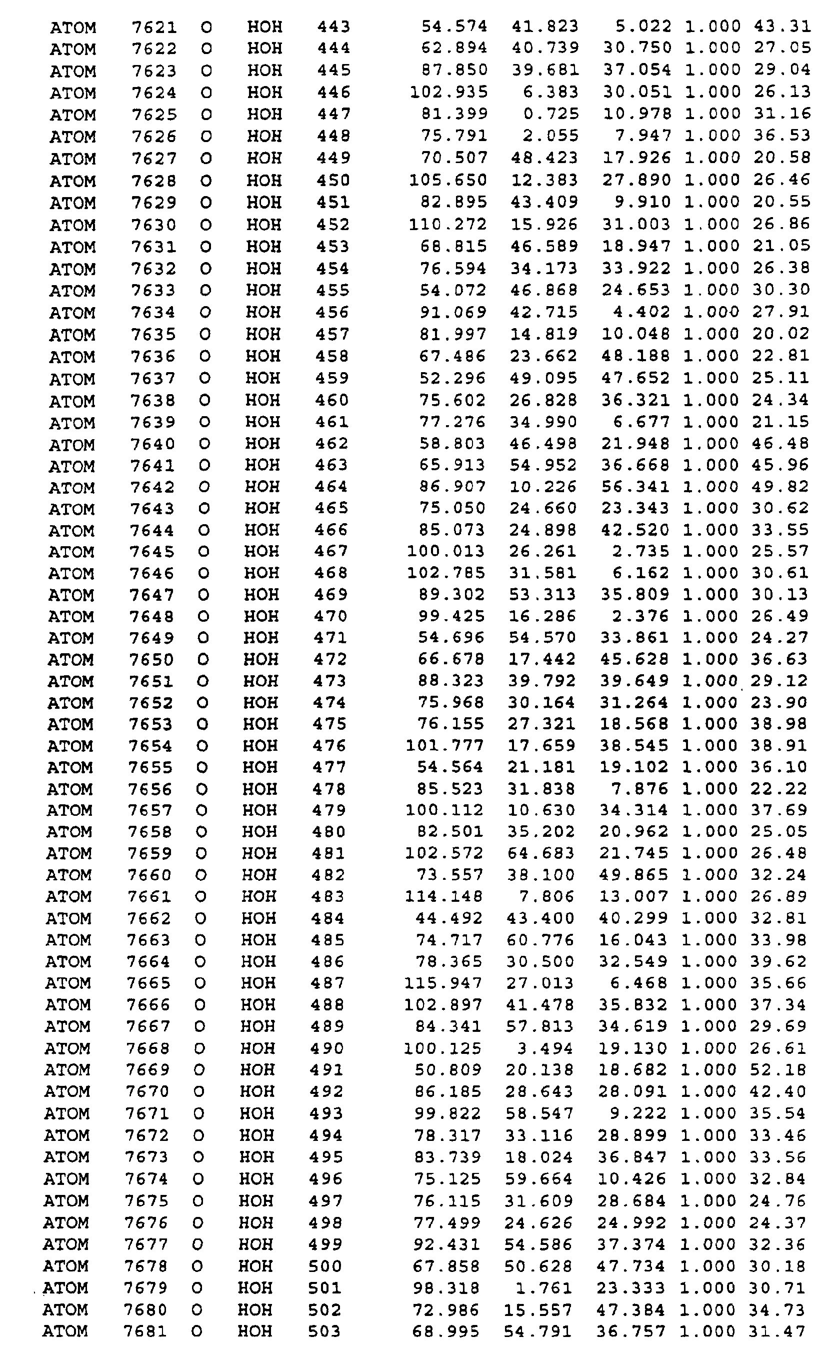

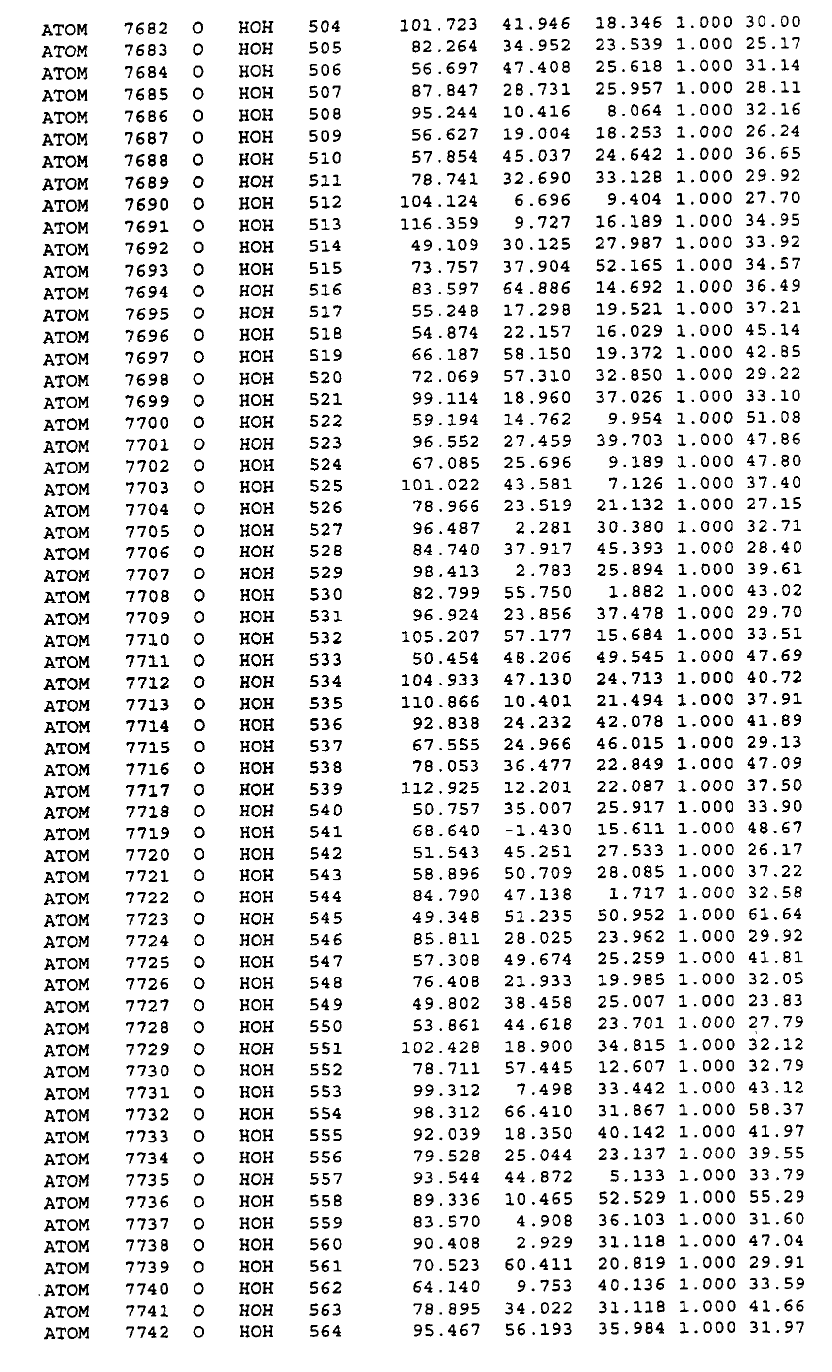

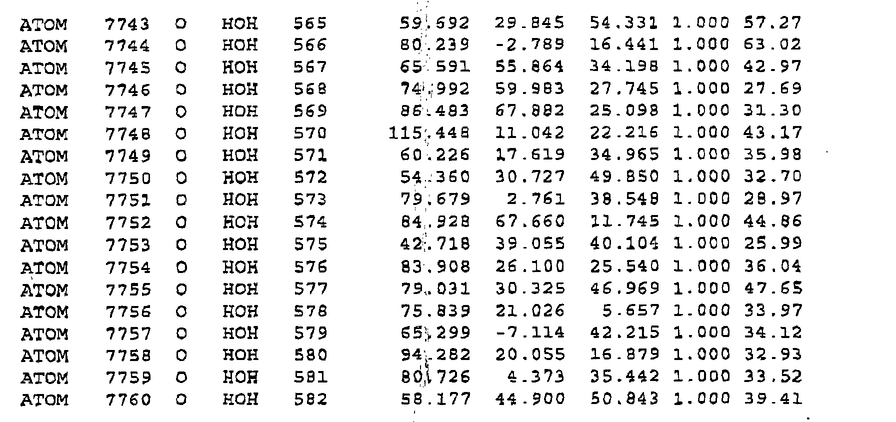

All data collection was carried out on an MAR Research (Hamburg, Germany) 345-mm image plate system installed on a Rigaku RU-200B rotating anode x-ray generator equipped with Osmic mirrors (Osmic, Inc., Troy, Michigan) and using an X-stream cryogenic system (obtained from Molecular Structure Corporation, The Woodlands, Texas). Data from 0.5° oscillations were integrated and scaled using DENZO and SCALEPACK (Otwinowski et al., *Methods Enzymology*, Vol. 276, pp. 307-326 (1997)). Crystals were mounted in a fiber loop, dipped in mother liquor with 25% glycerol added, and flash-cooled in liquid nitrogen for data collection at 100° K. An initial data set was collected to 2.4 Å resolution, which was used to obtain a molecular replacement solution. The space group was identified as C2, with an ERAB or HADH2 tetramer in the asymmetric unit.

The structure was determined by molecular replacement using the program EPMR (Kissinger et al., *Acta Crystallography*, Vol. D55, pp. 484-491 (1999)) with the wild-type protein tetramer as the search model. A solution was obtained with a correlation coefficient of 0.682 (R-factor = 0.361) for data between 15 and 4 Å.

A subsequent data set was collected to a resolution of 2.0 Å., and this data set was used for refinement. Initial refinement was carried out using standard rigid-body, simulated-annealing, positional, and temperature factor refinement protocols in X-PLOR Version 3.1 (Brünger, *XPLOR Manual, Version 3.1*, Yale University Press, New Haven, CT (1992)) with the application of non-crystallographic symmetry (NCS) restraints. The progress of the refinement was monitored by the free R-factor (Brunger, *Nature*, Vol. 355, pp. 472-475 (1992)) using 5% of the reflections taken from thin resolution shells. The final stages of refinement were performed using SHELX-97 (Sheldrick et al., *Methods Enzymology*, Vol. 277, pp. 319-344 (1997)). All data from 25.0 to 2.0 Å were used, and a bulk solvent correction was applied. Local NCS restraints were applied throughout the refinement to residues 5-205 and 220-261 of each monomer. X-FIT (McRee, *Journal of Molecular Graphics*, Vol. 10, pp. 44-46 (1992)) was used for rebuilding and visual analysis of the structural model. Two-hundred eighty-two (282) water molecules were fit using the automatic water fitting procedures in SHELX while monitoring the effect on the free R factor. The final, refined model includes residues 5-261 of each of the four monomers in the asymmetric unit, with one NAD molecule bound to each monomer and an inhibitor molecule bound to three of the monomers. The final crystallographic R-factor was 215 (R_{free} = .265) for all unique data between 25.0 and 2.0 Å resolution. The RMS deviations from target stereochemistry (Engh et al., *Acta Crystallography*, Vol. A47, pp. 392-400 (1991)) were 0.005 Å for bond lengths and 1.5° for bond angles. All backbone dihedral angles fall in allowed regions of the Ramachandran plot except those for Ala 154, which occurs in a generously allowed region in all four monomers, and which forms a part of the nucleotide-binding pocket. Proline 224 participates in a *cis* peptide bond.

### E. The ERAB or HADH2 Crystal Structure

Wild-type ERAB or HADH2 forms a tetramer in solution (He et al. (1998), *supra*). Likewise, a tetramer occupied the asymmetric unit of the crystal of the C214R mutant ERAB or HADH2. The conformations of the four subunits were essentially identical with the exception of a mobile loop near the substrate-binding site. Each ERAB or HADH2 monomer (Figure 1) formed a single domain with the overall polypeptide chain topology characteristic of the SDR family of enzymes (Jömvall et al., *Biochemistry*, Vol. 34, pp. 6003-6013 (1995)). The entire polypeptide chain of each of the four monomers had clearly defined electron density with the exception of the first five residues of each monomer, which were apparently disordered. ERAB or HADH2 contained a "Rossman fold" dinucleotide-binding motif (Rossman et al., *The Enzymes,* Academic Press, NY, pp. 61-102 (1975)), comprising a core β-sheet of seven parallel strands sandwiched between two sets of three α-helices.

Certain important aspects of the chain fold in ERAB or HADH2 arose from two insertions in the wild-type ERAB or HADH2 sequence relative to other members of the SDR family (see Figure 2). These insertions formed structural elements that extended from opposite ends of each monomer (Figure 1B). The first insertion residues (102-107) formed a short β hairpin structure (βDE1, βDE2) that extended the enzyme surface on one side of the substrate-binding cleft. This insertion also extended a monomer-monomer interface in the tetramer by contacting helix αE2 of an adjacent subunit. A second insertion (residues 141-146) extended the loop between αE2 and βE. This loop, which had extensive internal hydrogen bonding, did not lie near any active site region in the tetramer, but contacted Phe223 of an adjacent subunit and extends the subunit interface. Although the functional significance of these two structural elements of the protein is unknown, antibodies raised against peptides that encompass these insertion regions block the interaction of ERAB or HADH2 with β-amyloid (Yan et al. (1997), *supra*).

A ribbon representation of the ERAB or HADH2 tetramer is shown in Figure 3. The subunit interactions in the ERAB or HADH2 tetramer closely resembled those in the tetramers formed by certain other members of the SDR family, including 3α, 20β-hydroxysteroid dehydrogenase [Ghosh et al., *Proceedings of National Academy of Science, USA*, Vol. 88, pp. 10064-10068 (1991)] and 7α-hydroxysteroid dehydrogenase [Tanaka et al., *Biochemistry*, Vol. 35, pp. 7715-7730 (1996)]. The subunits in the tetramer were related by three mutually perpendicular two-fold axes, and each subunit contacted each of the other three subunits. The four active site regions were exposed on the outside of the tetramer, with a distance of about 31 Å between the reactive C4 atoms in the nicotinamide ring of each bound NAD⁺ cofactor.

Electron density for the bound NAD⁺ was clearly visible in all four subunits. Electron density for the inhibitor Compound I, however, was seen in only three of the four monomers in the asymmetric unit. The fourth monomer was involved in a crystal packing interaction that appeared to alter the conformation of the loop formed by residues 205 to 220, which formed one side of the substrate-binding cavity in the enzyme. The binding cleft was narrowed slightly in this monomer, which apparently precluded inhibitor binding. Therefore crystal formation and occupation of the fourth binding site by the inhibitor may be mutually exclusive.

Conversely, the corresponding region in other members of the SDR family has been observed to be mobile. In 7α-hydroxysteroid dehydrogenase, this region undergoes a large conformational change upon substrate binding (Tanaka et al. (1996) *supra*). The same region in *Drosophila* alcohol dehydrogenase is disordered in the enzyme-NAD⁺ complex and assumes differing conformations in the presence of different bound inhibitors (Benach et al., *Journal of Molecular Biology*, Vol. 289, No. 2, pp. 335-55 (1999)). In the crystal structure ofcis-biphenyl-2,3-dihydrodiol-2,3-dehydrogenase, this region is also observed to be disordered in the absence of substrate (Hülsmeyer et al., *Protein Science*, Vol. 7, pp. 1286-1293 (1998)). The flexibility of the substrate-binding flap may be important in allowing these enzymes to accommodate multiple substrates.

In the presence of inhibitor Compound I, the substrate-binding cavity formed a long, narrow cleft. One side of the cleft was composed of residues from two strands of the protein (residues 95-99 and 155-168). Residues 205-217, which formed part of the flexible substrate-binding flap, formed the other side of the cleft. Residue 214, which was mutated from Cys to Arg to facilitate crystallization, lay within this region but projected toward the exterior of the protein away from the substrate-binding cleft and was not in proximity to the bound inhibitor. The floor of the cleft was formed by the carboxy-terminus of the protein (residues 257-261). The substrate-binding cleft was largely hydrophobic in nature. A series of lysine residues (Lys 99, Lys 104, Lys 105) lay outside of the cleft at the site of the β-hairpin insertion region. These residues lay about 20 to 30 Å away from the reactive C4 atom of the NAD⁺, which corresponded to the distance between the phosphates and the reactive carbonyl group in an extended acetoacetyl-CoA molecule. This insertion in ERAB or HADH2 may therefore contribute to the affinity of the enzyme for acetoacetyl-CoA as a substrate.

While the invention has been described in conjunction with examples and preferred embodiments thereof, it is to be understood that the foregoing description is exemplary and explanatory in nature, and is intended to illustrate the invention and its preferred embodiments. Thus, the invention is intended to be defined not by the above description, but by the following claims and their equivalents.

## Claims

1. An isolated, purified polynucleotide that encodes a mutant Endoplasmic reticulum-associated amyloid β-peplide-binding protein (ERAB) or L-3-hydroxyacryl-CoA dehydrogenase Type II (HADH2) peptide, wherein said peptide is engineered to avoid cysteine oxidation.

2. The polynuclcotide of claim 1, wherein said polynucleotide encodes an ERAB or EIADH2 peptide which has an amino acid other than cysteine at one or all of positions 5, 58, and/or 214 of SEQ ID NO: 8.

3. An isolated mutant ERAB or HADH2 peptide which is engineered to avoid cysteine oxidation.

4. The peptide of claim 3 which has an amino acid other than cysteine at one or all of positions 5, 58, and/or 214 of SEQ ID NO: 8.

5. A crystal structure of a mutant ERAB or HADH2 peptide wherein said peptide is engineered to avoid cysteine oxidation.

6. The crystal structure of claim 5, wherein said peptide has an amino acid other than cysteine at one or all of positions 5, 58, and/or 214 of SEQ ID NO: 8.

7. An isolated polynucleotide that encodes a mutant ERAB or HADH2 peptide, wherein said polynucleotide is selected from the group consisting of SEQ ID NO: 1, SEQ ID NO: 3, SEQ ID NO: 5, SEQ ID NO: 19, and SEQ ID NO: 22, and functional variants thereof.

8. An isolated polynucleotide that encodes a mutant ERAB or HADH2 peptide, wherein said peptide has an amino acid sequence selected from the group consisting of SEQ JD NO: 2, SEQ ID NO: 4, SEQ ID NO: 6, SEQ ID NO: 20, and SEQ ID NO: 23, and functional variants thereof.

9. A crystal structure of a mutant ERAB or HADH2 peptide, wherein said peptide has an amino acid sequence selected from the group consisting of SEQ ID NO: 2, SEQ ID NO: 4, SEQ ID NO: 6, SEQ ID NO: 20, and SEQ ID NO: 23, and fragments or variants thereof.

10. The crystal structure of claim 9, wherein said mutant peptide has an amino acid sequence of SEQ ID NO: 2, or fragments or variants thereof.

11. , The crystal structure of claim 9, wherein said mutant peptide has an amino acid sequence of SEQ ID NO: 4, or fragments or variants thereof.

12. The crystal structure of claim 9, wherein said mutant peptide has an amino acid sequence of SEQ ID NO: 6, or fragments or variants thereof.

13. The crystal structure of claim 9, wherein said mutant peptide has an amino acid sequence of SEQ ID NO: 20, or fragments or variants thereof.

14. The crystal structure of claim 9, wherein said mutant peptide has an amino acid sequence of SEQ ID NO: 23, or fragments or variants thereof.

15. The crystal structure of claim 5, 6 or 9, wherein said crystal diffracts x-rays at a resolution value of ≤ 3 Å.

16. The crystal structure of claim 5, 6 or 9, wherein said crystal diffracts x-rays at a resolution value of ≤ 2 Å.

17. The crystal structure of claim 5, 6, or 9, wherein said crystal peptide mutant is tetrameric.

18. A crystal structure of a mutant ERAB or HADH2 peptide:ligand:NAD⁺ complex wherein said peptide is engineered to avoid cysteine oxidation.

19. The crystal structure of claim 18, wherein said peptide has an amino acid other than cysteine at one or all of positions 5, 58, and/or 214 of SEQ ID NO: 8.

20. A crystal structure of a mutant ERAB or HADH2 peptide:ligand:NAD⁺ complex, wherein said peptide has an amino acid sequence selected from the group consisting of SEQ ID NO: 2, SEQ ID NO: 4, SEQ ID NO: 6, SEQ ID NO: 20, and SEQ ID NO: 23, and functional variants thereof.

21. The crystal structure of claim 18, 19 or 20, wherein the crystal diffracts X-rays for the determination of the atomic coordinates of the complex to a resolution of greater than 3.0Å.

22. The crystal structure of claim 20 having the crystal coordinates shown in Table II.

23. The crystal structure of claim 18, 19 or 20, wherein said ligand is an ERAB or HADH2 inhibitor.

24. The crystal structure of claim 18, 19 or 20, wherein said ERAB or HADH2 inhibitor is a compound of the formula:

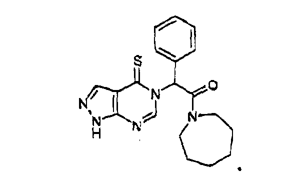

25. An isolated, purified peptide comprising a mutant ERAB or HADH2 peptide, wherein said mutant peptide has an amino acid sequence selected from the group consisting of SEQ ID NO: 2, SEQ ID NO: 4, SEQ ID NO: 6, SEQ ID NO: 20, and SEQ ID NO: 23, and functional variants thereof.

26. The peptide of claim 25 having an amino acid sequence comprising amino acids 90 to 261 of SEQ ID NO: 2 or functional variants thereof.

27. An isolated, purified ERAB or HADH2 peptide comprising a fold, wherein said fold comprises: a core β-sheet of seven strands sandwiched between two sets of three α-helices; and first and second insert domains, relative to other members of the short-chain dehydrogenase/reductase (SDR) family, the first insert domain forming a β hairpin that extends the surface of said ERAB or HADH2 peptide on one side of a substrate-binding cleft, and the second insert domain extending a loop between an α-helix and a β-sheet, wherein said α-helix comprises amino acid residues having a sequence of amino acid 123-136 of SEQ ID NO: 2, and said β-sheet comprises amino acid residues having a sequence of amino acid 148-153 of SEQ ID NO: 2.

28. An isolated, purified mutant ERAB or HADH2 peptide comprising a fold, wherein said fold comprises: a core β-sheet of seven strands sandwiched between two sets of three α-helices; and first and second insert domains, relative to other members of the short-chain dehydrogenase/reductase (SDR) family, the first insert domain forming a β hairpin that extends the surface of said ERAB or HADH2 peptide on one side of a substrate-binding cleft, and the second insert domain extending a loop between an α-helix and a β-sheet, wherein said peptide is engineered to avoid cysteine oxidation.

29. The peptide of claim 28, wherein said peptide has an amino acid other than cysteine at one or all of positions 5, 58, and/or 214 of SEQ ID NO: 8.

30. The peptide according to claim 28, wherein said α-helix comprises amino acid residues having a sequence of amino acid 123-136 of SEQ ID NO: 2 and said β-sheet comprises amino acid residues having a sequence of amino acid 148-153 of SEQ ID NO: 2, and further comprises a substrate-binding site involving residues selected from the group consisting of
(a) residues 95 to 99, 155 to 168, 205 to 217, and 257 to 261 of SEQ ID NO: 2;
(b) residues 95 to 99, 155 to 168, 205 to 217, and 257 to 261 of SEQ ID NO: 4;
(c) residues 95 to 99, 155 to 168, 205 to 217, and 257 to 261 of SEQ ID NO: 6;
(d) residues 95 to 99, 155 to 168, 205 to 217, and 257 to 261 of SEQ ID NO: 20; and
(e) residues 95 to 99, 155 to 168, 205 to 217, and 257 to 261 of SEQ ID NO: 23.

31. An isolated, purified mutant ERAB or HADH2 peptide comprising a fold, wherein said fold comprises: a core β-sheet of seven strands sandwiched between two sets of three α-helices; and first and second insert domains, relative to other members of the short-chain dehydrogenase/reductase (SDR) family, the first insert domain forming a β hairpin that extends the surface of said ERAB or HADH2 peptide on one side of a substrate-binding cleft, and the second insert domain extending a loop between an α-helix comprising residues 123-136 and a β-sheet comprising residues 148-153 of SEQ ID NO: 2.

32. The peptide according to claim 27, 28 or 31, further comprising a substrate-binding site involving residues selected from file group consisting of
(a) residues 95 to 99, 155 to 168, 205 to 217, and 257 to 261 of SEQ ID NO: 2;
(b) residues 95 to 99, 155 to 168, 205 to 217, and 257 to 261 of SEQ ID NO: 4;
(c) residues 95 to 99, 155 to 168, 205 to 217, and 257 to 261 of SEQ ID NO: 6;
(d) residues 95 to 99, 155 to 168, 205 to 217, and 257 to 261 of SEQ ID NO: 20; and
(e) residues 95 to 99, 155 to 168, 205 to 217, and 257 to 261 of SEQ ID NO: 23.

33. The peptide according to claim 31, wherein said first insert domain is at residues 102-107 of SEQ ID NO: 2.

34. The peptide according to claim 31, wherein said second insert domain is at residues 141-146 of SEQ ID NO: 2.

35. The peptide according to claim 31, wherein said ERAB or HADH2 is in the form of monomer.

36. The peptide according to claim 35, wherein said ERAB or HADH2 monomer forms a tetramer when crystallized.

37. The peptide according to claim 36, further comprising an ERAB or HADH2 cofactor-binding site.

38. The peptide according to claim 37, wherein said ERAB or HADH2 cofactor is NAD⁺.

39. An expression vector for producing a mutant ERAB or HADH2 peplide in a host cell, which vector comprises: a polynucleotide that encodes a mutant ERAB or HADH2 peptide, wherein said polynucleotide is the polynucleotide of claim 1, 2, or 7, or functional variants thereof; transcriptional regulatory sequences functional in said host cell operably linked to said polynucleotide; and a selectable marker.

40. The vector of claim 39, wherein said polynucleotide is selected from the group consisting of SEQ ID NO: 1, SEQ ID NO: 3, SEQ ID NO: 5, SEQ ID NO: 19, and SEQ ID NO: 22, and functional variants thereof.

41. A host cell stably transfected and transformed with the polynucleotide that encodes a mutant ERAB or HADH2 peptide, wherein said polynucleotide is the polynucleotide of claim 1, 2, or 7, or functional variants thereof.

42. The host cell of claim 41, wherein said polynucleotide is selected from the group consisting of SEQ ID NO: 1, SEQ ID NO: 3, SEQ ID NO: 5, SEQ ID NO: 19, and SEQ ID NO: 22, and functional variants thereof.

43. A method for identifying a candidate compound for its ability to interact with the human ERAB or HADH2 peptide comprising:
(a) expressing an isolated polynucleotide sequence which encodes a mutant ERAB or HADH2 peptide in a host cell capable of producing said peptide, wherein said polynucleotide is the polynucleotide of claim 1,2, or 7, or functional variants thereof;
(b) exposing said mutant ERAB or HADH2 peptide to said candidate compound; and
(c) evaluating the interaction of said mutant ERAB or HADH2 peptide with said candidate compound.

44. The method of claim 43, wherein said polynucleotide is selected from the group consisting of SEQ ID NO: 1, SEQ ID NO: 3, SEQ ID NO: 5, SEQ ID NO: 19 and SEQ ID NO: 22, and functional variants thereof.

45. The method of claim 43, wherein said evaluation step comprises conducting said x-ray crystallography on said ERAB or HADH2 peptide.

46. The method of claim 45, wherein the results of said x-ray crystallography step are used to determine the three-dimensional molecular structure of the configuration of the ERAB or HADH2 peptide and the binding pockets thereof.

47. A process of drug design for compounds which interact with an ERAB or HADH2 peptide comprising:
(a) crystallizing a mutant ERAB or HADH2 peptide, said peptide being a peptide of claim 3, 4, or 25, or functional variants thereof;
(b) resolving the x-ray crystallography of said peptide;
(c) applying the data, or a portion thereof, generated from resolving the x-ray crystallography of said peptide to a computer algorithm which generates a model of a three-dimensional structure, said model suitable for use in designing molecules that will interact with said peptide; and
(d) applying an interative process whereby various molecular structures are applied to said computer-generated model to identify the compounds which interact with said peptide.

48. The method of claim 47, wherein said peptide has an amino acid sequence selected from the group consisting of SEQ ID NO: 2, SEQ ID NO: 4, SEQ ID NO: 6, SEQ ID NO: 20, and SEQ ID NO: 23, and functional variants thereof.

49. The method of claim 47, wherein said process is utilized to identify inhibitors of an ERAB or HADH2 enzyme, said inhibitors serving as lead compounds for the design of potentially therapeutic compounds for the treatment of diseases or disorders associated with the ERAB or HADH2 action.

50. A method of assessing compounds which are agonists or antagonists of the activity of the human ERAB or HADH2 enzyme comprising:
(a) crystallizing a mutant ERAB or HADH2 peptide, said peptide being a peptide of claim 3, 4, or 25, or functional variants thereof;
(b) obtaining crystallography coordinates for said crystallized peptide;
(c) applying said crystallography coordinates or a portion thereof for said peptide to a computer algorithm such that said algorithm generates a model of a three-dimensional structure, said model suitable for use in designing molecules that will act as agonists or antagonists to said peptide; and
(d) applying an iterative process whereby various molecular structures are applied to said computer-generated model to identify potential agonists or antagonists to said peptide.

51. The method of claim 50, wherein said peptide has an amino acid sequence selected from the group consisting of SEQ ID NO: 2, SEQ ID NO: 4, SEQ ID NO: 6, SEQ ID NO: 20, and SEQ ID NO: 23, and functional variants thereof.

52. A method of identifying a compound that associates with an ERAB or HADH2 peptide, the method comprising the step of using the crystal coordinates of Table II or a portion thereof to computationally analyze a molecular structure to evaluate a chemical entity for associating with the substrate-binding site of ERAB or HADH2 and identifying those chemical entities that associate with said substrate-binding site, wherein said step of computationally analyzing the molecular sturucture comprises:
(a) storing instructions for processing machine readable data wherein said data comprises crystal coordinates of a mutant ERAB or HADH2 peptide molecule or complex of said peptide, said mutant ERAB or HADH2 peptide being the peptide of claim 3, 4, or 25, or functional variants thereof; and
(c) processing said data of crystal coordinates into a three-dimensional structure of said peptide molecule or complex.

53. The method of claim 52 further comprising the step of displaying said crystal coordinates of the three-dimensional structure.

54. The method of claim 52, wherein said peptide has an amino acid sequence selected from the group consisting of SEQ ID NO: 2, SEQ ID NO: 4, SEQ ID NO: 6, SEQ ID NO: 20, and SEQ ID NO: 23, and functional variants thereof.

55. The method of claim 52, wherein said complex is an ERAB or HADH2 peptide:ligand:NAD⁺ complex.

56. The method of claim 55, wherein said ligand is an ERAB or HADH2 inhibitor.

57. The method of claim 56, wherein said ligand is a compound of the formula:

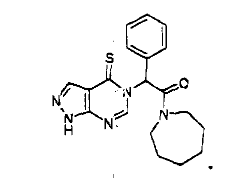

58. A method of using a computer processor for analyzing a molecular structure comprising:
(a) storing instructions for processing machine readable data wherein said data comprises crystal coordinates of a mutant ERAB or HADH2 peptide molecule or complex of said peptide, said mutant ERAB or HADH2 peptide being the peptide of claim 3, 4, or 25, or functional variants thereof; and
(b) processing said data of crystal coordinates into a three-dimensional structure of said peptide molecule or complex.

59. The method of claim 58 further comprising the step of displaying said crystal coordinates of the three-dimensional structure.

60. The method of claim 58, wherein said peptide has an amino acid sequence selected from the group consisting of SEQ ID NO; 2, SEQ ID NO: 4, SEQ ID NO: 6, SEQ ID NO: 20, and SEQ ID NO: 23, and functional variants thereo

61. The method of claim 58, wherein said complex is an ERAB or HADH2 peptide:ligand:NAD⁺ complex.

62. The method of claim 61, wherein said ligand is an ERAB or HADH2 inhibitor.

63. The method of claim 62, wherein said ligand is a compound of the formula:

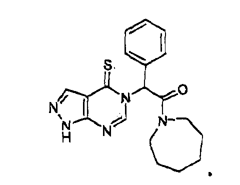

64. The method of claim 58, wherein said machine readable data storage medium is CD-ROM.

65. The method of claim 58, wherein said machine readable data storage medium is a magneto-optic disk.

66. A computer based method for processing X-ray coordinate data into a three-dimensional graphical display of mutant ERAB or HAHD2 peptide molecule or molecular complex which comprises a substrate binding domain, wherein said mutant ERAB or HADH2 peptide has an amino acid sequence selected from the group consisting of SEQ ID NO: 2, SEQ ID NO: 4, SEQ ID NO: 6, SEQ ID NO: 20 and SEQ ID NO: 23, and functional variants thereof.

67. The method of claim 66, wherein said complex is ERAB or HADH2 peptide:ligand:NAD⁺ complex.

68. The method of claim 67, wherein said ligand is an ERAB or HADH2 inhibitor.

69. The method of claim 66, wherein said X-ray coordinate data is stored in a machine readable storage medium.

70. The method of claim 66, wherein said three-dimensional graphical display is displayed on a computer monitor.

71. A crystallized ERAB or HADH2 peptide or an ERAB or HADH2 peptide:cofactor:ligand complex containing a substrate-binding site comprised of amino acid residues selected from the group consisting of
(a) residues 95 to 99, 155 to 168, 205 to 217, and 257 to 261 of SEQ ID NO: 2;
(b) residues 95 to 99, 155 to 168, 205 to 217, and 257 to 261 of SEQ ID NO: 4;
(c) residues 95 to 99, 155 to 168, 205 to 217, and 257 to 261 of SEQ ID NO: 6;
(d) residues 95 to 99, 155 to 168, 205 to 217, and 257 to 261 of SEQ ID NO: 8;
(e) residues 95 to 99, 155 to 168, 205 to 217, and 257 to 261 of SEQ ID NO: 20; and
(f) residues 95 to 99, 155 to 168, 205 to 217, and 257 to 261 of SEQ ID NO: 23.

72. The crystallized peptide or complex of claim 71, wherein said cofactor is NAD⁺.

73. A method of assessing compounds which are agonists or antagonists of the activity of the human ERAB or HADH2 pepetide comprising:
(a) crystallizing an ERAB or HADH2 peptide or ERAB or HADH2 peptide:NAD⁺:ligand complex;
(b) obtaining crystallography coordinates for said crystallized peptide or complex;
(c) applying said crystallography coordinates or a portion thereof for said peptide to a computer algorithm such that said algorithm generates a model of a substrate-binding site, said model suitable for use in designing molecules that will act as agonists or antagonists to said peptide; and
(d) applying an iterative process whereby various molecular structures are applied to said computer-generated model to identify potential agonists or antagonists to said peptide.

74. The method of claim 73, wherein said substrate-binding site comprises amino acid residues 95 to 99, 155 to 168, 205 to 213, 215-217, and 257 to 261 of SEQ ID NO: 2 according to Table II.

75. A method of assessing compounds which are agonists or antagonists of the activity of the human ERAB or HADH2 peptide comprising:
(a) employing computational means to perform a fining operation between the compounds and a substrate-binding site defined by crystallographic coordinates of amino acid residues 95 to 99, 155 to 168, 205 to 213, 215-217, and 257 to 261 of SEQ ID NO: 2 according to Table II; and
(b) applying an iterative process whereby various molecular structures are applied to said fitting operation to identify potential agonists or antagonists to said peptide.

76. The method of claim 75, wherein said substrate-binding site has amino acid residues selected from the group consisting of
(a) residues 95 to 99, 155 to 168, 205 to 217, and 257 to 261 of SEQ ID NO: 2;
(b) residues 95 to 99, 155 to 168, 205 to 217, and 257 to 261 of SEQ ID NO: 4;
(c) residues 95 to 99, 155 to 168, 205 to 217, and 257 to 261 of SEQ ID NO: 6;
(d) residues 95 to 99, 155 to 168, 205 to 217, and 257 to 261 of SEQ ID NO: 8;
(e) residues 95 to 99, 155 to 168, 205 to 217, and 257 to 261 of SEQ ID NO: 20; and
(f) residues 95 to 99, 155 to 168, 205 to 217, and 257 to 261 of SEQ IDNO: 23.

77. A method of identifying a compound that associates with ERAB or HADH2, the method comprising the step of using the crystal coordinates of amino acid residues 95 to 99, 155 to 168, 205 to 213, 215-217, and 257 to 261 of SEQ ID NO: 2 according to Table II to computationally analyze a molecular structure to evaluate a chemical entity for associating with the substrate-binding site of ERAB or HADH2 and identifying those chemical entities that associate with said substrate-binding site, wherein said step of computationally analyzing the molecular structure comprises:
(a) storing instructions for processing machine readable data wherein said data comprises crystal coordinates of amino acid residues 95 to 99, 155 to 168, 205 to 213, 215-217, and 257 to 261 of SEQ ID NO: 2 according to Table II; and
(b) processing said data of crystal coordinates into a three-dimensional structure of said peptide molecule or complex.

78. The method of claim 77 further comprising the step of displaying said crystal coordinates of the three-dimensional structure.

79. A method of using a computer processor for analyzing a substrate-binding site of an ERAB or HADH2 peptide or ERAB or HADH2 peptide:NAD⁺:ligand complex comprising:
(a) storing instructions for processing machine readable data wherein said data comprises X-ray crystallographic coordinates having crystal coordinates of amino acid residues 95 to 99, 155 to 168,205 to 213,215-217, and 257 to 261 of SEQ ID NO: 2 according to Table II; and
(b) processing said data of crystal coordinates into a three-dimensional structure of said peptide molecule or complex to analyze the substrate-binding site of said peptide.

80. The method of claim 79 further comprising the step of displaying said crystal coordinates of the three-dimensional structure.

81. The method of claim 79, wherein said ligand is an ERAB or HADH2 inhibitor.

82. The method of claim 79, wherein said machine readable data storage medium is CD-ROM.

83. The method of claim 79, wherein said machine readable data storage medium is a magneto-optic disk.

84. A computer based method for processing X-ray coordinate data into a three-dimensional graphical display of a substrate-binding site of an ERAB or HADH2 peptide molecule or ERAB or HADH2 peptide:NAD⁺:ligand complex using the crystal coordinates of amino acid residues 95 to 99, 155 to 168, 205 to 213, 215-217, and 257 to 261 of SEQ ID NO: 2 according to Table II.

85. The method of claim 84, wherein said ligand is an ERAB or HADH2 inhibitor.

86. The method of claim 84, wherein said X-ray coordinate data is stored in a machine readable storage medium.

87. The method of claim 84, wherein said three-dimensional graphical display is displayed on a computer monitor.

88. A process of drug design for compounds which associate with a substrate-binding site of an ERAB or HADH2 peptide comprising;
(a) employing computational means to perform a fitting operation between the compounds and a substrate-binding site defined by crystallographic coordinates of amino acid residues 95 to 99, 155 to 168, 205 to 213, 215-217, and 257 to 261 of SEQ ID NO: 2 according to Table II; and
(b) applying an iterative process whereby various molecular structures are applied to said fitting operation to identify the compounds which associate with the substrate-binding site.

89. The method of claim 88, wherein said substrate-binding site has amino acid residues selected from the group consisting of
(a) residues 95 to 99, 155 to 168, 205 to 217, and 257 to 261 of SEQ ID NO: 2;
(b) residues 95 to 99, 155 to 168, 205 to 217, and 257 to 261 of SEQ ID NO: 4;
(c) residues 95 to 99, 155 to 168, 205 to 217, and 257 to 261 of SEQ ID NO: 6;
(d) residues 95 to 99, 155 to 168, 205 to 217, and 257 to 261 of SEQ ID NO: 8;
(e) residues 95 to 99, 155 to 168, 205 to 217, and 257 to 261 of SEQ ID NO: 20; and
(f) residues 95 to 99, 155 to 168, 205 to 217, and 257 to 261 of SEQ ID NO: 23.

90. The method of claim 73 or 79, wherein said substrate-binding site has amino acid residues selected from the group consisting of
(a) residues 95 to 99, 155 to 168, 205 to 217, and 257 to 261 of SEQ ID NO: 2;
(b) residues 95 to 99, 155 to 168, 205 to 217, and 257 to 261 of SEQ ID NO: 4;
(c) residues 95 to 99, 155 to 168, 205 to 217, and 257 to 261 of SEQ ID NO: 6;
(d) residues 95 to 99, 155 to 168, 205 to 217, and 257 to 261 of SEQ ID NO: 8;
(e) residues 95 to 99, 155 to 168, 205 to 217, and 257 to 261 of SEQ ID NO: 20; and
(f) residues 95 to 99, 155 to 168, 205 to 217, and 257 to 261 of SEQ ID NO: 23.

91. A method of identifying a compound that associates with an ERAB or HADH2 peptide, the method comprising using the crystal coordinates from Table II, or portions thereof, to computationally evaluate a chemical entity for associating with the substrate-binding site of ERAB or HADH2.

92. A computer readable medium having stored thereon a model of a crystal structure comprising an ERAB or HADH2 peptide wherein said peptide has a substrate-binding site containing amino acid residues 95 to 99, 155 to 168, 205 to 217, and 257 to 261 of SEQ ID NO: 2.

93. A compound according to any one of claims 1 to 42 for use in medicine.
